Europäisches Patentamt
European Patent Office
Office européen des brevets

(19) (11) Publication number: 0 082 681 A2

# (12) EUROPEAN PATENT APPLICATION

(21) Application number: 82306724.4

(22) Date of filing: 16.12.82

(51) Int. Cl.³: C 07 D 239/42
C 07 D 239/46, C 07 D 239/52
C 07 D 251/22, C 07 D 251/16
C 07 D 251/46, C 07 D 405/12
C 07 D 409/12, C 07 D 411/12
A 01 N 47/36
//C07D339/00, C07D327/04,
C07D319/18, C07D339/08,
C07D317/62, C07D317/46

(30) Priority: 17.12.81 US 331891
10.11.82 US 440209
29.03.82 US 363379
01.11.82 US 437366

(43) Date of publication of application:
29.06.83 Bulletin 83/26

(84) Designated Contracting States:
AT BE CH DE FR GB IT LI LU NL SE

(71) Applicant: E.I. DU PONT DE NEMOURS AND COMPANY
Legal Department 1007 Market Street
Wilmington Delaware 19898(US)

(72) Inventor: Welch, John Thomas
13 Parkwood Street
McKowenville Albany New York 12203(US)

(72) Inventor: Carter, Linda Gay
1306 Grinnell Road
Wilmington Delaware 19803(US)

(74) Representative: Hildyard, Edward Martin et al,
Frank B. Dehn & Co. Imperial House 15-19 Kingsway
London WC2B 6UZ(GB)

## (54) Herbicidal sulfonamides.

(57) Sulfonylurea derivatives of the formula

$$ArSO_2NHC(=O)N(R_{10})R_1$$

wherein Ar is an optionally substituted bicyclic moiety;

$R_1$ is a substituted mono- or bicyclic heterocyclic moiety; and

$R_{10}$ is H or $CH_3$;

and their agriculturally suitable salts exhibit general and selective herbicidal activity. They may be formulated for use in conventional manner.

The novel compounds may be made e.g. by reacting an appropriate sulfonyl isocyanate of formula $ArSO_2NCO$ with a corresponding heterocyclic amine of formula $HNR_{10}R_1$.

EP 0 082 681 A2

Croydon Printing Company Ltd

0082681

BA-8451-A/ 8465-A

Title Cognate

## "Herbicidal sulfonamides"

## Background of the Invention

This invention relates to herbicidal phenyl-substituted sulfonylureas which are useful as general or selective herbicides, both pre-emergence and post-emergence.

U.S. Patent 4,127,405 teaches compounds which are useful for controlling weeds in wheat having the formula:

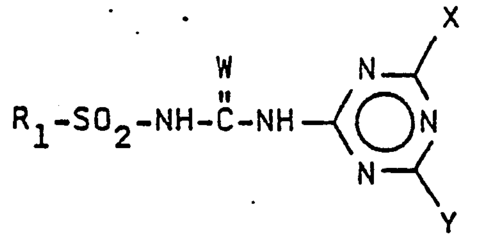

(I)

wherein

$R_1$ is

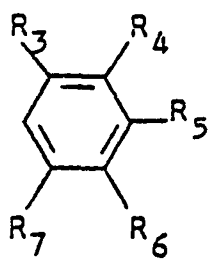

,

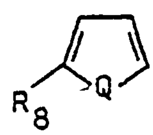

,

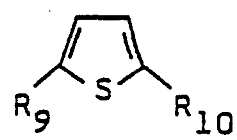

or

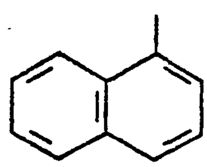

;

$R_3$ and $R_6$ are independently hydrogen, fluorine, chlorine, bromine, iodine, alkyl of 1-4 carbon atoms, alkoxy of 1-4 carbon atoms, nitro, trifluoromethyl, cyano, $CH_3S(O)_n$- or $CH_3CH_2S(O)_n$-;

$R_4$ is hydrogen, fluorine, chlorine, bromine or methyl;

$R_5$ is hydrogen, fluorine, chlorine, bromine, methyl or methoxy;

$R_7$ is hydrogen, fluorine, chlorine, bromine, alkyl of 1-2 carbon atoms or alkoxy of 1-2 carbon atom;

$R_8$ is hydrogen, methyl, chlorine or bromine;

$R_9$ and $R_{10}$ are independently hydrogen, methyl, chlorine or bromine;

W and Q are independently oxygen or sulfur;

n is 0, 1 or 2;

X is hydrogen, chlorine, bromine, methyl, ethyl, alkoxy of 1-3 carbon atoms, trifluoromethyl, $CH_3S$- or $CH_3OCH_2$-; and

Y is methyl or methoxy; or their agriculturally suitable salts; provided that:

(a) when $R_5$ is other than hydrogen, at least one of $R_3$, $R_4$, $R_6$ and $R_7$ is other than hydrogen and at least two of $R_3$, $R_4$, $R_6$ and $R_7$ must be hydrogen;

(b) when $R_5$ is hydrogen and all of $R_3$, $R_4$, $R_6$ and $R_7$ are other than hydrogen, then all of $R_3$, $R_4$, $R_6$ and $R_7$ must be either chlorine or methyl; and

(c) when $R_3$ and $R_7$ are both hydrogen, at least one of $R_4$, $R_5$ or $R_6$ must be hydrogen.

French Patent No. 1,468,747 discloses the following <u>para</u>-substituted phenylsulfonamides, useful as antidiabetic agents:

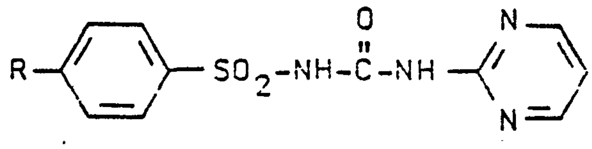

wherein

R = H, halogen, $CF_3$ or alkyl.

Logemann et al., Chem. Ab., 53, 18052g (1959), disclose a number of sulfonamides, including uracil derivatives and those having the formula:

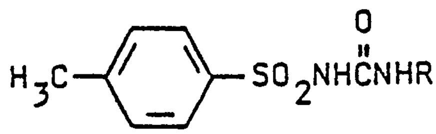

wherein

R is butyl, phenyl or 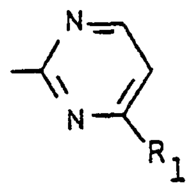 ; and

$R_1$ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm. 19, p. 121-5 (1962) [Chem. Ab., 59 1633 e] describes the synthesis of N-[(2,6-dimethoxypyrimidin-4-yl)aminocarbonyl]-4-methylbenzenesulfonamide:

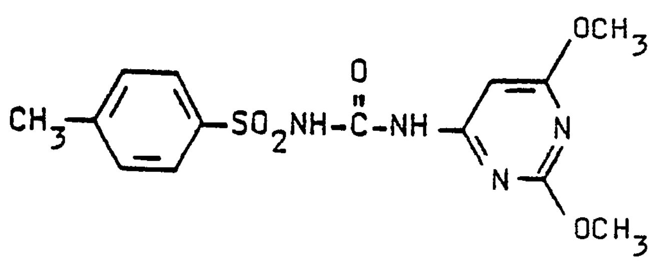

Based upon similarity to a known compound, the author predicted hypoglycemic activity for the foregoing compound.

Netherlands Patent 121,788, published September 15, 1966, teaches the preparation of compounds of Formula (i), and their use as general or selective herbicides,

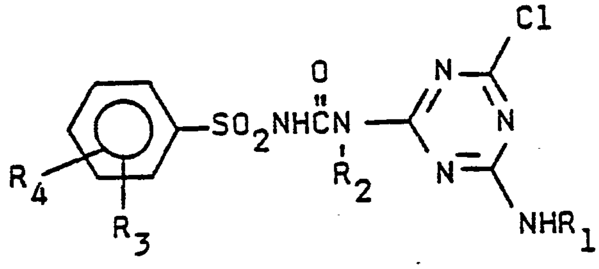
(i)

wherein

$R_1$ and $R_2$ may independently be alkyl of 1-4 carbon atoms; and

$R_3$ and $R_4$ may independently be hydrogen, chlorine or alkyl of 1-4 carbon atoms.

Compounds of Formula (ii), and their use as antidiabetic agents, are reported in J. Drug. Res. 6, 123 (1974),

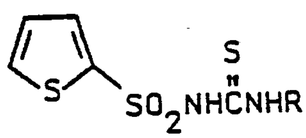
(ii)

wherein

R is pyridyl.

The presence of undesired vegetation causes substantial damage to useful crops, especially agricultural products that satisfy man's basic food needs, such as soybeans, barley, wheat, and the like. The current population explosion and concomitant world food shortage demand improvements in the efficiency of producing these crops. Prevention or minimizing the loss of a portion of valuable crops by killing, or inhibiting the growth of undesired vegetation is one way of improving this efficiency.

A wide variety of materials useful for killing, or inhibiting (controlling) the growth of undesired

vegetation is available; such materials are commonly referred to as herbicides. The need exists, however, for still more effective herbicides that destroy or retard weeds without causing significant damage to useful crops.

## Summary of the Invention

This invention relates to compounds of Formulae I, II, III, Ia and IIa, suitable agricultural compositions containing them, and their methods of use as general and/or selective pre-emergent and/or post-emergent herbicides.

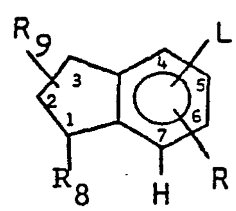

I

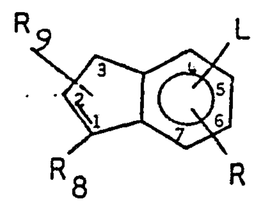

II

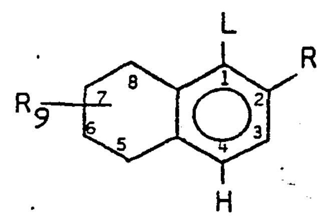

III

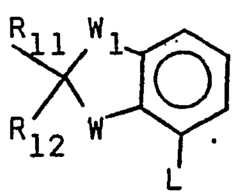

Ia

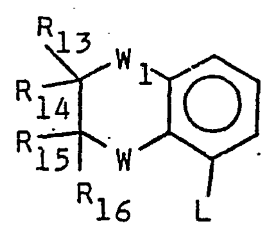

IIa

wherein

L is $SO_2NH\overset{O}{\overset{\|}{C}}\underset{R_{10}}{N}R_1$ ;

R is H, Cl, Br, $NO_2$, $QR_2$, $CO_2R_3$, $SO_2NR_5R_6$, $SO_2N(OCH_3)CH_3$, $OSO_2R_7$ or $C_1$-$C_3$ alkyl;

$R_2$ is $C_1$-$C_3$ alkyl, $CF_2H$, $CF_3$ or $CF_2CF_2H$;

$R_3$ is $C_1$-$C_3$ alkyl, $CH_2CH{=}CH_2$, $CH_2CH_2OCH_3$ or $CH_2CH_2Cl$;

$R_5$ and $R_6$ are independently $C_1$-$C_2$ alkyl;

$R_7$ is $C_1$-$C_3$ alkyl or $CF_3$;

$R_8$ is H, $CH_3$ or Cl;

$R_9$ is H or $CH_3$;

$R_{10}$ is H or $CH_3$;

Q is O, S or $SO_2$;

W is O, S, SO or $SO_2$;

$W_1$ is O, S, SO or $SO_2$;

$R_{11}$ is H or $CH_3$;

$R_{12}$ is H or $C_1$-$C_4$ alkyl;

$R_{13}$, $R_{14}$, $R_{15}$, $R_{16}$ and $R_{17}$ are independently H or $CH_3$;

$R_1$ is 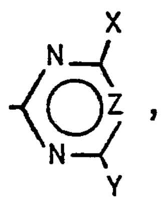 , 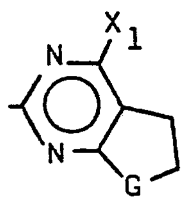 , 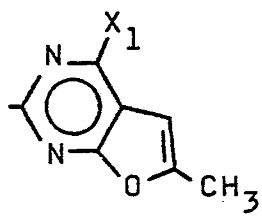 ,

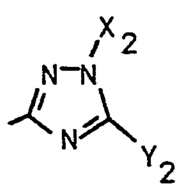 or 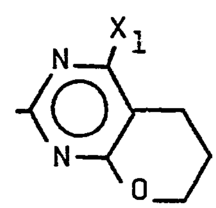 ;

X is $CH_3$, $OCH_3$ or Cl;

Y is $CH_3$, $C_2H_5$, $OCH_3$, $OC_2H_5$, $CH_2OCH_3$, $NH_2$, $NHCH_3$, $SCH_3$, $CH(OCH_3)_2$; 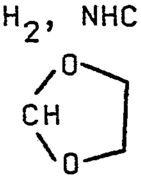 or $N(CH_3)_2$;

Z is CH or N;

$X_1$ is $CH_3$ or $OCH_3$;

G is O or $CH_2$;

$X_2$ is $C_1$-$C_3$ alkyl or $CH_2CF_3$; and

$Y_2$ is $SCH_3$, $SC_2H_5$, $OCH_3$ or $OC_2H_5$.

and their agriculturally suitable salts;

provided that

(1) in Formula I, L is not in the 6-position;

(2) in Formula I, when L is in the 4-position and R is in the 6-position, then R is H, Cl, Br, $NO_2$, $CH_3$ or $OCH_3$;

(3) in Formula II, when $R_8$ is Cl, then R is other than $C_1$-$C_3$ alkyl; and

(4) when X is Cl, then Z is CH and Y is $NH_2$, $NHCH_3$, $N(CH_3)_2$ or $OCH_3$.

Preferred for reasons of their higher herbicidal activity and/or more favorable ease of synthesis are:

(1) Compounds of the generic scope of Formula I.

(2) Compounds of the generic scope of Formula II.

(3) Compounds of the generic scope of Formula III.

(4) Compounds of <u>Formula Ia</u> where W and $W_1$ have identical values.

(5) Compounds of <u>Formula IIa</u> where W and $W_1$ have identical values.

(6) Compounds of <u>Preferred (1)</u> where L is fixed at the 4-position and $R_9$ is at the 2 or 3 position.

(7) Compounds of <u>Preferred (6)</u> where R is fixed at the 5-position and $R_{10}$ is H.

(8) Compounds of <u>Preferred (7)</u> where

$R_1$ is 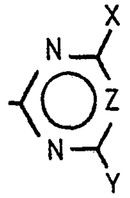 .

(9) Compounds of Preferred (8) where R is $CO_2CH_3$, $SO_2CH_3$, $SO_2N(CH_3)_2$, Cl, $NO_2$, $OSO_2CH_3$, $OCF_2H$, $SCF_2H$ or H.

(10) Compounds of Preferred 4 where

A is 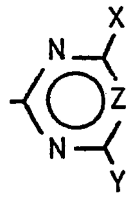 ; and

$R_{10}$ is H.

(11) Compounds of Preferred 5 where

A is 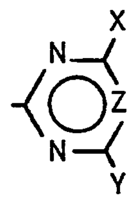 ; and

$R_{10}$ is H.

(12) Compounds of Preferred 10 where $R_{12}$ is H or $CH_3$.

(13) Compounds of Preferred 12 where Y is $CH_3$ or $OCH_3$.

(14) Compounds of Preferred 13 where W and $W_1$ are O or S.

(15) Compounds of Preferred 13 where W and $W_1$ are SO or $SO_2$.

Specifically Preferred are:

- 2,3-dihydro-N-[(4,6-dimethoxypyrimidin-2-yl)aminocarbonyl]-1H-indene-4-sulfonamide;
- 2,3-dihydro-N-[(4,6-dimethylpyrimidin-2-yl)aminocarbonyl]-1H-indene-4-sulfonamide;
- 2,3-dihydro-N-[(4-methoxy-6-methylpyrimidin-2-yl)-aminocarbonyl]-1H-indene-4-sulfonamide;

- 2,3-dihydro-N-[(4,6-dimethoxy-1,3,5-triazin-2-yl)-aminocarbonyl]-1H-indene-4-sulfonamide;
- 2,3-dihydro-N-[(4,6-dimethyl-1,3,5-triazin-2-yl)-aminocarbonyl]-1H-indene-4-sulfonamide;
- 2,3-dihydro-N-[(4-methoxy-6-methyl-1,3,5-triazin-2-yl)aminocarbonyl]-1H-indene-4-sulfonamide;
- N-[(4-methoxy-6-methylpyrimidin-2-yl)aminocarbonyl]-2,2-dimethyl-1,3-benzodioxole-4-sulfonamide;
- N-[(4,6-dimethylpyrimidin-2-yl)aminocarbonyl]-2,2-dimethyl-1,3-benzodioxole-4-sulfonamide;
- N-[(4,6-dimethoxypyrimidin-2-yl)aminocarbonyl-2,2-dimethyl-1,3-benzodioxole-4-sulfonamide;
- N-[(4,6-dimethoxy-1,3,5-triazin-2-yl)aminocarbonyl]-2,2-dimethyl-1,3-benzodioxole-4-sulfonamide;
- N-[(4-methoxy-6-methyl-1,3,5-triazin-2-yl)aminocarbonyl]-2,2-dimethyl-1,3-benzodioxole-4-sulfonamide;

and

- N-[(4,6-dimethyl-1,3,5-triazin-2-yl)aminocarbonyl]-2,2-dimethyl-1,3-benzodioxole-4-sulfonamide.

Synthesis

Many of the compounds of Formulae I, II or III may be prepared as shown in Equation 1 by the reaction of an appropriately substituted sulfonyl isocyanate,

$$L_1SO_2NCO$$

(IV)

wherein $L_1$ is a radical selected from:

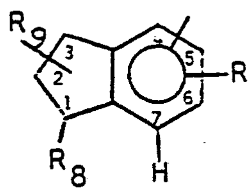

(A)

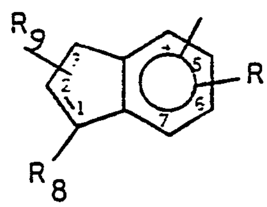

(B)

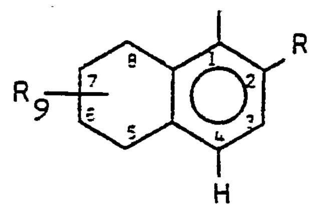

(C)

and the appropriate heterocyclic amine $R_1R_{10}NH$ (V); R, $R_1$, $R_8$, $R_9$ and $R_{10}$ being as previously defined.

Equation 1

$$\underset{(IV)}{L_1SO_2NCO} + \underset{(V)}{R_1R_{10}NH} \longrightarrow \underset{(VI)}{L_1SO_2NHCON(R_{10})\text{-}R_1}$$

The reaction of Equation 1 is generally carried out by adding a solution of the sulfonyl isocyanate (IV) in an inert solvent such as methylene chloride or acetonitrile to a suspension or solution of the heterocyclic amine (V) in the same solvent under anhydrous conditions. Since the sulfonyl isocyanate (IV) is usually a liquid or readily soluble in the solvent used, it is usually convenient to add this to a suspension or solution of the amine. The mixture

is then stirred from one to sixteen hours at temperatures from ambient to the reflux temperature of the solvent used. In some cases, the reaction is exothermic and the products crystallize from the reaction mixture on cooling. When the product is soluble in the reaction medium it can be isolated by evaporation of the solvent and trituration with a suitable solvent such as 1-chlorobutane, hexane or similar non-polar solvent.

Sulfonyl isocyanates can be prepared by the reaction of sulfonamides of structure IVa with phosgene in the presence of an alkyl isocyanate such as butyl isocyanate in an inert solvent, such as chlorobenzene or xylene, by the procedure of H. Ulrich and A. A. Y. Sayigh (Newer Methods of Preparative Organic Chemistry, Vol. VI, p. 223-41, Academic Press, New York and London, W. Foerst, Ed.) as shown by Equation 2.

<u>Equation 2</u>

$$\underset{\text{(IVa)}}{L_1SO_2NH_2} + COCl_2 \xrightarrow[\text{xylene}]{C_4H_9NCO} \underset{\text{(IV)}}{L_1SO_2NCO}$$

An alternative procedure for the preparation of the sulfonylureas of this invention is via the reaction of a carbamate of structure IVb with a heterocyclic amine (V) by the procedure of W. Meyer and W. Fory, EPO 44,807 as shown in Equation 2a.

<u>Equation 2a</u>

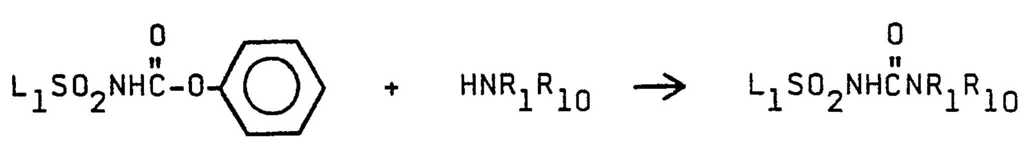

(IVb) (V) (VI)

The carbamates of structure IVb can be prepared by methods also described by M. Meyer and W. Fory (op. cit).

Alternatively, many of the sulfonylureas of this invention may be prepared by the reaction of sulfonamides of Formula IVa with heterocyclic isocyanates of Formula Va followed by replacement of the active halogens on the hetero ring as shown by the sequence in Equation 3.

Equation 3

a)

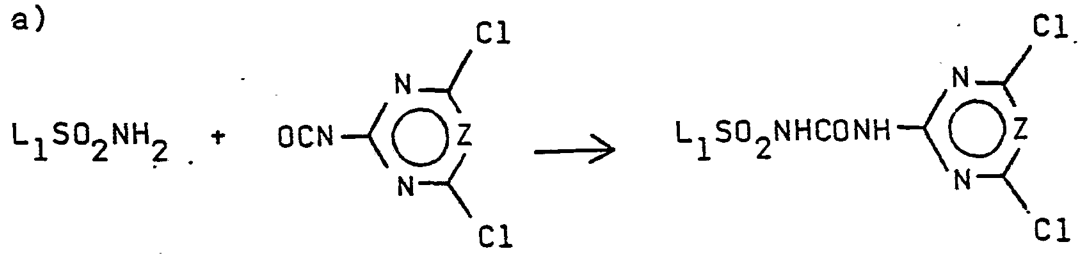

(IVa) (Va) (VIa)

b)

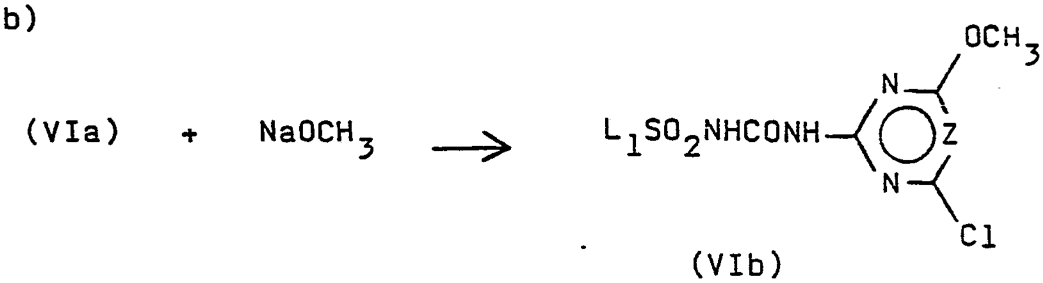

(VIb)

c)

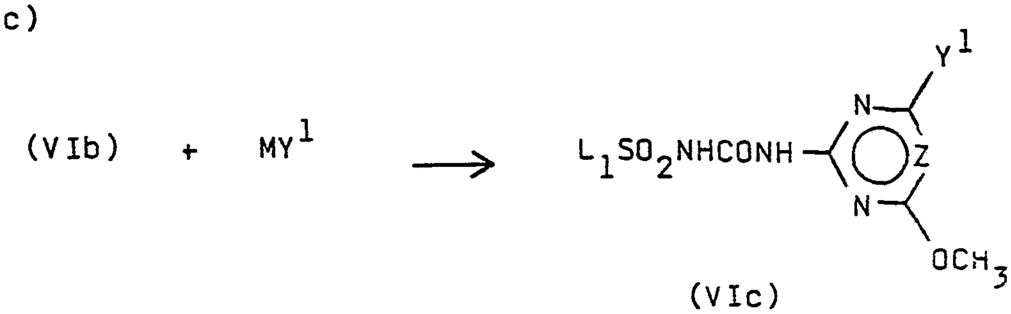

(VIc)

In Equation 3, $L_1$ and Z are as previously defined, $Y^1$ is $OCH_3$, $OC_2H_5$, $NH_2$, $NHCH_3$ or $N(CH_3)_2$ and M is H or an alkali metal cation. In Reaction

Step (3a), an appropriately substituted sulfonamide is reacted with a heterocyclic isocyanate of Formula Va to yield a sulfonylurea of Formula VIa. The heterocyclic isocyanates used in Reaction 3a may be prepared according to the method described in Swiss Patent 579,062; U.S. Patent 3,919,228, U.S. Patent 3,732,223 and Angew Chem. Int. Ed., 10, 402 (1976), the disclosures of which are herein incorporated by reference.

In Reaction Step (3a), an appropriately substituted sulfonamide is contacted with a heterocyclic isocyanate of Formula Va in the presence of an inert organic solvent such as methylene chloride, tetrahydrofuran, acetonitrile, acetone or butanone. Optionally, a catalytic amount of a base such as 1,4-diazabicyclo[2.2.2]octane (DABCO), potassium carbonate or sodium hydroxide may be added to the reaction mixture.

In Reaction Steps (3b) and (3c), one or two of the chlorine atoms on the pyrimidinyl or triazinyl ring of the compound of Formula VIa is displaced by an alcohol or amine (3c). Generally, this may be done by contacting the compound of Formula VIa with methanol or methoxide. Thus, in Reaction Step (3b), a compound of Formula VIa may be contacted with at least one equivalent of methanol. This reaction is sluggish, however, and it is preferred to contact the compound of Formula VIa with at least two equivalents of sodium methoxide in acetonitrile, THF or dimethylformamide.

It should be noted that two equivalents of methoxide are required for Reaction Step (3b) whereas only one equivalent of methanol is needed for the same process. When methoxide is used, the first equivalent of base removes a proton from the sulfonyl nitrogen, and it is only the second equivalent which effects displacement of the halogen. As a result, two equivalents of methoxide are required. The resulting salt

must be acidified, e.g., with sulfuric, hydrochloric or acetic acid, to yield a compound of Formula VIb. Applicant, of course, does not intend to be bound by the mechanism described above.

In Reaction Step (3c), a compound of Formula VIb is contacted with either one equivalent of alkanol, $CH_3OH$ or $C_2H_5OH$, or with two equivalents of alkoxide, $CH_3O-$ or $C_2H_5O-$ or two equivalents of $NH_3$, $H_2NCH_3$ or $HN(CH_3)_2$ to give sulfonylureas of Formula VIc.

The compounds of Formulae Ia and IIa can be prepared by one of the methods described below in Equations 4 and 5.

As shown in Equation 4, compounds of Formula Ib can be prepared by reaction of the sulfonamide of Formula VII with an appropriate methyl heterocyclic carbamate of Formula VIII in the presence of an equimolar amount of trimethylaluminum, wherein $R_{11}$, $R_{12}$, $R_{10}$, $W_1$, W and $R_1$ are as previously defined.

Equation 4

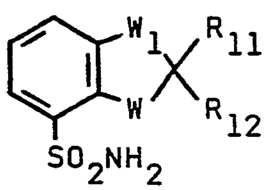

(VII)

+

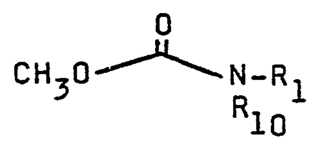

(VIII)

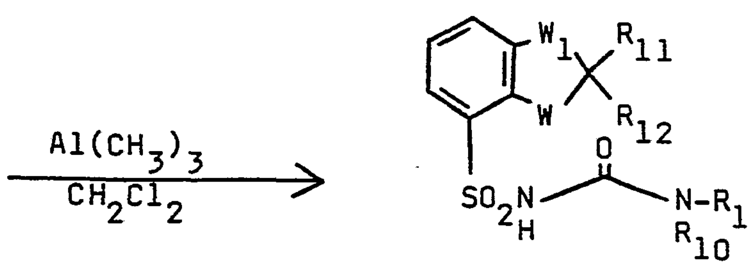


(Ib)

The reaction of Equation 4 takes place in methylene chloride at reflux or ambient temperature for 16-96 hours under an inert atmosphere. The product can be isolated after the sequential addition of aqueous acetic acid and hydrochloric acid to the reaction mixture followed by the partitioning of the product into methylene chloride. The product can be purified via crystallization from such solvents as n-butyl chloride, diethyl ether, tetrahydrofuran, or methylene chloride or by silica medium pressure liquid chromatography.

As shown in Equation 5, compounds of Formula IIb can be prepared by reaction of the sulfonamide of Formula IX with an appropriate methyl heterocyclic carbamate of Formula VIII in the presence of an equimolar amount of trimethylaluminum, wherein $R_{13}$ to $R_{16}$, $R_{10}$, $W_1$, W and $R_1$ are as previously defined.

Equation 5

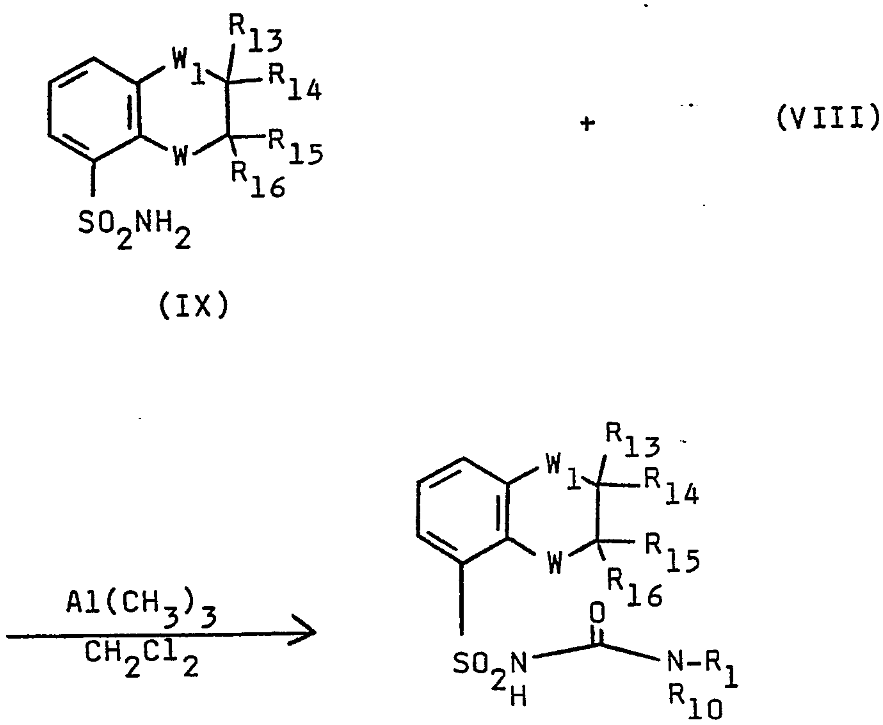


(IX) + (VIII)

(IIb)

The reaction of Equation 5 is performed in methylene chloride at reflux for 16-72 hours under an inert atmosphere. The product is isolated upon sequential addition of aqueous acetic acid and hydrochloric acid to the reaction mixture followed by the partitioning of the product into methylene chloride. The product is purified via crystallization from solvents such as n-butyl chloride, diethyl ether or methylene chloride or by silica medium pressure liquid chromatography.

Sulfonyl chlorides and sulfonamides of 1,2,3,4-tetrahydronaphthalene and 2,3-dihydro-1H-indene are well known in the art (c.f. Organic Chemistry of Sulfur, p. 484, C. M. Suter, John Wiley and Sons, New York, 1944). The preparation of sulfonamides of Formula IVa from the corresponding sulfonyl chlorides may be carried out using methods widely reported in the literature [e.g., Crossley et al., J. Am. Chem. Soc., 60, 2223 (1938); or Cross et al., J. Med. Chem., 21, 348 (1978)]. Sulfonyl chlorides may be prepared, for example, by direct chlorosulfonation of the aromatic ring using chlorosulfonic acid according to the method of R. T. Arnold and H. E. Zaugg, (J. Am. Chem. Soc., 63, 1317-20 (1941)).

Sulfonation also may be carried out with sulfuric acid according to the procedure of A. Courtin, Helv. Chim. Acta., 64, 572-78 (1981). The sulfonic acid thus obtained may be converted to the sulfonyl chloride by treatment with thionyl chloride, phosphorus trichloride or phosphorus pentachloride according to W. A. Gregory, U.S. 2,888,486. Alternatively, appropriately substituted amines, may also be converted to sulfonyl chlorides by preparing their diazonium chlorides and treating these with sulfur dioxide in the presence of a copper salt.

The preparation of 1,2,3,4-tetrahydro-5-nitronaphthalene-4-sulfonyl chloride via the diazonium chloride salt route (Equation 6) was reported by Courtin (op. cit.)

Equation 6

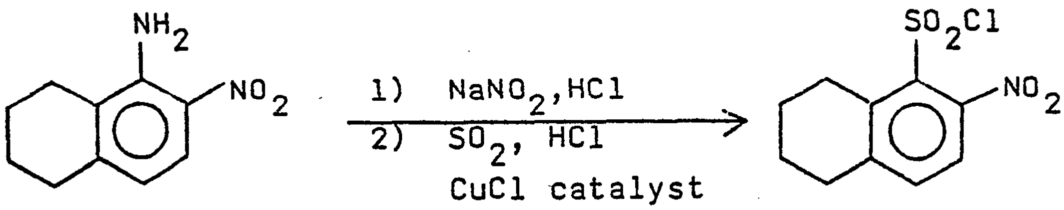


Nitration of 2,3-dihydro-1H-indene sulfonic acid derivatives, following the procedure of H. V. B. Joy and M. T. Bogart, J. Org. Chem., 1, 236-244 (1936) and J. Hornya, et al., Czech, 155,849 (15, Nov. 1974). 2,3-Dihydro-1H-indene-4-sulfonyl chloride yields the 6-nitro derivative and 2,3-dihydro-1H-indene-5-sulfonic acid yields a mixture of the 4,6- and 7-nitro derivatives which can be separated by fractional crystallization of the potassium salts.

Substituted benzenepropionic acids and benzenebutyric acids can be converted to 2,3-dihydro-1H-indenes and 1,2,3,4-tetrahydronaphthalene derivatives as shown in Equation 7 according to the procedure of M. Olivier and E. Marechal (Bull. Soc. Chim. Fr., 1973, 3092), wherein R is Cl, Br, $OR_2$, $SR_2$ or $C_1$-$C_3$ alkyl and $R_2$ is as previously defined.

Equation 7

a)

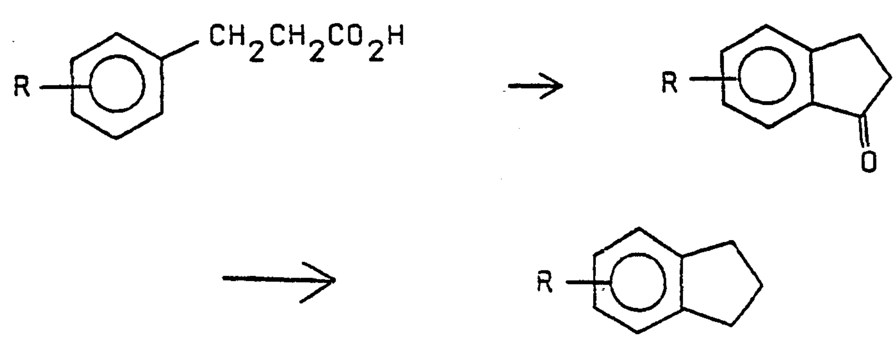

b)

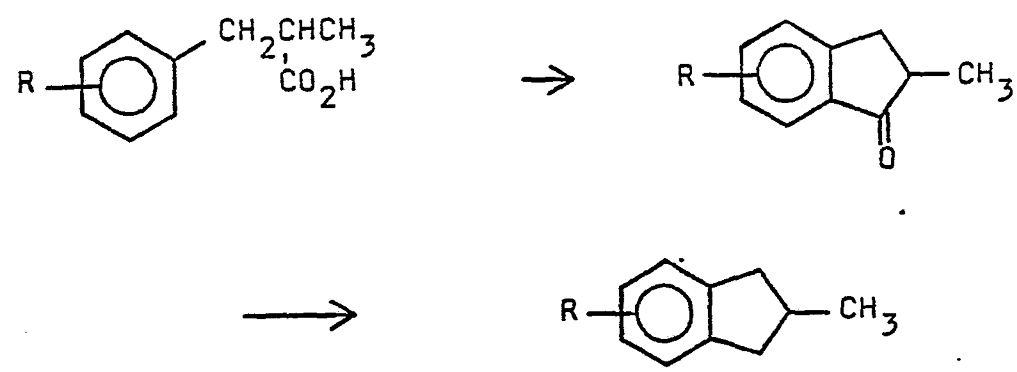

c)

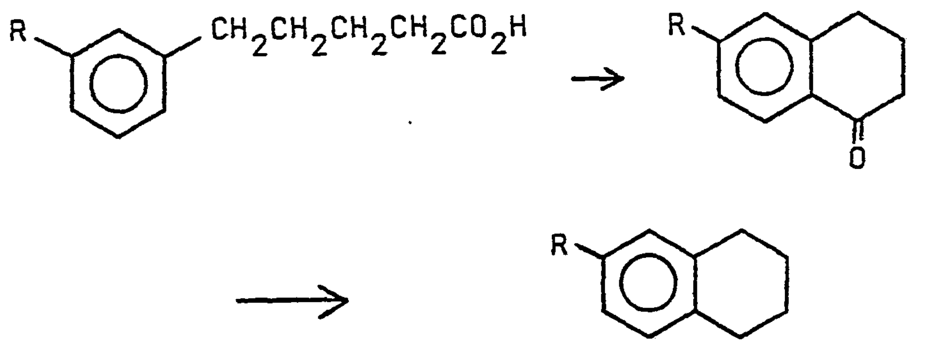

The intermediates obtained by the procedure of Equation 7 can be sulfonated or chlorosulfonated by the procedures previously described.

Nitro-2,3-dihydro-1H-indenylamines such as those reported by R. T. Arnold and J. Richter, J. Am. Chem. Soc., 70, 3505 (1948) and J. Majer et al., Czech. 152238 (15, Feb. 1974) C. A. 82, 97885 (1975) and 1,2,3,4-tetrahydro-4-nitro-5-naphthyl amine can be diazotized according to the procedure of A. Courtin (op. cit.) and the salt thus obtained can be reacted to obtain derivatives wherein the amino group has been replaced by R' where R' = Br, Cl, SH, $SO_2Cl$, CN or OH moieties as shown in Equation 8 by methods described by D. S. Wulfman (The Chemistry of Diazonium Salts, Part I, pages 274-312, Saul Patai, Editor, John Wiley and Sons, New York, 1978) or Courtin (op. cit.).

Equation 8

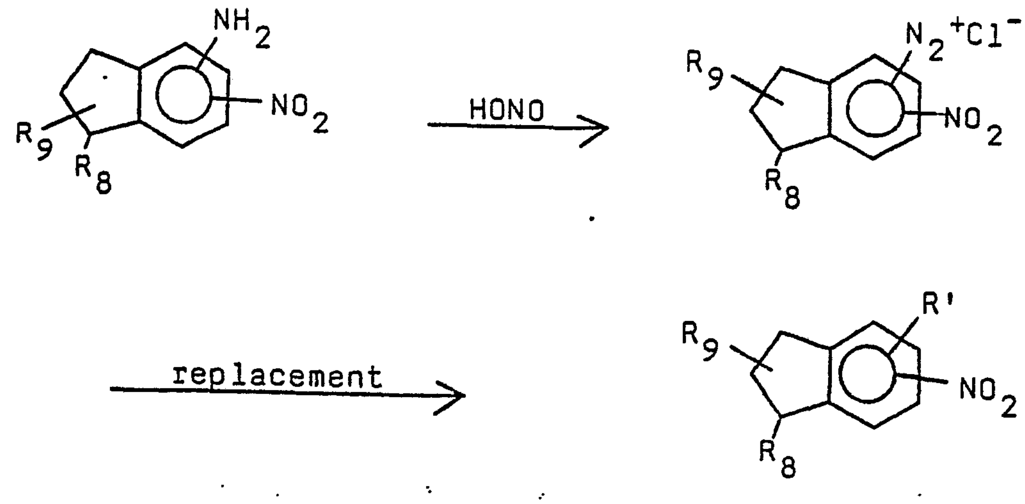


The intermediates thus prepared wherein R' is OH or SH can be alkylated by methods well known in the art to obtain derivatives where R' is $OR_2$ or $SR_2$. The thioethers thus obtained can be oxidized to yield intermediates with the $SO_2R_2$ moiety by warming the thioethers in acetic acid containing excess 30% hydrogen peroxide. In those instances where R'=$SO_2Cl$, those moieties can be converted to $SO_2NR_5R_6$ or $SO_2N(OCH_3)CH_3$ by methods well known in the art, such as Crossley et al. (op. cit.) or Cross et al. (op. cit.). Reaction of the intermediates where R'=OH with $R_7SO_2Cl$ in the presence of a base such as triethylamine yields intermediates with the $R_7SO_2O$-moiety.

Intermediates wherein R' is $CO_2R_3$ can be prepared from compounds where R' is CN by methods well known in the art. Hydrolysis of the nitrile group by aqueous sodium hydroxide yields the sodium salt of the acid (R' is $CO_2Na$) which can be converted to the desired carboxylic ester (where R' is $CO_2R_3$) by refluxing the product from the hydrolysis with the appropriate alcohol in the presence of a mineral acid.

As shown in Equation 9, the substituted 2,3-dihydro(4-, 5- or 6-)nitro-1H-indenes and 1,2,3,4-tetrahydro-5-nitronaphthalenes obtained above can be reduced to the corresponding amines which can be converted in turn to the desired sulfonyl chlorides.

Equation 9

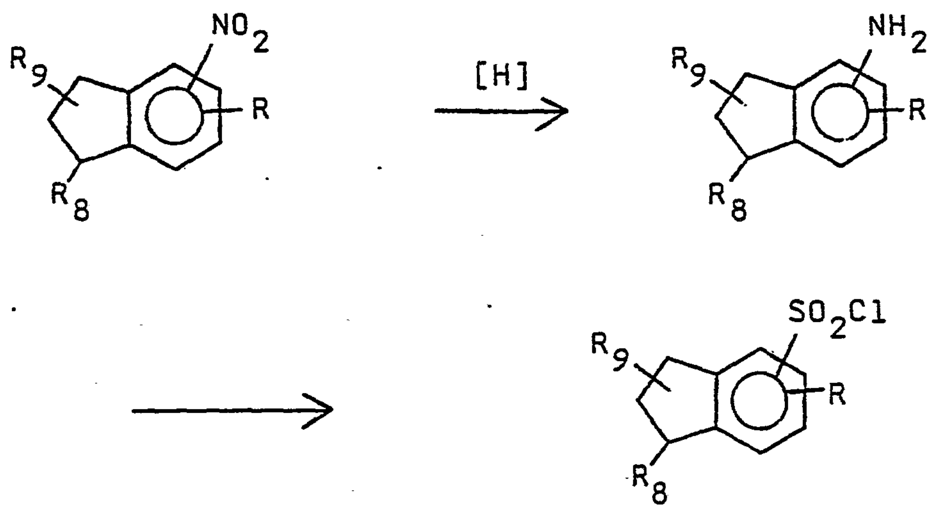


Reaction with hydrogen at a pressure of 100 to 1000 p.s.i.g. at a temperature of 80 to 150° in a solvent, such as ethanol, ethyl acetate or dimethylformamide, yields the desired substituted (4-, 5- or 6-)amino-2,3-dihydro-1H-indenes or 4-amino-1,2,3,4-tetrahydronaphthalenes. The product can be converted to the corresponding 2,3-dihydro-1H-indenyl-4-, 5- or 6-sulfonyl chlorides or 1,2,3,4-tetrahydronaphthyl-4-sulfonyl chloride via diazotization of the amine followed by reaction of the diazonium salt with sulfur dioxide and hydrochloric acid in the presence of cuprous or cupric chloride by the procedure of Courtin (op. cit.).

Using methods described in Organic Synthesis, V. 2, pages 924-5, V. Midrigician, Reinhold Publ. Co., New York, 1960, the 2,3-dihydro-1H-indenyl-(4-, 5- or 6-)sulfonamide intermediates prepared according to the above methods can be converted to the corresponding

1H-indenyl-(4-, 5- or 6-)sulfonamides by contacting the appropriate 2,3-dihydro derivative with a N-halogenated imide such as N-bromosuccinimide (NBS) followed by dehalogenation as shown in Equation 10 wherein R, $R_8$ and $R_9$ are as previously defined.

Equation 10

a)

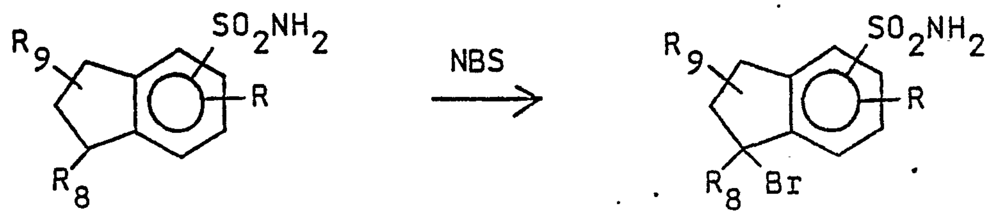


b)

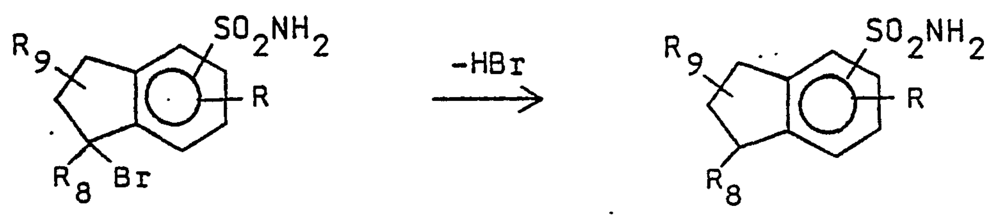


3-Chloro-1H-indene-7-sulfonyl chloride may be prepared by the chlorination of 2,3-dihydro-1H-indene-4-sulfonyl chloride with chlorine oxide in carbon tetrachloride as described in Example 9.

As shown in Equation 11, the sulfonamides of Formula VII wherein $R_{11}$ and $R_{12}$ are not H and $W_1$ and W are as previously defined can be prepared from the appropriate 1,3-benzodioxole of Formula X via the lithium sulfinate of Formula XI.

Equation 11

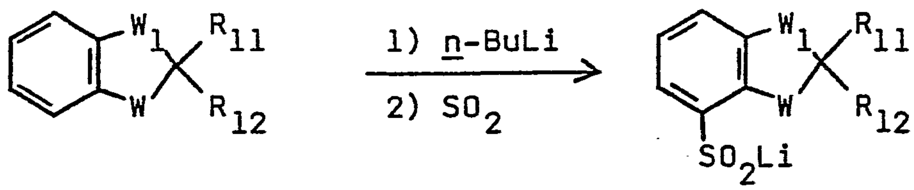


(X) (XI)

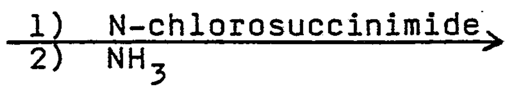


(VII)

Reaction of the arene of Formula X with n-butyllithium in ether or tetrahydrofuran in the presence or absence of a chelating agent such as N,N,N',N'-tetramethylethylene diamine at -78° to ambient temperature for 2-16 hours followed by contacting with sulfur dioxide at -78° to ambient temperature until no further exotherm is noted results in the formation of the lithium sulfinate of Formula XI. The 4-sulfonamide of Formula VII is isolated in a two-step sequence upon reaction of the lithium sulfinate of Formula XI with N-chlorosuccinimide in glacial acetic acid at 15 to 25°C followed by contacting the product with an aqueous solution to give the sulfonyl chloride. The sulfonyl chloride is then contacted with excess ammonia in diethyl ether or methylene chloride at 10-20°C. The 1,3-benzodioxoles of Formula X wherein $W_1$ and W are O can be prepared according to the procedures of C. M. Yoder and J. J. Zuckerman, J. Heterocycl. Chem., 1967, 4, 166; E. R. Cole et al., Aust. J. Chem., 1980, 33, 675; and W. Bonthrone and J. W. Cornforth, J. Chem. Soc. (C), 1969, 1202.

The 1,3-benzodithioles and the benzoxathioles of Formula X, wherein $W_1$ and W are O or S, can be prepared according to the procedures suggested by S. Cabiddu et al. Synthesis, 1976, 797; G. Scherowsky and J. Weiland, Chem. Ber. 1974, 3155; and A. Pelter et al. Tetrahed. Lett. 1978, 26, 2345. Compounds of Formula X wherein $W_1$ and/or W are higher oxidation states of sulfur can be prepared from compounds of Formula X, wherein $W_1$ and W are not both O, or $SO_2$ or a combination of O and $SO_2$, upon treatment with the appropriate equivalents of 30% hydrogen peroxide in acetic acid at ambient temperature or reflux for 4-17 hours as suggested by W. Parham et al. J. Am. Chem. Soc., 1953, 75, 1953; permanganate treatment according to the procedure of D. Greenwood and H. Stevenson, J. Chem. Soc., 1953, 1514, or via periodate oxidation. Mixtures of oxidized benzodithioles and benzoxathioles, obtained by these procedures, can be purified via fractional crystallization or column chromatography.

An alternative route to the preparation of the 4-sulfonamide of Formula VII, wherein $W_1$ and W are O or S and $R_{11}$ and $R_{12}$ are as previously defined, including H, from bromobenzene of Formula XII is illustrated below in Equation 12.

Equation 12

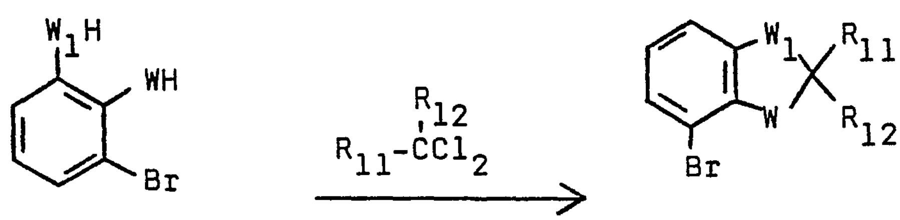


(XII) (XIII)

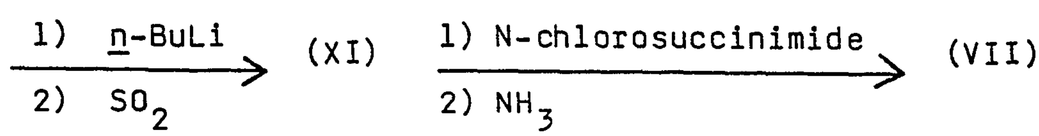

The 3-bromocatechol of Formula XII can be prepared according to the procedure of H. S. Mason, J. Am. Chem. Soc. 1947, 69, 2241. The 4-bromo-1,3-benzodioxole of Formula XIII can be prepared from 3-bromocatechol of Formula XII via procedures similar to those described by W. Bonthrone and J. W. Cornforth, J. Chem. Soc. (C), 1969, 1202. The bromo thiocatechols and bromothiophenols of Formula XII can be prepared according to the procedures of L. Horner et al., Phosphorus and Sulfur, 1982, 2, 353 and A. Ferretti, Org. Synth., 1973, Coll Vol V, 419. The 4-bromo-1,3-benzodithioles and the bromoxathioles of Formula XIII can be prepared according to the procedure of S. Cabiddu et al., Synthesis, loc. cit. A metal-halogen interchange of the bromo compound of Formula XIII in an ether solution at -70 to -78°C using 1.1 equivalents of n-butyllithium followed by sulfur dioxide contact at -45 to -78°C can afford the lithium sulfinate of Formula XI. The sulfonamide of Formula VII, can be prepared from the sulfinate of Formula XI by the N-chlorosuccinimide, ammonia procedures shown in Equation 11.

An alternative route to the preparation of 4-sulfonamides of Formula VII wherein $W_1$ and W can be higher oxidation states of sulfur is illustrated in Equation 13.

Equation 13

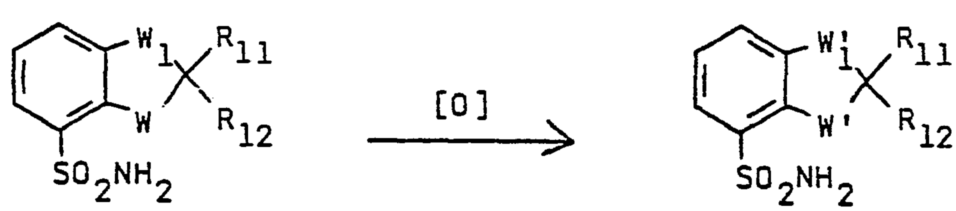


(VIIa) (VIIb)

Reaction of the 4-sulfonamides of Formula VIIa wherein $W_1$ and W are not both O or $SO_2$ or a combination of O and $SO_2$ and $R_{11}$ and $R_{12}$ are as previously defined, with 30% hydrogen peroxide as suggested by W. Parham et al., J. Am. Chem. Soc., loc. cit.; permangonate reaction according to the procedure of D. Greenwood and H. Stevenson, J. Chem. Soc., loc. cit.; or periodate reaction may afford the 4-sulfonamides of Formula VIIb wherein $W_1'$ and W' may be higher oxidation states of sulfur. Mixtures of oxidized sulfonamides, obtained by these procedures, can be purified via fractional crystallization or column chromatography.

As illustrated below in Equation 14, 5-sulfonamides of Formula IX, wherein $R_{13}$ through $R_{16}$ are not H and $W_1$ and W are as previously defined can be prepared from arenes of Formula XIV via the lithium sulfinate of Formula XV according to the procedures of Equation 11.

Equation 14

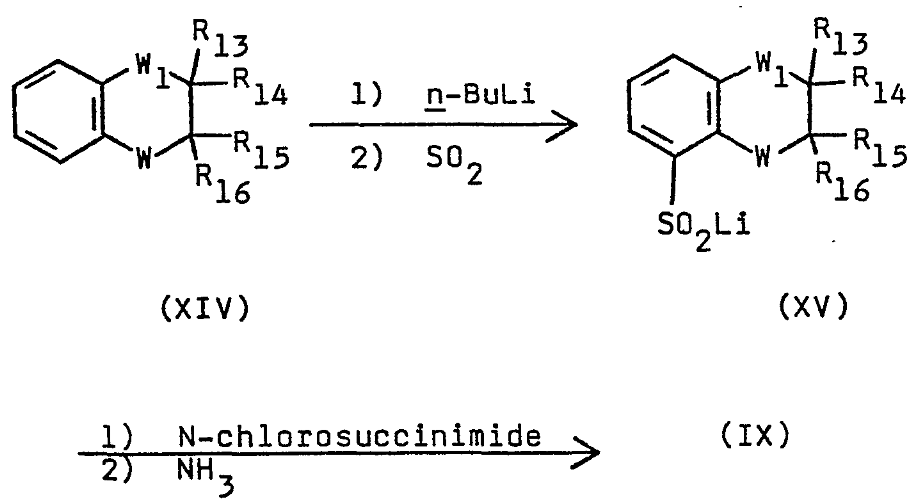


(XIV) (XV)

(IX)

The 1,4-benzodioxans of Formula XIV wherein $W_1$ and W are O can be prepared according to the procedures of Schroth et al., Z. Chem., 1967, 7, 152 and B. N. Ghosh, J. Chem. Soc., 1915, 107, 1591, W. Adam et al., Synthesis, 1982, 322, A. Bashall and J. Collins, Tetrahed. Lett., 1975, 40, 3489.

The 1,4-benzodithians and the benzoxathians of Formula XIV, wherein $W_1$ and W are both S or a combination of S and O can be prepared according to the procedures of W. Parham et al., J. Am. Chem. Soc., loc. cit. and S. Cabiddu et al., Synthesis, loc. cit. Compounds of Formula XIV wherein $W_1$ and/or W can include higher oxidation states of sulfur can be prepared upon hydrogen peroxide, permanganate or periodate treatment as suggested by W. Parham et al., J. Am. Chem. Soc., loc. cit. and D. Greenwood and H. Stevenson, J. Chem. Soc., loc. cit. Mixtures of oxidized benzodithians and benzoxathians, obtained by these procedures, can be purified via fractional crystallization or column chromatography.

An alternative route to the preparation of the 5-sulfonamides of Formula IX, wherein $W_1$ and W are O or S and $R_{13}$ through $R_{16}$ are as previously defined and include H from the bromobenzenes of Formula XII via the cyclic arene of Formula XVI and lithium sulfonate of Formula XV according to the procedures of Equation 12 is illustrated in Equation 15.

Equation 15

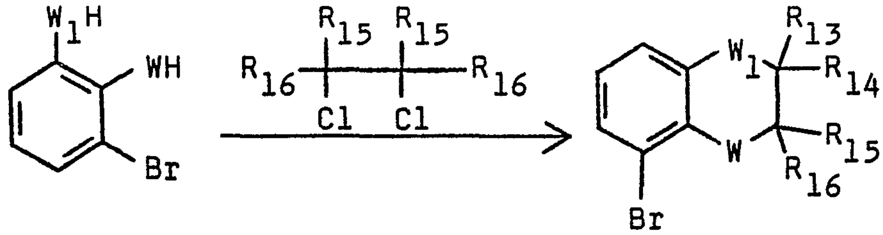

(XII) (XV)

1) n-BuLi
2) $SO_2$
(XV)
1) N-chlorosuccinimide
2) $NH_3$
(IX)

The preparation of the bromobenzenes is illustrated in the discussion of Equation 12. The bromobenzodioxans of Formula XVI can be prepared according to the procedures of Schroth et al., Z. Chem., loc. cit. and B. Ghosh, J. Chem. Soc., loc. cit. The bromobenzodithians and bromobenzoxathians of Formula XVI can be prepared according to the procedures of the benzodithians and benzoxathians of Formula XIV, discussed in Equation 14.

As illustrated in Equation 16, the 5-sulfonamides of Formula IXb wherein $R_{13}$ through $R_{16}$ are as previously defined and $W_1$ and W may include higher oxidation states of sulfur, may be prepared via the 5-sulfonamides of Formula IXa, wherein $W_1$ and W are not both O or $SO_2$ or a combination of O and $SO_2$ and $R_{13}$ through $R_{16}$ are as previously defined, according to the procedures of Equation 13.

Equation 16

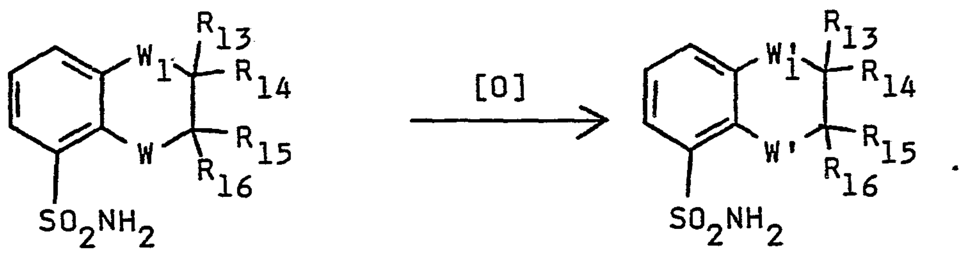


(IXa) (IXb)

Equation 17 illustrates the preparation of the requisite methyl heterocyclic carbamates of Formula VIII utilized in Equations 4 and 5, wherein $R_{10}$ and $R_1$ are as previously defined.

Equation 17

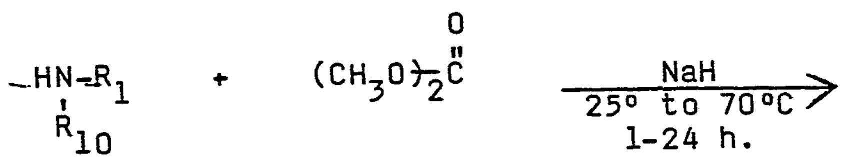


(VIII)

(V)

According to Equation 17, a heterocyclic amine of Formula V is contacted with two equivalents of sodium hydride and excess dimethylcarbonate to form VIII. This reaction is performed in an inert solvent such as tetrahydrofuran at 25 to 70°C for 1 to 24 hours. The product is isolated by (a) addition of two equivalents of concentrated hydrochloric acid and aqueous saturated sodium chloride and (b) separation of the organic phase followed by concentration to dryness *in vacuo*.

The synthesis of the pyrimidine and triazine amines of Formula V has been reviewed in "The Chemistry of Heterocyclic Compounds," a series published by Interscience Publ., New York and London. The 2-aminopyrimidines are described by D. J. Brown in

"The Pyrimidines," Vol. XXVI of this series. The 2-amino-1,3,5-triazines can be prepared according to methods described by E. M. Smolin and L. Rapaport in "s-Triazines and Derivatives," Vol. XIII of the same series. The synthesis of triazines is also described by F. C. Schaefer, U.S. 3,154,547 and by K. R. Huffman and F. C. Schaefer, J. Org. Chem., 1963, 28, 1816.

Braker, Sheehan, Spitzmiller and Lott, J. Am. Chem. Soc., 69, 3072 (1947) describe the preparation of 6,7-dihydro-4-methoxy-5H-cyclopentapyrimidin-2-amine by the following sequence of reactions.

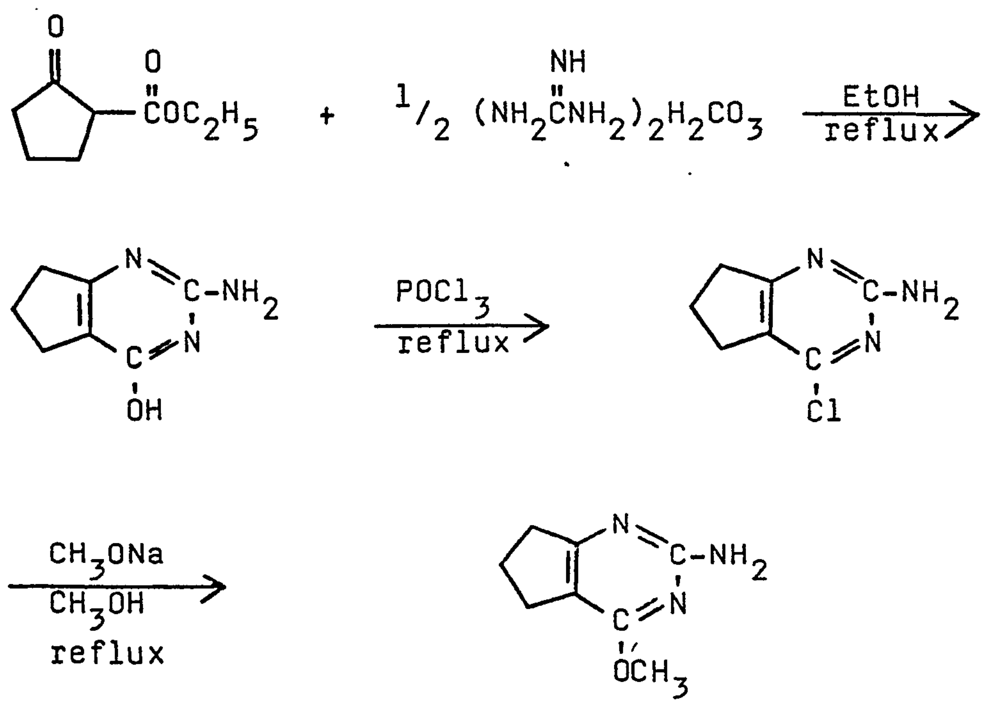


6,7-dihydro-4-methoxy-5H-cyclopentapyrimidin-2-amine.

An analogous sequence of reactions can be used to prepare 5,6,7,8-tetrahydro-4-methoxy-2-quinazolinamine.

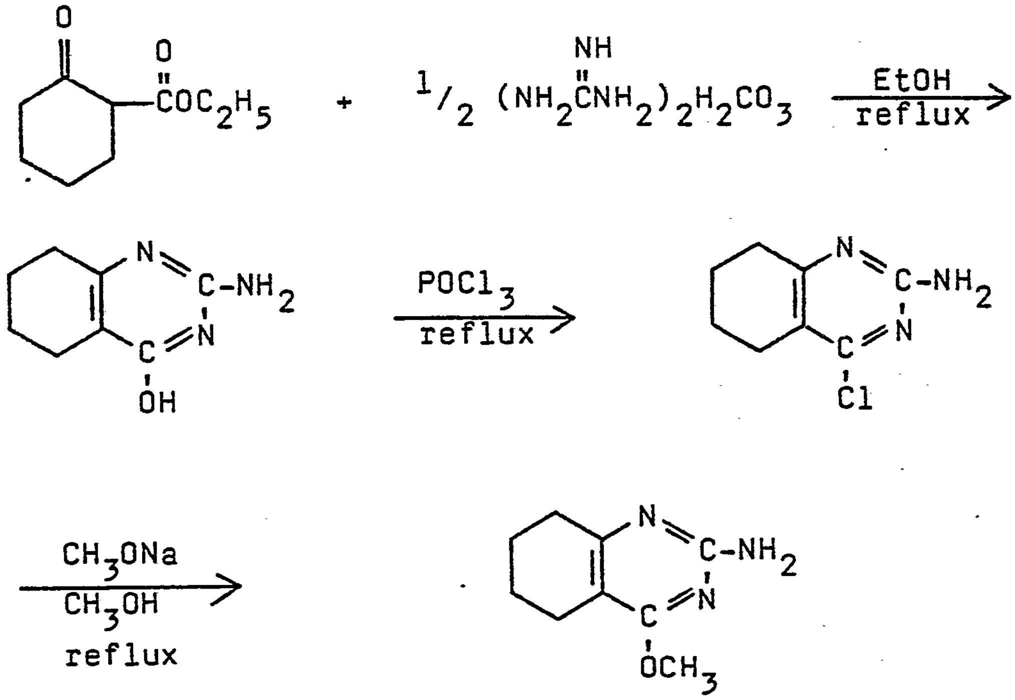


5,6,7,8-tetrahydro-4-methoxy-2-quinazolinamine.

Mitter and Bhattacharya, <u>Quart. J. Indian Chem. Soc.</u>, <u>4</u>, 152 (1927) describe the preparation of 5,6,7,8-tetrahydro-4-methyl-2-quinazolinamine as follows:

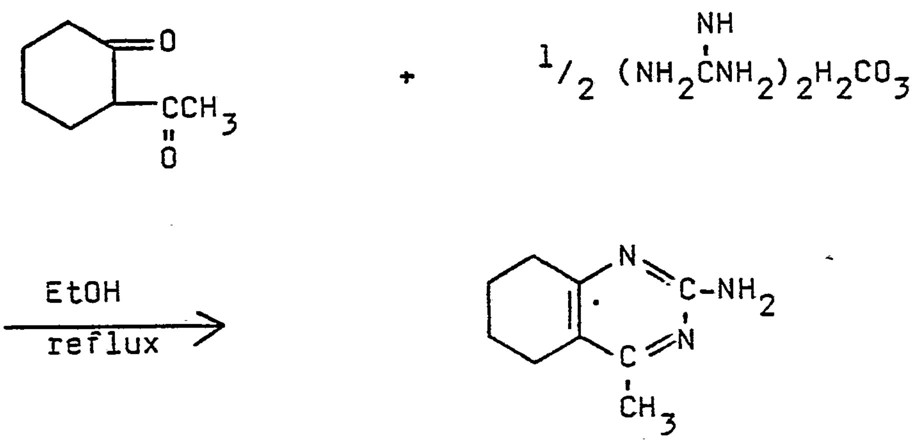


5,6,7,8-tetrahydro-4-methyl-2-quinazolinamine.

Similarly, 6,7-dihydro-4-methyl-5H-cyclopentapyrimidin-2-amine can be prepared by the condensation of 2-acetylcyclopentanone with guanidine carbonate, but preferably under acidic conditions, removing the water formed.

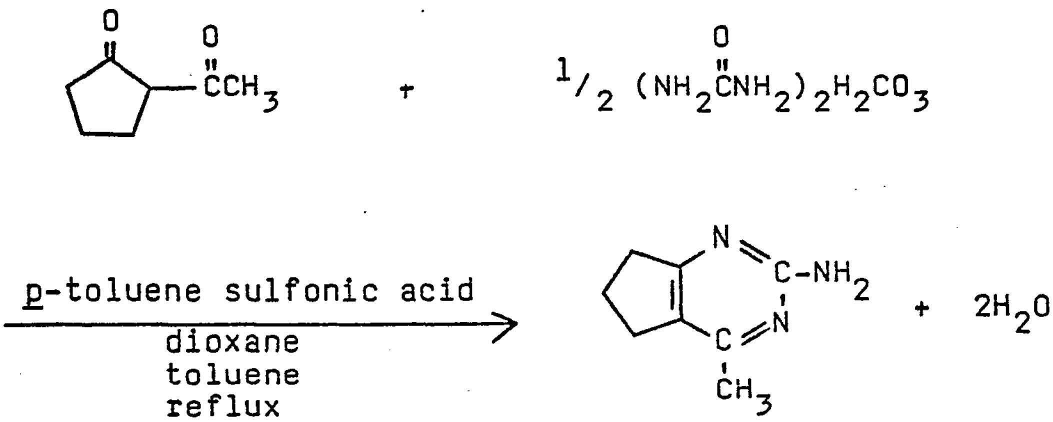


6,7-dihydro-4-methyl-5H-cyclopentapyrimidin-2-amine.

Shrage and Hitchings, J. Org. Chem., 16, 1153 (1951) describe the preparation of 5,6-dihydro-4-methylfuro[2,3-d]pyrimidin-2-amine by the following sequence of reactions

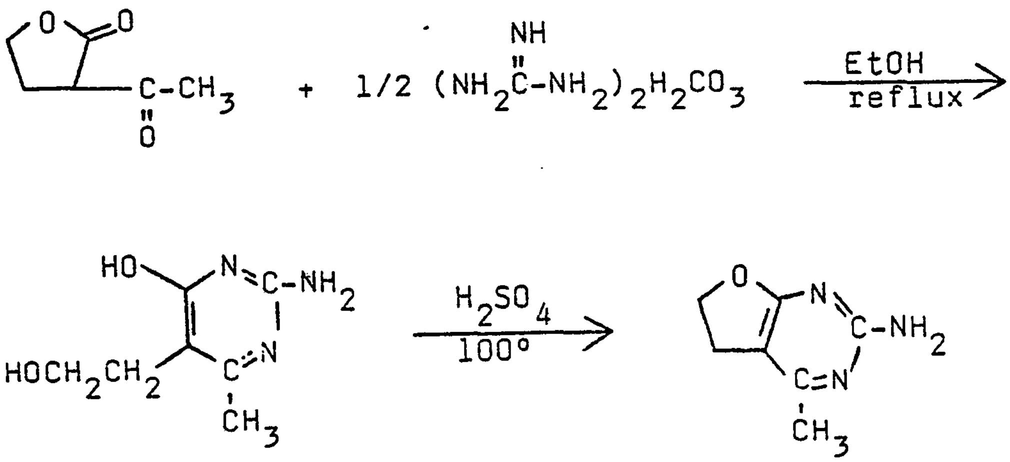


An analogous sequence of reactions can be used to prepare 6,7-dihydro-4-methyl-5H-pyrano[2,3-d]pyrimidin-2-amine starting with 2-acetyl-8 valerolactone [Korte and Wusten, Tetrahedron 19, 1423 (1963)].

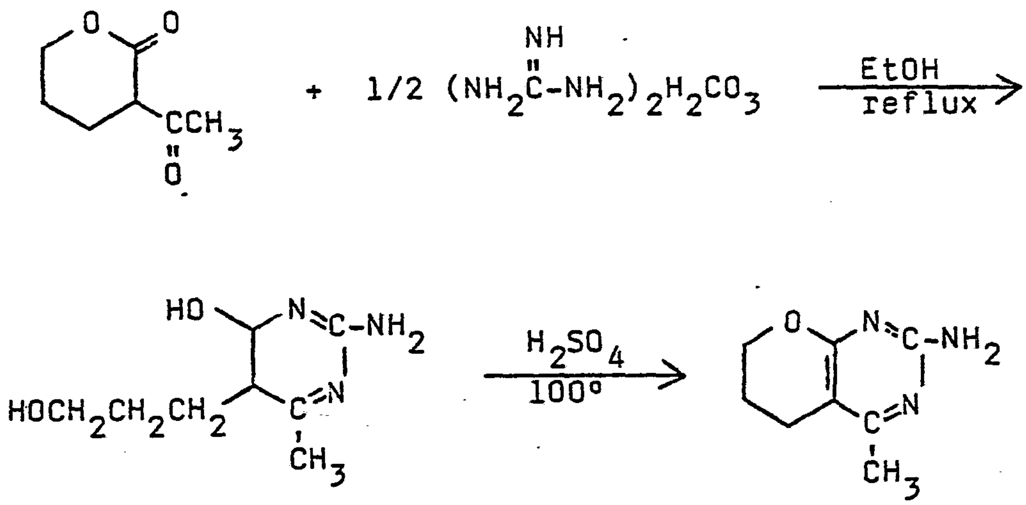


5,6-Dihydro-4-methoxyfuro[2,3-d]pyrimidin-2-amine can be prepared by the method of Braker et al., J. Am. Chem. Soc., 69, 3072 (1947), using 5,6-dihydro-4-hydroxyfuro[2,3-d]pyrimidin-2-amine [Svab, Budesinski and Vavrina, Collection Czech. Chem. Commun., 32, 1582 (1967)].

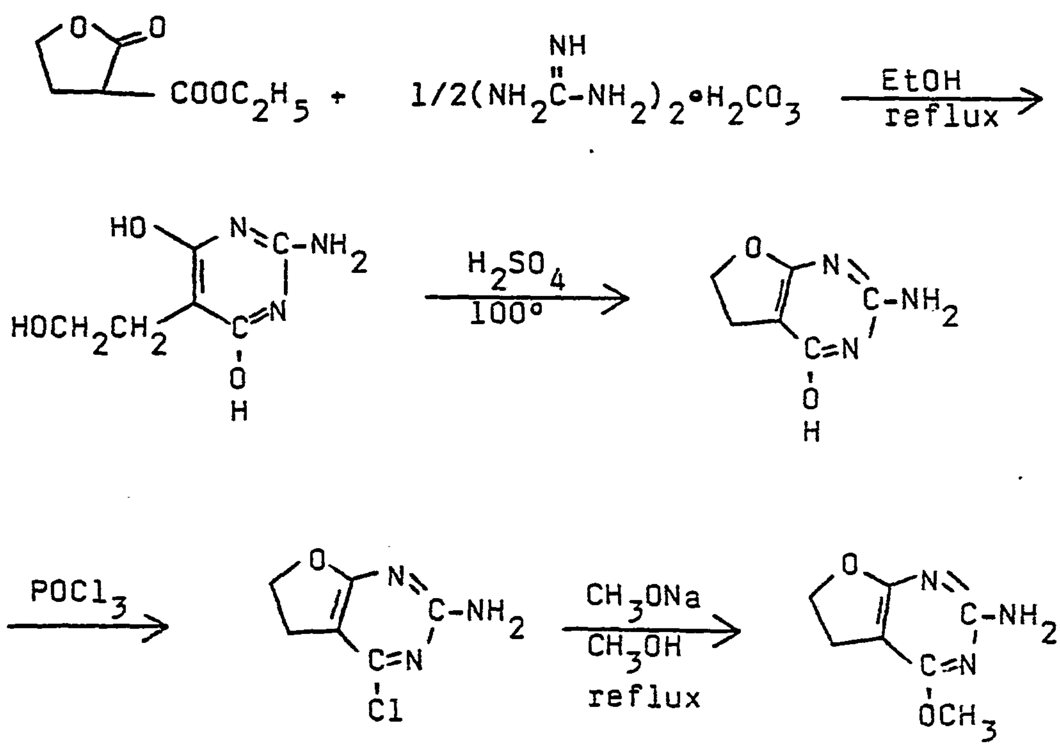

An analogous sequence of reactons can be used to prepare 6,7-dihydro-4-methoxy-5H-pyrano[2,3-d]pyrimidin-2-amine.

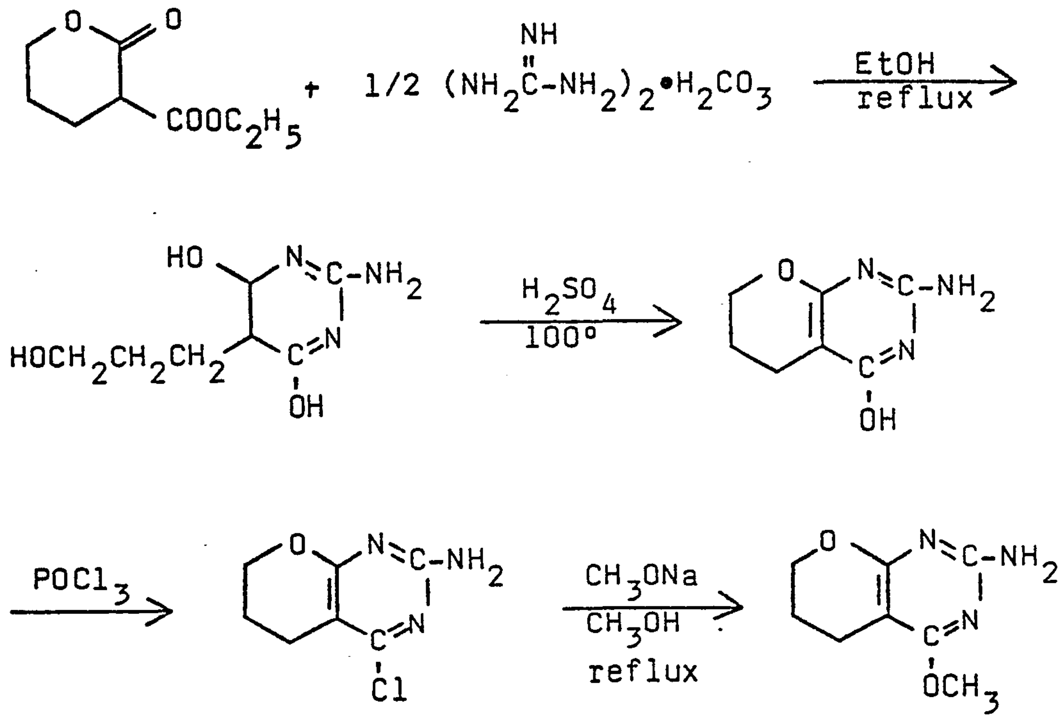


Caldwell, Kornfeld and Donnell, J. Am. Chem. Soc., 63, 2188 (1941), describe the preparation of 6,7-dihydro-5H-cyclopentapyrimidin-2-amine by the following sequence of reactions.

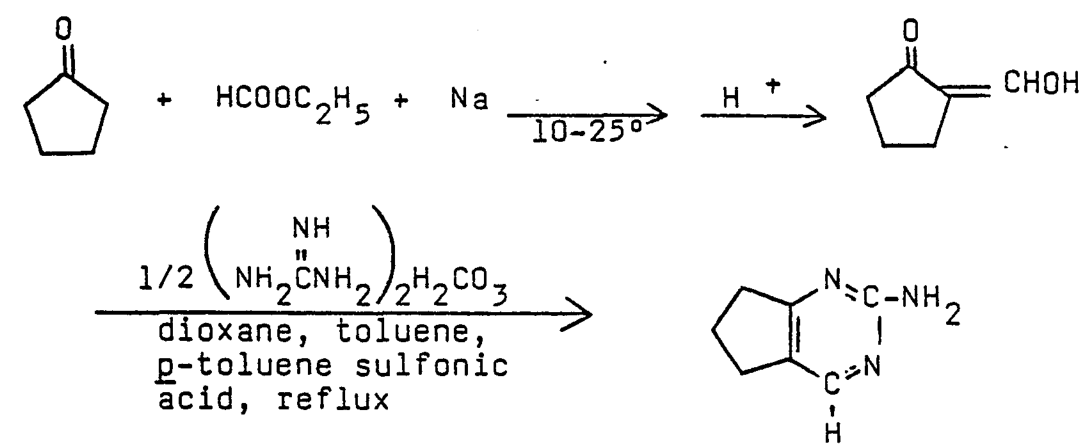

Fissekis, Myles and Brown, J. Org. Chem., 29, 2670 (1964), describe the preparation of 2-amino-4-hydroxy-5-(2-hydroxyethyl)pyrimidine which can be converted to 5,6-dihydrofuro[2,3-d]pyrimidin-2-amine by dehydration.

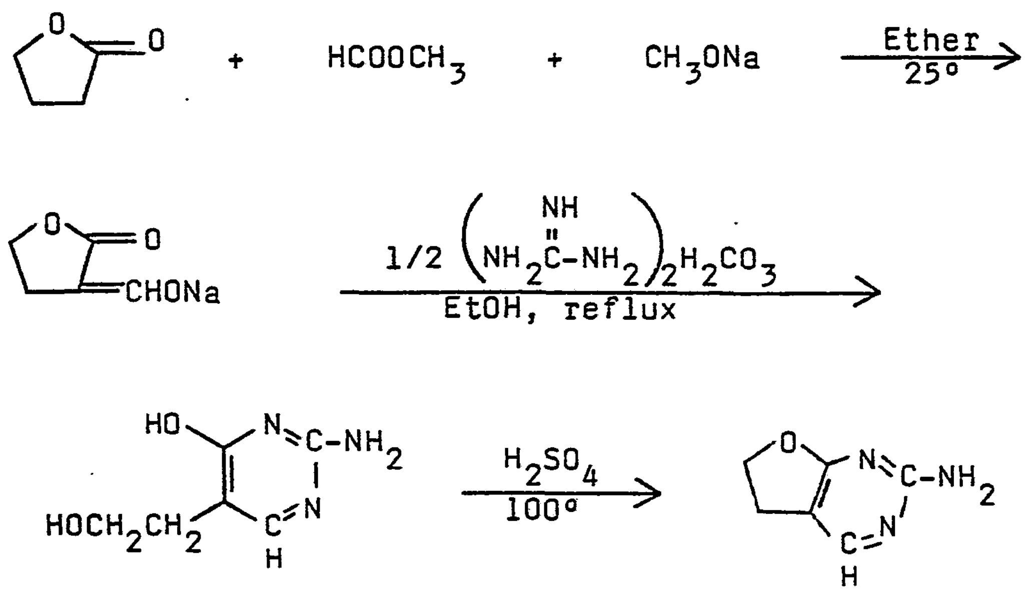


Preparations of 3-amino-1,2,4-triazoles are known in the art and 1,2,4-triazoles are reviewed in The Chemistry of Heterocyclic Compounds "Triazoles 1,2,4" (John Wiley and Sons, New York, 1981). Commonly used starting materials containing nitrogen are N-aminoguanidine, hydrazine, alkylhydrazines, cyanamide, ethyl cyanoacetimidate, dimethyl cyanodithioimidocarbonate, dimethyl cyanoimidocarbonate, ethoxymethylenecyanamide, and acylhydrazines. Some literature syntheses are illustrated below. Using these techniques or suitable modifications that would be apparent to one skilled in the art, the 3-amino-1,2,4-triazole intermediates can be readily prepared.

Heating equimolar amounts of ethyl propionimidate hydrochloride and N-aminoguanidine nitrate in pyridine gives 3-amino-5-ethyltriazole; German Patent 1,073,499 (1960); Berichte, 96, 1064 (1963).

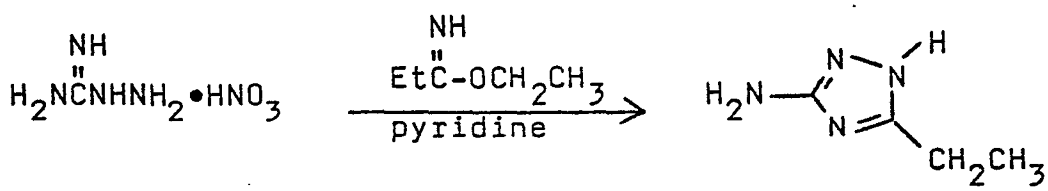


Condensation of hydrazine with ethyl N-cyanoacetimidate yields 3-amino-5-methyltriazole; Journal of Organic Chemistry, 28, 1816 (1963).

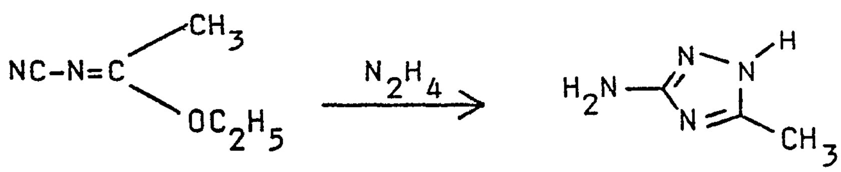


Trifluoromethyl-3-aminotriazole can be obtained by thermal dehydration of the hydrazide of trifluoroacetic acid. Zh. Obshch. Khim., 39, 2525 (1969); Chemical Abstracts, 72: 78954v (1970).

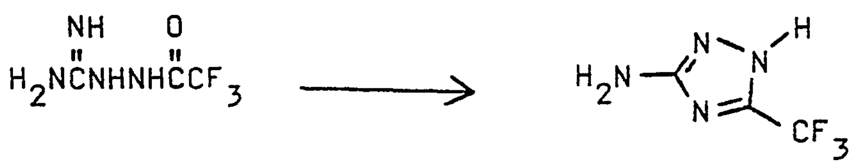

U.S. Patent 2,835,581 (1958) teaches the preparation of 3-amino-5-(hydroxymethyl)triazole from N-aminoguanidine and glycolic acid and British Patent 736,568 (1955) describes the synthesis of 3-amino-5-mercaptotriazole.

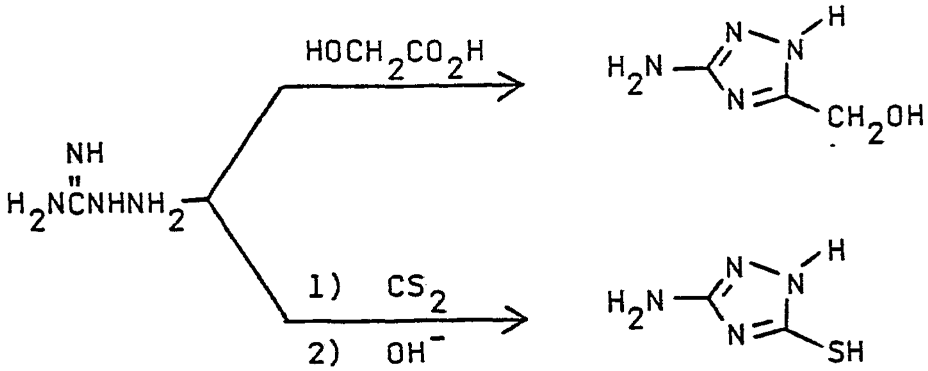


Condensing hydrazine with dimethyl cyanodithioimidocarbonate in acetonitrile gives 3-amino-5-methylthio-1,2,4-triazole while reaction of hydrazine with dimethyl N-cyanoimidocarbonate produces 3-amino-5-methoxy-1,2,4-triazole; Journal of Organic Chemistry, 39, 1522 (1974).

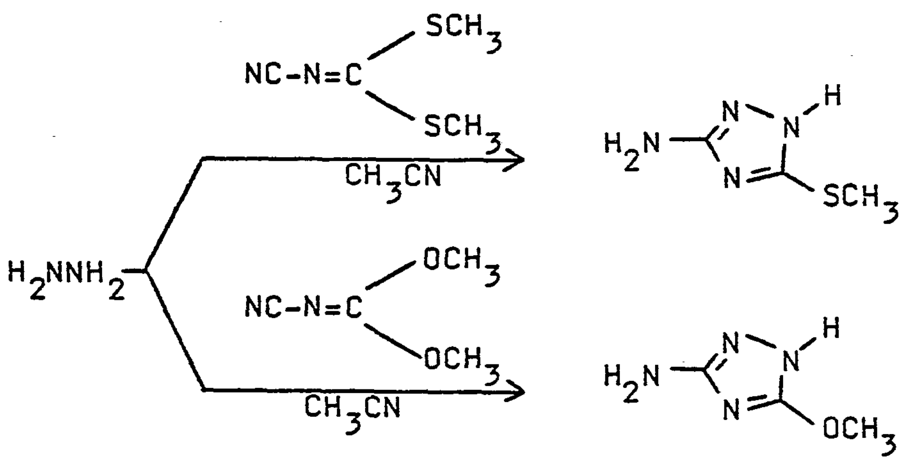


Reaction of substituted hydrazines with N-cyanothioimidocarbonates (prepared according to the procedure given in D. M. Wieland, Ph.D. Thesis, 1971, pp. 123-124) yields disubstituted aminotriazoles as shown below.

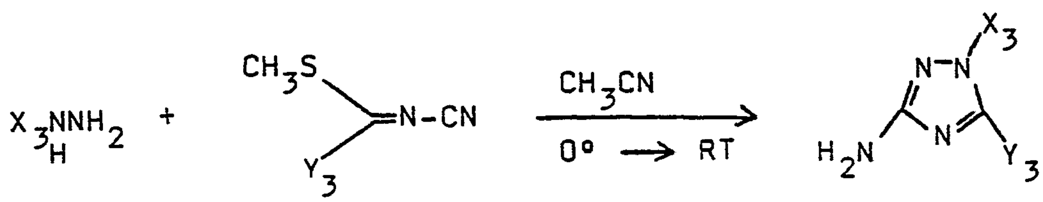


wherein

$X_3$ is $C_1$-$C_3$ alkyl; and

$Y_3$ is $C_1$-$C_2$ alkoxy.

The pyrimidine intermediates XIX shown below in which $X_1$ is methyl have been reported in the literature by E. Bisagni et al., [Bul. Soc. Chim. Fr., 803 (1969)]. An apparently more efficient procedure is depicted in the following sequence of reactions.

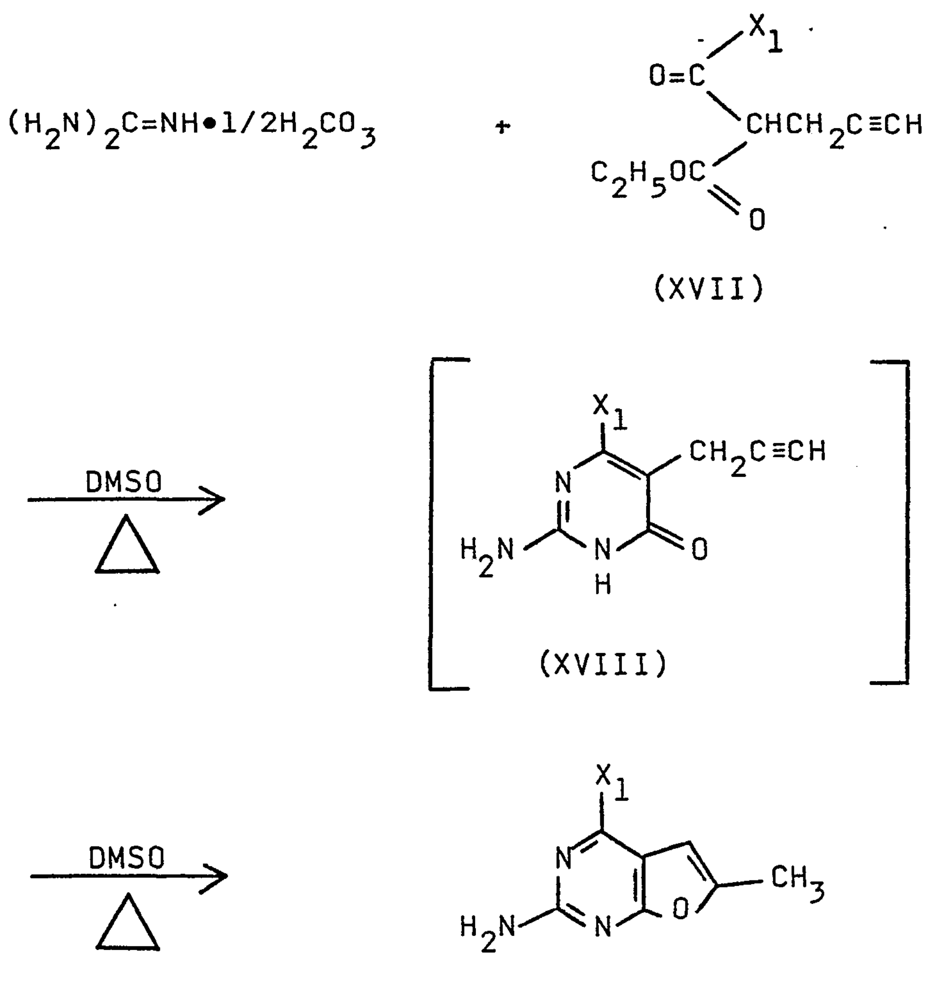


(XVII)

(XVIII)

(XIX)

The keto-ester precursors XVII are prepared by well known literature methods, e.g., J. F. Tinker and T. E. Whatmough, J. Amer. Chem. Soc., 74, 5235 (1952).

Treatment of XVII with an excess of guanidine carbonate in an organic solvent, preferably a polar aprotic solvent such as dimethylsulfoxide (DMSO), dimethylformamide (DMF), or N,N-dimethylacetamide, at a temperature of 80° to 200°, preferably 100° to 160°, ambient pressure and preferably under an inert atmosphere, yields both XIX and XVIII as products. The products are isolated upon dilution of the reaction mixture with, for example, acetone and water successively. Higher reaction temperatures and longer reaction times (e.g., in DMSO at 130°-150° for 2 to 8 hours) favor the production of the furopyrimidine XIX over the uncyclized pyrimidine XVIII.

Agriculturally suitable salts of compounds of Formulae I and II are also useful herbicides and can be prepared in a number of ways known to the art. For example, metal salts can be made by contacting compounds of Formulae I and II with a solution of an alkali or alkaline earth metal salt having a sufficiently basic anion (e.g. hydroxide, alkoxide, carbonate or hydroxide). Quaternary amine salts can be made by similar techniques.

Salts of compounds of Formulae I, Ia, II, IIa or III can also be prepared by exchange of one cation to another. Cationic exchange can be effected by direct treatment of an aqueous solution of a salt of a compound of Formulae I, Ia, II, IIa or III (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formulae I, Ia, II, IIa or III (e.g., an alkali metal or

quaternary amine salt) through a column packed with a cation exchange resin containing the cation to be exchanged. In this method, the cation of the resin is exchanged for that of the original salt and the desired product is eluted from the column. This method is particularly useful when the desired salt is watersoluble, e.g., a potassium, sodium or calcium salt.

Acid addition salts, useful in this invention, can be obtained by reacting a compound of Formulae I, Ia, II, IIa or III with a suitable acid, e.g., p-toluenesulfonic acid, trichloroacetic acid or the like.

The compounds of this invention and their preparation are further illustrated by the following examples wherein temperatures are given in degrees centigrade and all parts are by weight unless otherwise indicated.

Example 1

2,3-Dihydro-5-nitro-1H-indene-4-sulfonyl isocyanate

To 6.05 g of 2,3-dihydro-5-nitro-1H-indene-4-sulfonamide was added with stirring 50 ml of mixed xylenes with 2.47 g of butyl isocyanate and a catalytic amount of 1,4-diazabicyclo[2.2.2]octane (DABCO) at reflux. Phosgene was passed into the mixture slowly under a dry ice cooled reflux condenser until the temperature of the mixture fell to 124°C. The phosgene flow was discontinued until the reflux temperature rose to 134° whereupon the phosgene addition was resumed. Addition of phosgene was continued until the temperature once again fell to 124°. This cycling of phosgene addition was continued until the reflux temperature remained at 120-124° without further phosgene addition. Evaporation of the butyl isocyanate and xylene under reduced pressure yielded a residue. Infrared analysis of this residue showed a strong absorption peak at 2240 $cm^{-1}$ consistent for sulfonyl isocyanate and the disappearance of the urea carbonyl peak at 1700 $cm^{-1}$ and no NH peaks at 3360 and 3240 $cm^{-1}$. This residue was dissolved in 25 ml of dry methylene chloride and used in the following examples, as stated, without further purification.

Example 2

2,3-Dihydro-N-[(4,6-dimethylpyrimidin-2-yl)aminocarbonyl]-5-nitro-1H-indene-4-sulfonamide

To 0.615 g of 2-amino-4,6-dimethylpyrimidine in 5 ml of anhydrous methylene chloride, was added with stirring, one fifth of the 2,3-dihydro-5-nitro-1H-indenesulfonyl isocyanate, prepared in the preceding example, which was dissolved in anhydrous methylene chloride. After stirring at reflux, overnight, the solvent was removed by evaporation and the product triturated with chlorobutane and filtered. The solid thus obtained melted at 172° with decomposition and showed infrared absorption peaks at 3230-3160 $cm^{-1}$ (NH); 1700 $cm^{-1}$ (CO); 1540 $cm^{-1}$ ($NO_2$) and 1340-1370, 1160 ($SO_2$) consistent for the desired product. Proton Magnetic Resonance absorption peaks at 7.6-8.0 $\delta$ (aromatic CH), 6.9 $\delta$ (pyrimidine, 5-CH), 3.6-2.8 $\delta$ (cyclic $CH_2$), 2.4 $\delta$ ($CH_3$) confirmed the presence of the desired structure.

Example 3

2,3-Dihydro-N-[(4-methoxy-6-methylpyrimidin-2-yl)-aminocarbonyl]-5-nitro-1H-indene-4-sulfonamide

To 0.69 g of 2-amino-4-methoxy-6-methylpyrimidine suspended in 5 ml of anhydrous methylene chloride was added with stirring one fifth of the solution of 2,3-dihydro-5-nitro-1H-indenesulfonyl isocyanate prepared in Example 1. After stirring at reflux for 16 hours, the methylene chloride was removed *in vacuo* on a rotary evaporator and the residue was triturated

with 1-chlorobutane and methylene chloride to yield a solid which decomposed at 75-93°. This product showed absorption peaks by infrared spectroscopy at 3400 $cm^{-1}$ (NH), 1670 $cm^{-1}$ (CO), 1540 $cm^{-1}$ ($NO_2$) and 1360 $cm^{-1}$ ($SO_2$). The structure was confirmed by Proton Magnetic Resonance absorption peaks at 7.6 ppm (aromatic CH), 6.4 ppm (pyrimidine CH), 2.8-3.6 ppm (cyclic $CH_2$) and 3.9 ppm ($CH_3O$).

Example 4

2,3-Dihydro-N-[(4,6-dimethoxypyrimidin-2-yl)aminocarbonyl]-5-nitro-1H-indene-4-sulfonamide

To 0.78 g of 2-amino-4,6-dimethoxypyrimidine in 5 ml of anhydrous methylene chloride was added, with stirring, one fifth of the 2,3-dihydro-5-nitro-1H-indenesulfonyl isocyanate prepared in Example 1. The mixture was protected from moisture under nitrogen and heated to reflux for sixteen hours after which the solvent was removed in-vacuo. Trituration of the residual solid with 1-chlorobutane yielded a solid melting at 162-172°. Infrared spectroscopy analysis of this product showed absorption peaks at 3360 $cm^{-1}$ (NH), 1740 $cm^{-1}$ (C=O), 1540 $cm^{-1}$ ($NO_2$), 1360 and 1160 $cm^{-1}$ ($SO_2$) consistent for the desired structure and also absorption peaks by proton magnetic resonance at 7.5-7.7 ppm (aromatic CH), 6.0 ppm (pyrimidine CH), 3.9 ppm ($OCH_3$), 3.6-2.8 ppm (cyclic $CH_2$) confirmed the presence of the desired structure.

Example 5

N-Butylaminocarbonyl-5,6,7,8-tetrahydronaphthalene-1-sulfonamide

To 10.9 g of 5,6,7,8-tetrahydronaphthalene-1-sulfonamide dissolved in 25 ml of anhydrous acetonitrile was added 6.13 g of n-butyl isocyanate and the mixture was heated to reflux with stirring for 16 hours. The mixture was then filtered and concentrated

*in vacuo* to an oil which solidified to yield 15.5 g of the desired product melting at 173-175°. The structure was confirmed by infrared absorption peaks at 1680 $cm^{-1}$ (CO) and 3350 $cm^{-1}$ (NH) and proton magnetic absorption peaks at 7.2-8.0 ppm (aromatic CH) and 1-3.2 ppm ($CH_2$).

Example 6

5,6,7,8-Tetrahydronaphthalene-1-sulfonyl isocyanate

Into a solution of 15.5 g of N-butylaminocarbonyl-5,6,7,8-tetrahydronaphthalene-1-sulfonamide, and a catalytic amount of 1,4-diazabicyclo[2.2.2]octane dissolved in 50 ml of mixed xylenes at reflux (133°) was added enough phosgene to cause the reflux temperature to drop to 120-124°C. The addition of phosgene was discontinued until the temperature rose to 130°. Additional phosgene was added until the reflux temperature was depressed to 120-124° whereupon the phosgene addition was again halted. The cycling of the phosgene addition was continued until the reflux temperature remained at 120-122° without further phosgene addition. The formation of the desired product was monitored by periodically removing a small sample of the solution and checking for the appearance of an absorption peak by infrared at 2230 $cm^{-1}$, consistent for $SO_2NCO$ and the disappearance of the NH peak at 3350 $cm^{-1}$. The reaction mixture was concentrated *in vacuo* to remove the xylene solvent and butyl isocyanate by-product and the desired product was isolated as a residue. This residue was dissolved in anhydrous methylene chloride to a total volume of 60 ml and used without further purification.

## Example 7

N-[(4,6-Dimethylpyrimidin-2-yl)aminocarbonyl]-5,6,7,8-tetrahydronaphthalene-1-sulfonamide

To 1.02 g of 2-amino-4,6-dimethylpyrimidine dissolved in 6 ml of anhydrous methylene chloride was added 10 ml of the methylene chloride solution of 5,6,7,8-tetrahydronaphthalene-1-sulfonyl isocyanate prepared in Example 6. The mixture was heated to reflux for 16 hours. After evaporation of the solvent *in vacuo*, the product was isolated by trituration with 1-chlorobutane and filtration. It melted at 152-155° and showed absorption peaks in the infrared region at 1690 $cm^{-1}$ (CO) and by proton magnetic resonance at 7.2-7.6 ppm (aromatic CH), 6.3 (pyrimidine CH), 2.2 ppm ($CH_3$) consistent for the desired structure.

## Example 8

N-[(4,6-Dimethoxy-1,3,5-triazin-2-yl)aminocarbonyl]-5,6,7,8-tetrahydronaphthalene-1-sulfonamide

To a stirred suspension, 1.29 g of 2-amino-4,6-dimethoxy-1,3,5-triazine in 6 ml of methylene chloride was added 10 ml of the methylene chloride solution of 5,6,7,8-tetrahydronaphthalene-1-sulfonyl isocyanate prepared in Example 6. After the mixture was refluxed for sixteen hours, the solvent was removed by evaporation *in vacuo* and the residue triturated with 1-chlorobutane. The mixture was filtered and the filtrate partially evaporated to yield additional solid which melted at 171-173°. This product showed absorption peaks in the infrared region at 1710 $cm^{-1}$ (CO) and 3300 $cm^{-1}$ (NH) and by proton magnetic resonance at 7.2-7.6 ppm (aromatic CH), 4.1 ppm ($OCH_3$) and 1.0-3.2 ppm ($CH_2$) consistent for the desired structure.

Example 9

3-Chloro-1H-indene-4-sulfonyl chloride

To 8.64 g of 2,3-dihydro-1H-indene-4-sulfonyl chloride in 100 ml of carbon tetrachloride was added, with stirring, 75 ml of a 1.06 molar solution of chlorine oxide in carbon tetrachloride dropwise at such a rate that the temperature of the reaction mixture was allowed to rise to 40°. After the addition was completed, the mixture was stirred for 16 hours, then sparged with nitrogen for 10 minutes and the solvent evaporated *in vacuo*. The residue thus obtained was used in the following example without further purification.

Example 10

3-Chloro-1H-indene-4-sulfonamide

1-Chloro-3H-indene-4-sulfonyl chloride (7.0 g) in 50 ml of anhydrous tetrahydrofuran was cooled to -40° and 1.25 ml of liquified ammonia was added so that the reaction mixture temperature remained below -35°. After stirring for one half hour following the addition, the mixture was filtered, the filtered solids washed with tetrahydrofuran and the filtrate concentrated to yield an oil. The oil was purified by column chromatography on silica gel (200-400 mesh) and eluting with 150 ml hexane followed by 300 ml of 30% ethyl acetate in hexane. On standing, this oil solidified. It showed absorption peaks at 3250 and 3350 $cm^{-1}$ for $NH_2$ and 1380 and 1160 consistent for $SO_2$ and melted at 100-101°.

Example 11

3-Chloro-1H-indene-4-sulfonyl isocyanate

An anhydrous mixture of 6.0 g of 1-chloro-3H-indene-4-sulfonamide, 2.6 g of butyl isocyanate and 50 ml of mixed xylenes was heated to reflux at 135°. After 30 minutes, phosgene was passed into the solution under a dry ice cooled reflux condenser until the reflux temperature dropped to 120° whereupon the phosgene addition was discontinued. Heating was continued until the temperature rose to 130° and phosgene addition was resumed. This cycling addition of phosgene was continued until the reflux temperature remained at 120° for at least 30 minutes indicating the presence of an excess of phosgene in the system and completion of the reaction. The reaction mixture was cooled, filtered and evaporated in vacuo to remove xylene and butyl isocyanate. Infrared absorption analysis of the residue showed a peak at 2220 $cm^{-1}$, consistent for the desired structure. The product was diluted to 25 ml in methylene chloride.

## Example 12

3-Chloro-N-[(4-methoxy-6-methylpyrimidin-2-yl)aminocarbonyl]-1H-indene-4-sulfonamide

To 0.69 g of 2-amino-4-methoxy-6-methylpyrimidine in 5 ml of dry methylene chloride was added with stirring 5 ml of 1-chloro-3H-indene-4-sulfonyl isocyanate in methylene chloride from the preceding example. The mixture was heated to reflux for 16 hours and the solvent was evaporated in vacuo. Elutriation of the residue with 1-chlorobutane yielded the solid product which was removed by filtration; it melted at 168-172°. Infrared absorption peaks at 1700 $cm^{-1}$ (C=O), 1350 $cm^{-1}$ and 1160 $cm^{-1}$ ($SO_2$) and proton magnetic resonance absorption peaks at 2.1 ppm ($CH_3$), 3.6 ppm ($CH_2$), 3.9 ppm ($OCH_3$), 6.2 ppm (pyrimidine CH) and 7.2-8.5 (aromatic CH) confirmed the structure of the product.

Example 13

3-Chloro-N-[(4-methoxy-6-methyl-1,3,5-triazin-2-yl)-aminocarbonyl]-1H-indene-4-sulfonamide

To 0.70 g of 2-amino-4-methoxy-6-methyl-1,3,5-triazine in 5 ml of anhydrous methylene chloride with stirring was added 5 ml of methylene chloride solution of 1-chloro-3H-indene-4-sulfonyl isocyanate prepared above. The mixture was stirred and heated to reflux for 16 hours after which the solvent was removed by evaporation *in vacuo*. Trituration of the product with 1-chlorobutane dissolved the desired product, but did not dissolve unreacted amino.triazine, which was removed by filtration. Addition of hexane to the filtrate caused the desired product to precipitate as a solid which was filtered off, m.p. 168-170°. It showed absorption peaks in the infrared region at 1710 $cm^{-1}$ (C=O), 1360 and 1160 $cm^{-1}$ ($SO_2$), 3400 $cm^{-1}$ (NH) and by proton magnetic resonance at 2.5 ppm ($CH_3$), 3.5 ppm ($CH_2$), 3.9 ppm ($OCH_3$), and 7.4-8.5 ppm (aromatic CH) consistent for the desired structure.

Using the procedures of the previous Examples, 1-13, the compounds of Tables I-III can be prepared.

Example 14

2,2-Dimethyl-1,3-benzodioxole-4-sulfonamide

To a solution of 25 g 2,2-dimethyl-1,3-benzodioxole [prepared according to the procedure of C. M. Yoder and J. J. Zuckerman, J. Heterocycl. Chem., 1967, 4, 166] in 300 ml ether and 200 ml tetrahydrofuran at ambient temperature under nitrogen was added dropwise, 105 ml n-butyllithium (1.6N in hexane). The temperature was maintained at ≈25°C with ice bath cooling during the addition. The reaction mixture was stirred 16 hours at room temperature. Excess sulfur dioxide was bubbled through the reaction mixture, kept at ≈25°C with cooling, until no further exotherm was noted. The reaction mixture was stirred at room temperature for 18 hours followed by the addition of 10 ml isopropyl alcohol. The reaction mixture was concentrated and crystallized from ether/tetrahydrofuran to give 20.5 g tan solid. The solid was added to 150 ml acetic acid and the resultant mixture was cooled to 20°C. A slurry of 12.4 g N-chlorosuccinimide in 75 ml acetic acid was added portionwise with cooling to maintain the reaction mixture between 20° and 25°C. The reaction mixture was stirred for three hours at ambient temperature and the solvent was removed under reduced pressure to give an oil. The oil was dissolved in methylene chloride and washed with water. The aqueous phase was washed with methylene chloride. The organic phases were combined, dried and the solvent removed under reduced pressure to give an oil which was dissolved in 250 ml ether and cooled to 10°C. Excess ammonia (anhy.) was bubbled through the reaction mixture using ice bath cooling. The reaction mixture was warmed to ambient temperature, stirred for 1 hour, and 150 ml ether was added. The reaction mixture was washed with water, dried and concentrated

under reduced pressure to give 8.1 g tan oily solid, m.p. 100-112°C. IR (NH) 3240 (NH), 3420 (NH) $cm^{-1}$.

$^{1}$HNMR (DMSO-$d_6$): 1.7 (s, 6H); and
6.5-7.4 (m, 5H).

## Example 15

1,3-Benzodioxole-4-sulfonamide

To a solution of 18.8 g 3-bromocatechol (prepared according to the procedure of H. S. Mason, J. Am. Chem. Soc., 1947, 69, 2241) and 10 g methylene chloride in 150 ml dimethyl sulfoxide at ambient temperature under nitrogen is added 8.3 g of sodium hydroxide (powdered). The reaction mixture is heated at 120°C for 2 hours. Steam distillation gives 13.5 g of the solid, 4-bromo-1,3-benzodioxole. The solid is dissolved in 200 ml of tetrahydrofuran under a nitrogen atmosphere, cooled to -78°C and treated sequentially with 42.2 ml n-butyllithium (1.6N in hexane) for 16 hours and excess sulfur dioxide until no further exotherm is noted. The reaction mixture is contacted with 10 ml isopropyl alcohol, concentrated *in vacuo* and crystallized from ether/tetrahydrofuran to give a solid. The solid is added to 150 ml of acetic acid and cooled to 20°C. A slurry of 12 g N-chlorosuccinimide in 75 ml acetic acid is added portionwise with cooling to maintain a reaction temperature of 20-25°C. The reaction mixture is stirred for three hours at room temperature and the solvent is removed under reduced pressure to give an oil which is dissolved in methylene chloride and washed with water. The organic phase is dried, and the solvent removed under reduced pressure to give an oil which is dissolved in 250 ml ether and cooled to 10°C. Excess ammonia (anhy.) is bubbled through the reaction mixture with external cooling. The reaction mixture is washed with water, dried, and concentrated under reduced pressure to give a solid.

## Example 16

Methyl (4,6-dimethoxypyrimidin-2-yl)carbamate

2-Amino-4,6-dimethoxypyrimidine (56 g) was added portionwise to 50% sodium hydride (42.8 g) in 1000 ml of dry tetrahydrofuran. After stirring for 0.5 hour, dimethylcarbonate (58.5 g) was added dropwise with cooling. The mixture was stirred under nitrogen for about 16 hours at ambient temperature. Concentrated HCl (80 ml) was added slowly as external cooling was used to maintain a pot temperature of about 25°C. Saturated aqueous sodium chloride (80 ml) was then added. The solvents were decanted from the precipitated solids and dried over sodium sulfate. Filtration and evaporation of the solvents afforded the crude material which was recrystallized from hexane to yield 54 g of the title compound, m.p. 81-83°C. The IR spectrum showed characteristic absorption bands at 3400 and 1760 $cm^{-1}$.

## Example 17

N-[(4-Methoxy-6-methylpyrimidin-2-yl)aminocarbonyl]-2,2-dimethyl-1,3-benzodioxole-4-sulfonamide

To a solution of 0.55 g 2,2-dimethyl-1,3-benzodioxole-4-sulfonamide, prepared in Example 14, in 30 ml methylene chloride at ambient temperature under nitrogen was added 1.2 ml of trimethylaluminum (2M in toluene). After stirring 15 minutes at ambient temperature, 0.47 g of methyl [4-methoxy-6-methylpyrimidin-2-yl]carbamate, prepared according to the procedure of Example 3, was added and the reaction mixture was heated at reflux 16 hours. The reaction mixture was cooled to ambient temperature and 75 ml $H_2O$, 10 ml glacial acetic acid, and 10 drops hydrochloric acid were sequentially added. The organic phase was separated, dried, and the solvent removed under reduced pressure to give an oil. Crystallization of the oil

from a mixture of butyl chloride, hexane and ether gave 0.25 g white solid, m.p. 180-183°C. The infrared spectrum showed a carbonyl absorption at 1705 $cm^{-1}$ indicative of the title compound.

$^1$HNMR ($CDCl_3$): δ 1.6 (s, 6H);
2.5 (s, 3H);
4.0 (s, 3H);
6.3 (s, 1H); and
6.9-7.6 (m, 3H).

Example 18

Methyl (4,6-dimethoxy-1,3,5-triazin-2-yl)carbamate

2-Amino-4,6-dimethoxy-1,3,5-triazine (55.3 g) was added portionwise to 50% sodium hydride (38.0 g) in 1000 ml dry tetrahydrofuran at ambient temperature under nitrogen. After the reaction mixture was stirred for 1.25 hours, dimethylcarbonate (50.0 g) was added dropwise. The reaction mixture was stirred 16 hours at ambient temperature under nitrogen and concentrated hydrochloric acid (68 ml) was slowly added followed by 100 ml tetrahydrofuran. Insoluble material was removed via filtration and discarded. The filtrate was dried and the solvent removed under reduced pressure to give a solid which upon crystallization from ethanol afforded 43.9 g of the title compound, m.p. 118-122°C.

Example 19

N-[(4-Methoxy-6-methyl-1,3,5-triazin-2-yl)aminocarbonyl]-2,2-dimethyl-1,3-benzodioxole-4-sulfonamide

To a solution of 1.0 g 2,2-dimethyl-1,3-benzodioxole-4-sulfonamide, prepared in Example 14, in 50 ml methylene chloride at room temperature under nitrogen was added 2.5 ml trimethylaluminum (2M in toluene). After stirring 15 minutes at room temperature, 0.7 g of methyl [4-methoxy-6-methyl-1,3,5-triazin-2-yl]carbamate, prepared according to the procedure of Example

18, was added and the reaction mixture was heated at reflux 72 hours. The reaction mixture was cooled to room temperature and 75 ml ice, 3 ml glacial acetic acid, and 10 drops of hydrochloric acid were sequentially added. The organic phase was separated, dried and the solvent removed under reduced pressure to give an oil. Crystallization of the oil from a mixture of ether, methylene chloride, and hexane gave 0.1 g white solid, m.p. 160-168°C. The infrared spectrum showed a carbonyl absorption at 1705 $cm^{-1}$ indicative of the title compound.

$^1$HNMR ($(CD_3)_2CO$):δ 1.6 (s, 6H);
2.5 (s, 3H);
4.0 (s, 3H); and
6.9-7.6 (m, 3H).

Example 20

2,2-Dimethyl-1,3-Benzodithiole-4-Sulfonamide

To a solution of 69 ml *s*-butyllithium and 15 ml N,N,N',N'-tetramethylethylenediamine in 200 ml THF stirring at -78°C under nitrogen was added a THF solution of 18.2 g 2,2-dimethyl-1,3-benzodithiole [prepared according to the procedure of Pelter et al., Tetrahedron Lett., 1977, 225]. The temperature was maintained at -78°C to -65°C with external dry ice bath cooling during the addition. The reaction mixture was stirred 1 hour at -78°C. Excess sulfur dioxide was bubbled through the reaction mixture, kept at -70°C to -60°C with cooling until no further exotherm was noted. The reaction mixture was warmed to ambient temperature, stirred for 18 hours and 10 ml isopropyl alcohol was added. The reaction mixture was concentrated and crystallized from ether/methylene chloride to give a solid. The solid is added to 150 ml of acetic acid and cooled to 20°C. A slurry of 12 g N-chlorosuccinimide in 75 ml acetic acid is added

portionwise with cooling to maintain a reaction temperature of 20-25°C. The reaction mixture is stirred for 2 hours at room temperature and the solvent is removed under reduced pressure to give an oil which is dissolved in methylene chloride and washed with water. The organic phase is dried and the solvent removed under reduced pressure to give an oil which is dissolved in 250 ml ether and cooled to 10°C. Excess ammonia (anhy.) is bubbled through the reaction mixture with external cooling. The reaction mixture is warmed to room temperature, washed with water, dried, and concentrated under reduced pressure to give a tan solid.

Example 21

1,3-Benzodithiole-4-sulfonamide

To a solution of 1.7 g dibromomethane and Aliquat 336® in 75 ml toluene was added dropwise a solution of 2.2 g 3-bromothiocatechol (prepared according to the procedures of L. Horner et al., Phosphorus and Sulfur, 1982, 2, 353 and A. Ferretti, Org. Synth., 1973, Coll. Vol. V, 419), 2.4 g of 50% sodium hydroxide, and 25 ml water. The reaction mixture was heated at reflux 1.5 hour, cooled to ambient temperature and stirred 16 hours. The organic phase was separated, washed with 1*N* sodium hydroxide and water, dried and concentrated under reduced pressure to give an oil. The oil was purified via distillation to give 1.4 g 4-bromo-1,3-benzodithiole. The oil was dissolved in 60 ml of tetrahydrofuran under a nitrogen atmosphere, cooled to -70°C and treated sequentially with 4.5 ml *n*-butyllithium (1.6 N in hexanes) for 0.5 hour at -70°C and excess sulfur dioxide until no further exotherm was noted. The reaction mixture was contacted with 3 ml isopropyl alcohol, concentrated *in vacuo* and crystallized from ether/tetrahydrofuran to give a

solid. The solid was added to 40 ml acetic acid and cooled to 20°C. A slurry of 0.8 g N-chlorosuccinimide in 20 ml acetic acid was added portionwise with cooling to maintain a reaction temperature of 20-25°C. The reaction mixture was stirred for three hours at room temperature and the solvent was removed under reduced pressure to give an oil which was dissolved in methylene chloride and washed with water. The organic phase was dried, and the solvent removed under reduced pressure to give an oil which was dissolved in 250 ml ether and cooled to 10°C. Excess ammonia (anhy.) was bubbled through the reaction mixture with external cooling. The reaction mixture was washed with water, dried, and concentrated under reduced pressure to give a solid.

## Example 22

### 2,2-Dimethyl-1,3-benzodithiole-4-sulfonamide, 1,1,3,3-tetraoxide

To a solution of 10.0 g 2,2-dimethyl-1,3-benzodithiole, prepared in Example 20, in 150 ml glacial acetic acid was added dropwise with external cooling 34.5 ml 30% hydrogen peroxide. The reaction mixture was stirred at room temperature one hour, heated at reflux 1 hour, cooled to ambient temperature and stirred 16 hours. Aqueous sodium bisulfite and ether were added to the reaction mixture and the resultant mixture washed with sodium bisulfite, solid sodium chloride, dried and concentrated under reduced pressure to give 7.0 g white solid, m.p. 146-148°C. The solid was dissolved in 40 ml THF at -70°C under nitrogen and 22 ml of butyllithium (1.6 N in hexanes) was added dropwise with external cooling to maintain a maximum temperature of -65°C. The reaction mixture was stirred 1 hour at -70°C. Excess sulfur dioxide was bubbled through the reaction mixture, kept at

-70°C with cooling until no further exotherm was noted. The reaction mixture was warmed to ambient temperature, stirred at -70°C for 18 hours and 10 ml isopropyl alcohol was added. The reaction mixture was concentrated and crystallized from ether/methylene chloride to give a solid. The solid was added to 150 ml of acetic acid and cooled to 20°C. A slurry of 3.7 g N-chlorosuccinimide in 75 ml acetic acid was added portionwise with cooling to maintain a reaction temperature of 20-25°C. The reaction mixture was stirred for 2 hours at room temperature and the solvent was removed under reduced pressure to give an oil which is dissolved in methylene chloride and washed with water. The organic phase was dried and the solvent removed under reduced pressure to give an oil which is dissolved in 250 ml ether and cooled to 10°C. Excess ammonia (anhy.) was bubbled through the reaction mixture with external cooling. The reaction mixture was warmed to room temperature, washed with water, dried, and concentrated under reduced pressure to give a solid, m.p. 215-220°C.

## Example 23

N-[(4-Methoxy-6-methylpyrimidin-2-yl)aminocarbonyl)]-2,2-dimethyl-1,3-benzodithiole-4-sulfonamide

To a solution of 2.61 g 2,2-dimethyl-1,3-benzodithiole-4-sulfonamide, prepared in Example 20, in 45 ml methylene chloride at ambient temperature under nitrogen was added 5 ml of trimethylaluminum (2M in toluene). After stirring 15 minutes at ambient temperature, 2.0 g of methyl (4-methoxy-6-methylpyrimidin-2-yl)carbamate, prepared according to the procedure of Example 16, was added and the reaction mixture was heated at reflux 16 hours. The reaction mixture was cooled to ambient temperature and 75 ml $H_2O$, 10 ml glacial acetic acid, and 10 drops hydrochloric acid

were sequentially added. The organic phase was separated, dried, and the solvent removed under reduced pressure to give an oil. Crystallization of the oil from an ether solvent gave a solid.

Example 24

N-[(4-Methoxy-6-methyl-1,3,5-triazin-2-yl)aminocarbonyl]-2,2-dimethyl-1,3-benzodithiole-4-sulfonamide

To a solution of 2.6 g 2,2-dimethyl-1,3-benzodithiole-4-sulfonamide prepared in Example 20, in 60 ml methylene chloride at room temperature under nitrogen is added 5.5 ml trimethylaluminum (2M in toluene). After stirring 15 minutes at room temperature, 2.0 g of methyl [4-methoxy-6-methyl-1,3,5-triazin-2-yl]carbamate, prepared according to the procedure of Example 18, was added and the reaction mixture was heated at reflux for 72 hours. The reaction mixture was cooled to room temperature and 75 ml ice, 3 ml glacial acetic acid, and 10 drops of hydrochloric acid were sequentially added. The organic phase was separated, dried and the solvent removed under reduced pressure to give an oil. Crystallization of the oil from an appropriate solvent gave a solid.

Example 25

N-[(4-Methoxy-6-methylpyrimidin-2-yl)aminocarbonyl]-2,2-dimethyl-1,3-benzodithiole-4-sulfonamide 1,1,3,3-tetraoxide

To a solution of 3.25 g 2,2-dimethyl-1,3-benzodithiole-4-sulfonamide, 1,1,3,3-tetraoxide, prepared in Example 14, in 50 ml methylene chloride at ambient temperature under nitrogen was added 5.5 ml trimethylaluminum (2M in toluene). After stirring 15 minutes at ambient temperature, 2.0 g of methyl 4-methoxy-6-methylpyrimidin-2-yl)carbamate, prepared according to the procedure of Example 16, was added and the reaction mixture was heated at reflux for 72 hours. The

reaction mixture was cooled to ambient temperature and 75 ml water, 10 ml glacial acetic acid, and 10 drops hydrochloric acid were sequentially added. The organic phase was separated, dried, and the solvent removed under reduced pressure to give an oil. Crystallization of the oil from n-butyl chloride/ether gave a solid, m.p. 191-200°C.

IR(mull): 1690 (c=o) $cm^{-1}$

NMR ($(CD_3)_2CO$):δ 1.6 (s, 3H);
2.1 (s, 6H);
4.0 (s, 3H);
6.5 (s, 1H); and
8.1-9.0 (m, 3H).

Example 26

1,3-Benzoxathiole-4-sulfonamide

To a solution of 17 g dibromomethane and Aliquat 336® in 200 ml toluene is added dropwise a solution of 21 g 3-bromo-2-mercapto phenol [prepared according to the procedure of S. Cabiddu et al., Synthesis, loc. cit.] and A. Bashall and J. Collins, Tetrahed. Lett., loc. cit.] 24 g of 50% sodium hydroxide and 50 ml $H_2O$. The reaction mixture is then heated at reflux for 16 hours and cooled to ambient temperature. The organic phase is separated, washed with 1N sodium hydroxide and water, dried and concentrated under reduced pressure to give an oil. The oil is purified via distillation to give 14 g 4-bromo-1,3-benzoxathiole. The oil is dissolved in 60 ml of tetrahydrofuran under a nitrogen atmosphere, cooled to -70°C and treated sequentially with 45 ml n-butyllithium (1.6N in hexanes) for 1 hour and excess sulfur dioxide until no further exotherm is noted. The reaction mixture is contacted with 2 ml isopropyl alcohol, concentrated in vacuo and crystallized from ether/tetrahydrofuran to give a solid. The solid is added to 150 ml acetic

acid and cooled to 20°C. A slurry of 8.0 g N-chlorosuccinimide in 30 ml acetic acid is added portionwise with cooling to maintain a reaction temperature of 20-25°C. The reaction mixture is stirred for three hours at room temperature and the solvent is removed under reduced pressure to give an oil which is dissolved in methylene chloride and washed with water. The organic phase is dried, and the solvent removed under reduced pressure to give an oil which is dissolved in 250 ml ether and cooled to 10°C. Excess ammonia (anhy.) is bubbled through the reaction mixture with external cooling. The reaction mixture is washed with water, dried, and concentrated under reduced pressure to give a solid.

## Example 27

N-[(4-Methoxy-6-methylpyrimidin-2-yl)aminocarbonyl]-1,3-benzoxathiole-4-sulfonamide

To a solution of 2.2 g 1,3-benzoxathiole-4-sulfonamide, prepared in Example 26, in 50 ml methylene chloride at ambient temperature under nitrogen is added 5.5 ml trimethylaluminum (2*M* in toluene). After stirring 15 minutes at ambient temperature, 2.0 g of methyl [4-methoxy-6-methylpyrimidin-2-yl]carbamate, prepared according to the procedure of Example 16, is added and the reaction mixture is heated at reflux for 16 hours. The reaction mixture is cooled to ambient temperature and 75 ml water, 10 ml glacial acetic acid, and 10 drops hydrochloric acid are sequentially added. The organic phase is separated, dried, and the solvent removed under reduced pressure to give an oil. Crystallization of the oil from *n*-butyl chloride/ether gives a solid.

The compounds of Table IV can be prepared according to the procedures of Examples 17, 19, 23, 24, 25 and 27 using the appropriate 4-sulfonamide and carbamate.

Example 28

1,4-Benzodioxan-5-sulfonamide

To a solution of 43.6 g 5-bromo-1,4-benzodioxan (prepared according to the procedures of S. Cabiddu et al., Synthesis, loc. cit.) and in 400 ml tetrahydrofuran -70° under nitrogen was added dropwise 127 ml of *n*-butyllithium (1.6N in hexane). The temperature was maintained at -70°C with dry ice/acetone cooling during the addition. The reaction mixture was stirred 0.5 hour at -70°C. Excess sulfur dioxide was bubbled through the reaction mixture, kept at -60 to -70°C with cooling until no further exotherm was noted. The reaction mixture was stirred at -70°C for 2.5 hours, warmed to room temperature, stirred overnight, and 10 ml isopropyl alcohol is added. The reaction mixture was concentrated *in vacuo* and crystallized from ether/tetrahydrofuran to give a solid. The solid was added to 150 ml acetic acid and the resultant mixture was cooled to 20°C. A slurry of 20.5 g of N-chlorosuccinimide in 125 ml acetic acid was added portionwise with cooling to maintain the reaction mixture between 20-25°C. The reaction mixture was stirred 3 hours at ambient temperature and the solvent was removed under reduced pressure to give an oil. The oil was dissolved in methylene chloride and washed with water. The organic phase was dried and the solvent removed under reduced pressure to give an oil which was dissolved in 300 ml ether and cooled to 10°C. Excess ammonia (anhy.) was bubbled through the reaction mixture using ice bath cooling. The reaction mixture was

warmed to ambient temperature, stirred for 1 hour, and 100 ml ether added. The reaction mixture was washed with water, dried, and concentrated *in vacuo* to give a solid, m.p. 114-117°C, IR(mull).

$^1$HNMR (DMSO-$d_6$): 4.3 (s, 4H); and
6.7-7.6 (m, 5H).

Example 29

N-[(4-Methoxy-6-methylpyrimidin-2-yl)aminocarbonyl]-1,4-benzodioxan-5-sulfonamide

To a solution of 1.0 g 1,4-benzodioxan-5-sulfonamide, prepared in Example 28, in 50 ml methylene chloride at ambient temperature under nitrogen was added 2.5 ml trimethylaluminum (2M in toluene). After stirring 15 minutes at ambient temperature, 1.0 g of methyl [4-methoxy-6-methylpyrimidin-2-yl]carbamate, prepared according to the procedure of Example 16, was added and the reaction mixture was heated at reflux for 72 hours. The reaction mixture was cooled to ambient temperature and 75 ml water, 10 ml glacial acetic acid and 10 drops hydrochloric acid were sequentially added. The organic phase was separated, dried, and the solvent removed under reduced pressure to give an oil. Crystallization of the oil from *n*-butyl chloride/ether gave a solid, m.p. 203-205°C. IR(mull) 1700 (c=o) $cm^{-1}$.

$^1$HNMR ($(CD_3)_2CO$): 2.5 (s, 3H),
4.0 (s, 3H);
4.4 (s, 3H);
6.4 (s, 1H);
7.1 (m, 3H); and
7.7 (m, 2H).

Example 30

N-[(4,6-Dimethoxy-1,3,5-triazin-2-yl)aminocarbonyl]-1,4-benzodioxan-5-sulfonamide

To a solution of 1.0 g 1,4-benzodioxan-5-sulfonamide, prepared in Example 28, in 50 ml methylene chloride at ambient temperature under nitrogen, is added 2.5 ml trimethylaluminum (2M in toluene). After stirring 15 minutes at ambient temperature, 1.1 g of methyl [4,6-dimethoxytriazin-2-yl]carbamate, prepared in Example 18, is added and the reaction mixture is heated at reflux 16 hours. The reaction mixture is cooled to ambient temperature and 75 ml water, 10 ml glacial acetic acid, and 10 drops hydrochloric acid are sequentially added. The organic phase is separated, dried and the solvent removed under reduced pressure to give an oil. Crystallization of the oil from an appropriate organic solvent gives a solid.

Example 31

1,4-Benzodithian-5-sulfonamide

To a solution of 17 g dibromo ethane and Aliquat 336® in 200 ml toluene is added dropwise a solution of 22 g 3-bromothiocatechol [prepared according to the procedures of L. Horner et al., loc. cit. and A. Ferretti, loc. cit., 24 g of 50% sodium hydroxide, and 50 ml $H_2O$. The reaction mixture was then treated at reflux for 1.5 hour, cooled to ambient temperature and stirred 16 hours. The organic phase was separated, washed with 1N sodium hydroxide and water, dried and concentrated under reduced pressure to give an oil. The oil was purified via distillation to give 15 g 4-bromo-1,3-benzodithiole. The oil was dissolved in 60 ml of tetrahydrofuran under a nitrogen atmosphere, cooled to -70°C and treated sequentially with 50 ml n-butyllithium (1.6N in hexanes) for 1 hour and excess sulfur dioxide until no further exotherm was noted.

The reaction mixture was contacted with 3 ml isopropyl alcohol, concentrated *in vacuo* and crystallized from ether/tetrahydrofuran to give a solid. The solid was added to 150 ml acetic acid and cooled to 20°C. A slurry of 8.0 g N-chlorosuccinimide in 30 ml acetic acid was added portionwise with cooling to maintain a reaction temperature of 20-25°C. The reaction mixture was stirred for three hours at room temperature and the solvent was removed under reduced pressure to give an oil which was dissolved in methylene chloride and washed with water. The organic phase was dried, and the solvent removed under reduced pressure to give an oil which was dissolved in 250 ml ether and cooled to 10°C. Excess ammonia (anhy.) was bubbled through the reaction mixture with external cooling. The reaction mixture was washed with water, dried, and concentrated under reduced pressure to give a solid.

## Example 32

### N-[(4-methoxy-6-methylpyrimidin-2-yl)aminocarbonyl]-1,4-benzodithian-5-sulfonamide

To a solution of 2.5 g 1,4-benzodithian-5-sulfonamide, prepared in Example 31, in 50 ml methylene chloride at ambient temperature under nitrogen is added 5.5 ml trimethylaluminum (2*M* in toluene). After stirring 15 minutes at ambient temperature 2.0 g of methyl [4-methoxy-6-methylpyrimidin-2-yl]carbamate, prepared according to the procedure of Example 16, is added and the reaction mixture is heated at reflux for 16 hours. The reaction mixture is cooled to ambient temperature and 75 ml water, 10 ml glacial acetic acid, and 10 drops hydrochloric acid are sequentially added. The organic phase is separated, dried, and the solvent removed under reduced pressure to give an oil. Crystallization of the oil from *n*-butyl chloride/ether gives a solid.

Example 33

N-[(4,6-Dimethoxy-1,3,5-triazin-2-yl)aminocarbonyl]-1,4-benzodithian-5-sulfonamide

To a solution of 2.5 g 1,4-benzodithian-5-sulfonamide, prepared in Example 29, in 50 ml methylene chloride at ambient temperature under nitrogen is added 5.5 ml trimethylaluminum (2M in toluene). After stirring 15 minutes at ambient temperature 2.0 g of methyl [4,6-Dimethoxy-1,3,5-triazin-2-yl]carbamate, prepared according to the procedure of Example 16 is added and the reaction mixture is heated at reflux for 16 hours. The reaction mixture is cooled to ambient temperature and 75 ml water, 10 ml glacial acetic acid, and 10 drops hydrochloric acid are sequentially added. The organic phase is separated, dried and the solvent removed under reduced pressure to give an oil. Crystallization of the oil from an appropriate organic solvent gives a solid.

The compounds of Table V can be prepared according to the procedures of Examples 29, 30, 32 and 33 using the appropriate 5-sulfonamide and heterocyclic carbamate.

Table Ia

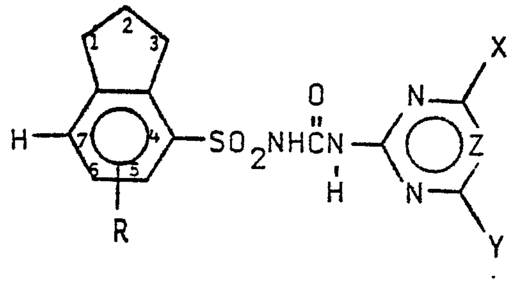

| R | X | Y | Z | m.p.(°C) |
|---|---|---|---|---|
| H | $CH_3$ | $CH_3$ | CH | 107-110° |
| H | $OCH_3$ | $CH_3$ | CH | 186-188° |
| H | $OCH_3$ | $OCH_3$ | CH | 165-167° |
| H | $CH_3$ | $C_2H_5$ | CH | |
| H | $OCH_3$ | $C_2H_5$ | CH | |
| H | $CH_3$ | $OC_2H_5$ | CH | |
| H | $OCH_3$ | $OC_2H_5$ | CH | |
| H | $CH_3$ | $CH_2OCH_3$ | CH | |
| H | $OCH_3$ | $CH_2OCH_3$ | CH | |
| H | $OCH_3$ | $NH_2$ | CH | |
| H | $OCH_3$ | $NHCH_3$ | CH | |
| H | $OCH_3$ | $N(CH_3)_2$ | CH | |
| H | $CH_3$ | $CH_3$ | N | 167-169° |
| H | $OCH_3$ | $CH_3$ | N | 205-207° |
| H | $OCH_3$ | $OCH_3$ | N | 164-166° |
| H | $CH_3$ | $C_2H_5$ | N | |
| H | $OCH_3$ | $C_2H_5$ | N | |
| H | $CH_3$ | $OC_2H_5$ | N | |
| H | $OCH_3$ | $OC_2H_5$ | N | |
| H | $CH_3$ | $CH_2OCH_3$ | N | |
| H | $OCH_3$ | $CH_2OCH_3$ | N | |
| H | $OCH_3$ | $NH_2$ | N | |
| H | $OCH_3$ | $NHCH_3$ | N | |
| H | $OCH_3$ | $N(CH_3)_2$ | N | |
| H | $CH_3$ | $CH(OCH_3)_2$ | CH | |

Table Ia (continued)

| R | X | Y | Z | m.p.(°C) |
|---|---|---|---|---|
| H | $OCH_3$ | $CH(OCH_3)_2$ | CH | |
| H | $CH_3$ | 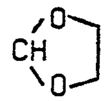 | CH | |
| H | $OCH_3$ | 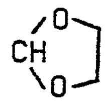 | CH | |
| H | $CH_3$ | $CH(OCH_3)_2$ | N | |
| H | $OCH_3$ | $CH(OCH_3)_2$ | N | |
| H | $CH_3$ | 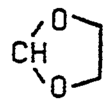 | N | |
| H | $OCH_3$ | 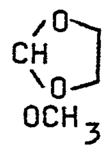 | N | |
| H | Cl | $OCH_3$ | CH | |
| H | Cl | $NH_2$ | CH | |
| H | Cl | $NHCH_3$ | CH | |
| H | Cl | $N(CH_3)_2$ | CH | |
| H | $CH_3$ | $SCH_3$ | N | |
| H | $OCH_3$ | $SCH_3$ | N | |
| H | $CH_3$ | $SCH_3$ | CH | |
| H | $OCH_3$ | $SCH_3$ | CH | |
| 5-Cl | $CH_3$ | $CH_3$ | CH | |
| 5-Cl | $OCH_3$ | $CH_3$ | CH | |
| 5-Cl | $OCH_3$ | $OCH_3$ | CH | |
| 5-Cl | $CH_3$ | $C_2H_5$ | CH | |
| 5-Cl | $OCH_3$ | $C_2H_5$ | CH | |
| 5-Cl | $CH_3$ | $OC_2H_5$ | CH | |
| 5-Cl | $OCH_3$ | $OC_2H_5$ | CH | |
| 5-Cl | $CH_3$ | $CH_2OCH_3$ | CH | |
| 5-Cl | $OCH_3$ | $CH_2OCH_3$ | CH | |
| 5-Cl | $OCH_3$ | $NH_2$ | CH | |
| 5-Cl | $OCH_3$ | $NHCH_3$ | CH | |
| 5-Cl | $OCH_3$ | $N(CH_3)_2$ | CH | |
| 5-Cl | $CH_3$ | $CH_3$ | N | |

Table Ia (continued)

| R | X | Y | Z | m.p.(°C) |
|---|---|---|---|---|
| 5-Cl | $OCH_3$ | $CH_3$ | N | |
| 5-Cl | $OCH_3$ | $OCH_3$ | N | |
| 5-Cl | $CH_3$ | $C_2H_5$ | N | |
| 5-Cl | $OCH_3$ | $C_2H_5$ | N | |
| 5-Cl | $CH_3$ | $OC_2H_5$ | N | |
| 5-Cl | $OCH_3$ | $OC_2H_5$ | N | |
| 5-Cl | $CH_3$ | $CH_2OCH_3$ | N | |
| 5-Cl | $OCH_3$ | $CH_2OCH_3$ | N | |
| 5-Cl | $OCH_3$ | $NH_2$ | N | |
| 5-Cl | $OCH_3$ | $NHCH_3$ | N | |
| 5-Cl | $OCH_3$ | $N(CH_3)_2$ | N | |
| 5-Cl | $CH_3$ | $CH(OCH_3)_2$ | CH | |
| 5-Cl | $CH_3$ | $CH(OCH_3)_2$ | N | |
| 5-Cl | $OCH_3$ | $CH(OCH_3)_2$ | CH | |
| 5-Cl | $OCH_3$ | $CH(OCH_3)_2$ | N | |
| 5-Cl | $CH_3$ | 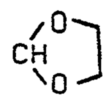 | CH | |
| 5-Cl | $OCH_3$ | 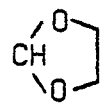 | CH | |
| 5-Cl | Cl | $OCH_3$ | CH | |
| 5-Cl | Cl | $NH_2$ | CH | |
| 5-Cl | Cl | $N(CH_3)_2$ | CH | |
| 6-Cl | $CH_3$ | $CH_3$ | CH | |
| 6-Cl | $OCH_3$ | $CH_3$ | CH | |
| 6-Cl | $OCH_3$ | $OCH_3$ | CH | |
| 6-Cl | $CH_3$ | $C_2H_5$ | CH | |
| 6-Cl | $OCH_3$ | $C_2H_5$ | CH | |
| 6-Cl | $CH_3$ | $OC_2H_5$ | CH | |
| 6-Cl | $OCH_3$ | $OC_2H_5$ | CH | |
| 6-Cl | $CH_3$ | $CH_2OCH_3$ | CH | |
| 6-Cl | $OCH_3$ | $CH_2OCH_3$ | CH | |
| 6-Cl | $OCH_3$ | $NH_2$ | CH | |

Table Ia (continued)

| R | X | Y | Z | m.p.(°C) |
|---|---|---|---|---|
| 6-Cl | $OCH_3$ | $NHCH_3$ | CH | |
| 6-Cl | $OCH_3$ | $N(CH_3)_2$ | CH | |
| 6-Cl | $CH_3$ | $NHCH_3$ | CH | |
| 6-Cl | $CH_3$ | $NH_2$ | CH | |
| 6-Cl | $CH_3$ | $N(CH_3)_2$ | CH | |
| 6-Cl | $CH_3$ | $CH_3$ | N | |
| 6-Cl | $OCH_3$ | $CH_3$ | N | |
| 6-Cl | $OCH_3$ | $OCH_3$ | N | |
| 6-Cl | $CH_3$ | $C_2H_5$ | N | |
| 6-Cl | $OCH_3$ | $C_2H_5$ | N | |
| 6-Cl | $CH_3$ | $OC_2H_5$ | N | |
| 6-Cl | $OCH_3$ | $OC_2H_5$ | N | |
| 6-Cl | $CH_3$ | $CH_2OCH_3$ | N | |
| 6-Cl | $OCH_3$ | $CH_2OCH_3$ | N | |
| 6-Cl | $OCH_3$ | $NH_2$ | N | |
| 6-Cl | $OCH_3$ | $NHCH_3$ | N | |
| 6-Cl | $OCH_3$ | $N(CH_3)_2$ | N | |
| 5-$NO_2$ | $CH_3$ | 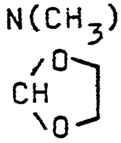 | CH | |
| 5-$NO_2$ | $OCH_3$ | 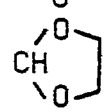 | CH | |
| 5-$NO_2$ | $CH_3$ | 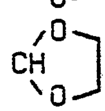 | N | |
| 5-$NO_2$ | $OCH_3$ | 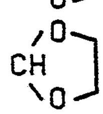 | N | |
| 5-$NO_2$ | Cl | $OCH_3$ | CH | |
| 5-$NO_2$ | $CH_3$ | $CH_3$ | CH | 172 dec. |
| 5-$NO_2$ | $OCH_3$ | $CH_3$ | CH | 65 dec. |
| 5-$NO_2$ | $OCH_3$ | $OCH_3$ | CH | 162 dec. |
| 5-$NO_2$ | $CH_3$ | $C_2H_5$ | CH | |
| 5-$NO_2$ | $OCH_3$ | $C_2H_5$ | CH | |
| 5-$NO_2$ | $CH_3$ | $OC_2H_5$ | CH | |

Table Ia (continued)

| R | X | Y | Z | m.p.(°C) |
|---|---|---|---|---|
| 5-$NO_2$ | $OCH_3$ | $OC_2H_5$ | CH | |
| 5-$NO_2$ | $CH_3$ | $CH_2OCH_3$ | CH | |
| 5-$NO_2$ | $OCH_3$ | $CH_2OCH_3$ | CH | |
| 5-$NO_2$ | $OCH_3$ | $NH_2$ | CH | |
| 5-$NO_2$ | $OCH_3$ | $NHCH_3$ | CH | |
| 5-$NO_2$ | $OCH_3$ | $N(CH_3)_2$ | CH | |
| 5-$NO_2$ | $CH_3$ | $CH_3$ | N | |
| 5-$NO_2$ | $OCH_3$ | $CH_3$ | N | 163 dec. |
| 5-$NO_2$ | $OCH_3$ | $OCH_3$ | N | 170-175° |
| 5-$NO_2$ | $CH_3$ | $C_2H_5$ | N | |
| 5-$NO_2$ | $OCH_3$ | $C_2H_5$ | N | |
| 5-$NO_2$ | $CH_3$ | $OC_2H_5$ | N | |
| 5-$NO_2$ | $OCH_3$ | $OC_2H_5$ | N | |
| 5-$NO_2$ | $CH_3$ | $CH_2OCH_3$ | N | |
| 5-$NO_2$ | $OCH_3$ | $CH_2OCH_3$ | N | |
| 5-$NO_2$ | $OCH_3$ | $NH_2$ | N | |
| 5-$NO_2$ | $OCH_3$ | $NHCH_3$ | N | |
| 5-$NO_2$ | $OCH_3$ | $N(CH_3)_2$ | N | |
| 6-$NO_2$ | $CH_3$ | $CH_3$ | CH | 170-178° |
| 6-$NO_2$ | $OCH_3$ | $CH_3$ | CH | 85 dec. |
| 6-$NO_2$ | $OCH_3$ | $OCH_3$ | CH | 200-206° |
| 6-$NO_2$ | $CH_3$ | $C_2H_5$ | CH | |
| 6-$NO_2$ | $OCH_3$ | $C_2H_5$ | CH | |
| 6-$NO_2$ | $CH_3$ | $OC_2H_5$ | CH | |
| 6-$NO_2$ | $OCH_3$ | $OC_2H_5$ | CH | |
| 6-$NO_2$ | $CH_3$ | $CH_2OCH_3$ | CH | |
| 6-$NO_2$ | $OCH_3$ | $CH_2OCH_3$ | CH | |
| 6-$NO_2$ | $OCH_3$ | $NH_2$ | CH | |
| 6-$NO_2$ | $OCH_3$ | $NHCH_3$ | CH | |
| 6-$NO_2$ | $OCH_3$ | $N(CH_3)_2$ | CH | |
| 6-$NO_2$ | $CH_3$ | $NHCH_3$ | CH | |
| 6-$NO_2$ | $CH_3$ | $NH_2$ | CH | |

Table Ia (continued)

| R | X | Y | Z | m.p.(°C) |
|---|---|---|---|---|
| 6-$NO_2$ | $CH_3$ | $N(CH_3)_2$ | CH | |
| 6-$NO_2$ | $CH_3$ | $CH_3$ | N | 140-143° |
| 6-$NO_2$ | $OCH_3$ | $CH_3$ | N | |
| 6-$NO_2$ | $OCH_3$ | $OCH_3$ | N | 170-172° |
| 6-$NO_2$ | $CH_3$ | $C_2H_5$ | N | |
| 6-$NO_2$ | $OCH_3$ | $C_2H_5$ | N | |
| 6-$NO_2$ | $CH_3$ | $OC_2H_5$ | N | |
| 6-$NO_2$ | $OCH_3$ | $OC_2H_5$ | N | |
| 6-$NO_2$ | $CH_3$ | $CH_2OCH_3$ | N | |
| 6-$NO_2$ | $OCH_3$ | $CH_2OCH_3$ | N | |
| 6-$NO_2$ | $OCH_3$ | $NH_2$ | N | |
| 6-$NO_2$ | $OCH_3$ | $NHCH_3$ | N | |
| 6-$NO_2$ | $OCH_3$ | $N(CH_3)_2$ | N | |
| 6-$NO_2$ | $CH_3$ | $NHCH_3$ | N | |
| 6-$NO_2$ | $CH_3$ | $NH_2$ | N | |
| 6-$NO_2$ | $CH_3$ | $N(CH_3)_2$ | N | |
| 6-Br | $CH_3$ | $CH_3$ | CH | |
| 6-Br | $CH_3$ | $OCH_3$ | CH | |
| 6-Br | $OCH_3$ | $OCH_3$ | CH | |
| 6-$OCH_3$ | $CH_3$ | $CH_3$ | CH | |
| 6-$OCH_3$ | $OCH_3$ | $CH_3$ | CH | |
| 6-$OCH_3$ | $OCH_3$ | $OCH_3$ | CH | |
| 6-Br | $CH_3$ | $CH_3$ | N | |
| 6-Br | $CH_3$ | $OCH_3$ | N | |
| 6-Br | $OCH_3$ | $OCH_3$ | N | |
| 6-$OCH_3$ | $CH_3$ | $CH_3$ | N | |
| 6-$OCH_3$ | $OCH_3$ | $CH_3$ | N | |
| 6-$OCH_3$ | $OCH_3$ | $OCH_3$ | N | |
| 5-Br | $CH_3$ | $CH_3$ | CH | |
| 5-Br | $CH_3$ | $OCH_3$ | CH | |
| 5-Br | $OCH_3$ | $OCH_3$ | CH | |

Table Ia (continued)

| R | X | Y | Z | m.p.(°C) |
|---|---|---|---|---|
| 5-$OCH_3$ | $CH_3$ | $CH_3$ | CH | |
| 5-$OCH_3$ | $OCH_3$ | $CH_3$ | CH | |
| 5-$OCH_3$ | $OCH_3$ | $OCH_3$ | CH | |
| 5-$OC_2H_5$ | $CH_3$ | $CH_3$ | CH | |
| 5-$OC_2H_5$ | $CH_3$ | $OCH_3$ | CH | |
| 5-$OC_2H_5$ | $OCH_3$ | $OCH_3$ | CH | |
| 5-$OCH(CH_3)_2$ | $CH_3$ | $CH_3$ | CH | |
| 5-$OCH(CH_3)_2$ | $OCH_3$ | $CH_3$ | CH | |
| 5-$OCH(CH_3)_2$ | $OCH_3$ | $OCH_3$ | CH | |
| 5-$OCH_2CH_2CH_3$ | $CH_3$ | $CH_3$ | CH | |
| 5-$OCH_2CH_2CH_3$ | $OCH_3$ | $CH_3$ | CH | |
| 5-$OCH_2CH_2CH_3$ | $OCH_3$ | $OCH_3$ | CH | |
| 5-Br | $CH_3$ | $CH_3$ | N | |
| 5-Br | $CH_3$ | $OCH_3$ | N | |
| 5-Br | $OCH_3$ | $OCH_3$ | N | |
| 5-$OCH_3$ | $CH_3$ | $CH_3$ | N | |
| 5-$OCH_3$ | $OCH_3$ | $CH_3$ | N | |
| 5-$OCH_3$ | $OCH_3$ | $OCH_3$ | N | |
| 5-$OC_2H_5$ | $CH_3$ | $CH_3$ | N | |
| 5-$OC_2H_5$ | $CH_3$ | $OCH_3$ | N | |
| 5-$OC_2H_5$ | $OCH_3$ | $OCH_3$ | N | |
| 5-$OCH(CH_3)_2$ | $CH_3$ | $CH_3$ | N | |
| 5-Br | $CH_3$ | $CH(OCH_3)_2$ | CH | |
| 5-Br | Cl | $OCH_3$ | CH | |
| 5-$OCH_3$ | $OCH_3$ | $CH(OCH_3)_2$ | CH | |
| 5-$OCH_3$ | $CH_3$ | $CH(OCH_3)_2$ | N | |
| 5-$OCH_3$ | Cl | $OCH_3$ | CH | |
| 5-$OC_2H_5$ | Cl | $OCH_3$ | CH | |
| 5-$OCH(CH_3)_2$ | Cl | $OCH_3$ | CH | |
| 5-$OCH(CH_3)_2$ | $CH_3$ | $CH(OCH_3)_2$ | CH | |
| 5-$OCH(CH_3)_2$ | $OCH_3$ | $CH_3$ | N | |
| 5-$OCH(CH_3)_2$ | $OCH_3$ | $OCH_3$ | N | |

Table Ia (continued)

| R | X | Y | Z | m.p.(°C) |
|---|---|---|---|---|
| 5-$OCH_2CH_2CH_3$ | $CH_3$ | $CH_3$ | N | |
| 5-$OCH_2CH_2CH_3$ | $OCH_3$ | $CH_3$ | N | |
| 5-$OCH_2CH_2CH_3$ | $OCH_3$ | $OCH_3$ | N | |
| 5-$SO_2N(CH_3)_2$ | $CH_3$ | $CH_3$ | CH | |
| 5-$SO_2N(CH_3)_2$ | $CH_3$ | $OCH_3$ | CH | |
| 5-$SO_2N(CH_3)_2$ | $OCH_3$ | $OCH_3$ | CH | |
| 5-$SO_2N(C_2H_5)_2$ | $CH_3$ | $CH_3$ | CH | |
| 5-$SO_2N(C_2H_5)_2$ | $CH_3$ | $OCH_3$ | CH | |
| 5-$SO_2N(C_2H_5)_2$ | $OCH_3$ | $OCH_3$ | CH | |
| 5-$SO_2N(CH_3)C_2H_5$ | $CH_3$ | $CH_3$ | CH | |
| 5-$SO_2N(CH_3)C_2H_5$ | $CH_3$ | $OCH_3$ | CH | |
| 5-$SO_2N(CH_3)C_2H_5$ | $OCH_3$ | $OCH_3$ | CH | |
| 5-$SO_2N(CH_3)OCH_3$ | $CH_3$ | $CH_3$ | CH | |
| 5-$SO_2N(CH_3)OCH_3$ | $CH_3$ | $OCH_3$ | CH | |
| 5-$SO_2N(CH_3)OCH_3$ | $OCH_3$ | $OCH_3$ | CH | |
| 5-$OSO_2CF_3$ | $CH_3$ | $CH_3$ | CH | |
| 5-$OSO_2CF_3$ | $CH_3$ | $OCH_3$ | CH | |
| 5-$OSO_2CF_3$ | $OCH_3$ | $OCH_3$ | CH | |
| 5-$OSO_2CH_3$ | $CH_3$ | $CH_3$ | CH | |
| 5-$OSO_2CH_3$ | $CH_3$ | $OCH_3$ | CH | |
| 5-$OSO_2CH_3$ | $OCH_3$ | $OCH_3$ | CH | |
| 5-$OSO_2C_2H_5$ | $CH_3$ | $CH_3$ | CH | |
| 5-$OSO_2C_2H_5$ | $CH_3$ | $OCH_3$ | CH | |
| 5-$OSO_2C_2H_5$ | $OCH_3$ | $OCH_3$ | CH | |
| 5-$OSO_2$-n-$C_3H_7$ | $CH_3$ | $CH_3$ | CH | |
| 5-$OSO_2$-n-$C_3H_7$ | $CH_3$ | $OCH_3$ | CH | |
| 5-$OSO_2$-n-$C_3H_7$ | $OCH_3$ | $OCH_3$ | CH | |
| 5-$CO_2CH_3$ | $CH_3$ | $CH_3$ | CH | |
| 5-$CO_2CH_3$ | $CH_3$ | $OCH_3$ | CH | |
| 5-$CO_2CH_3$ | $OCH_3$ | $OCH_3$ | CH | |
| 5-$SO_2N(CH_3)_2$ | Cl | $OCH_3$ | CH | |
| 5-$SO_2N(CH_3)_2$ | $CH_3$ | $CH(OCH_3)_2$ | CH | |

Table Ia (continued)

| R | X | Y | Z | m.p.(°C) |
|---|---|---|---|---|
| 5-$SO_2N(CH_3)_2$ | $CH_3$ | $CH(OCH_3)_2$ | N | |
| 5-$SO_2N(CH_3)_2$ | $CH_3$ | 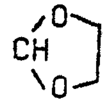 | N | |
| 5-$SO_2N(CH_3)_2$ | $CH_3$ | 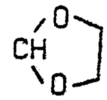 | CH | |
| 5-$SO_2N(C_2H_5)_2$ | $CH_3$ | 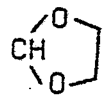 | CH | |
| 5-$SO_2N(C_2H_5)_2$ | $OCH_3$ | $CH(OCH_3)_2$ | CH | |
| 5-$SO_2N(C_2H_5)_2$ | Cl | $OCH_3$ | CH | |
| 5-$SO_2N(OCH_3)CH_3$ | Cl | $OCH_3$ | CH | |
| 5-$SO_2N(OCH_3)CH_3$ | $CH_3$ | $CH(OCH_3)_2$ | CH | |
| 5-$SO_2N(OCH_3)CH_3$ | $OCH_3$ | $CH(OCH_3)_2$ | CH | |
| 5-$OSO_2CH_3$ | Cl | $OCH_3$ | CH | |
| 5-$OSO_2CH_3$ | $CH_3$ | $CH(OCH_3)_2$ | CH | |
| 5-$OSO_2CH_3$ | $OCH_3$ | $CH(OCH_3)_2$ | CH | |
| 5-$OSO_2CF_3$ | Cl | $OCH_3$ | CH | |
| 5-$OSO_2CF_3$ | $OCH_3$ | $CH(OCH_3)_2$ | CH | |
| 5-$OSO_2$-$CH_2CH_2CH_3$ | $OCH_3$ | $CH(OCH_3)_2$ | CH | |
| 5-$OSO_2$-$CH_2CH_2CH_3$ | Cl | $OCH_3$ | CH | |
| 5-$CO_2CH_3$ | $CH_3$ | $OCH_3$ | N | |
| 5-$CO_2CH_3$ | $OCH_3$ | $OCH_3$ | N | |
| 5-$CO_2CH_3$ | Cl | $OCH_3$ | CH | |
| 5-$CO_2CH_3$ | $CH_3$ | $CH(OCH_3)_2$ | CH | |
| 5-$CO_2CH_3$ | $OCH_3$ | $CH(OCH_3)_2$ | CH | |
| 5-$CO_2C_2H_5$ | Cl | $OCH_3$ | CH | |
| 5-$CO_2C_2H_5$ | $CH_3$ | $CH(OCH_3)_2$ | CH | |
| 5-$CO_2C_2H_5$ | $OCH_3$ | $CH(OCH_3)_2$ | N | |
| 5-$CO_2C_2H_5$ | $OCH_3$ | 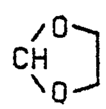 | N | |
| 5-$CO_2C_2H_5$ | $OCH_3$ | 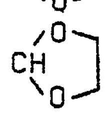 | CH | |
| 5-$CO_2CH_2CH_2OCH_3$ | Cl | $OCH_3$ | CH | |

Table Ia (continued)

| R | X | Y | Z | m.p.(°C) |
|---|---|---|---|---|
| 5-$CO_2CH(CH_3)_2$ | Cl | $OCH_3$ | CH | |
| 5-$CO_2CH(CH_3)_2$ | $OCH_3$ | $CH_3$ | CH | |
| 5-$CO_2CH(CH_3)_2$ | $CH_3$ | 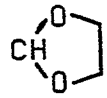 | CH | |
| 5-$CO_2C_2H_5$ | $CH_3$ | $CH_3$ | CH | |
| 5-$CO_2C_2H_5$ | $CH_3$ | $OCH_3$ | CH | |
| 5-$CO_2C_2H_5$ | $OCH_3$ | $OCH_3$ | CH | |
| 5-$CO_2$-n-$C_3H_7$ | $CH_3$ | $CH_3$ | CH | |
| 5-$CO_2$-n-$C_3H_7$ | $CH_3$ | $OCH_3$ | CH | |
| 5-$CO_2$-n-$C_3H_7$ | $OCH_3$ | $OCH_3$ | CH | |
| 5-$CO_2$-i-$C_3H_7$ | $CH_3$ | $CH_3$ | CH | |
| 5-$CO_2$-i-$C_3H_7$ | $CH_3$ | $OCH_3$ | CH | |
| 5-$CO_2$-i-$C_3H_7$ | $OCH_3$ | $OCH_3$ | CH | |
| 5-$CO_2CH_2CH=CH_2$ | $CH_3$ | $CH_3$ | CH | |
| 5-$CO_2CH_2CH=CH_2$ | $CH_3$ | $OCH_3$ | CH | |
| 5-$CO_2CH_2CH=CH_2$ | $OCH_3$ | $OCH_3$ | CH | |
| 5-$CO_2CH_2CH_2OCH_3$ | $CH_3$ | $CH_3$ | CH | |
| 5-$CO_2CH_2CH_2OCH_3$ | $CH_3$ | $OCH_3$ | CH | |
| 5-$CO_2CH_2CH_2OCH_3$ | $OCH_3$ | $OCH_3$ | CH | |
| 5-$CO_2CH_2CH_2Cl$ | $CH_3$ | $CH_3$ | CH | |
| 5-$CO_2CH_2CH_2Cl$ | $CH_3$ | $OCH_3$ | CH | |
| 5-$CO_2CH_2CH_2Cl$ | $OCH_3$ | $OCH_3$ | CH | |
| 5-$SO_2CH_3$ | $CH_3$ | $CH_3$ | CH | |
| 5-$SO_2CH_3$ | $CH_3$ | $OCH_3$ | CH | |
| 5-$SO_2CH_3$ | $OCH_3$ | $OCH_3$ | CH | |
| 5-$SO_2N(CH_3)_2$ | $CH_3$ | $CH_3$ | N | |
| 5-$SO_2N(CH_3)_2$ | $CH_3$ | $OCH_3$ | N | |
| 5-$SO_2N(CH_3)_2$ | $OCH_3$ | $OCH_3$ | N | |
| 5-$SO_2N(C_2H_5)_2$ | $CH_3$ | $CH_3$ | N | |
| 5-$SO_2N(C_2H_5)_2$ | $CH_3$ | $OCH_3$ | N | |
| 5-$SO_2N(C_2H_5)_2$ | $OCH_3$ | $OCH_3$ | N | |
| 5-$SO_2N(CH_3)C_2H_5$ | $CH_3$ | $CH_3$ | N | |

Table Ia (continued)

| R | X | Y | Z | m.p.(°C) |
|---|---|---|---|---|
| 5-$SO_2N(CH_3)C_2H_5$ | $CH_3$ | $OCH_3$ | N | |
| 5-$SO_2N(CH_3)C_2H_5$ | $OCH_3$ | $OCH_3$ | N | |
| 5-$SO_2N(CH_3)OCH_3$ | $CH_3$ | $CH_3$ | N | |
| 5-$SO_2N(CH_3)OCH_3$ | $CH_3$ | $OCH_3$ | N | |
| 5-$SO_2N(CH_3)OCH_3$ | $OCH_3$ | $OCH_3$ | N | |
| 5-$OSO_2CF_3$ | $CH_3$ | $CH_3$ | N | |
| 5-$OSO_2CF_3$ | $CH_3$ | $OCH_3$ | N | |
| 5-$OSO_2CF_3$ | $OCH_3$ | $OCH_3$ | N | |
| 5-$OSO_2CH_3$ | $CH_3$ | $CH_3$ | N | |
| 5-$OSO_2CH_3$ | $CH_3$ | $OCH_3$ | N | |
| 5-$OSO_2CH_3$ | $OCH_3$ | $OCH_3$ | N | |
| 5-$OSO_2C_2H_5$ | $CH_3$ | $CH_3$ | N | |
| 5-$OSO_2C_2H_5$ | $CH_3$ | $OCH_3$ | N | |
| 5-$OSO_2C_2H_5$ | $OCH_3$ | $OCH_3$ | N | |
| 5-$OSO_2$-n-$C_3H_7$ | $CH_3$ | $CH_3$ | N | |
| 5-$OSO_2$-n-$C_3H_7$ | $CH_3$ | $OCH_3$ | N | |
| 5-$OSO_2$-n-$C_3H_7$ | $OCH_3$ | $OCH_3$ | N | |
| 5-$CO_2CH_3$ | $CH_3$ | $CH_3$ | N | |
| 5-$CO_2CH_3$ | $CH_3$ | $OCH_3$ | N | |
| 5-$CO_2CH_3$ | $OCH_3$ | $OCH_3$ | N | |
| 5-$CO_2C_2H_5$ | $CH_3$ | $CH_3$ | N | |
| 5-$CO_2C_2H_5$ | $CH_3$ | $OCH_3$ | N | |
| 5-$CO_2C_2H_5$ | $OCH_3$ | $OCH_3$ | N | |
| 5-$CO_2$-n-$C_3H_7$ | $CH_3$ | $CH_3$ | N | |
| 5-$CO_2$-n-$C_3H_7$ | $CH_3$ | $OCH_3$ | N | |
| 5-$CO_2$-n-$C_3H_7$ | $OCH_3$ | $OCH_3$ | N | |
| 5-$CO_2$-i-$C_3H_7$ | $CH_3$ | $CH_3$ | N | |
| 5-$CO_2$-i-$C_3H_7$ | $CH_3$ | $OCH_3$ | N | |
| 5-$CO_2$-i-$C_3H_7$ | $OCH_3$ | $OCH_3$ | N | |
| 5-$CO_2CH_2CH{=}CH_2$ | $CH_3$ | $CH_3$ | N | |
| 5-$CO_2CH_2CH{=}CH_2$ | $CH_3$ | $OCH_3$ | N | |
| 5-$CO_2CH_2CH{=}CH_2$ | $OCH_3$ | $OCH_3$ | N | |

Table Ia (continued)

| R | X | Y | Z | m.p.(°C) |
|---|---|---|---|---|
| 5-$CO_2CH_2CH_2OCH_3$ | $CH_3$ | $CH_3$ | N | |
| 5-$CO_2CH_2CH_2OCH_3$ | $CH_3$ | $OCH_3$ | N | |
| 5-$CO_2CH_2CH_2OCH_3$ | $OCH_3$ | $OCH_3$ | N | |
| 5-$CO_2CH_2CH_2Cl$ | $CH_3$ | $CH_3$ | N | |
| 5-$CO_2CH_2CH_2Cl$ | $CH_3$ | $OCH_3$ | N | |
| 5-$CO_2CH_2CH_2Cl$ | $OCH_3$ | $OCH_3$ | N | |
| 5-$SO_2CH_3$ | $CH_3$ | $CH_3$ | N | |
| 5-$SO_2CH_3$ | $CH_3$ | $OCH_3$ | N | |
| 5-$SO_2CH_3$ | $OCH_3$ | $OCH_3$ | N | |
| 5-$SCH_3$ | $CH_3$ | $CH_3$ | N | |
| 5-$SCH_3$ | $OCH_3$ | $OCH_3$ | N | |
| 5-$SCH_3$ | $OCH_3$ | $CH_3$ | N | |
| 5-$SCH_3$ | $CH_3$ | $CH_3$ | CH | |
| 5-$SCH_3$ | $OCH_3$ | $OCH_3$ | CH | |
| 5-$SCH_3$ | $OCH_3$ | $CH_3$ | CH | |
| 5-$C_2H_5$ | $CH_3$ | $OCH_3$ | CH | |
| 5-$C_2H_5$ | $OCH_3$ | $OCH_3$ | CH | |
| 5-$C_2H_5$ | $CH_3$ | $OCH_3$ | N | |
| 5-$C_2H_5$ | $OCH_3$ | $OCH_3$ | N | |
| 5-$CH(CH_3)_2$ | $OCH_3$ | $CH_3$ | CH | |
| 5-$CH(CH_3)_2$ | $OCH_3$ | $OCH_3$ | CH | |
| 5-$CH(CH_3)_2$ | $CH_3$ | $OCH_3$ | N | |
| 5-$CH(CH_3)_2$ | $OCH_3$ | $OCH_3$ | N | |
| 5-n-$C_3H_7$ | $OCH_3$ | $OCH_3$ | N | |
| 5-n-$C_3H_7$ | $CH_3$ | $OCH_3$ | N | |
| 5-n-$C_3H_7$ | $CH_3$ | $CH_2OCH_3$ | N | |
| 5-$SCF_2H$ | $CH_3$ | $OCH_3$ | N | |
| 5-$SCF_2H$ | $OCH_3$ | $OCH_3$ | N | |
| 5-$SCF_2H$ | Cl | $OCH_3$ | CH | |
| 5-$OCF_3$ | $OCH_3$ | $OCH_3$ | CH | |
| 5-$OCF_3$ | $CH_3$ | $OCH_3$ | CH | |
| 5-$OCF_2H$ | $OCH_3$ | $CH_3$ | N | |

Table Ia (continued)

| R | X | Y | Z | m.p.(°C) |
|---|---|---|---|---|
| 5-$OCF_2H$ | $OCH_3$ | $OCH_3$ | CH | |
| $OCF_2HCF_2H$ | $CH_3$ | $CH_3$ | CH | |
| $OCF_2HCF_2H$ | Cl | $OCH_3$ | CH | |
| $OCF_2HCF_2H$ | $OCH_3$ | $CH_3$ | CH | |
| $OCF_2HCF_2H$ | $OCH_3$ | $OCH_3$ | CH | |
| $OCF_2HCF_2H$ | $CH_3$ | $OCH_3$ | N | |
| $C_2H_5$ | $CH_3$ | $OCH_3$ | N | |
| $C_2H_5$ | $CH_3$ | $CH_3$ | CH | |
| $C_2H_5$ | $OCH_3$ | $CH_3$ | CH | |
| $C_2H_5$ | $OCH_3$ | $OCH_3$ | CH | |
| $C_2H_5$ | Cl | $OCH_3$ | CH | |
| $CH(CH_3)_2$ | $OCH_3$ | $OCH_3$ | CH | |
| $CH(CH_3)_2$ | $CH_3$ | $OCH_3$ | CH | |
| $CH(CH_3)_2$ | $CH_3$ | $CH_3$ | CH | |
| n-$C_3H_7$ | $CH_3$ | $CH_3$ | CH | |
| n-$C_3H_7$ | $OCH_3$ | $CH_3$ | CH | |
| n-$C_3H_7$ | $OCH_3$ | $OCH_3$ | CH | |

## Table Ib

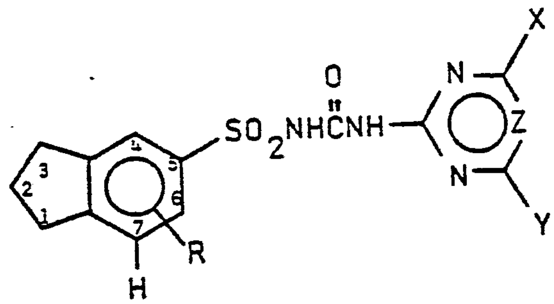

| R | X | Y | Z | m.p.(°C) |
|---|---|---|---|---|
| H | $CH_3$ | $CH_3$ | CH | 168-170° |
| H | $OCH_3$ | $CH_3$ | CH | 177-178° |
| H | $OCH_3$ | $OCH_3$ | CH | 177-178° |
| H | $CH_3$ | $C_2H_5$ | CH | |
| H | $OCH_3$ | $C_2H_5$ | CH | |
| H | $CH_3$ | $OC_2H_5$ | CH | |
| H | $OCH_3$ | $OC_2H_5$ | CH | |
| H | $CH_3$ | $CH_2OCH_3$ | CH | |
| H | $OCH_3$ | $CH_2OCH_3$ | CH | |
| H | $OCH_3$ | $NH_2$ | CH | |
| H | $OCH_3$ | $NHCH_3$ | CH | |
| H | $OCH_3$ | $N(CH_3)_2$ | CH | |
| H | $CH_3$ | $CH_3$ | N | 167-170° |
| H | $OCH_3$ | $CH_3$ | N | >150 dec |
| H | $OCH_3$ | $OCH_3$ | N | 180-182° |
| H | $CH_3$ | $C_2H_5$ | N | |
| H | $OCH_3$ | $C_2H_5$ | N | |
| H | $CH_3$ | $OC_2H_5$ | N | |
| H | $OCH_3$ | $OC_2H_5$ | N | |
| H | $CH_3$ | $CH_2OCH_3$ | N | |
| H | $OCH_3$ | $CH_2OCH_3$ | N | |
| H | $OCH_3$ | $NH_2$ | N | |
| H | $OCH_3$ | $NHCH_3$ | N | |
| H | $OCH_3$ | $N(CH_3)_2$ | N | |

Table Ib (continued)

| R | X | Y | Z | m.p.(°C) |
|---|---|---|---|---|
| H | $OCH_3$ | $SCH_3$ | N | |
| H | $CH_3$ | $SCH_3$ | N | |
| H | $OCH_3$ | $SCH_3$ | CH | |
| H | $CH_3$ | $SCH_3$ | CH | |
| 4-Cl | $CH_3$ | $CH_3$ | CH | |
| 4-Cl | $OCH_3$ | $CH_3$ | CH | |
| 4-Cl | $OCH_3$ | $OCH_3$ | CH | |
| 4-Cl | $CH_3$ | $C_2H_5$ | CH | |
| 4-Cl | $OCH_3$ | $C_2H_5$ | CH | |
| 4-Cl | $CH_3$ | $OC_2H_5$ | CH | |
| 4-Cl | $OCH_3$ | $OC_2H_5$ | CH | |
| 4-Cl | $CH_3$ | $CH_2OCH_3$ | CH | |
| 4-Cl | $OCH_3$ | $CH_2OCH_3$ | CH | |
| 4-Cl | $OCH_3$ | $NH_2$ | CH | |
| 4-Cl | $OCH_3$ | $NHCH_3$ | CH | |
| 4-Cl | $OCH_3$ | $N(CH_3)_2$ | CH | |
| 4-Cl | $CH_3$ | $CH_3$ | N | |
| 4-Cl | $OCH_3$ | $CH_3$ | N | |
| 4-Cl | $OCH_3$ | $OCH_3$ | N | |
| 4-Cl | $CH_3$ | $C_2H_5$ | N | |
| 4-Cl | $OCH_3$ | $C_2H_5$ | N | |
| 4-Cl | $CH_3$ | $OC_2H_5$ | N | |
| 4-Cl | $OCH_3$ | $OC_2H_5$ | N | |
| 4-Cl | $CH_3$ | $CH_2OCH_3$ | N | |
| 4-Cl | $OCH_3$ | $CH_2OCH_3$ | N | |
| 4-Cl | $OCH_3$ | $NH_2$ | N | |
| 4-Cl | $OCH_3$ | $NHCH_3$ | N | |
| 4-Cl | $OCH_3$ | $N(CH_3)_2$ | N | |
| 6-Cl | $CH_3$ | $CH_3$ | CH | |
| 6-Cl | $OCH_3$ | $CH_3$ | CH | |
| 6-Cl | $OCH_3$ | $OCH_3$ | CH | |
| 6-Cl | $CH_3$ | $C_2H_5$ | CH | |

Table Ib (continued)

| R | X | Y | Z | m.p.(°C) |
|---|---|---|---|---|
| 6-Cl | $OCH_3$ | $C_2H_5$ | CH | |
| 6-Cl | $CH_3$ | $OC_2H_5$ | CH | |
| 6-Cl | $OCH_3$ | $OC_2H_5$ | CH | |
| 6-Cl | $CH_3$ | $CH_2OCH_3$ | CH | |
| 6-Cl | $OCH_3$ | $CH_2OCH_3$ | CH | |
| 6-Cl | $OCH_3$ | $NH_2$ | CH | |
| 6-Cl | $OCH_3$ | $NHCH_3$ | CH | |
| 6-Cl | $OCH_3$ | $N(CH_3)_2$ | CH | |
| 6-Cl | $CH_3$ | $CH_3$ | N | |
| 6-Cl | $OCH_3$ | $CH_3$ | N | |
| 6-Cl | $OCH_3$ | $OCH_3$ | N | |
| 6-Cl | $CH_3$ | $C_2H_5$ | N | |
| 6-Cl | $OCH_3$ | $C_2H_5$ | N | |
| 6-Cl | $CH_3$ | $OC_2H_5$ | N | |
| 6-Cl | $OCH_3$ | $OC_2H_5$ | N | |
| 6-Cl | $CH_3$ | $CH_2OCH_3$ | N | |
| 6-Cl | $OCH_3$ | $CH_2OCH_3$ | N | |
| 6-Cl | $OCH_3$ | $NH_2$ | N | |
| 6-Cl | $OCH_3$ | $NHCH_3$ | N | |
| 6-Cl | $OCH_3$ | $N(CH_3)_2$ | N | |
| $4-NO_2$ | $CH_3$ | $CH_3$ | CH | |
| $4-NO_2$ | $OCH_3$ | $CH_3$ | CH | |
| $4-NO_2$ | $OCH_3$ | $OCH_3$ | CH | |
| $4-NO_2$ | $CH_3$ | $C_2H_5$ | CH | |
| $4-NO_2$ | $OCH_3$ | $C_2H_5$ | CH | |
| $4-NO_2$ | $CH_3$ | $OC_2H_5$ | CH | |
| $4-NO_2$ | $OCH_3$ | $OC_2H_5$ | CH | |
| $4-NO_2$ | $CH_3$ | $CH_2OCH_3$ | CH | |
| $4-NO_2$ | $OCH_3$ | $CH_2OCH_3$ | CH | |
| $4-NO_2$ | $OCH_3$ | $NH_2$ | CH | |
| $4-NO_2$ | $OCH_3$ | $NHCH_3$ | CH | |
| $4-NO_2$ | $OCH_3$ | $N(CH_3)_2$ | CH | |

Table Ib (continued)

| R | X | Y | Z | m.p.(°C) |
|---|---|---|---|---|
| 4-$NO_2$ | $CH_3$ | $CH_3$ | N | |
| 4-$NO_2$ | $OCH_3$ | $CH_3$ | N | |
| 4-$NO_2$ | $OCH_3$ | $OCH_3$ | N | |
| 4-$NO_2$ | $CH_3$ | $C_2H_5$ | N | |
| 4-$NO_2$ | $OCH_3$ | $C_2H_5$ | N | |
| 4-$NO_2$ | $CH_3$ | $OC_2H_5$ | N | |
| 4-$NO_2$ | $OCH_3$ | $OC_2H_5$ | N | |
| 4-$NO_2$ | $CH_3$ | $CH_2OCH_3$ | N | |
| 4-$NO_2$ | $OCH_3$ | $CH_2OCH_3$ | N | |
| 4-$NO_2$ | $OCH_3$ | $NH_2$ | N | |
| 4-$NO_2$ | $OCH_3$ | $NHCH_3$ | N | |
| 4-$NO_2$ | $OCH_3$ | $N(CH_3)_2$ | N | |
| 6-$NO_2$ | $CH_3$ | $CH_3$ | CH | |
| 6-$NO_2$ | $OCH_3$ | $CH_3$ | CH | |
| 6-$NO_2$ | $OCH_3$ | $OCH_3$ | CH | |
| 6-$NO_2$ | $CH_3$ | $C_2H_5$ | CH | |
| 6-$NO_2$ | $OCH_3$ | $C_2H_5$ | CH | |
| 6-$NO_2$ | $CH_3$ | $OC_2H_5$ | CH | |
| 6-$NO_2$ | $OCH_3$ | $OC_2H_5$ | CH | |
| 6-$NO_2$ | $CH_3$ | $CH_2OCH_3$ | CH | |
| 6-$NO_2$ | $OCH_3$ | $CH_2OCH_3$ | CH | |
| 6-$NO_2$ | $OCH_3$ | $NH_2$ | CH | |
| 6-$NO_2$ | $OCH_3$ | $NHCH_3$ | CH | |
| 6-$NO_2$ | $OCH_3$ | $N(CH_3)_2$ | CH | |
| 6-$NO_2$ | $CH_3$ | $CH_3$ | N | |
| 6-$NO_2$ | $OCH_3$ | $CH_3$ | N | |
| 6-$NO_2$ | $OCH_3$ | $OCH_3$ | N | |
| 6-$NO_2$ | $CH_3$ | $C_2H_5$ | N | |
| 6-$NO_2$ | $OCH_3$ | $C_2H_5$ | N | |
| 6-$NO_2$ | $CH_3$ | $OC_2H_5$ | N | |
| 6-$NO_2$ | $OCH_3$ | $OC_2H_5$ | N | |
| 6-$NO_2$ | $CH_3$ | $CH_2OCH_3$ | N | |

Table Ib (continued)

| R | X | Y | Z | m.p.(°C) |
|---|---|---|---|---|
| 6-$NO_2$ | $OCH_3$ | $CH_2OCH_3$ | N | |
| 6-$NO_2$ | $OCH_3$ | $NH_2$ | N | |
| 6-$NO_2$ | $OCH_3$ | $NHCH_3$ | N | |
| 6-$NO_2$ | $OCH_3$ | $N(CH_3)_2$ | N | |
| 6-Br | $CH_3$ | $CH_3$ | CH | |
| 6-Br | $CH_3$ | $OCH_3$ | CH | |
| 6-Br | $OCH_3$ | $OCH_3$ | CH | |
| 6-$OCH_3$ | $CH_3$ | $CH_3$ | CH | |
| 6-$OCH_3$ | $OCH_3$ | $CH_3$ | CH | |
| 6-$OCH_3$ | $OCH_3$ | $OCH_3$ | CH | |
| 6-$OC_2H_5$ | $CH_3$ | $CH_3$ | CH | |
| 6-$OC_2H_5$ | $CH_3$ | $OCH_3$ | CH | |
| 6-$OC_2H_5$ | $OCH_3$ | $OCH_3$ | CH | |
| 6-$OCH(CH_3)_2$ | $CH_3$ | $CH_3$ | CH | |
| 6-$OCH(CH_3)_2$ | $OCH_3$ | $CH_3$ | CH | |
| 6-$OCH(CH_3)_2$ | $OCH_3$ | $OCH_3$ | CH | |
| 6-$OCH_2CH_2CH_3$ | $CH_3$ | $CH_3$ | CH | |
| 6-$OCH_2CH_2CH_3$ | $OCH_3$ | $CH_3$ | CH | |
| 6-$OCH_2CH_2CH_3$ | $OCH_3$ | $OCH_3$ | CH | |
| 6-Br | $CH_3$ | $CH_3$ | N | |
| 6-Br | $CH_3$ | $OCH_3$ | N | |
| 6-Br | $OCH_3$ | $OCH_3$ | N | |
| 6-$OCH_3$ | $CH_3$ | $CH_3$ | N | |
| 6-$OCH_3$ | $OCH_3$ | $CH_3$ | N | |
| 6-$OCH_3$ | $OCH_3$ | $OCH_3$ | N | |
| 6-$OC_2H_5$ | $CH_3$ | $CH_3$ | N | |
| 6-$OC_2H_5$ | $CH_3$ | $OCH_3$ | N | |
| 6-$OC_2H_5$ | $OCH_3$ | $OCH_3$ | N | |
| 6-$OCH(CH_3)_2$ | $CH_3$ | $CH_3$ | N | |
| 6-$OCH(CH_3)_2$ | $OCH_3$ | $CH_3$ | N | |
| 6-$OCH(CH_3)_2$ | $OCH_3$ | $OCH_3$ | N | |
| 6-$OCH_2CH_2CH_3$ | $CH_3$ | $CH_3$ | N | |

Table Ib (continued)

| R | X | Y | Z | m.p.(°C) |
|---|---|---|---|---|
| 6-$OCH_2CH_2CH_3$ | $OCH_3$ | $CH_3$ | N | |
| 6-$OCH_2CH_2CH_3$ | $OCH_3$ | $OCH_3$ | N | |
| 4-Br | $CH_3$ | $CH_3$ | CH | |
| 4-Br | $CH_3$ | $OCH_3$ | CH | |
| 4-Br | $OCH_3$ | $OCH_3$ | CH | |
| 4-$OCH_3$ | $CH_3$ | $CH_3$ | CH | |
| 4-$OCH_3$ | $OCH_3$ | $CH_3$ | CH | |
| 4-$OCH_3$ | $OCH_3$ | $OCH_3$ | CH | |
| 4-$OC_2H_5$ | $CH_3$ | $CH_3$ | CH | |
| 4-$OC_2H_5$ | $CH_3$ | $OCH_3$ | CH | |
| 4-$OC_2H_5$ | $OCH_3$ | $OCH_3$ | CH | |
| 4-$OCH(CH_3)_2$ | $CH_3$ | $CH_3$ | CH | |
| 4-$OCH(CH_3)_2$ | $OCH_3$ | $CH_3$ | CH | |
| 4-$OCH(CH_3)_2$ | $OCH_3$ | $OCH_3$ | CH | |
| 4-$OCH_2CH_2CH_3$ | $CH_3$ | $CH_3$ | CH | |
| 4-$OCH_2CH_2CH_3$ | $OCH_3$ | $CH_3$ | CH | |
| 4-$OCH_2CH_2CH_3$ | $OCH_3$ | $OCH_3$ | CH | |
| 4-Br | $CH_3$ | $CH_3$ | N | |
| 4-Br | $CH_3$ | $OCH_3$ | N | |
| 4-Br | $OCH_3$ | $OCH_3$ | N | |
| 4-$OCH_3$ | $CH_3$ | $CH_3$ | N | |
| 4-$OCH_3$ | $OCH_3$ | $CH_3$ | N | |
| 4-$OCH_3$ | $OCH_3$ | $OCH_3$ | N | |
| 4-$OC_2H_5$ | $CH_3$ | $CH_3$ | N | |
| 4-$OC_2H_5$ | $CH_3$ | $OCH_3$ | N | |
| 4-$OC_2H_5$ | $OCH_3$ | $OCH_3$ | N | |
| 4-$OCH(CH_3)_2$ | $CH_3$ | $CH_3$ | N | |
| 4-$OCH(CH_3)_2$ | $OCH_3$ | $CH_3$ | N | |
| 4-$OCH(CH_3)_2$ | $OCH_3$ | $OCH_3$ | N | |
| 4-$OCH_2CH_2CH_3$ | $CH_3$ | $CH_3$ | N | |
| 4-$OCH_2CH_2CH_3$ | $OCH_3$ | $CH_3$ | N | |
| 4-$OCH_2CH_2CH_3$ | $OCH_3$ | $OCH_3$ | N | |

Table Ib (continued)

| R | X | Y | Z | m.p.(°C) |
|---|---|---|---|---|
| 4-$SO_2N(CH_3)_2$ | $CH_3$ | $CH_3$ | CH | |
| 4-$SO_2N(CH_3)_2$ | $CH_3$ | $OCH_3$ | CH | |
| 4-$SO_2N(CH_3)_2$ | $OCH_3$ | $OCH_3$ | CH | |
| 4-$SO_2N(C_2H_5)_2$ | $CH_3$ | $CH_3$ | CH | |
| 4-$SO_2N(C_2H_5)_2$ | $CH_3$ | $OCH_3$ | CH | |
| 4-$SO_2N(C_2H_5)_2$ | $OCH_3$ | $OCH_3$ | CH | |
| 4-$SO_2N(CH_3)C_2H_5$ | $CH_3$ | $CH_3$ | CH | |
| 4-$SO_2N(CH_3)C_2H_5$ | $CH_3$ | $OCH_3$ | CH | |
| 4-$SO_2N(CH_3)C_2H_5$ | $OCH_3$ | $OCH_3$ | CH | |
| 4-$SO_2N(CH_3)OCH_3$ | $CH_3$ | $CH_3$ | CH | |
| 4-$SO_2N(CH_3)OCH_3$ | $CH_3$ | $OCH_3$ | CH | |
| 4-$SO_2N(CH_3)OCH_3$ | $OCH_3$ | $OCH_3$ | CH | |
| 4-$OSO_2CF_3$ | $CH_3$ | $CH_3$ | CH | |
| 4-$OSO_2CF_3$ | $CH_3$ | $OCH_3$ | CH | |
| 4-$OSO_2CF_3$ | $OCH_3$ | $OCH_3$ | CH | |
| 4-$OSO_2CH_3$ | $CH_3$ | $CH_3$ | CH | |
| 4-$OSO_2CH_3$ | $CH_3$ | $OCH_3$ | CH | |
| 4-$OSO_2CH_3$ | $OCH_3$ | $OCH_3$ | CH | |
| 4-$OSO_2C_2H_5$ | $CH_3$ | $CH_3$ | CH | |
| 4-$OSO_2C_2H_5$ | $CH_3$ | $OCH_3$ | CH | |
| 4-$OSO_2C_2H_5$ | $OCH_3$ | $OCH_3$ | CH | |
| 4-$OSO_2$-n-$C_3H_7$ | $CH_3$ | $CH_3$ | CH | |
| 4-$OSO_2$-n-$C_3H_7$ | $CH_3$ | $OCH_3$ | CH | |
| 4-$OSO_2$-n-$C_3H_7$ | $OCH_3$ | $OCH_3$ | CH | |
| 4-$CO_2CH_3$ | $CH_3$ | $CH_3$ | CH | |
| 4-$CO_2CH_3$ | $CH_3$ | $OCH_3$ | CH | |
| 4-$CO_2CH_3$ | $OCH_3$ | $OCH_3$ | CH | |
| 4-$CO_2C_2H_5$ | $CH_3$ | $CH_3$ | CH | |
| 4-$CO_2C_2H_5$ | $CH_3$ | $OCH_3$ | CH | |
| 4-$CO_2C_2H_5$ | $OCH_3$ | $OCH_3$ | CH | |
| 4-$CO_2$-n-$C_3H_7$ | $CH_3$ | $CH_3$ | CH | |
| 4-$CO_2$-n-$C_3H_7$ | $CH_3$ | $OCH_3$ | CH | |

Table Ib (continued)

| R | X | Y | Z | m.p.(°C) |
|---|---|---|---|---|
| 4-$CO_2$-n-$C_3H_7$ | $OCH_3$ | $OCH_3$ | CH | |
| 4-$CO_2$-i-$C_3H_7$ | $CH_3$ | $CH_3$ | CH | |
| 4-$CO_2$-i-$C_3H_7$ | $CH_3$ | $OCH_3$ | CH | |
| 4-$CO_2$-i-$C_3H_7$ | $OCH_3$ | $OCH_3$ | CH | |
| 4-$CO_2CH_2CH=CH_2$ | $CH_3$ | $CH_3$ | CH | |
| 4-$CO_2CH_2CH=CH_2$ | $CH_3$ | $OCH_3$ | CH | |
| 4-$CO_2CH_2CH=CH_2$ | $OCH_3$ | $OCH_3$ | CH | |
| 4-$CO_2CH_2CH_2OCH_3$ | $CH_3$ | $CH_3$ | CH | |
| 4-$CO_2CH_2CH_2OCH_3$ | $CH_3$ | $OCH_3$ | CH | |
| 4-$CO_2CH_2CH_2OCH_3$ | $OCH_3$ | $OCH_3$ | CH | |
| 4-$CO_2CH_2CH_2Cl$ | $CH_3$ | $CH_3$ | CH | |
| 4-$CO_2CH_2CH_2Cl$ | $CH_3$ | $OCH_3$ | CH | |
| 4-$CO_2CH_2CH_2Cl$ | $OCH_3$ | $OCH_3$ | CH | |
| 4-$SO_2CH_3$ | $CH_3$ | $CH_3$ | CH | |
| 4-$SO_2CH_3$ | $CH_3$ | $OCH_3$ | CH | |
| 4-$SO_2CH_3$ | $OCH_3$ | $OCH_3$ | CH | |
| 6-$SO_2N(CH_3)_2$ | $CH_3$ | $CH_3$ | CH | |
| 6-$SO_2N(CH_3)_2$ | $CH_3$ | $OCH_3$ | CH | |
| 6-$SO_2N(CH_3)_2$ | $OCH_3$ | $OCH_3$ | CH | |
| 6-$SO_2N(C_2H_5)_2$ | $CH_3$ | $CH_3$ | CH | |
| 6-$SO_2N(C_2H_5)_2$ | $CH_3$ | $OCH_3$ | CH | |
| 6-$SO_2N(C_2H_5)_2$ | $OCH_3$ | $OCH_3$ | CH | |
| 6-$SO_2N(CH_3)C_2H_5$ | $CH_3$ | $CH_3$ | CH | |
| 6-$SO_2N(CH_3)C_2H_5$ | $CH_3$ | $OCH_3$ | CH | |
| 6-$SO_2N(CH_3)C_2H_5$ | $OCH_3$ | $OCH_3$ | CH | |
| 6-$SO_2N(CH_3)OCH_3$ | $CH_3$ | $CH_3$ | CH | |
| 6-$SO_2N(CH_3)OCH_3$ | $CH_3$ | $OCH_3$ | CH | |
| 6-$SO_2N(CH_3)OCH_3$ | $OCH_3$ | $OCH_3$ | CH | |
| 6-$OSO_2CF_3$ | $CH_3$ | $CH_3$ | CH | |
| 6-$OSO_2CF_3$ | $CH_3$ | $OCH_3$ | CH | |
| 6-$OSO_2CF_3$ | $OCH_3$ | $OCH_3$ | CH | |
| 6-$OSO_2CH_3$ | $CH_3$ | $CH_3$ | CH | |

Table Ib (continued)

| R | X | Y | Z | m.p.(°C) |
|---|---|---|---|---|
| 6-$OSO_2CH_3$ | $CH_3$ | $OCH_3$ | CH | |
| 6-$OSO_2CH_3$ | $OCH_3$ | $OCH_3$ | CH | |
| 6-$OSO_2C_2H_5$ | $CH_3$ | $CH_3$ | CH | |
| 6-$OSO_2C_2H_5$ | $CH_3$ | $OCH_3$ | CH | |
| 6-$OSO_2C_2H_5$ | $OCH_3$ | $OCH_3$ | CH | |
| 6-$OSO_2$-n-$C_3H_7$ | $CH_3$ | $CH_3$ | CH | |
| 6-$OSO_2$-n-$C_3H_7$ | $CH_3$ | $OCH_3$ | CH | |
| 6-$OSO_2$-n-$C_3H_7$ | $OCH_3$ | $OCH_3$ | CH | |
| 6-$CO_2CH_3$ | $CH_3$ | $CH_3$ | CH | |
| 6-$CO_2CH_3$ | $CH_3$ | $OCH_3$ | CH | |
| 6-$CO_2CH_3$ | $OCH_3$ | $OCH_3$ | CH | |
| 6-$CO_2C_2H_5$ | $CH_3$ | $CH_3$ | CH | |
| 6-$CO_2C_2H_5$ | $CH_3$ | $OCH_3$ | CH | |
| 6-$CO_2C_2H_5$ | $OCH_3$ | $OCH_3$ | CH | |
| 6-$CO_2$-n-$C_3H_7$ | $CH_3$ | $CH_3$ | CH | |
| 6-$CO_2$-n-$C_3H_7$ | $CH_3$ | $OCH_3$ | CH | |
| 6-$CO_2$-n-$C_3H_7$ | $OCH_3$ | $OCH_3$ | CH | |
| 6-$CO_2$-i-$C_3H_7$ | $CH_3$ | $CH_3$ | CH | |
| 6-$CO_2$-i-$C_3H_7$ | $CH_3$ | $OCH_3$ | CH | |
| 6-$CO_2$-i-$C_3H_7$ | $OCH_3$ | $OCH_3$ | CH | |
| 6-$CO_2CH_2CH{=}CH_2$ | $CH_3$ | $CH_3$ | CH | |
| 6-$CO_2CH_2CH{=}CH_2$ | $CH_3$ | $OCH_3$ | CH | |
| 6-$CO_2CH_2CH{=}CH_2$ | $OCH_3$ | $OCH_3$ | CH | |
| 6-$CO_2CH_2CH_2OCH_3$ | $CH_3$ | $CH_3$ | CH | |
| 6-$CO_2CH_2CH_2OCH_3$ | $CH_3$ | $OCH_3$ | CH | |
| 6-$CO_2CH_2CH_2OCH_3$ | $OCH_3$ | $OCH_3$ | CH | |
| 6-$CO_2CH_2CH_2Cl$ | $CH_3$ | $CH_3$ | CH | |
| 6-$CO_2CH_2CH_2Cl$ | $CH_3$ | $OCH_3$ | CH | |
| 6-$CO_2CH_2CH_2Cl$ | $OCH_3$ | $OCH_3$ | CH | |
| 6-$SO_2CH_3$ | $CH_3$ | $CH_3$ | CH | |
| 6-$SO_2CH_3$ | $CH_3$ | $OCH_3$ | CH | |
| 6-$SO_2CH_3$ | $OCH_3$ | $OCH_3$ | CH | |

Table Ib (continued)

| R | X | Y | Z | m.p.(°C) |
|---|---|---|---|---|
| 4-$SO_2N(CH_3)_2$ | $CH_3$ | $CH_3$ | N | |
| 4-$SO_2N(CH_3)_2$ | $CH_3$ | $OCH_3$ | N | |
| 4-$SO_2N(CH_3)_2$ | $OCH_3$ | $OCH_3$ | N | |
| 4-$SO_2N(C_2H_5)_2$ | $CH_3$ | $CH_3$ | N | |
| 4-$SO_2N(C_2H_5)_2$ | $CH_3$ | $OCH_3$ | N | |
| 4-$SO_2N(C_2H_5)_2$ | $OCH_3$ | $OCH_3$ | N | |
| 4-$SO_2N(CH_3)C_2H_5$ | $CH_3$ | $CH_3$ | N | |
| 4-$SO_2N(CH_3)C_2H_5$ | $CH_3$ | $OCH_3$ | N | |
| 4-$SO_2N(CH_3)C_2H_5$ | $OCH_3$ | $OCH_3$ | N | |
| 4-$SO_2N(CH_3)OCH_3$ | $CH_3$ | $CH_3$ | N | |
| 4-$SO_2N(CH_3)OCH_3$ | $CH_3$ | $OCH_3$ | N | |
| 4-$SO_2N(CH_3)OCH_3$ | $OCH_3$ | $OCH_3$ | N | |
| 4-$OSO_2CF_3$ | $CH_3$ | $CH_3$ | N | |
| 4-$OSO_2CF_3$ | $CH_3$ | $OCH_3$ | N | |
| 4-$OSO_2CF_3$ | $OCH_3$ | $OCH_3$ | N | |
| 4-$OSO_2CH_3$ | $CH_3$ | $CH_3$ | N | |
| 4-$OSO_2CH_3$ | $CH_3$ | $OCH_3$ | N | |
| 4-$OSO_2CH_3$ | $OCH_3$ | $OCH_3$ | N | |
| 4-$OSO_2C_2H_5$ | $CH_3$ | $CH_3$ | N | |
| 4-$OSO_2C_2H_5$ | $CH_3$ | $OCH_3$ | N | |
| 4-$OSO_2C_2H_5$ | $OCH_3$ | $OCH_3$ | N | |
| 4-$OSO_2$-n-$C_3H_7$ | $CH_3$ | $CH_3$ | N | |
| 4-$OSO_2$-n-$C_3H_7$ | $CH_3$ | $OCH_3$ | N | |
| 4-$OSO_2$-n-$C_3H_7$ | $OCH_3$ | $OCH_3$ | N | |
| 4-$CO_2CH_3$ | $CH_3$ | $CH_3$ | N | |
| 4-$CO_2CH_3$ | $CH_3$ | $OCH_3$ | N | |
| 4-$CO_2CH_3$ | $OCH_3$ | $OCH_3$ | N | |
| 4-$CO_2C_2H_5$ | $CH_3$ | $CH_3$ | N | |
| 4-$CO_2C_2H_5$ | $CH_3$ | $OCH_3$ | N | |
| 4-$CO_2C_2H_5$ | $OCH_3$ | $OCH_3$ | N | |
| 4-$CO_2$-n-$C_3H_7$ | $CH_3$ | $CH_3$ | N | |
| 4-$CO_2$-n-$C_3H_7$ | $CH_3$ | $OCH_3$ | N | |

Table Ib (continued)

| R | X | Y | Z | m.p.(°C) |
|---|---|---|---|---|
| 4-$CO_2$-n-$C_3H_7$ | $OCH_3$ | $OCH_3$ | N | |
| 4-$CO_2$-i-$C_3H_7$ | $CH_3$ | $CH_3$ | N | |
| 4-$CO_2$-i-$C_3H_7$ | $CH_3$ | $OCH_3$ | N | |
| 4-$CO_2$-i-$C_3H_7$ | $OCH_3$ | $OCH_3$ | N | |
| 4-$CO_2CH_2CH=CH_2$ | $CH_3$ | $CH_3$ | N | |
| 4-$CO_2CH_2CH=CH_2$ | $CH_3$ | $OCH_3$ | N | |
| 4-$CO_2CH_2CH=CH_2$ | $OCH_3$ | $OCH_3$ | N | |
| 4-$CO_2CH_2CH_2OCH_3$ | $CH_3$ | $CH_3$ | N | |
| 4-$CO_2CH_2CH_2OCH_3$ | $CH_3$ | $OCH_3$ | N | |
| 4-$CO_2CH_2CH_2OCH_3$ | $OCH_3$ | $OCH_3$ | N | |
| 4-$CO_2CH_2CH_2Cl$ | $CH_3$ | $CH_3$ | N | |
| 4-$CO_2CH_2CH_2Cl$ | $CH_3$ | $OCH_3$ | N | |
| 4-$CO_2CH_2CH_2Cl$ | $OCH_3$ | $OCH_3$ | N | |
| 4-$SO_2CH_3$ | $CH_3$ | $CH_3$ | N | |
| 4-$SO_2CH_3$ | $CH_3$ | $OCH_3$ | N | |
| 4-$SO_2CH_3$ | $OCH_3$ | $OCH_3$ | N | |
| 6-$SO_2N(CH_3)_2$ | $CH_3$ | $CH_3$ | N | |
| 6-$SO_2N(CH_3)_2$ | $CH_3$ | $OCH_3$ | N | |
| 6-$SO_2N(CH_3)_2$ | $OCH_3$ | $OCH_3$ | N | |
| 6-$SO_2N(C_2H_5)_2$ | $CH_3$ | $CH_3$ | N | |
| 6-$SO_2N(C_2H_5)_2$ | $CH_3$ | $OCH_3$ | N | |
| 6-$SO_2N(C_2H_5)_2$ | $OCH_3$ | $OCH_3$ | N | |
| 6-$SO_2N(CH_3)C_2H_5$ | $CH_3$ | $CH_3$ | N | |
| 6-$SO_2N(CH_3)C_2H_5$ | $CH_3$ | $OCH_3$ | N | |
| 6-$SO_2N(CH_3)C_2H_5$ | $OCH_3$ | $OCH_3$ | N | |
| 6-$SO_2N(CH_3)OCH_3$ | $CH_3$ | $CH_3$ | N | |
| 6-$SO_2N(CH_3)OCH_3$ | $CH_3$ | $OCH_3$ | N | |
| 6-$SO_2N(CH_3)OCH_3$ | $OCH_3$ | $OCH_3$ | N | |
| 6-$OSO_2CF_3$ | $CH_3$ | $CH_3$ | N | |
| 6-$OSO_2CF_3$ | $CH_3$ | $OCH_3$ | N | |
| 6-$OSO_2CF_3$ | $OCH_3$ | $OCH_3$ | N | |
| 6-$OSO_2CH_3$ | $CH_3$ | $CH_3$ | N | |

Table Ib (continued)

| R | X | Y | Z | m.p.(°C) |
|---|---|---|---|---|
| 6-$OSO_2CH_3$ | $CH_3$ | $OCH_3$ | N | |
| 6-$OSO_2CH_3$ | $OCH_3$ | $OCH_3$ | N | |
| 6-$OSO_2C_2H_5$ | $CH_3$ | $CH_3$ | N | |
| 6-$OSO_2C_2H_5$ | $CH_3$ | $OCH_3$ | N | |
| 6-$OSO_2C_2H_5$ | $OCH_3$ | $OCH_3$ | N | |
| 6-$OSO_2$-n-$C_3H_7$ | $CH_3$ | $CH_3$ | N | |
| 6-$OSO_2$-n-$C_3H_7$ | $CH_3$ | $OCH_3$ | N | |
| 6-$OSO_2$-n-$C_3H_7$ | $OCH_3$ | $OCH_3$ | N | |
| 6-$CO_2CH_3$ | $CH_3$ | $CH_3$ | N | |
| 6-$CO_2CH_3$ | $CH_3$ | $OCH_3$ | N | |
| 6-$CO_2CH_3$ | $OCH_3$ | $OCH_3$ | N | |
| 6-$CO_2C_2H_5$ | $CH_3$ | $CH_3$ | N | |
| 6-$CO_2C_2H_5$ | $CH_3$ | $OCH_3$ | N | |
| 6-$CO_2C_2H_5$ | $OCH_3$ | $OCH_3$ | N | |
| 6-$CO_2$-n-$C_3H_7$ | $CH_3$ | $CH_3$ | N | |
| 6-$CO_2$-n-$C_3H_7$ | $CH_3$ | $OCH_3$ | N | |
| 6-$CO_2$-n-$C_3H_7$ | $OCH_3$ | $OCH_3$ | N | |
| 6-$CO_2$-i-$C_3H_7$ | $CH_3$ | $CH_3$ | N | |
| 6-$CO_2$-i-$C_3H_7$ | $CH_3$ | $OCH_3$ | N | |
| 6-$CO_2$-i-$C_3H_7$ | $OCH_3$ | $OCH_3$ | N | |
| 6-$CO_2CH_2CH=CH_2$ | $CH_3$ | $CH_3$ | N | |
| 6-$CO_2CH_2CH=CH_2$ | $CH_3$ | $OCH_3$ | N | |
| 6-$CO_2CH_2CH=CH_2$ | $OCH_3$ | $OCH_3$ | N | |
| 6-$CO_2CH_2CH_2OCH_3$ | $CH_3$ | $CH_3$ | N | |
| 6-$CO_2CH_2CH_2OCH_3$ | $CH_3$ | $OCH_3$ | N | |
| 6-$CO_2CH_2CH_2OCH_3$ | $OCH_3$ | $OCH_3$ | N | |
| 6-$CO_2CH_2CH_2Cl$ | $CH_3$ | $CH_3$ | N | |
| 6-$CO_2CH_2CH_2Cl$ | $CH_3$ | $OCH_3$ | N | |
| 6-$CO_2CH_2CH_2Cl$ | $OCH_3$ | $OCH_3$ | N | |
| 6-$SO_2CH_3$ | $CH_3$ | $CH_3$ | N | |
| 6-$SO_2CH_3$ | $CH_3$ | $OCH_3$ | N | |
| 6-$SO_2CH_3$ | $OCH_3$ | $OCH_3$ | N | |

Table Ib (continued)

| R | X | Y | Z | m.p.(°C) |
|---|---|---|---|---|
| H | $CH_3$ | $CH(OCH_3)_2$ | N | |
| H | $OCH_3$ | $CH(OCH_3)_2$ | N | |
| H | $CH_3$ | 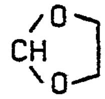 | N | |
| H | $OCH_3$ | 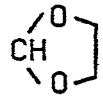 | N | |
| H | Cl | $OCH_3$ | CH | |
| H | Cl | $NH_2$ | CH | |
| H | Cl | $NHCH_3$ | CH | |
| H | Cl | $N(CH_3)_2$ | CH | |
| 4-Cl | $CH_3$ | $CH(OCH_3)_2$ | CH | |
| 4-Cl | $CH_3$ | $CH(OCH_3)_2$ | N | |
| 4-Cl | $OCH_3$ | $CH(OCH_3)_2$ | CH | |
| 4-Cl | $OCH_3$ | $CH(OCH_3)_2$ | N | |
| 4-Cl | $CH_3$ | 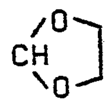 | CH | |
| 4-Cl | $CH_3$ | 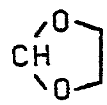 | N | |
| 4-Cl | $OCH_3$ | 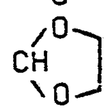 | CH | |
| 4-Cl | $OCH_3$ | 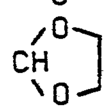 | N | |
| 4-Cl | Cl | $OCH_3$ | CH | |
| 6-Cl | $CH_3$ | $CH(OCH_3)_2$ | CH | |
| 6-Cl | Cl | $OCH_3$ | CH | |
| 6-Cl | $OCH_3$ | $CH(OCH_3)_2$ | N | |
| 4-$NO_2$ | $CH_3$ | $CH(OCH_3)_2$ | N | |
| 4-$NO_2$ | $CH_3$ | $CH(OCH_3)_2$ | CH | |
| 4-$NO_2$ | $OCH_3$ | $CH(OCH_3)_2$ | N | |
| 4-$NO_2$ | $OCH_3$ | $CH(OCH_3)_2$ | CH | |
| 4-$NO_2$ | Cl | $OCH_3$ | CH | |
| 4-Br | $CH_3$ | $CH(OCH_3)_2$ | CH | |

Table Ib (continued)

| R | X | Y | Z | m.p.(°C) |
|---|---|---|---|---|
| 4-Br | $CH_3$ | $CH(OCH_3)_2$ | N | |
| 4-Br | $OCH_3$ | $CH(OCH_3)_2$ | CH | |
| 4-Br | $OCH_3$ | $CH(OCH_3)_2$ | N | |
| 4-Br | Cl | $OCH_3$ | CH | |
| 4-$OCH_3$ | Cl | $OCH_3$ | CH | |
| 4-$OC_2H_5$ | Cl | $OCH_3$ | CH | |
| 4-$OCH(CH_3)_2$ | Cl | $OCH_3$ | CH | |
| 4-$SO_2N(CH_3)_2$ | Cl | $OCH_3$ | CH | |
| 4-$SO_2N(C_2H_5)_2$ | Cl | $OCH_3$ | CH | |
| 4-$OSO_2CH_3$ | Cl | $OCH_3$ | CH | |
| 4-$OSO_2CF_3$ | Cl | $OCH_3$ | CH | |
| 4-$SO_2CH_3$ | Cl | $OCH_3$ | CH | |
| 4-$SO_2C_2H_5$ | Cl | $OCH_3$ | CH | |
| 4-$SO_2CH_2CH_2CH_3$ | Cl | $OCH_3$ | CH | |
| 4-$OSO_2CH_2CH_2CH_3$ | Cl | $OCH_3$ | CH | |
| 4-$CO_2CH_3$ | Cl | $OCH_3$ | CH | |
| 4-$CO_2C_2H_5$ | Cl | $OCH_3$ | CH | |
| 4-$CO_2CH(CH_3)_2$ | Cl | $OCH_3$ | CH | |
| 4-$CO_2CH_2CH_2OCH_3$ | Cl | $OCH_3$ | CH | |
| 4-$OCF_2CF_2H$ | Cl | $OCH_3$ | CH | |
| 4-$C_2H_5$ | Cl | $OCH_3$ | CH | |
| 4-$CH(CH_3)_2$ | Cl | $OCH_3$ | CH | |
| 4-$CH_2CH_2CH_3$ | Cl | $OCH_3$ | CH | |
| 4-$SCH_3$ | Cl | $OCH_3$ | CH | |
| 4-$SC_2H_5$ | Cl | $OCH_3$ | CH | |
| 4-$SCH_2CH_2CH_3$ | Cl | $OCH_3$ | CH | |
| 6-Br | Cl | $OCH_3$ | CH | |
| 6-$OCH_3$ | Cl | $OCH_3$ | CH | |
| 6-$OC_2H_5$ | Cl | $OCH_3$ | CH | |
| 6-$OCH(CH_3)_2$ | Cl | $OCH_3$ | CH | |
| 6-$SO_2N(CH_3)_2$ | Cl | $OCH_3$ | CH | |
| 6-$SO_2N(C_2H_5)_2$ | Cl | $OCH_3$ | CH | |

Table Ib (continued)

| R | X | Y | Z | m.p.(°C) |
|---|---|---|---|---|
| 6-$OSO_2CH_3$ | Cl | $OCH_3$ | CH | |
| 6-$OSO_2CF_3$ | Cl | $OCH_3$ | CH | |
| 6-$SO_2CH_3$ | Cl | $OCH_3$ | CH | |
| 6-$SO_2C_2H_5$ | Cl | $OCH_3$ | CH | |
| 6-$SO_2CH_2CH_2CH_3$ | Cl | $OCH_3$ | CH | |
| 6-$OSO_2CH_2CH_2CH_3$ | Cl | $OCH_3$ | CH | |
| 6-$CO_2CH_3$ | Cl | $OCH_3$ | CH | |
| 6-$CO_2C_2H_5$ | Cl | $OCH_3$ | CH | |
| 6-$CO_2CH(CH_3)_2$ | Cl | $OCH_3$ | CH | |
| 6-$CO_2CH_2CH_2OCH_3$ | Cl | $OCH_3$ | CH | |
| 6-$OCF_2CF_2H$ | Cl | $OCH_3$ | CH | |
| 6-$SCF_2H$ | Cl | $OCH_3$ | CH | |
| 6-$OCF_2H$ | Cl | $OCH_3$ | CH | |
| 6-$OCF_3$ | Cl | $OCH_3$ | CH | |
| 6-$C_2H_5$ | Cl | $OCH_3$ | CH | |
| 6-$CH(CH_3)_2$ | Cl | $OCH_3$ | CH | |
| 6-$SCH_3$ | Cl | $OCH_3$ | CH | |
| 6-$SC_2H_5$ | Cl | $OCH_3$ | CH | |
| 4-$SCF_2H$ | Cl | $OCH_3$ | CH | |
| 4-$OCF_2H$ | Cl | $OCH_3$ | CH | |
| 4-$OCF_3$ | Cl | $OCH_3$ | CH | |

Table Ic

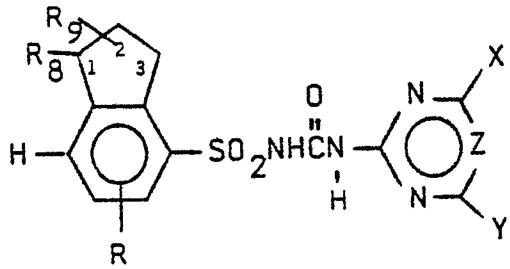

| R | $R_9$ | $R_8$ | X | Y | Z | m.p.(°C) |
|---|---|---|---|---|---|---|
| H | 1-$CH_3$ | H | $CH_3$ | $CH_3$ | CH | |
| H | 1-$CH_3$ | H | $OCH_3$ | $OCH_3$ | CH | |
| H | 1-$CH_3$ | H | $OCH_3$ | $CH_3$ | CH | |
| H | 1-$CH_3$ | H | $OCH_3$ | $SCH_3$ | CH | |
| H | 2-$CH_3$ | $CH_3$ | $OCH_3$ | $CH_3$ | CH | |
| H | 2-$CH_3$ | $CH_3$ | $OCH_3$ | $OCH_3$ | CH | |
| H | 2-$CH_3$ | H | $CH_3$ | $CH_3$ | CH | |
| H | 3-$CH_3$ | $CH_3$ | $CH_3$ | $CH_3$ | CH | |
| H | 3-$CH_3$ | H | $OCH_3$ | $CH_3$ | CH | |
| H | 3-$CH_3$ | H | $OCH_3$ | $OCH_3$ | CH | |
| 5-Cl | 1-$CH_3$ | H | $CH_3$ | $CH_3$ | CH | |
| 5-Cl | 1-$CH_3$ | H | $OCH_3$ | $OCH_3$ | CH | |
| 5-Cl | 1-$CH_3$ | H | $OCH_3$ | $CH_3$ | CH | |
| 6-Cl | 1-$CH_3$ | H | $CH_3$ | $CH_3$ | CH | |
| 6-Cl | 1-$CH_3$ | H | $OCH_3$ | $OCH_3$ | CH | |
| 6-Cl | 1-$CH_3$ | H | $OCH_3$ | $CH_3$ | CH | |
| 5-$NO_2$ | 1-$CH_3$ | H | $CH_3$ | $CH_3$ | CH | |
| 5-$NO_2$ | 1-$CH_3$ | H | $OCH_3$ | $OCH_3$ | CH | |
| 5-$NO_2$ | 1-$CH_3$ | H | $OCH_3$ | $CH_3$ | CH | |
| 5-Br | 1-$CH_3$ | H | $CH_3$ | $CH_3$ | CH | |
| 5-Br | 1-$CH_3$ | H | $OCH_3$ | $OCH_3$ | CH | |
| 5-Br | 1-$CH_3$ | H | $OCH_3$ | $CH_3$ | CH | |
| 5-$OCH_3$ | 1-$CH_3$ | H | $CH_3$ | $CH_3$ | CH | |
| 5-$OCH_3$ | 1-$CH_3$ | H | $OCH_3$ | $OCH_3$ | CH | |
| 5-$OCH_3$ | 1-$CH_3$ | H | $OCH_3$ | $CH_3$ | CH | |
| H | 1-$CH_3$ | H | $OCH_3$ | $SCH_3$ | N | |

Table Ic (continued)

| R | $R_9$ | $R_8$ | X | Y | Z | m.p.(°C) |
|---|---|---|---|---|---|---|
| 5-$CO_2CH_3$ | 1-$CH_3$ | H | $CH_3$ | $CH_3$ | CH | |
| 5-$CO_2CH_3$ | 1-$CH_3$ | H | $OCH_3$ | $CH_3$ | CH | |
| 5-$CO_2CH_3$ | 1-$CH_3$ | H | $OCH_3$ | $OCH_3$ | CH | |
| 5-$CO_2CH_3$ | 1-$CH_3$ | H | Cl | $OCH_3$ | CH | |
| 5-$CO_2CH_3$ | 1-$CH_3$ | H | Cl | $N(CH_3)_2$ | CH | |
| 5-$CO_2C_2H_5$ | 1-$CH_3$ | H | Cl | $OCH_3$ | CH | |
| 5-$CO_2C_2H_5$ | 1-$CH_3$ | H | $CH_3$ | $OCH_3$ | N | |
| 5-$CO_2C_2H_5$ | 1-$CH_3$ | H | $OCH_3$ | $CH_3$ | N | |
| 5-$CO_2C_2H_5$ | 1-$CH_3$ | H | $OCH_3$ | $OCH_3$ | N | |
| 5-$SO_2CH_3$ | 1-$CH_3$ | H | $OCH_3$ | $CH_3$ | CH | |
| 5-$SO_2CH_3$ | 1-$CH_3$ | H | $OCH_3$ | $CH_3$ | N | |
| 5-$SO_2CH_3$ | 1-$CH_3$ | H | $OCH_3$ | $OCH_3$ | CH | |
| 5-$SO_2CH_3$ | 1-$CH_3$ | $CH_3$ | $OCH_3$ | $OCH_3$ | N | |
| 5-$SO_2CH_3$ | 1-$CH_3$ | H | Cl | $OCH_3$ | CH | |
| 5-$OSO_2CF_3$ | 1-$CH_3$ | $CH_3$ | $OCH_3$ | $OCH_3$ | CH | |
| 5-$OSO_2CF_3$ | 1-$CH_3$ | H | $OCH_3$ | $CH_3$ | N | |
| 5-$OSO_2CH_3$ | 1-$CH_3$ | $CH_3$ | Cl | $OCH_3$ | CH | |
| 5-$SO_2N(CH_3)_2$ | 1-$CH_3$ | $CH_3$ | Cl | $OCH_3$ | CH | |
| 5-$SCH_3$ | 1-$CH_3$ | $CH_3$ | $CH_3$ | $OCH_3$ | CH | |
| 5-$SCH_3$ | 1-$CH_3$ | $CH_3$ | $CH_3$ | $CH_3$ | CH | |
| 5-$SCH_3$ | 1-$CH_3$ | $CH_3$ | $OCH_3$ | $OCH_3$ | CH | |
| 5-$SO_2CH_3$ | 3-$CH_3$ | $CH_3$ | $OCH_3$ | $OCH_3$ | CH | |
| 5-$SO_2CH_3$ | 3-$CH_3$ | $CH_3$ | $OCH_3$ | $CH_3$ | CH | |
| 5-$SO_2CH_3$ | 3-$CH_3$ | $CH_3$ | $CH_3$ | $CH_3$ | CH | |
| 5-$SCF_2H$ | 3-$CH_3$ | H | $CH_3$ | $CH_3$ | CH | |
| 5-$SCF_2H$ | 3-$CH_3$ | H | $OCH_3$ | $CH_3$ | CH | |
| 5-$SCF_2H$ | 3-$CH_3$ | H | $OCH_3$ | $OCH_3$ | CH | |
| 5-$CO_2CH_3$ | 2-$CH_3$ | $CH_3$ | $OCH_3$ | $CH_3$ | CH | |
| 5-$CO_2CH_3$ | 2-$CH_3$ | $CH_3$ | Cl | $OCH_3$ | CH | |
| 5-$CO_2CH_3$ | 2-$CH_3$ | $CH_3$ | $OCH_3$ | $OCH_3$ | CH | |
| 5-$CO_2CH_3$ | 2-$CH_3$ | $CH_3$ | $CH_3$ | $OCH_3$ | N | |
| 5-$CO_2CH_3$ | 2-$CH_3$ | $CH_3$ | $OCH_3$ | $OCH_3$ | N | |

Table Id

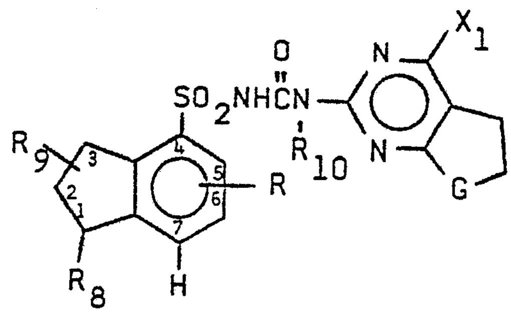

| R | $R_8$ | $R_9$ | $R_{10}$ | G | $X_1$ | m.p.(°C) |
|---|---|---|---|---|---|---|
| H | H | H | H | O | $CH_3$ | |
| 5-Cl | H | H | H | O | $CH_3$ | |
| 5-Br | H | H | H | O | $CH_3$ | |
| 5-$OCH_3$ | H | H | H | O | $CH_3$ | |
| 5-$SO_2CH_3$ | H | H | H | O | $CH_3$ | |
| 5-$SCH_3$ | H | H | H | O | $CH_3$ | |
| 5-$CO_2CH_3$ | H | H | H | O | $CH_3$ | |
| 5-$SO_2N(CH_3)_2$ | H | H | H | O | $CH_3$ | |
| 5-$SO_2N(OCH_3)CH_3$ | H | H | H | O | $CH_3$ | |
| 5-$OSO_2CH_3$ | H | H | H | O | $CH_3$ | |
| 5-$OSO_2CF_3$ | H | H | H | O | $CH_3$ | |
| 5-$CH_3$ | $CH_3$ | H | H | O | $CH_3$ | |
| 6-Cl | $CH_3$ | H | H | O | $CH_3$ | |
| 6-$CH_3$ | $CH_3$ | H | H | O | $CH_3$ | |
| 6-$OCH_3$ | $CH_3$ | H | H | O | $CH_3$ | |
| 6-$NO_2$ | $CH_3$ | H | H | O | $CH_3$ | |
| 6-Br | $CH_3$ | H | H | O | $CH_3$ | |
| H | $CH_3$ | H | H | O | $OCH_3$ | |
| 5-Cl | Cl | H | H | O | $OCH_3$ | |
| 5-Br | Cl | H | H | O | $OCH_3$ | |
| 5-$CO_2CH_3$ | Cl | H | H | O | $OCH_3$ | |
| 5-$SO_2CH_3$ | $CH_3$ | H | H | O | $OCH_3$ | |
| 5-$SCH_3$ | $CH_3$ | H | H | O | $OCH_3$ | |
| 5-$OCH_3$ | Cl | H | H | O | $OCH_3$ | |

Table Id (continued)

| R | $R_8$ | $R_9$ | $R_{10}$ | G | $X_1$ | m.p.(°C) |
|---|---|---|---|---|---|---|
| 5-$SO_2N(CH_3)_2$ | $CH_3$ | H | H | O | $OCH_3$ | |
| 5-$OSO_2CH_3$ | $CH_3$ | H | H | O | $OCH_3$ | |
| 5-$OSO_2CF_3$ | Cl | H | H | O | $OCH_3$ | |
| 6-Cl | $CH_3$ | H | H | O | $OCH_3$ | |
| 6-$CH_3$ | H | H | H | O | $OCH_3$ | |
| 6-$OCH_3$ | H | H | H | O | $OCH_3$ | |
| 6-$NO_2$ | H | H | H | O | $OCH_3$ | |
| 6-Br | H | H | H | O | $OCH_3$ | |
| H | H | H | H | $CH_2$ | $CH_3$ | |
| 5-Cl | H | H | H | $CH_2$ | $CH_3$ | |
| 5-Br | H | H | H | $CH_2$ | $CH_3$ | |
| 5-$OCH_3$ | H | H | H | $CH_2$ | $CH_3$ | |
| 5-$SO_2CH_3$ | H | H | H | $CH_2$ | $CH_3$ | |
| 5-$SCH_3$ | H | H | H | $CH_2$ | $CH_3$ | |
| 5-$SO_2N(CH_3)_2$ | H | H | H | $CH_2$ | $CH_3$ | |
| 5-$SO_2N(OCH_3)CH_3$ | H | H | H | $CH_2$ | $CH_3$ | |
| 5-$OSO_2CH_3$ | H | H | H | $CH_2$ | $CH_3$ | |
| 5-$OSO_2CF_3$ | H | H | H | $CH_2$ | $CH_3$ | |
| 5-$CH_3$ | H | H | H | $CH_2$ | $CH_3$ | |
| 6-Cl | $CH_3$ | H | H | $CH_2$ | $CH_3$ | |
| 6-$CH_3$ | $CH_3$ | H | H | $CH_2$ | $CH_3$ | |
| 6-$OCH_3$ | $CH_3$ | H | H | $CH_2$ | $CH_3$ | |
| 6-$NO_2$ | $CH_3$ | H | H | $CH_2$ | $CH_3$ | |
| 6-Br | H | H | H | $CH_2$ | $CH_3$ | |
| H | H | H | H | $CH_2$ | $OCH_3$ | |
| 5-Cl | H | H | H | $CH_2$ | $OCH_3$ | |
| 5-Br | H | H | H | $CH_2$ | $OCH_3$ | |
| 5-$OCH_3$ | H | H | H | $CH_2$ | $OCH_3$ | |
| 5-$SO_2CH_3$ | H | H | H | $CH_2$ | $OCH_3$ | |
| 5-$SCH_3$ | H | H | H | $CH_2$ | $OCH_3$ | |
| 5-$CO_2CH_3$ | H | H | H | $CH_2$ | $OCH_3$ | |
| 5-$OSO_2CH_3$ | H | H | H | $CH_2$ | $OCH_3$ | |

Table Id (continued)

| R | $R_8$ | $R_9$ | $R_{10}$ | G | $X_1$ | m.p.(°C) |
|---|---|---|---|---|---|---|
| 5-$CH_3$ | H | H | H | $CH_2$ | $OCH_3$ | |
| 6-Cl | H | H | H | $CH_2$ | $OCH_3$ | |
| 6-$CH_3$ | H | H | H | $CH_2$ | $OCH_3$ | |
| 6-$OCH_3$ | H | H | H | $CH_2$ | $OCH_3$ | |
| 6-$NO_2$ | H | H | H | $CH_2$ | $OCH_3$ | |
| 6-Br | H | H | H | $CH_2$ | $OCH_3$ | |
| H | H | 1-$CH_3$ | H | O | $CH_3$ | |
| H | H | 1-$CH_3$ | H | O | $OCH_3$ | |
| H | H | 1-$CH_3$ | H | $CH_2$ | $CH_3$ | |
| H | H | 1-$CH_3$ | H | $CH_2$ | $OCH_3$ | |
| H | H | 2-$CH_3$ | H | O | $CH_3$ | |
| H | H | 2-$CH_3$ | H | O | $OCH_3$ | |
| H | H | 2-$CH_3$ | H | $CH_2$ | $CH_3$ | |
| H | H | 2-$CH_3$ | H | $CH_2$ | $OCH_3$ | |
| H | H | 3-$CH_3$ | H | O | $CH_3$ | |
| H | H | 3-$CH_3$ | H | O | $OCH_3$ | |
| H | H | 3-$CH_3$ | H | $CH_2$ | $CH_3$ | |
| H | H | 3-$CH_3$ | H | $CH_2$ | $OCH_3$ | |
| H | H | H | $CH_3$ | O | $CH_3$ | |
| H | H | H | $CH_3$ | O | $OCH_3$ | |
| H | H | H | $CH_3$ | $CH_2$ | $CH_3$ | |
| H | H | H | $CH_3$ | $CH_2$ | $OCH_3$ | |

Table Ie

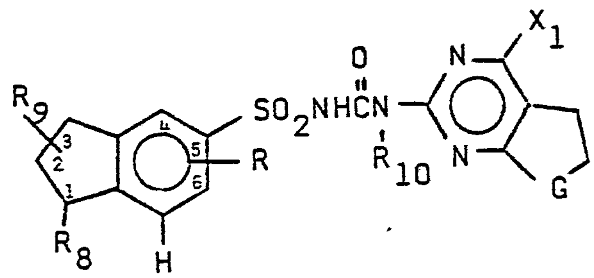

| R | $R_8$ | $R_9$ | $R_{10}$ | G | $X_1$ | m.p.(°C) |
|---|---|---|---|---|---|---|
| H | H | H | H | O | $CH_3$ | |
| 4-Cl | H | H | H | O | $CH_3$ | |
| 4-Br | H | H | H | O | $CH_3$ | |
| 4-$NO_2$ | H | H | H | O | $CH_3$ | |
| 4-$OCH_3$ | H | H | H | O | $CH_3$ | |
| 4-$SCH_3$ | H | H | H | O | $CH_3$ | |
| 4-$SO_2CH_3$ | H | H | H | O | $CH_3$ | |
| 4-$CO_2CH_3$ | H | H | H | O | $CH_3$ | |
| 4-$SO_2N(CH_3)_2$ | H | H | H | O | $CH_3$ | |
| 4-$OSO_2CH_3$ | H | H | H | O | $CH_3$ | |
| 6-Cl | H | H | H | O | $CH_3$ | |
| 6-Br | H | H | H | O | $CH_3$ | |
| 6-$NO_2$ | H | H | H | O | $CH_3$ | |
| 6-$OCH_3$ | H | H | H | O | $CH_3$ | |
| 6-$SCH_3$ | H | H | H | O | $CH_3$ | |
| 6-$SO_2CH_3$ | H | H | H | O | $CH_3$ | |
| 6-$CO_2CH_3$ | H | H | H | O | $CH_3$ | |
| 6-$SO_2N(CH_3)_2$ | H | H | H | O | $CH_3$ | |
| 6-$OSO_2CH_3$ | H | H | H | O | $CH_3$ | |
| H | H | H | H | O | $OCH_3$ | |
| 4-Cl | H | H | H | O | $OCH_3$ | |
| 4-Br | H | H | H | O | $OCH_3$ | |
| 4-$NO_2$ | H | H | H | O | $OCH_3$ | |
| 4-$OCH_3$ | H | H | H | O | $OCH_3$ | |
| 4-$SCH_3$ | H | H | H | O | $OCH_3$ | |
| 4-$SO_2CH_3$ | H | H | H | O | $OCH_3$ | |

Table Ie (continued)

| R | $R_8$ | $R_9$ | $R_{10}$ | G | $X_1$ | m.p.(°C) |
|---|---|---|---|---|---|---|
| 4-$CO_2CH_3$ | $CH_3$ | H | H | O | $OCH_3$ | |
| 4-$SO_2N(CH_3)_2$ | $CH_3$ | H | H | O | $OCH_3$ | |
| 4-$OSO_2CH_3$ | $CH_3$ | H | H | O | $OCH_3$ | |
| 6-Cl | $CH_3$ | H | H | O | $OCH_3$ | |
| 6-Br | $CH_3$ | H | H | O | $OCH_3$ | |
| 6-$NO_2$ | $CH_3$ | H | H | O | $OCH_3$ | |
| 6-$OCH_3$ | H | H | H | O | $OCH_3$ | |
| 6-$SCH_3$ | H | H | H | O | $OCH_3$ | |
| 6-$SO_2CH_3$ | H | H | H | O | $OCH_3$ | |
| 6-$CO_2CH_3$ | H | H | H | O | $OCH_3$ | |
| 6-$SO_2N(CH_3)_2$ | H | H | H | O | $OCH_3$ | |
| 6-$OSO_2CH_3$ | H | H | H | O | $OCH_3$ | |
| H | H | H | H | $CH_2$ | $CH_3$ | |
| 4-Cl | H | H | H | $CH_2$ | $CH_3$ | |
| 4-Br | H | H | H | $CH_2$ | $CH_3$ | |
| 4-$NO_2$ | H | H | H | $CH_2$ | $CH_3$ | |
| 4-$OCH_3$ | H | H | H | $CH_2$ | $CH_3$ | |
| 4-$SCH_3$ | H | H | H | $CH_2$ | $CH_3$ | |
| 4-$SO_2CH_3$ | H | H | H | $CH_2$ | $CH_3$ | |
| 4-$CO_2CH_3$ | H | H | H | $CH_2$ | $CH_3$ | |
| 4-$SO_2N(CH_3)_2$ | H | H | H | $CH_2$ | $CH_3$ | |
| 4-$OSO_2CH_3$ | H | H | H | $CH_2$ | $CH_3$ | |
| 6-Cl | H | H | H | $CH_2$ | $CH_3$ | |
| 6-Br | H | H | H | $CH_2$ | $CH_3$ | |
| 6-$NO_2$ | H | H | H | $CH_2$ | $CH_3$ | |
| 6-$OCH_3$ | H | H | H | $CH_2$ | $CH_3$ | |
| 6-$SCH_3$ | H | H | H | $CH_2$ | $CH_3$ | |
| 6-$SO_2CH_3$ | H | H | H | $CH_2$ | $CH_3$ | |
| 6-$CO_2CH_3$ | H | H | H | $CH_2$ | $CH_3$ | |
| 6-$SO_2N(CH_3)_2$ | H | H | H | $CH_2$ | $CH_3$ | |
| 6-$OSO_2CH_3$ | H | H | H | $CH_2$ | $CH_3$ | |
| H | H | H | H | $CH_2$ | $OCH_3$ | |

Table Ie (continued)

| R | $R_8$ | $R_9$ | $R_{10}$ | G | $X_1$ | m.p.(°C) |
|---|---|---|---|---|---|---|
| 4-Cl | H | H | H | $CH_2$ | $OCH_3$ | |
| 4-Br | H | H | H | $CH_2$ | $OCH_3$ | |
| 4-$NO_2$ | H | H | H | $CH_2$ | $OCH_3$ | |
| 4-$OCH_3$ | H | H | H | $CH_2$ | $OCH_3$ | |
| 4-$SCH_3$ | H | H | H | $CH_2$ | $OCH_3$ | |
| 4-$SO_2CH_3$ | H | H | H | $CH_2$ | $OCH_3$ | |
| 4-$CO_2CH_3$ | H | H | H | $CH_2$ | $OCH_3$ | |
| 4-$SO_2N(CH_3)_2$ | H | H | H | $CH_2$ | $OCH_3$ | |
| 4-$OSO_2CH_3$ | H | H | H | $CH_2$ | $OCH_3$ | |
| 6-Cl | H | H | H | $CH_2$ | $OCH_3$ | |
| 6-Br | H | H | H | $CH_2$ | $OCH_3$ | |
| 6-$NO_2$ | H | H | H | $CH_2$ | $OCH_3$ | |
| 6-$OCH_3$ | H | H | H | $CH_2$ | $OCH_3$ | |
| 6-$SCH_3$ | H | H | H | $CH_2$ | $OCH_3$ | |
| 6-$SO_2CH_3$ | H | H | H | $CH_2$ | $OCH_3$ | |
| 6-$CO_2CH_3$ | H | H | H | $CH_2$ | $OCH_3$ | |
| 6-$SO_2N(CH_3)_2$ | H | H | H | $CH_2$ | $OCH_3$ | |
| 6-$OSO_2CH_3$ | H | H | H | $CH_2$ | $OCH_3$ | |
| H | H | 1-$CH_3$ | H | O | $CH_3$ | |
| H | H | 1-$CH_3$ | H | O | $OCH_3$ | |
| H | H | 1-$CH_3$ | H | $CH_2$ | $CH_3$ | |
| H | H | 1-$CH_3$ | H | $CH_2$ | $OCH_3$ | |
| H | H | 2-$CH_3$ | H | O | $CH_3$ | |
| H | H | 2-$CH_3$ | H | O | $OCH_3$ | |
| H | H | 2-$CH_3$ | H | $CH_2$ | $CH_3$ | |
| H | H | 2-$CH_3$ | H | $CH_2$ | $OCH_3$ | |
| H | H | 3-$CH_3$ | H | O | $CH_3$ | |
| H | H | 3-$CH_3$ | H | O | $OCH_3$ | |
| H | H | 3-$CH_3$ | H | $CH_2$ | $CH_3$ | |
| H | H | 3-$CH_3$ | H | $CH_2$ | $OCH_3$ | |
| H | H | H | $CH_3$ | O | $CH_3$ | |
| H | H | H | $CH_3$ | O | $OCH_3$ | |
| H | H | H | $CH_3$ | $CH_2$ | $CH_3$ | |
| H | H | H | $CH_3$ | $CH_2$ | $OCH_3$ | |

Table If

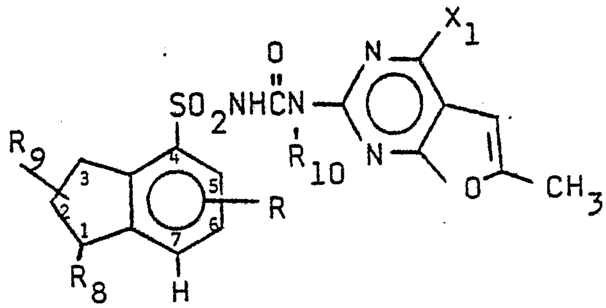

| R | $R_8$ | $R_9$ | $R_{10}$ | $X_1$ | m.p.(°C) |
|---|---|---|---|---|---|
| H | H | H | H | $CH_3$ | |
| 5-Cl | H | H | H | $CH_3$ | |
| 5-Br | H | H | H | $CH_3$ | |
| 5-$NO_2$ | H | H | H | $CH_3$ | |
| 5-$OCH_3$ | H | H | H | $CH_3$ | |
| 5-$SCH_3$ | H | H | H | $CH_3$ | |
| 5-$SO_2CH_3$ | H | H | H | $CH_3$ | |
| 5-$CO_2CH_3$ | H | H | H | $CH_3$ | |
| 5-$SO_2N(CH_3)_2$ | H | H | H | $CH_3$ | |
| 5-$SO_2N(OCH_3)CH_3$ | H | H | H | $CH_3$ | |
| 5-$OSO_2CH_3$ | H | H | H | $CH_3$ | |
| 5-$CH_3$ | H | H | H | $CH_3$ | |
| 6-Cl | H | H | H | $CH_3$ | |
| 6-Br | H | H | H | $CH_3$ | |
| 6-$NO_2$ | H | H | H | $CH_3$ | |
| 6-$CH_3$ | H | H | H | $CH_3$ | |
| 6-$OCH_3$ | H | H | H | $CH_3$ | |
| H | H | H | H | $OCH_3$ | |
| 5-Cl | H | H | H | $OCH_3$ | |
| 5-Br | H | H | H | $OCH_3$ | |
| 5-$NO_2$ | H | H | H | $OCH_3$ | |
| 5-$OCH_3$ | H | H | H | $OCH_3$ | |
| 5-$SCH_3$ | H | H | H | $OCH_3$ | |
| 5-$SO_2CH_3$ | H | H | H | $OCH_3$ | |

Table If (continued)

| R | $R_8$ | $R_9$ | $R_{10}$ | $X_1$ | m.p.(°C) |
|---|---|---|---|---|---|
| $5-CO_2CH_3$ | H | H | H | $OCH_3$ | |
| $5-SO_2N(CH_3)_2$ | H | H | H | $OCH_3$ | |
| $5-SO_2N(OCH_3)CH_3$ | H | H | H | $OCH_3$ | |
| $5-OSO_2CH_3$ | H | H | H | $OCH_3$ | |
| $5-CH_3$ | H | H | H | $OCH_3$ | |
| 6-Cl | H | H | H | $OCH_3$ | |
| 6-Br | H | H | H | $OCH_3$ | |
| $6-NO_2$ | H | H | H | $OCH_3$ | |
| $6-CH_3$ | H | H | H | $OCH_3$ | |
| $6-OCH_3$ | H | H | H | $OCH_3$ | |
| H | H | $1-CH_3$ | H | $CH_3$ | |
| H | H | $2-CH_3$ | H | $CH_3$ | |
| H | H | $3-CH_3$ | H | $CH_3$ | |
| H | H | $1-CH_3$ | H | $OCH_3$ | |
| H | $CH_3$ | $2-CH_3$ | H | $OCH_3$ | |
| H | $CH_3$ | $3-CH_3$ | H | $OCH_3$ | |
| H | H | H | $CH_3$ | $CH_3$ | |
| H | H | H | $CH_3$ | $OCH_3$ | |

Table Ig

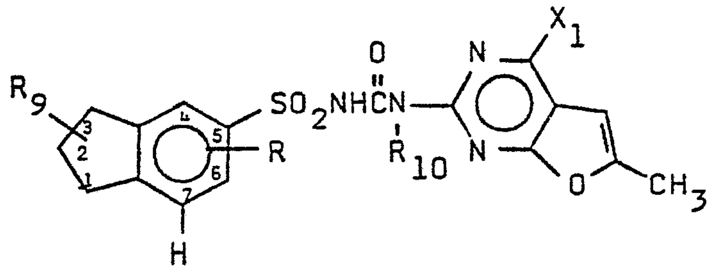

| R | $R_9$ | $R_{10}$ | $X_1$ | m.p.(°C) |
|---|---|---|---|---|
| H | H | H | $CH_3$ | |
| 4-Cl | H | H | $CH_3$ | |
| 4-Br | H | H | $CH_3$ | |
| 4-$NO_2$ | H | H | $CH_3$ | |
| 4-$OCH_3$ | H | H | $CH_3$ | |
| 4-$SCH_3$ | H | H | $CH_3$ | |
| 4-$SO_2CH_3$ | H | H | $CH_3$ | |
| 4-$CO_2CH_3$ | H | H | $CH_3$ | |
| 4-$SO_2N(CH_3)_2$ | H | H | $CH_3$ | |
| 4-$SO_2N(OCH_3)CH_3$ | H | H | $CH_3$ | |
| 4-$OSO_2CH_3$ | H | H | $CH_3$ | |
| 6-Cl | H | H | $CH_3$ | |
| 6-Br | H | H | $CH_3$ | |
| 6-$NO_2$ | H | H | $CH_3$ | |
| 6-$OCH_3$ | H | H | $CH_3$ | |
| 6-$SCH_3$ | H | H | $CH_3$ | |
| 6-$SO_2CH_3$ | H | H | $CH_3$ | |
| 6-$CO_2CH_3$ | H | H | $CH_3$ | |
| 6-$SO_2N(CH_3)_2$ | H | H | $CH_3$ | |
| 6-$OSO_2CH_3$ | H | H | $CH_3$ | |
| H | H | H | $OCH_3$ | |
| 4-Cl | H | H | $OCH_3$ | |
| 4-Br | H | H | $OCH_3$ | |
| 4-$NO_2$ | H | H | $OCH_3$ | |
| 4-$OCH_3$ | H | H | $OCH_3$ | |
| 4-$SCH_3$ | H | H | $OCH_3$ | |

Table Ig (continued)

| R | $R_9$ | $R_{10}$ | $X_1$ | m.p.(°C) |
|---|---|---|---|---|
| 4-$SO_2CH_3$ | H | H | $OCH_3$ | |
| 4-$CO_2CH_3$ | H | H | $OCH_3$ | |
| 4-$SO_2N(CH_3)_2$ | H | H | $OCH_3$ | |
| 3-$OSO_2CH_3$ | H | H | $OCH_3$ | |
| 6-Cl | H | H | $OCH_3$ | |
| 6-Br | H | H | $OCH_3$ | |
| 6-$NO_2$ | H | H | $OCH_3$ | |
| 6-$OCH_3$ | H | H | $OCH_3$ | |
| 6-$SCH_3$ | H | H | $OCH_3$ | |
| 6-$SO_2CH_3$ | H | H | $OCH_3$ | |
| 6-$CO_2CH_3$ | H | H | $OCH_3$ | |
| 6-$SO_2N(CH_3)_2$ | H | H | $OCH_3$ | |
| 6-$OSO_2CH_3$ | H | H | $OCH_3$ | |
| H | 1-$CH_3$ | H | $CH_3$ | |
| H | 1-$CH_3$ | H | $OCH_3$ | |
| H | 2-$CH_3$ | H | $CH_3$ | |
| H | 2-$CH_3$ | H | $OCH_3$ | |
| H | 3-$CH_3$ | H | $CH_3$ | |
| H | 3-$CH_3$ | H | $OCH_3$ | |
| H | H | $CH_3$ | $CH_3$ | |
| H | H | $CH_3$ | $OCH_3$ | |

Table 1h

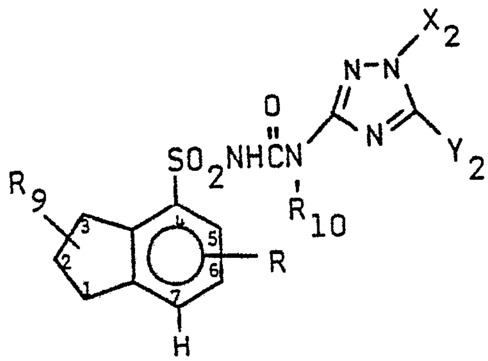

| R | $R_9$ | $R_{10}$ | $X_2$ | $Y_2$ |
|---|---|---|---|---|
| H | H | H | $CH_3$ | $SCH_3$ |
| H | H | H | $CH_3$ | $SC_2H_5$ |
| H | H | H | $CH_3$ | $OCH_3$ |
| H | H | H | $CH_3$ | $OC_2H_5$ |
| H | H | H | $C_2H_5$ | $SCH_3$ |
| H | H | H | $C_2H_5$ | $SC_2H_5$ |
| H | H | H | $C_2H_5$ | $OCH_3$ |
| H | H | H | $C_2H_5$ | $OC_2H_5$ |
| H | H | H | $\underline{n}$-$C_3H_7$ | $SCH_3$ |
| H | H | H | $\underline{n}$-$C_3H_7$ | $SC_2H_5$ |
| H | H | H | $\underline{n}$-$C_3H_7$ | $OCH_3$ |
| H | H | H | $\underline{n}$-$C_3H_7$ | $OC_2H_5$ |
| H | H | H | $CH_2CF_3$ | $SCH_3$ |
| H | H | H | $CH_2CF_3$ | $SC_2H_5$ |
| H | H | H | $CH_2CF_3$ | $OCH_3$ |
| H | H | H | $CH_2CF_3$ | $OC_2H_5$ |
| 5-$CO_2CH_3$ | H | H | $CH_3$ | $OCH_3$ |
| 5-$SO_2N(CH_3)_2$ | H | H | $CH_3$ | $OCH_3$ |
| 5-$SCH_3$ | H | H | $CH_3$ | $OCH_3$ |
| 5-$SO_2CH_3$ | H | H | $CH_3$ | $OCH_3$ |
| 5-$OSO_2CH_3$ | H | H | $CH_3$ | $OCH_3$ |
| 5-$SO_2N(OCH_3)CH_3$ | H | H | $CH_3$ | $OCH_3$ |

Table 1h (continued)

| R | $R_9$ | $R_{10}$ | $X_2$ | $Y_2$ |
|---|---|---|---|---|
| 5-$OCH_3$ | H | H | $CH_3$ | $OCH_3$ |
| 6-Cl | H | H | $CH_3$ | $OCH_3$ |
| 6-Br | H | H | $CH_3$ | $OCH_3$ |
| 6-$CH_3$ | H | H | $CH_3$ | $OCH_3$ |
| 6-$NO_2$ | H | H | $CH_3$ | $OCH_3$ |
| 6-$OCH_3$ | H | H | $CH_3$ | $OCH_3$ |
| H | 1-$CH_3$ | H | $CH_3$ | $OCH_3$ |
| H | 2-$CH_3$ | H | $C_2H_5$ | $SCH_3$ |
| H | 3-$CH_3$ | H | $CH_2CF_3$ | $OC_2H_5$ |
| H | H | $CH_3$ | $CH_3$ | $OCH_3$ |
| H | H | $CH_3$ | $CH_3$ | $SCH_3$ |

Table 1i

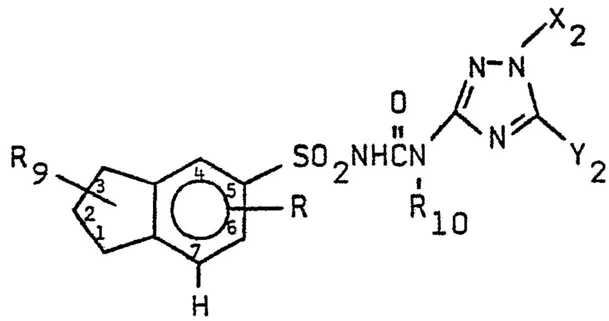

| R | $R_9$ | $R_{10}$ | $X_2$ | $Y_2$ |
|---|---|---|---|---|
| H | H | H | $CH_3$ | $SCH_3$ |
| H | H | H | $CH_3$ | $SC_2H_5$ |
| H | H | H | $CH_3$ | $OCH_3$ |
| H | H | H | $CH_3$ | $OC_2H_5$ |
| H | H | H | $C_2H_5$ | $SCH_3$ |
| H | H | H | $C_2H_5$ | $SC_2H_5$ |
| H | H | H | $C_2H_5$ | $OCH_3$ |
| H | H | H | $C_2H_5$ | $OC_2H_5$ |
| H | H | H | $\underline{n}$-$C_3H_7$ | $OCH_3$ |
| H | H | H | $CH_2CF_3$ | $OCH_3$ |
| 4-$CO_2CH_3$ | H | H | $CH_3$ | $OCH_3$ |
| 4-$SO_2N(CH_3)_2$ | H | H | $CH_3$ | $OCH_3$ |
| 4-$OCH_3$ | H | H | $CH_3$ | $OCH_3$ |
| 4-$SO_2CH_3$ | H | H | $CH_3$ | $OCH_3$ |
| 6-$CO_2CH_3$ | H | H | $CH_3$ | $OCH_3$ |
| 6-$SO_2N(CH_3)_2$ | H | H | $CH_3$ | $OCH_3$ |
| 6-$OCH_3$ | H | H | $CH_3$ | $OCH_3$ |
| 6-$SO_2CH_3$ | H | H | $CH_3$ | $OCH_3$ |
| H | 1-$CH_3$ | H | $CH_3$ | $OCH_3$ |
| H | 2-$CH_3$ | H | $CH_3$ | $OCH_3$ |
| H | 3-$CH_3$ | H | $CH_3$ | $OCH_3$ |
| H | H | $CH_3$ | $CH_3$ | $OCH_3$ |

Table IIa

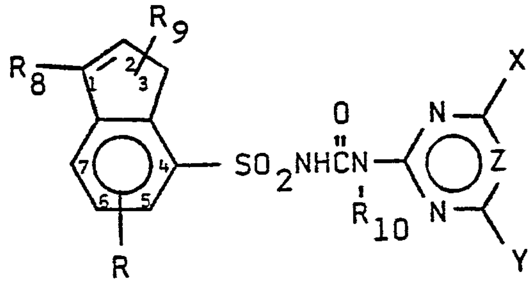

| $R_8$ | $R_9$ | R | $R_{10}$ | X | Y | Z | m.p.(°C) |
|---|---|---|---|---|---|---|---|
| Cl | H | H | H | $CH_3$ | $CH_3$ | CH | 78-80° |
| Cl | H | H | H | $OCH_3$ | $CH_3$ | CH | 168-172° |
| Cl | H | H | H | $OCH_3$ | $OCH_3$ | CH | 88-91° |
| Cl | H | H | H | $CH_3$ | $C_2H_5$ | CH | |
| Cl | H | H | H | $OCH_3$ | $C_2H_5$ | CH | |
| Cl | H | H | H | $CH_3$ | $OC_2H_5$ | CH | |
| Cl | H | H | H | $OCH_3$ | $OC_2H_5$ | CH | |
| Cl | H | H | H | $CH_3$ | $CH_2OCH_3$ | CH | |
| Cl | H | H | H | $OCH_3$ | $CH_2OCH_3$ | CH | |
| Cl | H | H | H | $OCH_3$ | $NH_2$ | CH | |
| Cl | H | H | H | $OCH_3$ | $NHCH_3$ | CH | |
| Cl | H | H | H | $OCH_3$ | $N(CH_3)_2$ | CH | |
| Cl | H | H | H | $CH_3$ | $CH_3$ | N | |
| Cl | H | H | H | $OCH_3$ | $CH_3$ | N | 168-170° |
| Cl | H | H | H | $OCH_3$ | $OCH_3$ | N | |
| Cl | H | H | H | $CH_3$ | $C_2H_5$ | N | |
| Cl | H | H | H | $OCH_3$ | $C_2H_5$ | N | |
| Cl | H | H | H | $CH_3$ | $OC_2H_5$ | N | |
| Cl | H | H | H | $OCH_3$ | $OC_2H_5$ | N | |
| Cl | H | H | H | $CH_3$ | $CH_2OCH_3$ | N | |
| Cl | H | H | H | $OCH_3$ | $CH_2OCH_3$ | N | |
| Cl | H | H | H | $OCH_3$ | $NH_2$ | N | |
| Cl | H | H | H | $OCH_3$ | $NHCH_3$ | N | |
| Cl | H | H | H | $OCH_3$ | $N(CH_3)_2$ | N | |

Table IIa (continued)

| $R_8$ | $R_9$ | R | $R_{10}$ | X | Y | Z | m.p.(°C) |
|---|---|---|---|---|---|---|---|
| Cl | H | 5-Cl | H | $CH_3$ | $CH_3$ | CH | |
| Cl | H | 5-Cl | H | $CH_3$ | $OCH_3$ | CH | |
| Cl | H | 5-Cl | H | $OCH_3$ | $OCH_3$ | CH | |
| Cl | H | 5-$NO_2$ | H | $CH_3$ | $CH_3$ | CH | |
| Cl | H | 5-$NO_2$ | H | $CH_3$ | $OCH_3$ | CH | |
| Cl | H | 5-$NO_2$ | H | $OCH_3$ | $OCH_3$ | CH | |
| Cl | H | 5-$CO_2CH_3$ | H | $CH_3$ | $CH_3$ | CH | |
| Cl | H | 5-$CO_2CH_3$ | H | $CH_3$ | $OCH_3$ | CH | |
| Cl | H | 5-$CO_2CH_3$ | H | $OCH_3$ | $OCH_3$ | CH | |
| Cl | H | 5-$SO_2CH_3$ | H | $CH_3$ | $CH_3$ | CH | |
| Cl | H | 5-$SO_2CH_3$ | H | $CH_3$ | $OCH_3$ | CH | |
| Cl | H | 5-$SO_2CH_3$ | H | $OCH_3$ | $OCH_3$ | CH | |
| Cl | H | 5-$SO_2N(CH_3)_2$ | H | $CH_3$ | $CH_3$ | CH | |
| Cl | H | 5-$SO_2N(CH_3)_2$ | H | $CH_3$ | $OCH_3$ | CH | |
| Cl | H | 5-$SO_2N(CH_3)_2$ | H | $OCH_3$ | $OCH_3$ | CH | |
| Cl | H | 5-$OSO_2CF_3$ | H | $CH_3$ | $CH_3$ | CH | |
| Cl | H | 5-$OSO_2CF_3$ | H | $CH_3$ | $OCH_3$ | CH | |
| Cl | H | 5-$OSO_2CF_3$ | H | $OCH_3$ | $OCH_3$ | CH | |
| Cl | H | 5-$OCH_3$ | H | $CH_3$ | $CH_3$ | CH | |
| Cl | H | 5-$OCH_3$ | H | $CH_3$ | $OCH_3$ | CH | |
| Cl | H | 5-$OCH_3$ | H | $OCH_3$ | $OCH_3$ | CH | |
| Cl | H | 5-$OC_2H_5$ | H | $CH_3$ | $CH_3$ | CH | |
| Cl | H | 5-$OC_2H_5$ | H | $CH_3$ | $OCH_3$ | CH | |
| Cl | H | 5-$OC_2H_5$ | H | $OCH_3$ | $OCH_3$ | CH | |
| Cl | H | 5-Br | H | $CH_3$ | $CH_3$ | CH | |
| Cl | H | 5-Br | H | $CH_3$ | $OCH_3$ | CH | |
| Cl | H | 5-Br | H | $OCH_3$ | $OCH_3$ | CH | |
| Cl | H | 5-$OSO_2CH_3$ | H | $CH_3$ | $CH_3$ | CH | |
| Cl | H | 5-$OSO_2CH_3$ | H | $CH_3$ | $OCH_3$ | CH | |
| Cl | H | 5-$OSO_2CH_3$ | H | $OCH_3$ | $OCH_3$ | CH | |
| Cl | H | 5-Cl | H | $CH_3$ | $CH_3$ | N | |
| Cl | H | 5-Cl | H | $CH_3$ | $OCH_3$ | N | |

Table IIa (continued)

| $R_8$ | $R_9$ | R | $R_{10}$ | X | Y | Z | m.p.(°C) |
|---|---|---|---|---|---|---|---|
| Cl | H | 5-Cl | H | $OCH_3$ | $OCH_3$ | N | |
| Cl | H | 5-$NO_2$ | H | $CH_3$ | $CH_3$ | N | |
| Cl | H | 5-$NO_2$ | H | $CH_3$ | $OCH_3$ | N | |
| Cl | H | 5-$NO_2$ | H | $OCH_3$ | $OCH_3$ | N | |
| Cl | H | 5-$CO_2CH_3$ | H | $CH_3$ | $CH_3$ | N | |
| Cl | H | 5-$CO_2CH_3$ | H | $CH_3$ | $OCH_3$ | N | |
| Cl | H | 5-$CO_2CH_3$ | H | $OCH_3$ | $OCH_3$ | N | |
| Cl | H | 5-$SO_2CH_3$ | H | $CH_3$ | $CH_3$ | N | |
| Cl | H | 5-$SO_2CH_3$ | H | $CH_3$ | $OCH_3$ | N | |
| Cl | H | 5-$SO_2CH_3$ | H | $OCH_3$ | $OCH_3$ | N | |
| Cl | H | 5-$SO_2N(CH_3)_2$ | H | $CH_3$ | $CH_3$ | N | |
| Cl | H | 5-$SO_2N(CH_3)_2$ | H | $CH_3$ | $OCH_3$ | N | |
| Cl | H | 5-$SO_2N(CH_3)_2$ | H | $OCH_3$ | $OCH_3$ | N | |
| Cl | H | 5-$OSO_2CF_3$ | H | $CH_3$ | $CH_3$ | N | |
| Cl | H | 5-$OSO_2CF_3$ | H | $CH_3$ | $OCH_3$ | N | |
| Cl | H | 5-$OSO_2CF_3$ | H | $OCH_3$ | $OCH_3$ | N | |
| Cl | H | 6-Cl | H | $CH_3$ | $CH_3$ | CH | |
| Cl | H | 6-Cl | H | $CH_3$ | $OCH_3$ | CH | |
| Cl | H | 6-Cl | H | $OCH_3$ | $OCH_3$ | CH | |
| Cl | H | 6-$OCH_3$ | H | $CH_3$ | $CH_3$ | CH | |
| Cl | H | 6-$OCH_3$ | H | $CH_3$ | $OCH_3$ | CH | |
| Cl | H | 6-$OCH_3$ | H | $OCH_3$ | $OCH_3$ | CH | |
| Cl | H | 6-$NO_2$ | H | $CH_3$ | $CH_3$ | CH | |
| Cl | H | 6-$NO_2$ | H | $CH_3$ | $OCH_3$ | CH | |
| Cl | H | 6-$NO_2$ | H | $OCH_3$ | $OCH_3$ | CH | |
| Cl | H | 6-$SO_2CH_3$ | H | $CH_3$ | $CH_3$ | CH | |
| Cl | H | 6-$SO_2CH_3$ | H | $CH_3$ | $OCH_3$ | CH | |
| Cl | H | 6-$SO_2CH_3$ | H | $OCH_3$ | $OCH_3$ | CH | |
| Cl | H | 6-$CO_2CH_3$ | H | $CH_3$ | $CH_3$ | CH | |
| Cl | H | 6-$CO_2CH_3$ | H | $CH_3$ | $OCH_3$ | CH | |
| Cl | H | 6-$CO_2CH_3$ | H | $OCH_3$ | $OCH_3$ | CH | |
| Cl | H | 6-$SO_2N(CH_3)_2$ | H | $CH_3$ | $CH_3$ | CH | |

Table IIa (continued)

| $R_8$ | $R_9$ | R | $R_{10}$ | X | Y | Z | m.p.(°C) |
|---|---|---|---|---|---|---|---|
| Cl | H | 6-$SO_2N(CH_3)_2$ | H | $CH_3$ | $OCH_3$ | CH | |
| Cl | H | 6-$SO_2N(CH_3)_2$ | H | $OCH_3$ | $OCH_3$ | CH | |
| Cl | H | 6-Cl | H | $CH_3$ | $CH_3$ | N | |
| Cl | H | 6-Cl | H | $CH_3$ | $OCH_3$ | N | |
| Cl | H | 6-Cl | H | $OCH_3$ | $OCH_3$ | N | |
| Cl | H | 6-$OCH_3$ | H | $CH_3$ | $CH_3$ | N | |
| Cl | H | 6-$OCH_3$ | H | $CH_3$ | $OCH_3$ | N | |
| Cl | H | 6-$OCH_3$ | H | $OCH_3$ | $OCH_3$ | N | |
| Cl | H | 6-$NO_2$ | H | $CH_3$ | $CH_3$ | N | |
| Cl | H | 6-$NO_2$ | H | $CH_3$ | $OCH_3$ | N | |
| Cl | H | 6-$NO_2$ | H | $OCH_3$ | $OCH_3$ | N | |
| Cl | H | 6-$SO_2CH_3$ | H | $CH_3$ | $CH_3$ | N | |
| Cl | H | 6-$SO_2CH_3$ | H | $CH_3$ | $OCH_3$ | N | |
| Cl | H | 6-$SO_2CH_3$ | H | $OCH_3$ | $OCH_3$ | N | |
| Cl | H | 6-$CO_2CH_3$ | H | $CH_3$ | $CH_3$ | N | |
| Cl | H | 6-$CO_2CH_3$ | H | $CH_3$ | $OCH_3$ | N | |
| Cl | H | 6-$CO_2CH_3$ | H | $OCH_3$ | $OCH_3$ | N | |
| Cl | H | 6-$SO_2N(CH_3)_2$ | H | $CH_3$ | $CH_3$ | N | |
| Cl | H | 6-$SO_2N(CH_3)_2$ | H | $CH_3$ | $OCH_3$ | N | |
| Cl | H | 6-$SO_2N(CH_3)_2$ | H | $OCH_3$ | $OCH_3$ | N | |
| $CH_3$ | H | H | H | $CH_3$ | $CH_3$ | CH | |
| $CH_3$ | H | H | H | $CH_3$ | $OCH_3$ | CH | |
| $CH_3$ | H | H | H | $OCH_3$ | $OCH_3$ | CH | |
| $CH_3$ | H | 5-Cl | H | $CH_3$ | $CH_3$ | CH | |
| $CH_3$ | H | 5-Cl | H | $CH_3$ | $OCH_3$ | CH | |
| $CH_3$ | H | 5-Cl | H | $OCH_3$ | $OCH_3$ | CH | |
| $CH_3$ | H | 5-Br | H | $CH_3$ | $CH_3$ | CH | |
| $CH_3$ | H | 5-Br | H | $CH_3$ | $OCH_3$ | CH | |
| $CH_3$ | H | 5-Br | H | $OCH_3$ | $OCH_3$ | CH | |
| $CH_3$ | H | 5-$NO_2$ | H | $CH_3$ | $CH_3$ | CH | |
| $CH_3$ | H | 5-$NO_2$ | H | $CH_3$ | $OCH_3$ | CH | |
| $CH_3$ | H | 5-$NO_2$ | H | $OCH_3$ | $OCH_3$ | CH | |

Table IIa (continued)

| $R_8$ | $R_9$ | R | $R_{10}$ | X | Y | Z | m.p.(°C) |
|---|---|---|---|---|---|---|---|
| $CH_3$ | H | $5-SO_2CH_3$ | H | $CH_3$ | $CH_3$ | CH | |
| $CH_3$ | H | $5-SO_2CH_3$ | H | $CH_3$ | $OCH_3$ | CH | |
| $CH_3$ | H | $5-SO_2CH_3$ | H | $OCH_3$ | $OCH_3$ | CH | |
| H | H | $5-CH_3$ | H | $CH_3$ | $CH_3$ | CH | |
| H | H | $5-CH_3$ | H | $CH_3$ | $OCH_3$ | CH | |
| H | H | $5-CH_3$ | H | $OCH_3$ | $OCH_3$ | CH | |
| H | H | 5-Cl | H | $CH_3$ | $CH_3$ | CH | |
| H | H | 5-Cl | H | $CH_3$ | $OCH_3$ | CH | |
| H | H | 5-Cl | H | $OCH_3$ | $OCH_3$ | CH | |
| H | H | $6-CH_3$ | H | $CH_3$ | $CH_3$ | CH | |
| H | H | $6-CH_3$ | H | $CH_3$ | $OCH_3$ | CH | |
| H | H | $6-CH_3$ | H | $OCH_3$ | $OCH_3$ | CH | |
| H | H | $5-NO_2$ | H | $CH_3$ | $CH_3$ | CH | |
| H | H | $5-NO_2$ | H | $CH_3$ | $OCH_3$ | CH | |
| H | H | $5-NO_2$ | H | $OCH_3$ | $OCH_3$ | CH | |
| H | H | $5-CO_2CH_3$ | H | $CH_3$ | $CH_3$ | CH | |
| H | H | $5-CO_2CH_3$ | H | $CH_3$ | $OCH_3$ | CH | |
| H | H | $5-CO_2CH_3$ | H | $OCH_3$ | $OCH_3$ | CH | |
| H | H | $5-OC_2H_5$ | H | $CH_3$ | $OCH_3$ | CH | |
| H | H | $6-O-\underline{n}-C_3H_7$ | H | $OCH_3$ | $OCH_3$ | N | |
| H | H | $5-OCF_2H$ | H | $CH_3$ | $CH_3$ | CH | |
| H | H | $6-OCF_3$ | H | $OCH_3$ | $OCH_3$ | CH | |
| H | H | $5-OCF_2CF_2H$ | H | $CH_3$ | $OCH_3$ | N | |
| H | H | $5-SO_2N(CH_3)C_2H_5$ | H | $OCH_3$ | $OCH_3$ | N | |
| H | H | $6-SO_2N(C_2H_5)_2$ | H | $CH_3$ | $CH_3$ | CH | |
| H | H | $6-SO_2N(OCH_3)CH_3$ | H | $CH_3$ | $OCH_3$ | CH | |
| H | H | $5-CO_2C_2H_5$ | H | $CH_3$ | $OCH_3$ | CH | |
| H | H | $6-CO_2-\underline{n}-C_3H_7$ | H | $OCH_3$ | $OCH_3$ | N | |
| H | H | $5-CO_2CH_2CH=CH_2$ | H | $CH_3$ | $CH_3$ | CH | |
| H | H | $6-CO_2CH_2CH_2OCH_3$ | H | $OCH_3$ | $OCH_3$ | N | |
| H | H | $5-CO_2CH_2CH_2Cl$ | H | $OCH_3$ | $CH_3$ | CH | |
| H | H | $5-OSO_2C_2H_5$ | H | $OCH_3$ | $OCH_3$ | N | |

Table IIa (continued)

| $R_8$ | $R_9$ | R | $R_{10}$ | X | Y | Z | m.p.(°C) |
|---|---|---|---|---|---|---|---|
| H | H | 5-$OSO_2$-n-$C_3H_7$ | H | $CH_3$ | $OCH_3$ | N | |
| H | H | 5-$C_2H_5$ | H | $CH_3$ | $CH_3$ | N | |
| H | H | 6-n-$C_3H_7$ | H | $CH_3$ | $OCH_3$ | N | |
| Cl | H | H | H | Cl | $OCH_3$ | CH | |
| Cl | H | H | H | Cl | $NH_2$ | CH | |
| Cl | H | H | H | Cl | $NCH_3$ | CH | |
| Cl | H | H | H | Cl | $N(CH_3)_2$ | CH | |
| Cl | H | H | H | $OCH_3$ | $SCH_3$ | CH | |
| Cl | H | H | H | $CH_3$ | $CH(OCH_3)_2$ | N | |
| Cl | H | H | H | $CH_3$ | 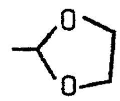 | CH | |
| H | 1-$CH_3$ | H | H | $CH_3$ | $OCH_3$ | CH | |
| H | 2-$CH_3$ | H | H | $OCH_3$ | $OCH_3$ | N | |
| H | 3-$CH_3$ | H | H | $CH_3$ | $OCH_3$ | N | |
| H | H | H | $CH_3$ | $OCH_3$ | $OCH_3$ | N | |

Table IIb

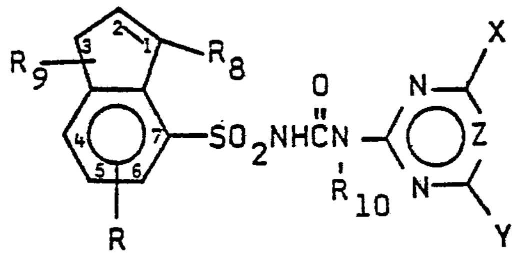

| $R_8$ | $R_9$ | R | $R_{10}$ | X | Y | Z | m.p.(°C) |
|---|---|---|---|---|---|---|---|
| Cl | H | H | H | $CH_3$ | $CH_3$ | CH | |
| Cl | H | H | H | $OCH_3$ | $CH_3$ | CH | |
| Cl | H | H | H | $OCH_3$ | $OCH_3$ | CH | |
| Cl | H | H | H | $CH_3$ | $C_2H_5$ | CH | |
| Cl | H | H | H | $OCH_3$ | $C_2H_5$ | CH | |
| Cl | H | H | H | $CH_3$ | $OC_2H_5$ | CH | |
| Cl | H | H | H | $OCH_3$ | $OC_2H_5$ | CH | |
| Cl | H | H | H | $CH_3$ | $CH_2OCH_3$ | CH | |
| Cl | H | H | H | $OCH_3$ | $CH_2OCH_3$ | CH | |
| Cl | H | H | H | $OCH_3$ | $NH_2$ | CH | |
| Cl | H | H | H | $OCH_3$ | $NHCH_3$ | CH | |
| Cl | H | H | H | $OCH_3$ | $N(CH_3)_2$ | CH | |
| Cl | H | H | H | $CH_3$ | $CH_3$ | N | |
| Cl | H | H | H | $OCH_3$ | $CH_3$ | N | |
| Cl | H | H | H | $OCH_3$ | $OCH_3$ | N | |
| Cl | H | H | H | $CH_3$ | $C_2H_5$ | N | |
| Cl | H | H | H | $OCH_3$ | $C_2H_5$ | N | |
| Cl | H | H | H | $CH_3$ | $OC_2H_5$ | N | |
| Cl | H | H | H | $OCH_3$ | $OC_2H_5$ | N | |
| Cl | H | H | H | $CH_3$ | $CH_2OCH_3$ | N | |
| Cl | H | H | H | $OCH_3$ | $CH_2OCH_3$ | N | |
| Cl | H | H | H | $OCH_3$ | $NH_2$ | N | |
| Cl | H | H | H | $OCH_3$ | $NHCH_3$ | N | |
| Cl | H | H | H | $OCH_3$ | $N(CH_3)_2$ | N | |

Table IIb (continued)

| $R_8$ | $R_9$ | R | $R_{10}$ | X | Y | Z | m.p.(°C) |
|---|---|---|---|---|---|---|---|
| Cl | H | H | H | Cl | $OCH_3$ | CH | |
| H | $2-CH_3$ | H | H | $OCH_3$ | $OCH_3$ | CH | |
| H | H | H | $CH_3$ | $OCH_3$ | $OCH_3$ | N | |
| $CH_3$ | H | H | H | $CH_3$ | $OCH_3$ | CH | |

Table IIc

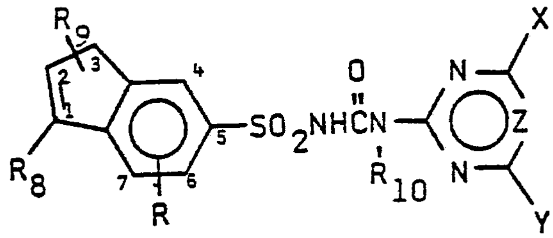

| $R_8$ | $R_9$ | R | $R_{10}$ | X | Y | Z | m.p.(°C) |
|---|---|---|---|---|---|---|---|
| Cl | H | H | H | $CH_3$ | $CH_3$ | CH | |
| Cl | H | H | H | $OCH_3$ | $CH_3$ | CH | |
| Cl | H | H | H | $OCH_3$ | $OCH_3$ | CH | |
| Cl | H | H | H | $CH_3$ | $C_2H_5$ | CH | |
| Cl | H | H | H | $OCH_3$ | $C_2H_5$ | CH | |
| Cl | H | H | H | $CH_3$ | $OC_2H_5$ | CH | |
| Cl | H | H | H | $OCH_3$ | $OC_2H_5$ | CH | |
| Cl | H | H | H | $CH_3$ | $CH_2OCH_3$ | CH | |
| Cl | H | H | H | $OCH_3$ | $CH_2OCH_3$ | CH | |
| Cl | H | H | H | $OCH_3$ | $NH_2$ | CH | |
| Cl | H | H | H | $OCH_3$ | $NHCH_3$ | CH | |
| Cl | H | H | H | $OCH_3$ | $N(CH_3)_2$ | CH | |
| Cl | H | H | H | $CH_3$ | $CH_3$ | N | |
| Cl | H | H | H | $OCH_3$ | $CH_3$ | N | |
| Cl | H | H | H | $OCH_3$ | $OCH_3$ | N | |
| Cl | H | H | H | $CH_3$ | $C_2H_5$ | N | |
| Cl | H | H | H | $OCH_3$ | $C_2H_5$ | N | |
| Cl | H | H | H | $CH_3$ | $OC_2H_5$ | N | |
| Cl | H | H | H | $OCH_3$ | $OC_2H_5$ | N | |
| Cl | H | H | H | $CH_3$ | $CH_2OCH_3$ | N | |
| Cl | H | H | H | $OCH_3$ | $CH_2OCH_3$ | N | |
| Cl | H | H | H | $OCH_3$ | $NH_2$ | N | |
| Cl | H | H | H | $OCH_3$ | $NHCH_3$ | N | |
| Cl | H | H | H | $OCH_3$ | $N(CH_3)_2$ | N | |

Table IIc (continued)

| $R_8$ | $R_9$ | R | $R_{10}$ | X | Y | Z | m.p.(°C) |
|---|---|---|---|---|---|---|---|
| Cl | H | H | H | Cl | $OCH_3$ | CH | |
| H | H | H | H | $OCH_3$ | $CH_3$ | N | |
| H | H | H | $CH_3$ | $OCH_3$ | $OCH_3$ | N | |
| H | 3-$CH_3$ | H | H | $CH_3$ | $OCH_3$ | CH | |
| H | H | H | H | $CH_3$ | 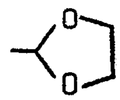 | CH | |

Table IId

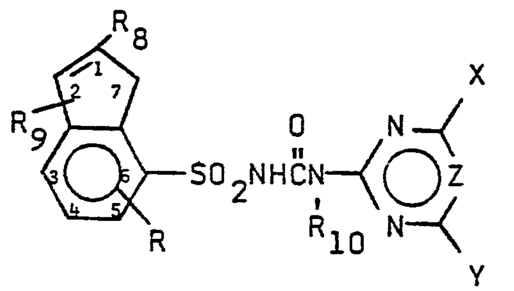

| $R_8$ | $R_9$ | R | $R_{10}$ | X | Y | Z | m.p.(°C) |
|---|---|---|---|---|---|---|---|
| Cl | H | H | H | $CH_3$ | $CH_3$ | CH | |
| Cl | H | H | H | $OCH_3$ | $CH_3$ | CH | |
| Cl | H | H | H | $OCH_3$ | $OCH_3$ | CH | |
| Cl | H | H | H | $CH_3$ | $C_2H_5$ | CH | |
| Cl | H | H | H | $OCH_3$ | $C_2H_5$ | CH | |
| Cl | H | H | H | $CH_3$ | $OC_2H_5$ | CH | |
| Cl | H | H | H | $OCH_3$ | $OC_2H_5$ | CH | |
| Cl | H | H | H | $CH_3$ | $CH_2OCH_3$ | CH | |
| Cl | H | H | H | $OCH_3$ | $CH_2OCH_3$ | CH | |
| Cl | H | H | H | $OCH_3$ | $NH_2$ | CH | |
| Cl | H | H | H | $OCH_3$ | $NHCH_3$ | CH | |
| Cl | H | H | H | $OCH_3$ | $N(CH_3)_2$ | CH | |
| Cl | H | H | H | $CH_3$ | $CH_3$ | N | |
| Cl | H | H | H | $OCH_3$ | $CH_3$ | N | |
| Cl | H | H | H | $OCH_3$ | $OCH_3$ | N | |
| Cl | H | H | H | $CH_3$ | $C_2H_5$ | N | |
| Cl | H | H | H | $OCH_3$ | $C_2H_5$ | N | |
| Cl | H | H | H | $CH_3$ | $OC_2H_5$ | N | |
| Cl | H | H | H | $OCH_3$ | $OC_2H_5$ | N | |
| Cl | H | H | H | $CH_3$ | $CH_2OCH_3$ | N | |
| Cl | H | H | H | $OCH_3$ | $CH_2OCH_3$ | N | |
| Cl | H | H | H | $OCH_3$ | $NH_2$ | N | |
| Cl | H | H | H | $OCH_3$ | $NHCH_3$ | N | |
| Cl | H | H | H | $OCH_3$ | $N(CH_3)_2$ | N | |

Table IId (continued)

| $R_8$ | $R_9$ | R | $R_{10}$ | X | Y | Z | m.p.(°C) |
|---|---|---|---|---|---|---|---|
| H | H | H | H | Cl | $OCH_3$ | CH | |
| H | H | H | H | $OCH_3$ | $CH_3$ | N | |
| H | H | H | $CH_3$ | $OCH_3$ | $OCH_3$ | CH | |
| H | H | 7-$CH_3$ | H | $CH_3$ | $OCH_3$ | CH | |
| H | H | H | H | $CH_3$ | 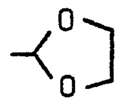 | CH | |

Table IIe

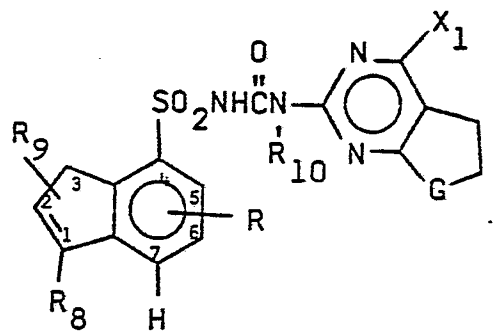

| R | $R_8$ | $R_9$ | $R_{10}$ | G | $X_1$ | m.p.(°C) |
|---|---|---|---|---|---|---|
| H | Cl | H | H | O | $CH_3$ | |
| H | Cl | H | H | O | $OCH_3$ | |
| H | Cl | H | H | $CH_2$ | $CH_3$ | |
| H | Cl | H | H | $CH_2$ | $OCH_3$ | |
| H | H | H | H | O | $OCH_3$ | |
| H | H | H | H | O | $CH_3$ | |
| H | H | H | H | $CH_2$ | $OCH_3$ | |
| H | H | H | H | $CH_2$ | $CH_3$ | |
| 6-$NO_2$ | H | H | H | O | $OCH_3$ | |
| 5-Cl | H | H | H | O | $CH_3$ | |
| H | $CH_3$ | H | H | $CH_2$ | $OCH_3$ | |
| H | H | 2-$CH_3$ | H | O | $OCH_3$ | |
| H | H | H | $CH_3$ | O | $CH_3$ | |

Table IIf

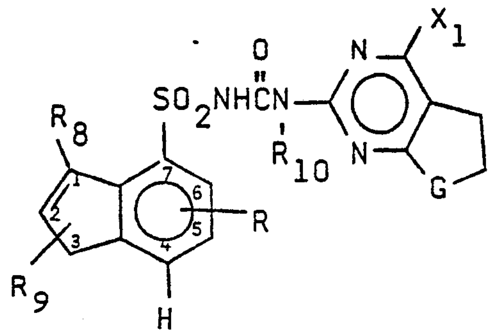

| R | $R_8$ | $R_9$ | $R_{10}$ | G | $X_1$ | m.p.(°C) |
|---|---|---|---|---|---|---|
| H | Cl | H | H | O | $CH_3$ | |
| H | Cl | H | H | O | $OCH_3$ | |
| H | Cl | H | H | $CH_2$ | $CH_3$ | |
| H | Cl | H | H | $CH_2$ | $OCH_3$ | |
| H | H | H | H | O | $OCH_3$ | |
| H | H | H | H | O | $CH_3$ | |
| H | H | H | H | $CH_2$ | $OCH_3$ | |
| H | H | H | H | $CH_2$ | $CH_3$ | |
| 6-$CH_3$ | H | H | H | O | $CH_3$ | |
| H | $CH_3$ | H | H | $CH_2$ | $OCH_3$ | |
| H | H | 3-$CH_3$ | H | O | $CH_3$ | |
| H | H | H | $CH_3$ | O | $CH_3$ | |

Table IIg

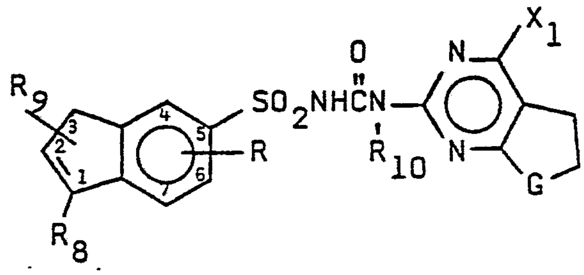

| R | $R_8$ | $R_9$ | $R_{10}$ | G | $X_1$ | m.p.(°C) |
|---|---|---|---|---|---|---|
| H | Cl | H | H | O | $CH_3$ | |
| H | Cl | H | H | O | $OCH_3$ | |
| H | Cl | H | H | $CH_2$ | $CH_3$ | |
| H | Cl | H | H | $CH_2$ | $OCH_3$ | |
| H | H | H | H | O | $OCH_3$ | |
| H | H | H | H | O | $CH_3$ | |
| H | H | H | H | $CH_2$ | $OCH_3$ | |
| H | H | H | H | $CH_2$ | $CH_3$ | |
| 6-$CH_3$ | H | H | H | O | $CH_3$ | |
| H | $CH_3$ | H | H | $CH_2$ | $OCH_3$ | |
| H | H | 3-$CH_3$ | H | O | $OCH_3$ | |
| H | H | H | $CH_3$ | O | $CH_3$ | |

Table IIh

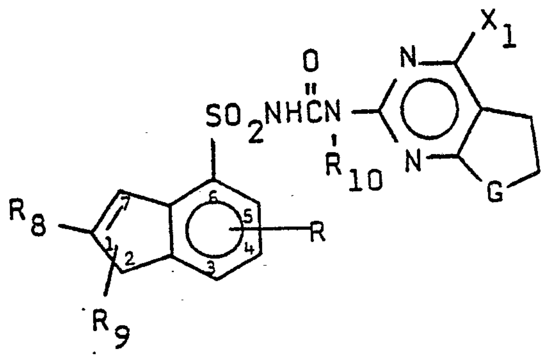

| R | $R_8$ | $R_9$ | $R_{10}$ | G | $X_1$ | m.p.(°C) |
|---|---|---|---|---|---|---|
| H | Cl | H | H | O | $CH_3$ | |
| H | Cl | H | H | O | $OCH_3$ | |
| H | Cl | H | H | $CH_2$ | $CH_3$ | |
| H | Cl | H | H | $CH_2$ | $OCH_3$ | |
| H | H | H | H | O | $OCH_3$ | |
| H | H | H | H | O | $CH_3$ | |
| H | H | H | H | $CH_2$ | $OCH_3$ | |
| H | H | H | H | $CH_2$ | $CH_3$ | |
| 5-$CH_3$ | H | H | H | O | $CH_3$ | |
| H | $CH_3$ | H | H | $CH_2$ | $OCH_3$ | |
| H | H | 2-$CH_3$ | H | O | $CH_3$ | |
| H | H | H | $CH_3$ | O | $OCH_3$ | |

Table IIi

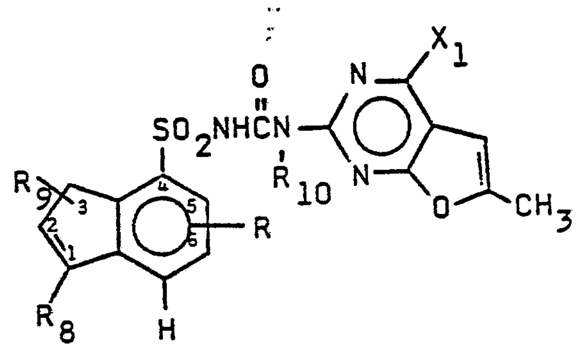

| R | $R_8$ | $R_9$ | $R_{10}$ | $X_1$ | m.p.(°C) |
|---|---|---|---|---|---|
| H | Cl | H | H | $CH_3$ | |
| H | Cl | H | H | $OCH_3$ | |
| H | H | H | H | $CH_3$ | |
| H | H | H | H | $OCH_3$ | |
| H | $CH_3$ | H | H | $CH_3$ | |
| H | $CH_3$ | H | H | $OCH_3$ | |
| 5-$CH_3$ | H | H | H | $CH_3$ | |
| H | H | 2-$CH_3$ | H | $OCH_3$ | |
| H | Cl | H | $CH_3$ | $OCH_3$ | |

Table IIj

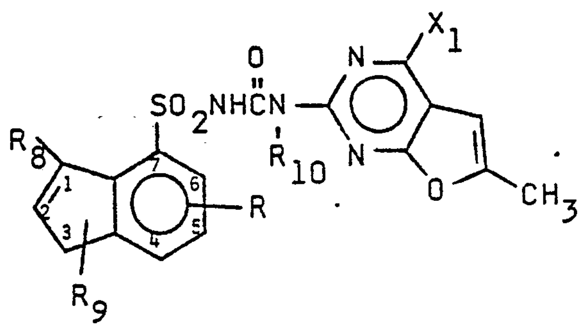

| R | $R_8$ | $R_9$ | $R_{10}$ | $X_1$ | m.p.(°C) |
|---|---|---|---|---|---|
| H | Cl | H | H | $CH_3$ | |
| H | Cl | H | H | $OCH_3$ | |
| H | H | H | H | $CH_3$ | |
| H | H | H | H | $OCH_3$ | |
| H | $CH_3$ | H | H | $CH_3$ | |
| H | $CH_3$ | H | H | $OCH_3$ | |
| 6-$CH_3$ | H | H | H | $CH_3$ | |
| H | H | 2-$CH_3$ | H | $OCH_3$ | |
| H | Cl | H | $CH_3$ | $OCH_3$ | |

Table IIk

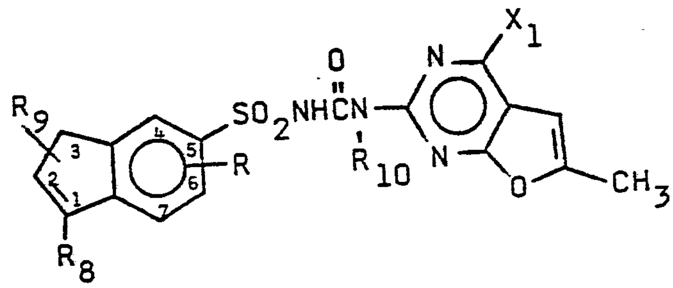

| R | $R_8$ | $R_9$ | $R_{10}$ | $X_1$ | m.p.(°C) |
|---|---|---|---|---|---|
| H | Cl | H | H | $CH_3$ | |
| H | Cl | H | H | $OCH_3$ | |
| H | H | H | H | $CH_3$ | |
| H | H | H | H | $OCH_3$ | |
| H | $CH_3$ | H | H | $CH_3$ | |
| H | $CH_3$ | H | H | $OCH_3$ | |
| 6-$CH_3$ | H | H | H | $CH_3$ | |
| H | H | 2-$CH_3$ | H | $OCH_3$ | |
| H | Cl | H | $CH_3$ | $OCH_3$ | |

Table III

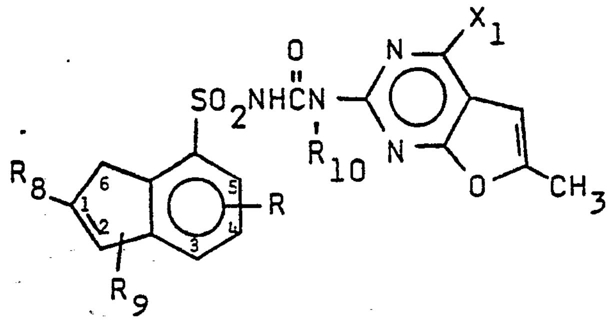

| R | $R_8$ | $R_9$ | $R_{10}$ | $X_1$ | m.p.(°C) |
|---|---|---|---|---|---|
| H | Cl | H | H | $CH_3$ | |
| H | Cl | H | H | $OCH_3$ | |
| H | H | H | H | $CH_3$ | |
| H | H | H | H | $OCH_3$ | |
| H | $CH_3$ | H | H | $CH_3$ | |
| H | $CH_3$ | H | H | $OCH_3$ | |
| 5-$CH_3$ | H | H | H | $CH_3$ | |
| H | H | 2-$CH_3$ | H | $OCH_3$ | |
| H | Cl | H | $CH_3$ | $OCH_3$ | |

Table IIm

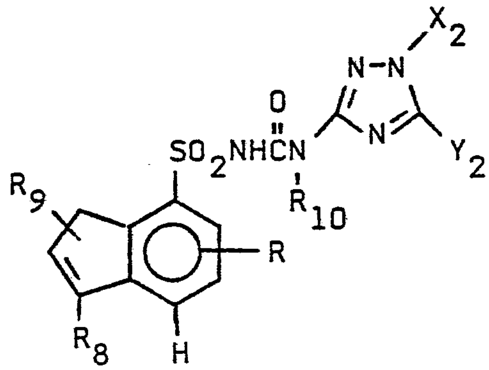

| R | $R_8$ | $R_9$ | $R_{10}$ | $X_2$ | $Y_2$ | m.p.(°C) |
|---|---|---|---|---|---|---|
| H | Cl | H | H | $CH_3$ | $SCH_3$ | |
| H | Cl | H | H | $CH_3$ | $OCH_3$ | |
| H | Cl | H | H | $CH_3$ | $OC_2H_5$ | |
| H | Cl | H | H | $CH_3$ | $SC_2H_5$ | |
| H | Cl | H | H | $C_2H_5$ | $OCH_3$ | |
| H | Cl | H | H | $\underline{n}$-$C_3H_7$ | $OCH_3$ | |
| H | Cl | H | H | $CH_2CF_3$ | $OCH_3$ | |
| H | H | H | H | $CH_3$ | $SCH_3$ | |
| H | H | H | H | $CH_3$ | $OCH_3$ | |
| H | H | H | H | $CH_3$ | $OC_2H_5$ | |
| H | H | H | H | $CH_3$ | $SC_2H_5$ | |
| H | $CH_3$ | H | H | $CH_3$ | $OCH_3$ | |
| $CH_3$ | H | H | H | $CH_3$ | $SCH_3$ | |
| H | H | 2-$CH_3$ | H | $CH_3$ | $OCH_3$ | |
| H | H | H | $CH_3$ | $CH_3$ | $OCH_3$ | |

Table IIn

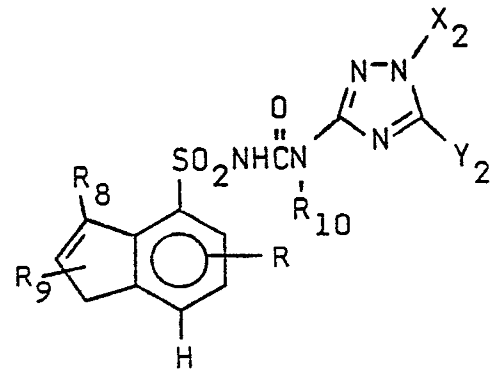

| R | $R_8$ | $R_9$ | $R_{10}$ | $X_2$ | $Y_2$ | m.p.(°C) |
|---|---|---|---|---|---|---|
| H | Cl | H | H | $CH_3$ | $SCH_3$ | |
| H | Cl | H | H | $CH_3$ | $OCH_3$ | |
| H | Cl | H | H | $CH_3$ | $OC_2H_5$ | |
| H | Cl | H | H | $CH_3$ | $SC_2H_5$ | |
| H | Cl | H | H | $C_2H_5$ | $OCH_3$ | |
| H | Cl | H | H | n-$C_3H_7$ | $OCH_3$ | |
| H | Cl | H | H | $CH_2CF_3$ | $OCH_3$ | |
| H | H | H | H | $CH_3$ | $SCH_3$ | |
| H | H | H | H | $CH_3$ | $OCH_3$ | |
| H | H | H | H | $CH_3$ | $OC_2H_5$ | |
| H | H | H | H | $CH_3$ | $SC_2H_5$ | |
| H | $CH_3$ | H | H | $CH_3$ | $OCH_3$ | |
| $CH_3$ | H | H | H | $CH_3$ | $SCH_3$ | |
| H | H | 2-$CH_3$ | H | $CH_3$ | $OCH_3$ | |
| H | H | H | $CH_3$ | $CH_3$ | $OCH_3$ | |

Table IIo

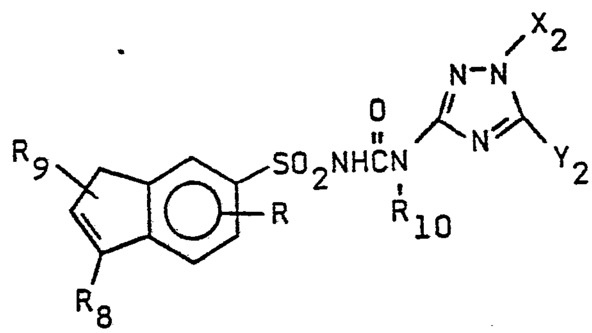

| R | $R_8$ | $R_9$ | $R_{10}$ | $X_2$ | $Y_2$ | m.p.(°C) |
|---|---|---|---|---|---|---|
| H | Cl | H | H | $CH_3$ | $SCH_3$ | |
| H | Cl | H | H | $CH_3$ | $OCH_3$ | |
| H | Cl | H | H | $CH_3$ | $OC_2H_5$ | |
| H | Cl | H | H | $CH_3$ | $SC_2H_5$ | |
| H | Cl | H | H | $C_2H_5$ | $OCH_3$ | |
| H | Cl | H | H | $\underline{n}$-$C_3H_7$ | $OCH_3$ | |
| H | Cl | H | H | $CH_2CF_3$ | $OCH_3$ | |
| H | H | H | H | $CH_3$ | $SCH_3$ | |
| H | H | H | H | $CH_3$ | $OCH_3$ | |
| H | H | H | H | $CH_3$ | $OC_2H_5$ | |
| H | H | H | H | $CH_3$ | $SC_2H_5$ | |
| H | $CH_3$ | H | H | $CH_3$ | $OCH_3$ | |
| $CH_3$ | H | H | H | $CH_3$ | $SCH_3$ | |
| H | H | 2-$CH_3$ | H | $CH_3$ | $OCH_3$ | |
| H | H | H | $CH_3$ | $CH_3$ | $OCH_3$ | |

Table IIp

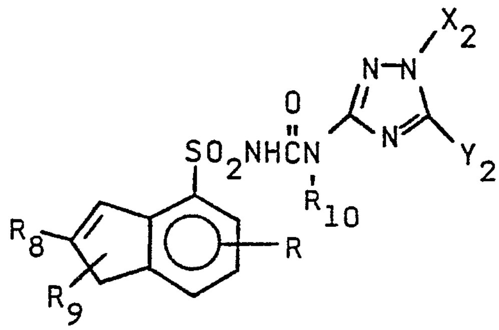

| R | $R_8$ | $R_9$ | $R_{10}$ | $X_2$ | $Y_2$ | m.p.(°C) |
|---|---|---|---|---|---|---|
| H | Cl | H | H | $CH_3$ | $SCH_3$ | |
| H | Cl | H | H | $CH_3$ | $OCH_3$ | |
| H | Cl | H | H | $CH_3$ | $OC_2H_5$ | |
| H | Cl | H | H | $CH_3$ | $SC_2H_5$ | |
| H | Cl | H | H | $C_2H_5$ | $OCH_3$ | |
| H | Cl | H | H | $\underline{n}$-$C_3H_7$ | $OCH_3$ | |
| H | Cl | H | H | $CH_2CF_3$ | $OCH_3$ | |
| H | H | H | H | $CH_3$ | $SCH_3$ | |
| H | H | H | H | $CH_3$ | $OCH_3$ | |
| H | H | H | H | $CH_3$ | $OC_2H_5$ | |
| H | H | H | H | $CH_3$ | $SC_2H_5$ | |
| H | $CH_3$ | H | H | $CH_3$ | $OCH_3$ | |
| $CH_3$ | H | H | H | $CH_3$ | $SCH_3$ | |
| H | H | 2-$CH_3$ | H | $CH_3$ | $OCH_3$ | |
| H | H | H | $CH_3$ | $CH_3$ | $OCH_3$ | |

Table IIIa

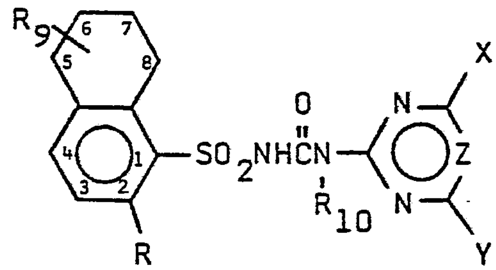

| $R_9$ | R | $R_{10}$ | X | Y | Z | m.p.(°C) |
|---|---|---|---|---|---|---|
| H | H | H | $CH_3$ | $CH_3$ | CH | 152-155° |
| H | H | H | $OCH_3$ | $CH_3$ | CH | 155-157° |
| H | H | H | $OCH_3$ | $OCH_3$ | CH | 145-147° |
| H | H | H | $CH_3$ | $C_2H_5$ | CH | |
| H | H | H | $OCH_3$ | $C_2H_5$ | CH | |
| H | H | H | $CH_3$ | $OC_2H_5$ | CH | |
| H | H | H | $OCH_3$ | $OC_2H_5$ | CH | |
| H | H | H | $CH_3$ | $CH_2OCH_3$ | CH | |
| H | H | H | $OCH_3$ | $CH_2OCH_3$ | CH | |
| H | H | H | $OCH_3$ | $NH_2$ | CH | |
| H | H | H | $OCH_3$ | $NHCH_3$ | CH | |
| H | H | H | $OCH_3$ | $N(CH_3)_2$ | CH | |
| H | H | H | $CH_3$ | $CH_3$ | N | 150-152° |
| H | H | H | $OCH_3$ | $CH_3$ | N | |
| H | H | H | $OCH_3$ | $OCH_3$ | N | 171-173° |
| H | H | H | $CH_3$ | $C_2H_5$ | N | |
| H | H | H | $OCH_3$ | $C_2H_5$ | N | |
| H | H | H | $CH_3$ | $OC_2H_5$ | N | |
| H | H | H | $OCH_3$ | $OC_2H_5$ | N | |
| H | H | H | $CH_3$ | $CH_2OCH_3$ | N | |
| H | H | H | $OCH_3$ | $CH_2OCH_3$ | N | |
| H | H | H | $OCH_3$ | $NH_2$ | N | |
| H | H | H | $OCH_3$ | $NHCH_3$ | N | |
| H | H | H | $OCH_3$ | $N(CH_3)_2$ | N | |

Table IIIa (continued)

| $R_9$ | R | $R_{10}$ | X | Y | Z | m.p.(°C) |
|---|---|---|---|---|---|---|
| H | H | H | Cl | $OCH_3$ | CH | |
| H | H | H | $OCH_3$ | $SCH_3$ | N | |
| H | H | H | $CH_3$ | $CH(OCH_3)_2$ | CH | |
| H | H | H | $CH_3$ | 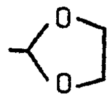 | CH | |
| H | Cl | H | $CH_3$ | $CH_3$ | CH | |
| H | Cl | H | $OCH_3$ | $CH_3$ | CH | |
| H | Cl | H | $OCH_3$ | $OCH_3$ | CH | |
| H | Cl | H | $CH_3$ | $C_2H_5$ | CH | |
| H | Cl | H | $OCH_3$ | $C_2H_5$ | CH | |
| H | Cl | H | $CH_3$ | $OC_2H_5$ | CH | |
| H | Cl | H | $OCH_3$ | $OC_2H_5$ | CH | |
| H | Cl | H | $CH_3$ | $CH_2OCH_3$ | CH | |
| H | Cl | H | $OCH_3$ | $CH_2OCH_3$ | CH | |
| H | Cl | H | $OCH_3$ | $NH_2$ | CH | |
| H | Cl | H | $OCH_3$ | $NHCH_3$ | CH | |
| H | Cl | H | $OCH_3$ | $N(CH_3)_2$ | CH | |
| H | Cl | H | $CH_3$ | $CH_3$ | N | |
| H | Cl | H | $OCH_3$ | $CH_3$ | N | |
| H | Cl | H | $OCH_3$ | $OCH_3$ | N | |
| H | Cl | H | $CH_3$ | $C_2H_5$ | N | |
| H | Cl | H | $OCH_3$ | $C_2H_5$ | N | |
| H | Cl | H | $CH_3$ | $OC_2H_5$ | N | |
| H | Cl | H | $OCH_3$ | $OC_2H_5$ | N | |
| H | Cl | H | $CH_3$ | $CH_2OCH_3$ | N | |
| H | Cl | H | $OCH_3$ | $CH_2OCH_3$ | N | |
| H | Cl | H | $OCH_3$ | $NH_2$ | N | |
| H | Cl | H | $OCH_3$ | $NHCH_3$ | N | |
| H | Cl | H | $OCH_3$ | $N(CH_3)_2$ | N | |
| H | Br | H | $CH_3$ | $CH_3$ | CH | |
| H | Br | H | $OCH_3$ | $CH_3$ | CH | |
| H | Br | H | $OCH_3$ | $OCH_3$ | CH | |

Table IIIa (continued)

| $R_9$ | R | $R_{10}$ | X | Y | Z | m.p.(°C) |
|---|---|---|---|---|---|---|
| H | Br | H | $CH_3$ | $C_2H_5$ | CH | |
| H | Br | H | $OCH_3$ | $C_2H_5$ | CH | |
| H | Br | H | $CH_3$ | $OC_2H_5$ | CH | |
| H | Br | H | $OCH_3$ | $OC_2H_5$ | CH | |
| H | Br | H | $CH_3$ | $CH_2OCH_3$ | CH | |
| H | Br | H | $OCH_3$ | $CH_2OCH_3$ | CH | |
| H | Br | H | $OCH_3$ | $NH_2$ | CH | |
| H | Br | H | $OCH_3$ | $NHCH_3$ | CH | |
| H | Br | H | $OCH_3$ | $N(CH_3)_2$ | CH | |
| H | Br | H | $CH_3$ | $CH_3$ | N | |
| H | Br | H | $OCH_3$ | $CH_3$ | N | |
| H | Br | H | $OCH_3$ | $OCH_3$ | N | |
| H | Br | H | $CH_3$ | $C_2H_5$ | N | |
| H | Br | H | $OCH_3$ | $C_2H_5$ | N | |
| H | Br | H | $CH_3$ | $OC_2H_5$ | N | |
| H | Br | H | $OCH_3$ | $OC_2H_5$ | N | |
| H | Br | H | $CH_3$ | $CH_2OCH_3$ | N | |
| H | Br | H | $OCH_3$ | $CH_2OCH_3$ | N | |
| H | Br | H | $OCH_3$ | $NH_2$ | N | |
| H | Br | H | $OCH_3$ | $NHCH_3$ | N | |
| H | Br | H | $OCH_3$ | $N(CH_3)_2$ | N | |
| H | $NO_2$ | H | $CH_3$ | $CH_3$ | CH | |
| H | $NO_2$ | H | $OCH_3$ | $CH_3$ | CH | |
| H | $NO_2$ | H | $OCH_3$ | $OCH_3$ | CH | |
| H | $NO_2$ | H | $CH_3$ | $C_2H_5$ | CH | |
| H | $NO_2$ | H | $OCH_3$ | $C_2H_5$ | CH | |
| H | $NO_2$ | H | $CH_3$ | $OC_2H_5$ | CH | |
| H | $NO_2$ | H | $OCH_3$ | $OC_2H_5$ | CH | |
| H | $NO_2$ | H | $CH_3$ | $CH_2OCH_3$ | CH | |
| H | $NO_2$ | H | $OCH_3$ | $CH_2OCH_3$ | CH | |
| H | $NO_2$ | H | $OCH_3$ | $NH_2$ | CH | |
| H | $NO_2$ | H | $OCH_3$ | $NHCH_3$ | CH | |

Table IIIa (continued)

| $R_9$ | R | $R_{10}$ | X | Y | Z | m.p.(°C) |
|---|---|---|---|---|---|---|
| H | $NO_2$ | H | $OCH_3$ | $N(CH_3)_2$ | CH | |
| H | $NO_2$ | H | $CH_3$ | $CH_3$ | N | |
| H | $NO_2$ | H | $OCH_3$ | $CH_3$ | N | |
| H | $NO_2$ | H | $OCH_3$ | $OCH_3$ | N | |
| H | $NO_2$ | H | $CH_3$ | $C_2H_5$ | N | |
| H | $NO_2$ | H | $OCH_3$ | $C_2H_5$ | N | |
| H | $NO_2$ | H | $CH_3$ | $OC_2H_5$ | N | |
| H | $NO_2$ | H | $OCH_3$ | $OC_2H_5$ | N | |
| H | $NO_2$ | H | $CH_3$ | $CH_2OCH_3$ | N | |
| H | $NO_2$ | H | $OCH_3$ | $CH_2OCH_3$ | N | |
| H | $NO_2$ | H | $OCH_3$ | $NH_2$ | N | |
| H | $NO_2$ | H | $OCH_3$ | $NHCH_3$ | N | |
| H | $NO_2$ | H | $OCH_3$ | $N(CH_3)_2$ | N | |
| H | $OCH_3$ | H | $CH_3$ | $CH_3$ | CH | |
| H | $OCH_3$ | H | $OCH_3$ | $CH_3$ | CH | |
| H | $OCH_3$ | H | $OCH_3$ | OCH | CH | |
| H | $OCH_3$ | H | $CH_3$ | $C_2H_5$ | CH | |
| H | $OCH_3$ | H | $OCH_3$ | $C_2H_5$ | CH | |
| H | $OCH_3$ | H | $CH_3$ | $OC_2H_5$ | CH | |
| H | $OCH_3$ | H | $OCH_3$ | $OC_2H_5$ | CH | |
| H | $OCH_3$ | H | $CH_3$ | $CH_2OCH_3$ | CH | |
| H | $OCH_3$ | H | $OCH_3$ | $CH_2OCH_3$ | CH | |
| H | $OCH_3$ | H | $OCH_3$ | $NH_2$ | CH | |
| H | $OCH_3$ | H | $OCH_3$ | $NHCH_3$ | CH | |
| H | $OCH_3$ | H | $OCH_3$ | $N(CH_3)_2$ | CH | |
| H | $OCH_3$ | H | $CH_3$ | $CH_3$ | N | |
| H | $OCH_3$ | H | $OCH_3$ | $CH_3$ | N | |
| H | $OCH_3$ | H | $OCH_3$ | $OCH_3$ | N | |
| H | $OCH_3$ | H | $CH_3$ | $C_2H_5$ | N | |
| H | $OCH_3$ | H | $OCH_3$ | $C_2H_5$ | N | |
| H | $OCH_3$ | H | $CH_3$ | $OC_2H_5$ | N | |
| H | $OCH_3$ | H | $OCH_3$ | $OC_2H_5$ | N | |

Table IIIa (continued)

| $R_9$ | R | $R_{10}$ | X | Y | Z | m.p.(°C) |
|---|---|---|---|---|---|---|
| H | $OCH_3$ | H | $CH_3$ | $CH_2OCH_3$ | N | |
| H | $OCH_3$ | H | $OCH_3$ | $CH_2OCH_3$ | N | |
| H | $OCH_3$ | H | $OCH_3$ | $NH_2$ | N | |
| H | $OCH_3$ | H | $OCH_3$ | $NHCH_3$ | N | |
| H | $OCH_3$ | H | $OCH_3$ | $N(CH_3)_2$ | N | |
| H | $OC_2H_5$ | H | $CH_3$ | $CH_3$ | CH | |
| H | $OCH(CH_3)_2$ | H | $OCH_3$ | $CH_3$ | CH | |
| H | $OCH_2CH_2CH_3$ | H | $OCH_3$ | $OCH_3$ | CH | |
| H | $OC_2H_5$ | H | $CH_3$ | $C_2H_5$ | CH | |
| H | $OC_2H_5$ | H | $OCH_3$ | $C_2H_5$ | CH | |
| H | $OCH(CH_3)_2$ | H | $CH_3$ | $OC_2H_5$ | CH | |
| H | $OC_2H_5$ | H | $OCH_3$ | $OC_2H_5$ | N | |
| H | $OC_2H_5$ | H | $CH_3$ | $CH_2OCH_3$ | N | |
| H | $OCH_2CH_2CH_3$ | H | $OCH_3$ | $CH_2OCH_3$ | N | |
| H | $OCH_2CH_2CH_3$ | H | $OCH_3$ | $NH_2$ | N | |
| H | $OC_2H_5$ | H | $OCH_3$ | $NHCH_3$ | N | |
| H | $OC_2H_5$ | H | $OCH_3$ | $N(CH_3)_2$ | N | |
| H | $SO_2N(CH_3)_2$ | H | $CH_3$ | $CH_3$ | CH | |
| H | $SO_2N(CH_3)_2$ | H | $CH_3$ | $OCH_3$ | CH | |
| H | $SO_2N(CH_3)_2$ | H | $OCH_3$ | $OCH_3$ | CH | |
| H | $SO_2N(C_2H_5)_2$ | H | $CH_3$ | $CH_3$ | CH | |
| H | $SO_2N(C_2H_5)_2$ | H | $CH_3$ | $OCH_3$ | CH | |
| H | $SO_2N(C_2H_5)_2$ | H | $OCH_3$ | $OCH_3$ | CH | |
| H | $SO_2N(CH_3)C_2H_5$ | H | $CH_3$ | $CH_3$ | CH | |
| H | $SO_2N(CH_3)C_2H_5$ | H | $CH_3$ | $OCH_3$ | CH | |
| H | $SO_2N(CH_3)C_2H_5$ | H | $OCH_3$ | $OCH_3$ | CH | |
| H | $SO_2N(CH_3)OCH_3$ | H | $CH_3$ | $CH_3$ | CH | |
| H | $SO_2N(CH_3)OCH_3$ | H | $CH_3$ | $OCH_3$ | CH | |
| H | $SO_2N(CH_3)OCH_3$ | H | $OCH_3$ | $OCH_3$ | CH | |
| H | $OSO_2CF_3$ | H | $CH_3$ | $CH_3$ | CH | |
| H | $OSO_2CF_3$ | H | $CH_3$ | $OCH_3$ | CH | |
| H | $OSO_2CF_3$ | H | $OCH_3$ | $OCH_3$ | CH | |

Table IIIa (continued)

| $R_9$ | R | $R_{10}$ | X | Y | Z | m.p.(°C) |
|---|---|---|---|---|---|---|
| H | $OSO_2CH_3$ | H | $CH_3$ | $CH_3$ | CH | |
| H | $OSO_2CH_3$ | H | $CH_3$ | $OCH_3$ | CH | |
| H | $OSO_2CH_3$ | H | $OCH_3$ | $OCH_3$ | CH | |
| H | $OSO_2C_2H_5$ | H | $CH_3$ | $CH_3$ | CH | |
| H | $OSO_2C_2H_5$ | H | $CH_3$ | $OCH_3$ | CH | |
| H | $OSO_2C_2H_5$ | H | $OCH_3$ | $OCH_3$ | CH | |
| H | $SO_2N(OCH_3)CH_3$ | H | $CH_3$ | $OCH_3$ | CH | |
| H | $OSO_2$-n-$C_3H_7$ | H | $CH_3$ | $CH_3$ | CH | |
| H | $OSO_2$-n-$C_3H_7$ | H | $CH_3$ | $OCH_3$ | CH | |
| H | $OSO_2$-n-$C_3H_7$ | H | $OCH_3$ | $OCH_3$ | CH | |
| H | $CO_2CH_3$ | H | $CH_3$ | $CH_3$ | CH | |
| H | $CO_2CH_3$ | H | $CH_3$ | $OCH_3$ | CH | |
| H | $CO_2CH_3$ | H | $OCH_3$ | $OCH_3$ | CH | |
| H | $CO_2C_2H_5$ | H | $CH_3$ | $CH_3$ | CH | |
| H | $CO_2C_2H_5$ | H | $CH_3$ | $OCH_3$ | CH | |
| H | $CO_2C_2H_5$ | H | $OCH_3$ | $OCH_3$ | CH | |
| H | $CO_2$-n-$C_3H_7$ | H | $CH_3$ | $CH_3$ | CH | |
| H | $CO_2$-n-$C_3H_7$ | H | $CH_3$ | $OCH_3$ | CH | |
| H | $CO_2$-n-$C_3H_7$ | H | $OCH_3$ | $OCH_3$ | CH | |
| H | $CO_2$-i-$C_3H_7$ | H | $CH_3$ | $CH_3$ | CH | |
| H | $CO_2$-i-$C_3H_7$ | H | $CH_3$ | $OCH_3$ | CH | |
| H | $CO_2$-i-$C_3H_7$ | H | $OCH_3$ | $OCH_3$ | CH | |
| H | $CO_2CH_2CH{=}CH_2$ | H | $CH_3$ | $CH_3$ | CH | |
| H | $CO_2CH_2CH{=}CH_2$ | H | $CH_3$ | $OCH_3$ | CH | |
| H | $CO_2CH_2CH{=}CH_2$ | H | $OCH_3$ | $OCH_3$ | CH | |
| H | $CO_2CH_2CH_2OCH_3$ | H | $CH_3$ | $CH_3$ | CH | |
| H | $CO_2CH_2CH_2OCH_3$ | H | $CH_3$ | $OCH_3$ | CH | |
| H | $CO_2CH_2CH_2OCH_3$ | H | $OCH_3$ | $OCH_3$ | CH | |
| H | $CO_2CH_2CH_2Cl$ | H | $CH_3$ | $CH_3$ | CH | |
| H | $CO_2CH_2CH_2Cl$ | H | $CH_3$ | $OCH_3$ | CH | |
| H | $CO_2CH_2CH_2Cl$ | H | $OCH_3$ | $OCH_3$ | CH | |
| H | $SO_2CH_3$ | H | $CH_3$ | $CH_3$ | CH | |

Table IIIa (continued)

| $R_9$ | R | $R_{10}$ | X | Y | Z | m.p.(°C) |
|---|---|---|---|---|---|---|
| H | $SO_2CH_3$ | H | $CH_3$ | $OCH_3$ | CH | |
| H | $SO_2CH_3$ | H | $OCH_3$ | $OCH_3$ | CH | |
| H | $CH_3$ | H | $OCH_3$ | $OCH_3$ | N | |
| H | $C_2H_5$ | H | $OCH_3$ | $OCH_3$ | N | |
| H | $n$-$C_3H_7$ | H | $OCH_3$ | $OCH_3$ | N | |
| H | $OCF_2H$ | H | $OCH_3$ | $OCH_3$ | N | |
| H | $OCF_3$ | H | $OCH_3$ | $OCH_3$ | N | |
| H | $OCF_2CF_2H$ | H | $OCH_3$ | $OCH_3$ | N | |
| H | $SCH_3$ | H | $OCH_3$ | $OCH_3$ | N | |
| H | S-$n$-$C_3H_7$ | H | $OCH_3$ | $OCH_3$ | N | |
| H | $OSO_2CF_3$ | H | $OCH_3$ | $OCH_3$ | N | |
| 5-$CH_3$ | H | H | $CH_3$ | $OCH_3$ | CH | |
| 6-$CH_3$ | H | H | $OCH_3$ | $OCH_3$ | CH | |
| 7-$CH_3$ | H | H | $CH_3$ | $OCH_3$ | N | |
| 8-$CH_3$ | H | H | $OCH_3$ | $OCH_3$ | N | |
| H | H | $CH_3$ | $OCH_3$ | $OCH_3$ | CH | |
| H | H | $CH_3$ | $CH_3$ | $OCH_3$ | CH | |
| H | H | $CH_3$ | $CH_3$ | $OCH_3$ | N | |
| H | H | $CH_3$ | $OCH_3$ | $OCH_3$ | N | |

Table IIIb

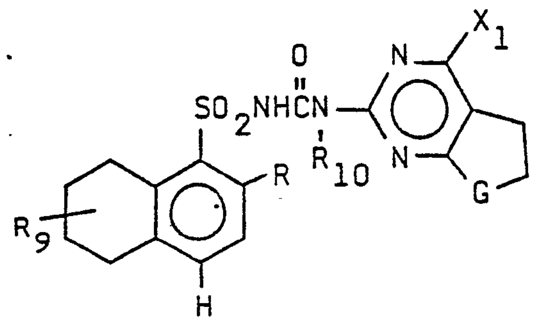

| $R_9$ | R | $R_{10}$ | G | $X_1$ | m.p.(°C) |
|---|---|---|---|---|---|
| H | H | H | O | $CH_3$ | |
| H | H | H | O | $OCH_3$ | |
| H | H | H | $CH_2$ | $CH_3$ | |
| H | H | H | $CH_2$ | $OCH_3$ | |
| 5-$CH_3$ | H | H | O | $OCH_3$ | |
| H | Cl | H | $CH_2$ | $CH_3$ | |
| H | $CH_3$ | H | O | $CH_3$ | |
| H | H | $CH_3$ | O | $OCH_3$ | |

Table IIIc

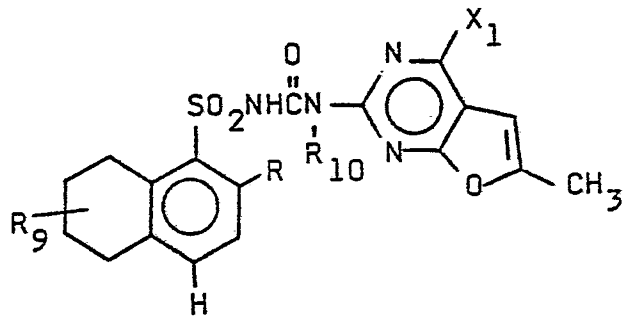

| $R_9$ | R | $R_{10}$ | $X_1$ | m.p.(°C) |
|---|---|---|---|---|
| H | H | H | $CH_3$ | |
| H | H | H | $OCH_3$ | |
| 5-$CH_3$ | H | H | $CH_3$ | |
| H | Cl | H | $OCH_3$ | |
| H | $CH_3$ | H | $OCH_3$ | |
| H | H | $CH_3$ | $CH_3$ | |

Table IIId

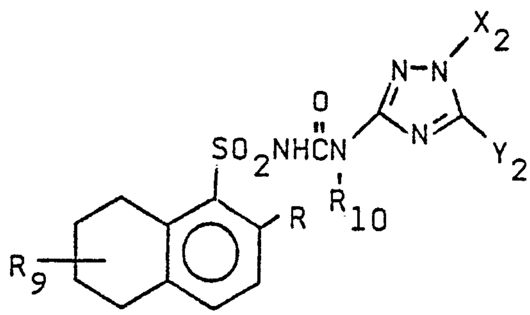

| $R_9$ | R | $R_{10}$ | $X_2$ | $Y_2$ | m.p.(°C) |
|---|---|---|---|---|---|
| H | H | H | $CH_3$ | $SCH_3$ | |
| H | H | H | $CH_3$ | $OCH_3$ | |
| H | H | H | $CH_3$ | $OC_2H_5$ | |
| H | H | H | $CH_3$ | $SC_2H_5$ | |
| H | H | H | $C_2H_5$ | $OCH_3$ | |
| H | H | H | $C_2H_5$ | $OC_2H_5$ | |
| H | H | H | $C_2H_5$ | $SCH_3$ | |
| H | H | H | $\underline{n}$-$C_3H_7$ | $OCH_3$ | |
| H | H | H | $CH_2CF_3$ | $OCH_3$ | |
| H | H | H | $CH_2CF_3$ | $SCH_3$ | |
| 5-$CH_3$ | H | H | $CH_3$ | $OCH_3$ | |
| H | Cl | H | $CH_3$ | $OCH_3$ | |
| H | $CH_3$ | H | $CH_3$ | $OCH_3$ | |
| H | H | $CH_3$ | $CH_3$ | $OCH_3$ | |

Table IVa

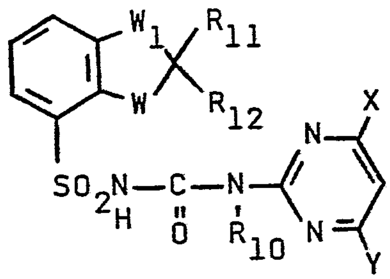

| $W_1$ | W | $R_{11}$ | $R_{12}$ | $R_{10}$ | X | Y | m.p.(°C) |
|---|---|---|---|---|---|---|---|
| O | O | H | H | H | $CH_3$ | $CH_3$ | |
| O | O | H | H | H | $CH_3$ | $OCH_3$ | |
| O | O | H | H | H | $CH_3$ | $OC_2H_5$ | |
| O | O | H | H | H | $OCH_3$ | $OCH_3$ | |
| O | O | H | H | H | $OCH_3$ | $OC_2H_3$ | |
| O | O | H | H | H | $OCH_3$ | $SCH_3$ | |
| O | O | H | H | H | $OCH_3$ | $CH(OCH_3)_2$ | |
| O | O | H | H | H | Cl | $NH_2$ | |
| O | O | H | H | H | Cl | $NHCH_3$ | |
| O | O | H | H | H | Cl | $N(CH_3)$ | |
| O | O | H | H | H | Cl | $OCH_3$ | |
| O | O | H | H | $CH_3$ | $CH_3$ | $CH_3$ | |
| O | O | H | H | $CH_3$ | $CH_3$ | $OCH_3$ | |
| O | O | H | $CH_3$ | H | $CH_3$ | $CH_3$ | |
| O | O | H | $CH_3$ | H | $CH_3$ | $OCH_3$ | |
| O | O | H | $CH_3$ | H | $CH_3$ | $SCH_3$ | |
| O | O | H | $CH_3$ | H | $CH_3$ | $CH(OCH_3)_2$ | |
| O | O | H | $CH_3$ | H | $OCH_3$ | $CH_3$ | |
| O | O | H | $CH_3$ | H | $OCH_3$ | $OCH_3$ | |
| O | O | H | $CH_3$ | H | $OCH_3$ | 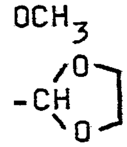 | |
| O | O | H | $CH_3$ | H | Cl | $N(CH_3)_2$ | |
| O | O | H | $C_2H_5$ | H | $CH_3$ | $NH_2$ | |
| O | O | H | $C_2H_5$ | H | $CH_3$ | $OC_2H_5$ | |
| O | O | H | $C_2H_5$ | H | $CH_3$ | $SCH_3$ | |
| O | O | H | $C_2H_5$ | H | $OCH_3$ | $CH_2OCH_3$ | |
| O | O | H | $C_2H_5$ | H | $OCH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| O | O | H | $CH_3CH_2CH_2$ | H | $CH_3$ | $N(CH_3)_2$ | |
| O | O | H | $CH_3CH_2CH_2$ | H | $CH_3$ | $OCH_3$ | |
| O | O | H | $CH_3CH_2CH_2$ | H | $OCH_3$ | $NH_2$ | |
| O | O | H | $CH(CH_3)_2$ | H | $OCH_3$ | $OCH_3$ | |
| O | O | H | $CH_3CH_2CH_2CH_2$ | H | $CH_3$ | $OCH_3$ | |
| O | O | H | $CH_3CH(CH_3)CH_2$ | H | $CH_3$ | $NH(CH_3)$ | |
| O | O | H | $CH_3CH_2CH(CH_3)$ | H | $OCH_3$ | $OC_2H_5$ | |
| O | O | H | $(CH_3)_3C$ | H | $CH_3$ | $OCH_3$ | |
| O | O | H | $(CH_3)_3C$ | H | $OCH_3$ | $OCH_3$ | |
| O | O | $CH_3$ | $CH_3$ | H | $CH_3$ | H | |
| O | O | $CH_3$ | $CH_3$ | H | $CH_3$ | $CH_3$ | 168-171° |
| O | O | $CH_3$ | $CH_3$ | H | $CH_3$ | $OCH_3$ | 180-183° |
| O | O | $CH_3$ | $CH_3$ | H | $CH_3$ | $OC_2H_5$ | |
| O | O | $CH_3$ | $CH_3$ | H | $CH_3$ | $CH_2OCH_3$ | |
| O | O | $CH_3$ | $CH_3$ | H | $CH_3$ | $SCH_3$ | |
| O | O | $CH_3$ | $CH_3$ | H | $OCH_3$ | $OCH_3$ | 194-203° |
| O | O | $CH_3$ | $CH_3$ | H | Cl | $OCH_3$ | |
| O | O | $CH_3$ | $CH_3$ | H | Cl | $NH_2$ | |
| O | O | $CH_3$ | $CH_3$ | H | Cl | $NH(CH_3)$ | |
| O | O | $CH_3$ | $CH_3$ | H | Cl | $N(CH_3)_2$ | |
| O | O | $CH_3$ | $CH_3$ | $CH_3$ | $CH_3$ | $CH_3$ | |
| O | O | $CH_3$ | $CH_3$ | $CH_3$ | $CH_3$ | $OCH_3$ | |
| O | O | $CH_3$ | $CH_3$ | $CH_3$ | $OCH_3$ | $OCH_3$ | |
| O | O | $CH_3$ | $CH_3CH_2$ | H | $CH_3$ | $CH_3$ | |
| O | O | $CH_3$ | $CH_3CH_2$ | H | $CH_3$ | 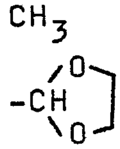 | |
| O | O | $CH_3$ | $CH_3CH_2$ | H | $OCH_3$ | $OCH_3$ | |
| O | O | $CH_3$ | $CH_3CH_2CH_2$ | H | $CH_3$ | $OCH_3$ | |
| O | O | $CH_3$ | $CH_3CH_2CH_2$ | H | $OCH_3$ | $OCH_3$ | |
| O | O | $CH_3$ | $CH_3CH_2CH_2$ | H | $OCH_3$ | $OC_2H_5$ | |
| O | O | $CH_3$ | $(CH_3)_2CH$ | H | $OCH_3$ | $OCH_3$ | |
| O | O | $CH_3$ | $CH_3CH_2CH_2CH_2-$ | H | $CH_3$ | $CH_3$ | |

Table IVa (continued)

| $W_1$ | W | $R_{11}$ | $R_{12}$ | $R_{10}$ | X | Y | m.p.(°C) |
|---|---|---|---|---|---|---|---|
| O | O | $CH_3$ | $CH_3CH_2CH_2CH_2$- | H | $CH_3$ | $N(CH_3)_2$ | |
| O | O | $CH_3$ | $CH_3CH_2CH_2CH_2$- | H | Cl | $N(CH_3)_2$ | |
| O | O | $CH_3$ | $CH_3CH(CH_3)CH_2$ | H | $CH_3$ | $OCH_3$ | |
| O | O | $CH_3$ | $CH_3CH(CH_3)CH_2$ | H | $CH_3$ | $NHCH_3$ | |
| O | O | $CH_3$ | $CH_3CH_2CH(CH_3)$ | H | $CH_3$ | $OCH_3$ | |
| O | O | $CH_3$ | $(CH_3)_3C$ | H | $CH_3$ | $CH(OCH_3)_2$ | |
| S | S | H | H | H | $CH_3$ | $CH_3$ | |
| S | S | H | H | H | $CH_3$ | $OCH_3$ | |
| S | S | H | H | H | $CH_3$ | $OC_2H_5$ | |
| S | S | H | H | H | $OCH_3$ | $OCH_3$ | |
| S | S | H | H | H | $OCH_3$ | $OC_2H_5$ | |
| S | S | H | H | H | $OCH_3$ | $SCH_3$ | |
| S | S | H | H | H | $OCH_3$ | $CH(OCH_3)_2$ | |
| S | S | H | H | H | Cl | $NH_2$ | |
| S | S | H | H | H | Cl | $NHCH_3$ | |
| S | S | H | H | H | Cl | $N(CH_3)_2$ | |
| S | S | H | H | H | Cl | $OCH_3$ | |
| S | S | H | H | $CH_3$ | $CH_3$ | $CH_3$ | |
| S | S | H | H | $CH_3$ | $CH_3$ | $OCH_3$ | |
| S | S | H | $CH_3$ | H | $CH_3$ | $CH_3$ | |
| S | S | H | $CH_3$ | H | $CH_3$ | $OCH_3$ | |
| S | S | H | $CH_3$ | H | $CH_3$ | $N(CH_3)_2$ | |
| S | S | H | $CH_3$ | H | $OCH_3$ | $OCH_3$ | |
| S | S | H | $CH_3$ | H | $OCH_3$ | 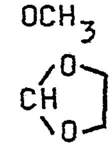 | |
| S | S | H | $CH_3$ | H | Cl | $N(CH_3)_2$ | |
| S | S | H | $C_2H_5$ | H | $CH_3$ | $CH_3$ | |
| S | S | H | $C_2H_5$ | H | $CH_3$ | $OCH_3$ | |
| S | S | H | $C_2H_5$ | H | $CH_3$ | $SCH_3$ | |
| S | S | H | $C_2H_5$ | H | $OCH_3$ | $OCH_3$ | |
| S | S | H | $CH_3CH_2CH_2$ | H | $CH_3$ | $CH_3$ | |
| S | S | H | $CH_3CH_2CH_2$ | H | $CH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| S | S | H | $CH(CH_3)_2$ | H | $OCH_3$ | $OCH_3$ | |
| S | S | H | $CH_3CH_2CH_2CH_2$ | H | $CH_3$ | $OCH_3$ | |
| S | S | H | $CH_3CH(CH_3)CH_2$ | H | $CH_3$ | $CH_3$ | |
| S | S | H | $CH_3CH_2CH(CH_3)$ | H | $OCH_3$ | $OCH_3$ | |
| S | S | H | $(CH_3)_3C$ | H | $CH_3$ | $OCH_3$ | |
| S | S | H | $(CH_3)_3C$ | H | $OCH_3$ | $OCH_3$ | |
| S | S | $CH_3$ | $CH_3$ | H | $CH_3$ | H | |
| S | S | $CH_3$ | $CH_3$ | H | $CH_3$ | $CH_3$ | |
| S | S | $CH_3$ | $CH_3$ | H | $CH_3$ | $OCH_3$ | |
| S | S | $CH_3$ | $CH_3$ | H | $CH_3$ | $OC_2H_5$ | |
| S | S | $CH_3$ | $CH_3$ | H | $CH_3$ | $CH_2OCH_3$ | |
| S | S | $CH_3$ | $CH_3$ | H | $CH_3$ | $SCH_3$ | |
| S | S | $CH_3$ | $CH_3$ | H | $OCH_3$ | $OCH_3$ | |
| S | S | $CH_3$ | $CH_3$ | H | $OCH_3$ | 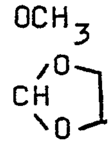 | |
| S | S | $CH_3$ | $CH_3$ | H | Cl | $OCH_3$ | |
| S | S | $CH_3$ | $CH_3$ | H | Cl | $NH_2$ | |
| S | S | $CH_3$ | $CH_3$ | H | Cl | $NH(CH_3)$ | |
| S | S | $CH_3$ | $CH_3$ | H | Cl | $N(CH_3)_2$ | |
| S | S | $CH_3$ | $CH_3$ | $CH_3$ | $CH_3$ | $OCH_3$ | |
| S | S | $CH_3$ | $CH_3$ | $CH_3$ | $OCH_3$ | $OCH_3$ | |
| S | S | $CH_3$ | $CH_3CH_2$ | H | $CH_3$ | $CH_3$ | |
| S | S | $CH_3$ | $CH_3CH_2$ | H | $CH_3$ | $OCH_3$ | |
| S | S | $CH_3$ | $CH_3CH_2$ | H | $OCH_3$ | $OCH_3$ | |
| S | S | $CH_3$ | $CH_3CH_2$ | H | $OCH_3$ | $CH(OCH_3)_2$ | |
| S | S | $CH_3$ | $CH_3CH_2CH_2$ | H | $CH_3$ | $CH_3$ | |
| S | S | $CH_3$ | $CH_3CH_2CH_2$ | H | $CH_3$ | $CH(OCH_3)_2$ | |
| S | S | $CH_3$ | $CH_3CH_2CH_2$ | H | $OCH_3$ | $OCH_3$ | |
| S | S | $CH_3$ | $CH_3CH_2CH_2$ | H | $OCH_3$ | $OC_2H_5$ | |
| S | S | $CH_3$ | $(CH_3)_2CH$ | H | $OCH_3$ | $OCH_3$ | |
| S | S | $CH_3$ | $CH_3CH_2CH_2CH_2-$ | H | $CH_3$ | $SCH_3$ | |
| S | S | $CH_3$ | $CH_3CH_2CH_2CH_2-$ | H | $OCH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| S | S | $CH_3$ | $CH_3CH(CH_3)CH_2$ | H | $CH_3$ | $OCH_3$ | |
| S | S | $CH_3$ | $CH_3CH(CH_3)CH_2$ | H | Cl | $N(CH_3)_2$ | |
| S | S | $CH_3$ | $CH_3CH_2CH(CH_3)$ | H | $CH_3$ | $OCH_3$ | |
| S | S | $CH_3$ | $(CH_3)_3C$ | H | $CH_3$ | $OCH_3$ | |
| $SO_2$ | $SO_2$ | H | H | H | $CH_3$ | $CH_3$ | |
| $SO_2$ | $SO_2$ | H | H | H | $CH_3$ | $OCH_3$ | |
| $SO_2$ | $SO_2$ | H | H | H | $CH_3$ | $OC_2H_5$ | |
| $SO_2$ | $SO_2$ | H | H | H | $OCH_3$ | $OCH_3$ | |
| $SO_2$ | $SO_2$ | H | H | H | $OCH_3$ | $OC_2H_5$ | |
| $SO_2$ | $SO_2$ | H | H | H | $OCH_3$ | $SCH_3$ | |
| $SO_2$ | $SO_2$ | H | H | H | $OCH_3$ | $CH(OCH_3)_2$ | |
| $SO_2$ | $SO_2$ | H | H | H | Cl | $N(CH_3)_2$ | |
| $SO_2$ | $SO_2$ | H | H | H | Cl | $OCH_3$ | |
| $SO_2$ | $SO_2$ | H | H | $CH_3$ | $CH_3$ | $CH_3$ | |
| $SO_2$ | $SO_2$ | H | H | $CH_3$ | $CH_3$ | $OCH_3$ | |
| $SO_2$ | $SO_2$ | H | $CH_3$ | H | $CH_3$ | $CH_3$ | |
| $SO_2$ | $SO_2$ | H | $CH_3$ | H | $CH_3$ | $SCH_3$ | |
| $SO_2$ | $SO_2$ | H | $CH_3$ | H | $OCH_3$ | $OCH_3$ | |
| $SO_2$ | $SO_2$ | $CH_3$ | $CH_3$ | H | $CH_3$ | H | |
| $SO_2$ | $SO_2$ | $CH_3$ | $CH_3$ | H | $CH_3$ | $CH_3$ | |
| $SO_2$ | $SO_2$ | $CH_3$ | $CH_3$ | H | $CH_3$ | $OCH_3$ | 191-200° |
| $SO_2$ | $SO_2$ | $CH_3$ | $CH_3$ | H | $CH_3$ | $OC_2H_5$ | |
| $SO_2$ | $SO_2$ | $CH_3$ | $CH_3$ | H | $OCH_3$ | $SCH_3$ | |
| $SO_2$ | $SO_2$ | $CH_3$ | $CH_3$ | H | $OCH_3$ | $OCH_3$ | |
| $SO_2$ | $SO_2$ | $CH_3$ | $CH_3$ | H | Cl | $OCH_3$ | |
| $SO_2$ | $SO_2$ | $CH_3$ | $CH_3$ | H | Cl | $NH_2$ | |
| $SO_2$ | $SO_2$ | $CH_3$ | $CH_3$ | H | Cl | $NH(CH_3)$ | |
| $SO_2$ | $SO_2$ | $CH_3$ | $CH_3$ | H | Cl | $N(CH_3)_2$ | |
| $SO_2$ | $SO_2$ | $CH_3$ | $CH_3$ | $CH_3$ | $CH_3$ | $OCH_3$ | |
| $SO_2$ | $SO_2$ | $CH_3$ | $CH_3$ | $CH_3$ | $OCH_3$ | $OCH_3$ | |
| $SO_2$ | $SO_2$ | $CH_3$ | $CH_3CH_2$ | H | $CH_3$ | $CH_3$ | |
| $SO_2$ | $SO_2$ | $CH_3$ | $CH_3CH_2$ | H | $CH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| $SO_2$ | $SO_2$ | $CH_3$ | $CH_3CH_2$ | H | $OCH_3$ | $OCH_3$ | |
| $SO_2$ | $SO_2$ | $CH_3$ | $CH_3CH_2$ | H | $OCH_3$ | $CH(OCH_3)_2$ | |
| $SO_2$ | $SO_2$ | $CH_3$ | $CH_3CH_2CH_2$ | H | $CH_3$ | $CH_3$ | |
| $SO_2$ | $SO_2$ | $CH_3$ | $CH_3CH_2CH_2$ | H | $CH_3$ | $OCH_3$ | |
| $SO_2$ | $SO_2$ | $CH_3$ | $CH_3CH_2CH_2$ | H | $OCH_3$ | H | |
| $SO_2$ | $SO_2$ | $CH_3$ | $CH_3CH_2CH_2$ | H | $OCH_3$ | $OCH_3$ | |
| $SO_2$ | $SO_2$ | $CH_3$ | $CH_3CH_2CH_2$ | H | $OCH_3$ | $OC_2H_5$ | |
| $SO_2$ | $SO_2$ | $CH_3$ | $(CH_3)_2CH$ | H | $OCH_3$ | $OCH_3$ | |
| $SO_2$ | $SO_2$ | $CH_3$ | $CH_3CH_2CH_2CH_2$- | H | $CH_3$ | $CH_3$ | |
| $SO_2$ | $SO_2$ | $CH_3$ | $CH_3CH_2CH_2CH_2$- | H | $CH_3$ | $SCH_3$ | |
| $SO_2$ | $SO_2$ | $CH_3$ | $CH_3CH_2CH_2CH_2$- | H | $OCH_3$ | H | |
| $SO_2$ | $SO_2$ | $CH_3$ | $CH_3CH_2CH_2CH_2$- | H | $OCH_3$ | $OCH_3$ | |
| $SO_2$ | $SO_2$ | $CH_3$ | $CH_3CH_2CH_2CH_2$- | H | Cl | $N(CH_3)_2$ | |
| $SO_2$ | $SO_2$ | $CH_3$ | $CH_3CH(CH_3)CH_2$ | H | $CH_3$ | $OCH_3$ | |
| $SO_2$ | $SO_2$ | $CH_3$ | $CH_3CH(CH_3)CH_2$ | H | $OCH_3$ | $OCH_3$ | |
| $SO_2$ | $SO_2$ | $CH_3$ | $CH_3CH(CH_3)CH_2$ | H | Cl | $N(CH_3)_2$ | |
| $SO_2$ | $SO_2$ | $CH_3$ | $CH_3CH_2CH(CH_3)$ | H | $CH_3$ | $CH_3$ | |
| $SO_2$ | $SO_2$ | $CH_3$ | $CH_3CH_2CH(CH_3)$ | H | $CH_3$ | $OCH_3$ | |
| $SO_2$ | $SO_2$ | $CH_3$ | $(CH_3)_3C$ | H | $CH_3$ | $OCH_3$ | |
| $SO_2$ | $SO_2$ | H | $CH_3$ | H | Cl | $N(CH_3)_2$ | |
| $SO_2$ | $SO_2$ | H | $C_2H_5$ | H | $CH_3$ | $CH_3$ | |
| $SO_2$ | $SO_2$ | H | $C_2H_5$ | H | $CH_3$ | $OCH_3$ | |
| $SO_2$ | $SO_2$ | H | $C_2H_5$ | H | $CH_3$ | $SCH_3$ | |
| $SO_2$ | $SO_2$ | H | $C_2H_5$ | H | $OCH_3$ | $OCH_3$ | |
| $SO_2$ | $SO_2$ | H | $CH_3CH_2CH_2$ | H | $CH_3$ | $CH_3$ | |
| $SO_2$ | $SO_2$ | H | $CH_3CH_2CH_2$ | H | $CH_3$ | $OCH_3$ | |
| $SO_2$ | $SO_2$ | H | $CH(CH_3)_2$ | H | $OCH_3$ | $OCH_3$ | |
| $SO_2$ | $SO_2$ | H | $CH_3CH_2CH_2CH_2$ | H | $CH_3$ | $OCH_3$ | |
| $SO_2$ | $SO_2$ | H | $CH_3CH(CH_3)CH_2$ | H | $CH_3$ | $CH_3$ | |
| $SO_2$ | $SO_2$ | H | $CH_3CH_2CH(CH_3)$ | H | $OCH_3$ | $OCH_3$ | |
| $SO_2$ | $SO_2$ | H | $(CH_3)_3C$ | H | $CH_3$ | $OCH_3$ | |
| $SO_2$ | $SO_2$ | H | $(CH_3)_3C$ | H | $OCH_3$ | $OCH_3$ | |
| $SO_2$ | $SO_2$ | H | $CH_3$ | H | $CH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| SO | SO | H | H | H | $CH_3$ | $CH_3$ | |
| SO | SO | H | H | H | $CH_3$ | $OCH_3$ | |
| SO | SO | H | H | H | $OCH_3$ | $SCH_3$ | |
| SO | SO | H | H | $CH_3$ | $CH_3$ | $CH_3$ | |
| SO | SO | H | H | $CH_3$ | $CH_3$ | $OCH_3$ | |
| SO | SO | H | $CH_3$ | H | $CH_3$ | $OCH_3$ | |
| SO | SO | $CH_3$ | $CH_3$ | H | $CH_3$ | $CH_3$ | |
| SO | SO | $CH_3$ | $CH_3$ | H | $CH_3$ | $OCH_3$ | |
| SO | SO | $CH_3$ | $CH_3$ | $CH_3$ | $OCH_3$ | $OCH_3$ | |
| SO | SO | $CH_3$ | $CH_3$ | $CH_3$ | $CH_3$ | $OCH_3$ | |
| SO | SO | H | $CH_3CH_2CH_2CH_2$ | H | $CH_3$ | $OCH_3$ | |
| SO | SO | H | $CH_3CH_2CH_2CH_2$ | H | $OCH_3$ | $OCH_3$ | |
| O | S | H | H | H | $CH_3$ | $CH_3$ | |
| O | S | H | H | H | $CH_3$ | $OCH_3$ | |
| O | S | H | $CH_3$ | H | $CH_3$ | $OCH_3$ | |
| O | S | $CH_3$ | $CH_3$ | H | $CH_3$ | $OCH_3$ | |
| O | S | $CH_3$ | $CH_3CH_2$ | H | $CH_3$ | $OCH_3$ | |
| O | SO | H | H | H | $CH_3$ | $CH_3$ | |
| O | SO | H | H | H | $CH_3$ | $OCH_3$ | |
| O | SO | H | $CH_3$ | H | $CH_3$ | $SCH_3$ | |
| O | SO | $CH_3$ | $CH_3$ | H | $CH_3$ | $CH_3$ | |
| O | SO | $CH_3$ | $CH_3$ | H | $OCH_3$ | $OCH_3$ | |
| O | SO | $CH_3$ | $CH_3$ | H | Cl | $N(CH_3)_2$ | |
| O | $SO_2$ | H | H | H | $CH_3$ | $CH_3$ | |
| O | $SO_2$ | H | H | H | $CH_3$ | $OCH_3$ | |
| O | $SO_2$ | H | H | H | Cl | $N(CH_3)_2$ | |
| O | $SO_2$ | H | $CH_3$ | H | $CH_3$ | $CH_3$ | |
| O | $SO_2$ | H | $CH_3$ | H | $CH_3$ | $OCH_3$ | |
| O | $SO_2$ | H | $CH_3$ | H | $CH_3$ | $CH(OCH_3)_2$ | |
| O | $SO_2$ | H | $CH_3CH_2CH_2CH_2$ | H | $CH_3$ | $OCH_3$ | |
| O | $SO_2$ | $CH_3$ | $CH_3$ | H | $CH_3$ | $CH_3$ | |
| O | $SO_2$ | $CH_3$ | $CH_3$ | H | $CH_3$ | $OCH_3$ | |

Table IVa (continued)

| $W_1$ | W | $R_{11}$ | $R_{12}$ | $R_{10}$ | X | Y | m.p.(°C) |
|---|---|---|---|---|---|---|---|
| O | $SO_2$ | $CH_3$ | $CH_3$ | H | $OCH_3$ | $SCH_3$ | |
| O | $SO_2$ | $CH_3$ | $CH_3CH_2CH_2$ | H | $CH_3$ | $OCH_3$ | |
| S | O | H | H | H | $CH_3$ | $OCH_3$ | |
| S | O | $CH_3$ | $CH_3$ | H | $CH_3$ | $OCH_3$ | |
| SO | O | H | H | H | $CH_3$ | $OCH_3$ | |
| SO | O | $CH_3$ | $CH_3$ | H | $CH_3$ | $OCH_3$ | |
| $SO_2$ | O | H | H | H | $CH_3$ | $CH_3$ | |
| $SO_2$ | O | $CH_3$ | $CH_3$ | H | $CH_3$ | $OCH_3$ | |
| SO | S | H | H | H | $CH_3$ | $OCH_3$ | |
| SO | S | $CH_3$ | $CH_3$ | H | $CH_3$ | $OCH_3$ | |
| $SO_2$ | S | H | H | H | $CH_3$ | $OCH_3$ | |
| $SO_2$ | S | $CH_3$ | $CH_3$ | H | $CH_3$ | $OCH_3$ | |
| S | SO | H | H | H | $CH_3$ | $OCH_3$ | |
| S | SO | H | $CH_3$ | H | $CH_3$ | $OCH_3$ | |
| S | SO | $CH_3$ | $CH_3$ | H | $CH_3$ | $OCH_3$ | |
| S | $SO_2$ | H | H | H | $CH_3$ | $CH_3$ | |
| S | $SO_2$ | H | H | H | $CH_3$ | $OCH_3$ | |
| S | $SO_2$ | H | $CH_3CH_2$ | H | $CH_3$ | $OCH_3$ | |
| S | $SO_2$ | $CH_3$ | $CH_3$ | H | $OCH_3$ | $OCH_3$ | |
| SO | $SO_2$ | H | H | H | $CH_3$ | $OCH_3$ | |
| SO | $SO_2$ | H | $CH_3$ | H | $CH_3$ | $OCH_3$ | |
| SO | $SO_2$ | $CH_3$ | $CH_3$ | H | $CH_3$ | $CH_3$ | |
| SO | $SO_2$ | $CH_3$ | $CH_3$ | H | $CH_3$ | $OCH_3$ | |
| SO | $SO_2$ | $CH_3$ | $CH_3$ | H | Cl | $N(CH_3)_2$ | |
| $SO_2$ | SO | H | H | H | $CH_3$ | $OCH_3$ | |
| $SO_2$ | SO | H | $CH_3$ | H | $CH_3$ | $OCH_3$ | |
| $SO_2$ | SO | H | $CH_3CH_2$ | H | $CH_3$ | $OCH_3$ | |
| $SO_2$ | SO | $CH_3$ | $CH_3$ | H | $CH_3$ | $OCH_3$ | |
| O | $SO_2$ | $CH_3$ | $CH_3$ | H | $OCH_3$ | $OCH_3$ | |
| S | $SO_2$ | H | H | H | $OCH_3$ | $OCH_3$ | |

Table IVb

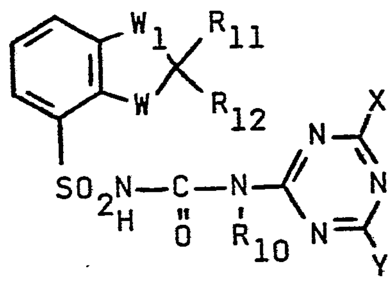

| $W_1$ | W | $R_{11}$ | $R_{12}$ | $R_{10}$ | X | Y | m.p.(°C) |
|---|---|---|---|---|---|---|---|
| O | O | H | H | H | $CH_3$ | $CH_3$ | |
| O | O | H | H | H | $CH_3$ | $OCH_3$ | |
| O | O | H | H | H | $CH_3$ | $SCH_3$ | |
| O | O | H | H | H | $CH_3$ | $OC_2H_5$ | |
| O | O | H | H | H | $CH_3$ | $CH_2OCH_3$ | |
| O | O | H | H | H | $OCH_3$ | $OCH_3$ | |
| O | O | H | H | H | $OCH_3$ | $N(CH_3)_2$ | |
| O | O | H | H | H | $OCH_3$ | $SCH_3$ | |
| O | O | H | H | $CH_3$ | $CH_3$ | $OCH_3$ | |
| O | O | H | H | $CH_3$ | $OCH_3$ | $OCH_3$ | |
| O | O | H | $CH_3$ | H | $CH_3$ | $CH_3$ | |
| O | O | H | $CH_3$ | H | $CH_3$ | $OCH_3$ | |
| O | O | H | $CH_3$ | H | $OCH_3$ | $OCH_3$ | |
| O | O | H | $CH_3$ | H | $OCH_3$ | $NH(CH_3)$ | |
| O | O | H | $CH_3CH_2$ | H | $CH_3$ | $OCH_3$ | |
| O | O | H | $CH_3CH_2$ | H | $OCH_3$ | $OCH_3$ | |
| O | O | H | $CH_3CH_2$ | H | $OCH_3$ | 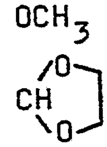 | |
| O | O | H | $CH_3CH_2CH_2$ | H | $CH_3$ | $CH_3$ | |
| O | O | H | $CH_3CH_2CH_2$ | H | $CH_3$ | $OCH_3$ | |
| O | O | H | $CH_3CH_2CH_2$ | H | $OCH_3$ | $CH_2OCH_3$ | |
| O | O | H | $(CH_3)_2CH$ | H | $CH_3$ | $OCH_3$ | |
| O | O | H | $CH_3CH_2CH_2CH_2$ | H | $CH_3$ | $CH_3$ | |
| O | O | H | $CH_3CH_2CH_2CH_2$ | H | $OCH_3$ | $OCH_3$ | |
| O | O | H | $CH_3CH(CH_3)CH_2$ | H | $OCH_3$ | $OCH_3$ | |

Table IVb (continued)

| $W_1$ | W | $R_{11}$ | $R_{12}$ | $R_{10}$ | X | Y | m.p.(°C) |
|---|---|---|---|---|---|---|---|
| O | O | H | $CH_3CH_2CH(CH_3)$ | H | $CH_3$ | $OCH_3$ | |
| O | O | H | $(CH_3)_3C$ | H | $CH_3$ | $OCH_3$ | |
| O | O | $CH_3$ | $CH_3$ | H | $CH_3$ | $CH_3$ | 168-171° |
| O | O | $CH_3$ | $CH_3$ | H | $CH_3$ | $OCH_3$ | 160-168° |
| O | O | $CH_3$ | $CH_3$ | H | $CH_3$ | $SCH_3$ | |
| O | O | $CH_3$ | $CH_3$ | H | $CH_3$ | $CH_2OCH_3$ | |
| O | O | $CH_3$ | $CH_3$ | H | $OCH_3$ | $OCH_3$ | 194-202° |
| O | O | $CH_3$ | $CH_3CH_2$ | H | $CH_2$ | $OC_2H_5$ | |
| O | O | $CH_3$ | $CH_3CH_2$ | H | $OCH_3$ | $OCH_3$ | |
| O | O | $CH_3$ | $CH_3CH_2CH_2$ | H | $CH_3$ | $SCH_3$ | |
| O | O | $CH_3$ | $CH_3CH_2CH_2$ | H | $CH_3$ | $OCH_3$ | |
| O | O | $CH_3$ | $(CH_3)_2CH$ | H | $CH_3$ | $OCH_3$ | |
| O | O | $CH_3$ | $CH_3CH_2CH_2CH_2-$ | H | $CH_3$ | $CH_3$ | |
| O | O | $CH_3$ | $CH_3CH_2CH_2CH_2-$ | H | $CH_3$ | $SCH_3$ | |
| O | O | $CH_3$ | $CH_3CH_2CH_2CH_2-$ | H | $CH_3$ | $OCH_3$ | |
| O | O | $CH_3$ | $CH_3(CH_3)CH_2$ | H | $OCH_3$ | $OCH_3$ | |
| O | O | $CH_3$ | $CH_3CH_2CH(CH_3)$ | H | $CH_3$ | $OCH_3$ | |
| O | O | $CH_3$ | $(CH_3)_3C$ | H | $CH_3$ | $OCH_3$ | |
| S | S | H | H | H | $CH_3$ | $CH_3$ | |
| S | S | H | H | H | $CH_3$ | $OCH_3$ | |
| S | S | H | H | H | $CH_3$ | $OC_2H_5$ | |
| S | S | H | H | H | $CH_3$ | $CH_2OCH_3$ | |
| S | S | H | H | H | $OCH_3$ | $OCH_3$ | |
| S | S | H | H | H | $OCH_3$ | $SCH_3$ | |
| S | S | H | H | H | $OCH_3$ | $CH(OCH_3)_2$ | |
| S | S | H | H | $CH_3$ | $CH_3$ | $OCH_3$ | |
| S | S | H | H | $CH_3$ | $OCH_3$ | $OCH_3$ | |
| S | S | H | $CH_3$ | H | $CH_3$ | $CH_3$ | |
| S | S | H | $CH_3$ | H | $CH_3$ | $OCH_3$ | |
| S | S | H | $CH_3$ | H | $OCH_3$ | $OCH_3$ | |
| S | S | H | $CH_3$ | H | $OCH_3$ | $OC_2H_5$ | |
| S | S | H | $CH_3CH_2$ | H | $CH_3$ | $OCH_3$ | |

Table IVb (continued)

| $W_1$ | W | $R_{11}$ | $R_{12}$ | $R_{10}$ | X | Y | m.p.(°C) |
|---|---|---|---|---|---|---|---|
| S | S | H | $CH_3CH_2$ | H | $OCH_3$ | $OC_2H_5$ | |
| S | S | H | $CH_3CH_2$ | H | $OCH_3$ | 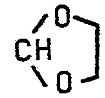 | |
| S | S | H | $CH_3CH_2CH_2$ | H | $CH_3$ | $CH_3$ | |
| S | S | H | $CH_3CH_2CH_2$ | H | $CH_3$ | 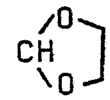 | |
| S | S | H | $CH_3CH_2CH_2$ | H | $OCH_3$ | $OCH_3$ | |
| S | S | H | $(CH_3)_2CH$ | H | $CH_3$ | $OCH_3$ | |
| S | S | H | $CH_3CH_2CH_2CH_2$ | H | $CH_3$ | $CH_3$ | |
| S | S | H | $CH_3CH_2CH_2CH_2$ | H | $CH_3$ | $OCH_3$ | |
| S | S | H | $CH_3CH_2CH_2CH_2$ | H | $OCH_3$ | $OCH_3$ | |
| S | S | H | $CH_3CH_2CH(CH_3)$ | H | $CH_3$ | $OCH_3$ | |
| S | S | H | $(CH_3)_3C$ | H | $CH_3$ | $OCH_3$ | |
| S | S | $CH_3$ | $CH_3$ | H | $CH_3$ | $CH_3$ | |
| S | S | $CH_3$ | $CH_3$ | H | $CH_3$ | $OCH_3$ | |
| S | S | $CH_3$ | $CH_3$ | H | $CH_3$ | $OC_2H_5$ | |
| S | S | $CH_3$ | $CH_3$ | H | $CH_3$ | $CH_2OCH_3$ | |
| S | S | $CH_3$ | $CH_3$ | H | $OCH_3$ | $OCH_3$ | |
| S | S | $CH_3$ | $CH_3$ | $CH_3$ | $CH_3$ | $CH_3$ | |
| S | S | $CH_3$ | $CH_3$ | $CH_3$ | $CH_3$ | $OCH_3$ | |
| S | S | $CH_3$ | $CH_3CH_2$ | H | $CH_3$ | $OCH_3$ | |
| S | S | $CH_3$ | $CH_3CH_2$ | H | $OCH_3$ | $OCH_3$ | |
| S | S | $CH_3$ | $CH_3CH_2CH_2$ | H | $CH_3$ | $CH_3$ | |
| S | S | $CH_3$ | $CH_3CH_2CH_2$ | H | $CH_3$ | $SCH_3$ | |
| S | S | $CH_3$ | $CH_3CH_2CH_2$ | H | $OCH_3$ | $OCH_3$ | |
| S | S | $CH_3$ | $(CH_3)_2CH$ | H | $CH_3$ | $OCH_3$ | |
| S | S | $CH_3$ | $CH_3CH_2CH_2CH_2$- | H | $CH_3$ | $CH_3$ | |
| S | S | $CH_3$ | $CH_3CH_2CH_2CH_2$- | H | $CH_3$ | $OCH_3$ | |
| S | S | $CH_3$ | $CH_3CH(CH_3)CH_2$ | H | $OCH_3$ | $OCH_3$ | |
| S | S | $CH_3$ | $CH_3CH_2CH(CH_3)$ | H | $CH_3$ | $OCH_3$ | |
| S | S | $CH_3$ | $(CH_3)_3C$ | H | $CH_3$ | $OCH_3$ | |
| $SO_2$ | $SO_2$ | H | H | H | $CH_3$ | $CH_3$ | |

Table IVb (continued)

| $W_1$ | W | $R_{11}$ | $R_{12}$ | $R_{10}$ | X | Y | m.p.(°C) |
|---|---|---|---|---|---|---|---|
| $SO_2$ | $SO_2$ | H | H | H | $CH_3$ | $OCH_3$ | |
| $SO_2$ | $SO_2$ | H | H | H | $CH_3$ | $OC_2H_5$ | |
| $SO_2$ | $SO_2$ | H | H | H | $CH_3$ | $CH_2OCH_3$ | |
| $SO_2$ | $SO_2$ | H | H | H | $OCH_3$ | $OCH_3$ | |
| $SO_2$ | $SO_2$ | H | H | H | $OCH_3$ | $SCH_3$ | |
| $SO_2$ | $SO_2$ | H | H | H | $OCH_3$ | $CH(OCH_3)_2$ | |
| $SO_2$ | $SO_2$ | H | H | $CH_3$ | $CH_3$ | $OCH_3$ | |
| $SO_2$ | $SO_2$ | H | H | $CH_3$ | $OCH_3$ | $OCH_3$ | |
| $SO_2$ | $SO_2$ | H | $CH_3$ | H | $CH_3$ | $CH_3$ | |
| $SO_2$ | $SO_2$ | H | $CH_3$ | H | $CH_3$ | $OCH_3$ | |
| $SO_2$ | $SO_2$ | H | $CH_3$ | H | $OCH_3$ | $OCH_3$ | |
| $SO_2$ | $SO_2$ | H | $CH_3$ | H | $OCH_3$ | $OC_2H_5$ | |
| $SO_2$ | $SO_2$ | H | $CH_3CH_2$ | H | $CH_3$ | $OCH_3$ | |
| $SO_2$ | $SO_2$ | H | $CH_3CH_2$ | H | $OCH_3$ | $OCH_3$ | |
| $SO_2$ | $SO_2$ | H | $CH_3CH_2$ | H | $OCH_3$ | $OCH_2H_5$ | |
| $SO_2$ | $SO_2$ | H | $CH_3CH_2$ | H | $OCH_3$ | 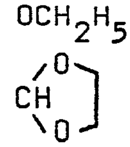 | |
| $SO_2$ | $SO_2$ | H | $CH_3CH_2CH_2$ | H | $CH_3$ | $CH_3$ | |
| $SO_2$ | $SO_2$ | H | $CH_3CH_2CH_2$ | H | $CH_3$ | $OCH_3$ | |
| $SO_2$ | $SO_2$ | H | $(CH_3)_2CH$ | H | $CH_3$ | $OCH_3$ | |
| $SO_2$ | $SO_2$ | H | $CH_3CH_2CH_2CH_2$ | H | $CH_3$ | $CH_3$ | |
| $SO_2$ | $SO_2$ | H | $CH_3CH_2CH_2CH_2$ | H | $CH_3$ | $OCH_3$ | |
| $SO_2$ | $SO_2$ | H | $CH_3CH_2CH_2CH_2$ | H | $OCH_3$ | $OCH_3$ | |
| $SO_2$ | $SO_2$ | H | $CH_3CH(CH_3)CH_2$ | H | $OCH_3$ | $OCH_3$ | |
| $SO_2$ | $SO_2$ | H | $CH_3CH_2CH(CH_3)$ | H | $CH_3$ | $OCH_3$ | |
| $SO_2$ | $SO_2$ | H | $(CH_3)_3C$ | H | $CH_3$ | $OCH_3$ | |
| $SO_2$ | $SO_2$ | $CH_3$ | $CH_3$ | H | $CH_3$ | $CH_3$ | |
| $SO_2$ | $SO_2$ | $CH_3$ | $CH_3$ | H | $CH_3$ | $OCH_3$ | |
| $SO_2$ | $SO_2$ | $CH_3$ | $CH_3$ | H | $CH_3$ | $OC_2H_5$ | |
| $SO_2$ | $SO_2$ | $CH_3$ | $CH_3$ | H | $CH_3$ | $CH_2OCH_3$ | |
| $SO_2$ | $SO_2$ | $CH_3$ | $CH_3$ | H | $OCH_3$ | $OCH_3$ | |
| $SO_2$ | $SO_2$ | $CH_3$ | $CH_3$ | $CH_3$ | $CH_3$ | $CH_3$ | |

Table IVb (continued)

| $W_1$ | W | $R_{11}$ | $R_{12}$ | $R_{10}$ | X | Y | m.p.(°C) |
|---|---|---|---|---|---|---|---|
| $SO_2$ | $SO_2$ | $CH_3$ | $CH_3$ | $CH_3$ | $CH_3$ | $OCH_3$ | |
| $SO_2$ | $SO_2$ | $CH_3$ | $CH_3CH_2$ | H | $CH_2$ | $OCH_3$ | |
| $SO_2$ | $SO_2$ | $CH_3$ | $CH_3CH_2$ | H | $OCH_3$ | $OCH_3$ | |
| $SO_2$ | $SO_2$ | $CH_3$ | $CH_3CH_2CH_2$ | H | $CH_3$ | $CH_3$ | |
| $SO_2$ | $SO_2$ | $CH_3$ | $CH_3CH_2CH_2$ | H | $CH_3$ | $SCH_3$ | |
| $SO_2$ | $SO_2$ | $CH_3$ | $CH_3CH_2CH_2$ | H | $OCH_3$ | $OCH_3$ | |
| $SO_2$ | $SO_2$ | $CH_3$ | $(CH_3)_2CH$ | H | $CH_2$ | $OCH_3$ | |
| $SO_2$ | $SO_2$ | $CH_3$ | $CH_3CH_2CH_2CH_2$- | H | $CH_3$ | $CH_3$ | |
| $SO_2$ | $SO_2$ | $CH_3$ | $CH_3CH_2CH_2CH_2$- | H | $CH_3$ | $OCH_3$ | |
| $SO_2$ | $SO_2$ | $CH_3$ | $CH_3CH_2CH_2CH_2$- | H | $CH_3$ | $OCH_3$ | |
| $SO_2$ | $SO_2$ | $CH_3$ | $CH_3CH(CH_3)CH_2$ | H | $OCH_3$ | $OCH_3$ | |
| $SO_2$ | $SO_2$ | $CH_3$ | $CH_3CH_2CH(CH_3)$ | H | $CH_3$ | $OCH_3$ | |
| $SO_2$ | $SO_2$ | $CH_3$ | $(CH_3)_3C$ | H | $CH_3$ | $OCH_3$ | |
| SO | SO | H | H | H | $CH_3$ | $CH_3$ | |
| SO | SO | H | H | H | $CH_3$ | $OCH_3$ | |
| SO | SO | H | $CH_3$ | H | $CH_3$ | $OCH_3$ | |
| SO | SO | H | $CH_3$ | H | $CH_3$ | $SCH_3$ | |
| SO | SO | $CH_3$ | $CH_3$ | H | $CH_3$ | $CH_3$ | |
| SO | SO | $CH_3$ | $CH_3$ | H | $CH_3$ | $OCH_3$ | |
| SO | SO | $CH_3$ | $CH_3$ | H | $CH_3$ | $SCH_3$ | |
| SO | SO | $CH_3$ | $CH_3$ | H | $OCH_3$ | $OCH_3$ | |
| SO | SO | $CH_3$ | $CH_3$ | H | $OCH_3$ | $OC_2H_5$ | |
| O | S | H | H | H | $CH_3$ | $CH_3$ | |
| O | S | H | H | H | $CH_3$ | $OCH_3$ | |
| O | S | H | H | H | $OCH_3$ | $OCH_3$ | |
| O | S | H | $CH_3$ | H | $CH_3$ | $OCH_3$ | |
| O | S | $CH_3$ | $CH_3$ | H | $CH_3$ | $CH_3$ | |
| O | S | $CH_3$ | $CH_3$ | H | $CH_3$ | $OCH_3$ | |
| O | S | $CH_3$ | $CH_3$ | H | $OCH_3$ | $OCH_3$ | |
| O | SO | H | H | H | $CH_3$ | $OCH_3$ | |
| O | SO | H | $CH_3$ | H | $CH_3$ | $OCH_3$ | |
| O | SO | $CH_3$ | $CH_3$ | H | $CH_3$ | $OCH_3$ | |

Table IVb (continued)

| $W_1$ | W | $R_{11}$ | $R_{12}$ | $R_{10}$ | X | Y | m.p.(°C) |
|---|---|---|---|---|---|---|---|
| O | $SO_2$ | H | H | H | $CH_3$ | $CH_3$ | |
| O | $SO_2$ | H | H | H | $CH_3$ | $OCH_3$ | |
| O | $SO_2$ | H | $CH_3$ | H | $CH_3$ | $OCH_3$ | |
| O | $SO_2$ | $CH_3$ | $CH_3$ | H | $CH_3$ | $OCH_3$ | |
| O | $SO_2$ | $CH_3$ | $CH_3$ | H | $OCH_3$ | $OCH_3$ | |
| S | O | $CH_3$ | $CH_3$ | H | $CH_3$ | $CH_3$ | |
| S | O | $CH_3$ | $CH_3$ | H | $CH_3$ | $OCH_3$ | |
| SO | S | $CH_3$ | $CH_3$ | H | $CH_3$ | $OCH_3$ | |
| $SO_2$ | O | H | H | H | $CH_3$ | $OCH_3$ | |
| $SO_2$ | O | $CH_3$ | H | H | $CH_3$ | $OCH_3$ | |
| SO | S | $CH_3$ | $CH_3$ | H | $CH_3$ | $OCH_3$ | |
| $SO_2$ | S | H | H | H | $CH_3$ | $OCH_3$ | |
| $SO_2$ | S | $CH_3$ | $CH_3$ | H | $CH_3$ | $OCH_3$ | |
| S | SO | H | H | H | $CH_3$ | $OCH_3$ | |
| S | SO | $CH_3$ | $CH_3$ | H | $CH_3$ | $OCH_3$ | |
| S | $SO_2$ | H | H | H | $CH_3$ | $OCH_3$ | |
| S | $SO_2$ | H | $CH_3$ | H | $CH_3$ | $OCH_3$ | |
| S | $SO_2$ | $CH_3$ | $CH_3$ | H | $CH_3$ | $OCH_3$ | |
| $SO_2$ | SO | $CH_3$ | $CH_3$ | H | $CH_3$ | $OCH_3$ | |
| SO | $SO_2$ | H | H | H | $CH_3$ | $OCH_3$ | |
| SO | $SO_2$ | $CH_3$ | $CH_3$ | H | $CH_3$ | $OCH_3$ | |

Table IVc

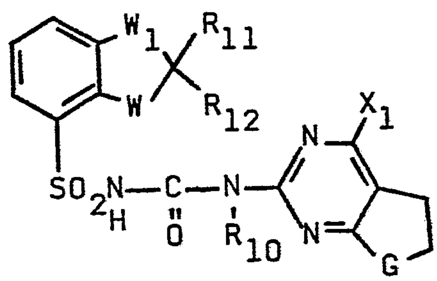

| $W_1$ | W | $R_{11}$ | $R_{12}$ | $R_{10}$ | G | $X_1$ | m.p.(°C) |
|---|---|---|---|---|---|---|---|
| O | O | H | H | H | O | $CH_3$ | |
| O | O | H | H | H | O | $OCH_3$ | |
| O | O | H | H | H | $CH_2$ | $CH_3$ | |
| O | O | H | H | $CH_3$ | $CH_2$ | $CH_3$ | |
| O | O | H | H | $CH_3$ | O | $CH_3$ | |
| O | O | H | H | $CH_3$ | O | $OCH_3$ | |
| O | O | H | $CH_3$ | H | O | $CH_3$ | |
| O | O | H | $CH_3$ | H | O | $OCH_3$ | |
| O | O | H | $CH_3CH_2$ | H | $CH_2$ | $OCH_3$ | |
| O | O | H | $CH_3CH_2CH_2$ | H | O | $OCH_3$ | |
| O | O | H | $(CH_3)_2CH$ | H | O | $OCH_3$ | |
| O | O | H | $CH_3CH_2CH_2CH_2$ | H | O | $OCH_3$ | |
| O | O | H | $CH_3CH(CH_3)CH_3$ | H | $CH_2$ | $CH_3$ | |
| O | O | H | $(CH_3)_3C$ | H | O | $CH_3$ | |
| O | O | $CH_3$ | $CH_3$ | H | O | $CH_3$ | |
| O | O | $CH_3$ | $CH_3$ | H | O | $OCH_3$ | |
| O | O | $CH_3$ | $CH_3$ | H | $CH_2$ | $CH_3$ | |
| O | O | $CH_3$ | $CH_3$ | H | $CH_2$ | $OCH_3$ | |
| O | O | $CH_3$ | $CH_3$ | $CH_3$ | $CH_2$ | $CH_3$ | |
| O | O | $CH_3$ | $CH_3$ | $CH_3$ | O | $CH_3$ | |
| O | O | $CH_3$ | $CH_3$ | $CH_3$ | O | $OCH_3$ | |
| O | O | $CH_3$ | $CH_3CH_2$ | H | O | $CH_3$ | |
| O | O | $CH_3$ | $CH_3CH_2CH_2$ | H | O | $CH_3$ | |
| O | O | $CH_3$ | $(CH_3)_2CH$ | H | O | $CH_3$ | |
| O | O | $CH_3$ | $CH_3CH_2CH_2CH_2$ | H | O | $CH_3$ | |

Table IVc (continued)

| $W_1$ | W | $R_{11}$ | $R_{12}$ | $R_{10}$ | G | $X_1$ | m.p.(°C) |
|---|---|---|---|---|---|---|---|
| O | O | $CH_3$ | $CH_3CH(CH_3)CH_3$ | H | O | $CH_3$ | |
| O | O | $CH_3$ | $(CH_3)_3C$ | H | O | $CH_3$ | |
| S | S | H | H | H | O | $CH_3$ | |
| S | S | H | H | H | O | $OCH_3$ | |
| S | S | H | H | H | $CH_2$ | $CH_3$ | |
| S | S | H | H | $CH_3$ | $CH_2$ | $CH_3$ | |
| S | S | H | H | $CH_3$ | O | $CH_3$ | |
| S | S | H | H | $CH_3$ | O | $OCH_3$ | |
| S | S | H | $CH_3$ | H | O | $CH_3$ | |
| S | S | H | $CH_3$ | H | O | $OCH_3$ | |
| S | S | H | $CH_3CH_2$ | H | $CH_2$ | $CH_3$ | |
| S | S | H | $CH_3CH_2CH_2$ | H | O | $OCH_3$ | |
| S | S | H | $CH_3CH_2CH_2CH_2$ | H | O | $OCH_3$ | |
| S | S | $CH_3$ | $CH_3$ | H | O | $CH_3$ | |
| S | S | $CH_3$ | $CH_3$ | H | O | $OCH_3$ | |
| S | S | $CH_3$ | $CH_3$ | H | $OCH_3$ | $CH_3$ | |
| S | S | $CH_3$ | $CH_3$ | H | $OCH_3$ | $OCH_3$ | |
| S | S | $CH_3$ | $CH_3$ | $CH_3$ | $CH_2$ | $CH_3$ | |
| S | S | $CH_3$ | $CH_3$ | $CH_3$ | O | $CH_3$ | |
| S | S | $CH_3$ | $CH_3$ | $CH_3$ | O | $OCH_3$ | |
| S | S | $CH_3$ | $CH_3CH_2$ | H | O | $CH_3$ | |
| S | S | $CH_3$ | $CH_3CH_2CH_2$ | H | O | $CH_3$ | |
| S | S | $CH_3$ | $(CH_3)_2CH$ | H | O | $CH_3$ | |
| S | S | $CH_3$ | $CH_3CH_2CH_2CH_2$ | H | O | $CH_3$ | |
| S | S | $CH_3$ | $CH_3CH(CH_3)CH_2$ | H | O | $CH_3$ | |
| S | S | $CH_3$ | $(CH_3)_3C$ | H | O | $CH_3$ | |
| $SO_2$ | $SO_2$ | H | H | H | O | $CH_3$ | |
| $SO_2$ | $SO_2$ | H | H | H | O | $OCH_3$ | |
| $SO_2$ | $SO_2$ | H | H | H | $CH_2$ | $CH_3$ | |
| $SO_2$ | $SO_2$ | H | H | H | $CH_2$ | $OCH_3$ | |
| $SO_2$ | $SO_2$ | H | H | $CH_3$ | $CH_2$ | $CH_3$ | |
| $SO_2$ | $SO_2$ | H | H | $CH_3$ | O | $CH_3$ | |

Table IVc (continued)

| $W_1$ | W | $R_{11}$ | $R_{12}$ | $R_{10}$ | G | $X_1$ | m.p.(°C) |
|---|---|---|---|---|---|---|---|
| $SO_2$ | $SO_2$ | H | H | $CH_3$ | O | $OCH_3$ | |
| $SO_2$ | $SO_2$ | H | $CH_3$ | H | O | $CH_3$ | |
| $SO_2$ | $SO_2$ | H | $CH_3$ | H | O | $OCH_3$ | |
| $SO_2$ | $SO_2$ | H | $CH_3CH_2$ | H | $CH_2$ | $OCH_3$ | |
| $SO_2$ | $SO_2$ | H | $CH_3CH_2CH_3$ | H | $CH_2$ | $OCH_3$ | |
| $SO_2$ | $SO_2$ | H | $CH_3CH_2CH_3$ | H | O | $OCH_3$ | |
| $SO_2$ | $SO_2$ | $CH_3$ | $CH_3$ | H | O | $CH_3$ | |
| $SO_2$ | $SO_2$ | $CH_3$ | $CH_3$ | H | O | $OCH_3$ | |
| $SO_2$ | $SO_2$ | $CH_3$ | $CH_3$ | H | $CH_2$ | $CH_3$ | |
| $SO_2$ | $SO_2$ | $CH_3$ | $CH_3$ | H | $CH_2$ | $OCH_3$ | |
| $SO_2$ | $SO_2$ | $CH_3$ | $CH_3$ | $CH_3$ | $CH_2$ | $CH_3$ | |
| $SO_2$ | $SO_2$ | $CH_3$ | $CH_3$ | $CH_3$ | O | $CH_3$ | |
| $SO_2$ | $SO_2$ | $CH_3$ | $CH_3$ | $CH_3$ | O | $OCH_3$ | |
| $SO_2$ | $SO_2$ | $CH_3$ | $CH_3CH_2$ | H | O | $CH_3$ | |
| $SO_2$ | $SO_2$ | $CH_3$ | $CH_3CH_2CH_2$ | H | O | $CH_3$ | |
| $SO_2$ | $SO_2$ | $CH_3$ | $(CH_3)_2CH$ | H | O | $CH_3$ | |
| $SO_2$ | $SO_2$ | $CH_3$ | $CH_3CH_2CH_2CH_2$ | H | O | $CH_3$ | |
| $SO_2$ | $SO_2$ | $CH_3$ | $CH_3CH(CH_3)CH_2$ | H | O | $CH_3$ | |
| $SO_2$ | $SO_2$ | $CH_3$ | $(CH_3)_3C$ | H | O | $CH_3$ | |
| SO | SO | H | H | H | $CH_2$ | $CH_3$ | |
| SO | SO | H | H | H | O | $OCH_3$ | |
| SO | SO | $CH_3$ | $CH_3$ | H | $CH_2$ | $CH_3$ | |
| SO | SO | $CH_3$ | $CH_3$ | H | O | $CH_3$ | |
| O | S | H | H | H | O | $CH_3$ | |
| O | S | $CH_3$ | $CH_3$ | H | O | $CH_3$ | |
| O | SO | H | H | H | O | $CH_3$ | |
| O | SO | $CH_3$ | $CH_3$ | H | O | $CH_3$ | |
| O | $SO_2$ | H | H | H | O | $CH_3$ | |
| O | $SO_2$ | $CH_3$ | $CH_3$ | H | O | $CH_3$ | |
| S | O | H | H | H | O | $CH_3$ | |
| SO | O | H | H | H | O | $CH_3$ | |
| $SO_2$ | O | H | H | H | O | $CH_3$ | |

Table IVc (continued)

| $W_1$ | W | $R_{11}$ | $R_{12}$ | $R_{10}$ | G | $X_1$ | m.p.(°C) |
|---|---|---|---|---|---|---|---|
| SO | S | $CH_3$ | $CH_3$ | H | O | $CH_3$ | |
| $SO_2$ | S | H | H | H | O | $CH_3$ | |
| $SO_2$ | S | $CH_3$ | $CH_3$ | H | O | $CH_3$ | |
| S | SO | $CH_3$ | $CH_3$ | H | O | $CH_3$ | |
| S | $SO_2$ | $CH_3$ | $CH_3$ | H | O | $CH_3$ | |
| $SO_2$ | SO | H | H | H | O | $CH_3$ | |
| SO | $SO_2$ | H | H | H | O | $CH_3$ | |

Table IVd

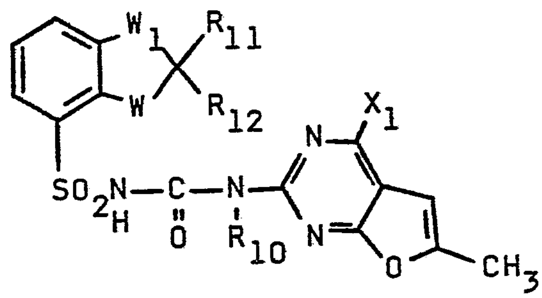

| $W_1$ | W | $R_{11}$ | $R_{12}$ | $R_{10}$ | $X_1$ | m.p.(°C) |
|---|---|---|---|---|---|---|
| O | O | H | H | H | $CH_3$ | |
| O | O | H | H | H | $OCH_3$ | |
| O | O | H | H | $CH_3$ | $CH_3$ | |
| O | O | H | H | $CH_3$ | $OCH_3$ | |
| O | O | H | $CH_3$ | H | $CH_3$ | |
| O | O | H | $CH_3CH_2$ | H | $CH_3$ | |
| O | O | H | $CH_3CH_2CH_2$ | H | $CH_3$ | |
| O | O | H | $(CH_3)_2CH$ | H | $CH_3$ | |
| O | O | H | $CH_3CH_2CH_2CH_2$ | H | $CH_3$ | |
| O | O | H | $CH_3CH(CH_3)CH_3$ | H | $CH_3$ | |
| O | O | H | $(CH_3)_3C$ | H | $CH_3$ | |
| O | O | $CH_3$ | $CH_3$ | H | $OCH_3$ | |
| O | O | $CH_3$ | $CH_3$ | H | $CH_3$ | |
| O | O | $CH_3$ | $CH_3$ | $CH_3$ | $CH_3$ | |
| O | O | $CH_3$ | $CH_3$ | $CH_3$ | $OCH_3$ | |
| O | O | $CH_3$ | $CH_3CH_2$ | H | $CH_3$ | |
| O | O | $CH_3$ | $CH_3CH_2CH_2$ | H | $CH_3$ | |
| O | O | $CH_3$ | $(CH_3)_2CH$ | H | CH | |
| O | O | $CH_3$ | $CH_3CH_2CH_2CH_2$ | H | $CH_3$ | |
| O | O | $CH_3$ | $CH_3CH(CH_3)CH_3$ | H | $CH_3$ | |
| O | O | $CH_3$ | $(CH_3)_3C$ | H | $CH_3$ | |
| S | S | H | H | H | $CH_3$ | |
| S | S | H | H | H | $OCH_3$ | |
| S | S | H | H | $CH_3$ | $CH_3$ | |
| S | S | H | H | $CH_3$ | $OCH_3$ | |

Table IVd (continued)

| $W_1$ | W | $R_{11}$ | $R_{12}$ | $R_{10}$ | $X_1$ | m.p.(°C) |
|---|---|---|---|---|---|---|
| S | S | H | $CH_3$ | H | $CH_3$ | |
| S | S | H | $CH_3CH_2$ | H | $CH_3$ | |
| S | S | H | $CH_3CH_2CH_2$ | H | $CH_3$ | |
| S | S | H | $(CH_3)_2CH$ | H | $CH_3$ | |
| S | S | H | $CH_3CH_2CH_2CH_2$ | H | $CH_3$ | |
| S | S | H | $CH_3CH(CH_3)CH_3$ | H | $CH_3$ | |
| S | S | H | $(CH_3)_3C$ | H | $CH_3$ | |
| S | S | $CH_3$ | $CH_3$ | H | $OCH_3$ | |
| S | S | $CH_3$ | $CH_3$ | H | $CH_3$ | |
| S | S | $CH_3$ | $CH_3$ | $CH_3$ | $CH_3$ | |
| S | S | $CH_3$ | $CH_3$ | $CH_3$ | $OCH_3$ | |
| S | S | $CH_3$ | $CH_3CH_2$ | H | $CH_3$ | |
| S | S | $CH_3$ | $CH_3CH_2CH_2$ | H | $CH_3$ | |
| S | S | $CH_3$ | $(CH_3)_2CH$ | H | $CH_3$ | |
| S | S | $CH_3$ | $CH_3CH_2CH_2CH_2$ | H | $CH_3$ | |
| S | S | $CH_3$ | $CH_3CH(CH_3)CH_3$ | H | $CH_3$ | |
| S | S | $CH_3$ | $(CH_3)_3C$ | H | $CH_3$ | |
| $SO_2$ | $SO_2$ | H | H | H | $CH_3$ | |
| $SO_2$ | $SO_2$ | H | H | H | $OCH_3$ | |
| $SO_2$ | $SO_2$ | H | H | $CH_3$ | $CH_3$ | |
| $SO_2$ | $SO_2$ | H | H | $CH_3$ | $OCH_3$ | |
| $SO_2$ | $SO_2$ | H | $CH_3$ | H | $CH_3$ | |
| $SO_2$ | $SO_2$ | H | $CH_3$ | H | $CH_3$ | |
| $SO_2$ | $SO_2$ | H | $CH_3CH_2$ | H | $CH_3$ | |
| $SO_2$ | $SO_2$ | H | $CH_3CH_2CH_2$ | H | $CH_3$ | |
| $SO_2$ | $SO_2$ | H | $(CH_3)_2CH$ | H | $CH_3$ | |
| $SO_2$ | $SO_2$ | H | $CH_3CH_2CH_2CH_2$ | H | $CH_3$ | |
| $SO_2$ | $SO_2$ | H | $CH_3CH(CH_3)CH_3$ | H | $CH_3$ | |
| $SO_2$ | $SO_2$ | H | $(CH_3)_3C$ | H | $CH_3$ | |
| $SO_2$ | $SO_2$ | $CH_3$ | $CH_3$ | H | $OCH_3$ | |
| $SO_2$ | $SO_2$ | $CH_3$ | $CH_3$ | H | $CH_3$ | |
| $SO_2$ | $SO_2$ | $CH_3$ | $CH_3$ | $CH_3$ | $CH_3$ | |

Table IVd (continued)

| $W_1$ | $W$ | $R_{11}$ | $R_{12}$ | $R_{10}$ | $X_1$ | m.p.(°C) |
|---|---|---|---|---|---|---|
| $SO_2$ | $SO_2$ | $CH_3$ | $CH_3$ | $CH_3$ | $OCH_3$ | |
| $SO_2$ | $SO_2$ | $CH_3$ | $CH_3CH_2$ | H | $CH_3$ | |
| $SO_2$ | $SO_2$ | $CH_3$ | $CH_3CH_2CH_2$ | H | $CH_3$ | |
| $SO_2$ | $SO_2$ | $CH_3$ | $(CH_3)_2CH$ | H | $CH_3$ | |
| $SO_2$ | $SO_2$ | $CH_3$ | $CH_3CH_2CH_2CH_2$ | H | $CH_3$ | |
| $SO_2$ | $SO_2$ | $CH_3$ | $CH_3(CH_3)CH_3$ | H | $CH_3$ | |
| $SO_2$ | $SO_2$ | $CH_3$ | $(CH_3)_3C$ | H | $CH_3$ | |
| SO | SO | $CH_3$ | $CH_3$ | H | $CH_3$ | |
| SO | SO | $CH_3$ | $CH_3$ | H | $OCH_3$ | |
| O | S | H | H | H | $CH_3$ | |
| O | SO | $CH_3$ | $CH_3$ | H | $CH_3$ | |
| O | $SO_2$ | $CH_3$ | $CH_3$ | H | $CH_3$ | |
| S | O | H | H | H | $CH_3$ | |
| SO | O | H | H | H | $CH_3$ | |
| $SO_2$ | O | H | H | H | $CH_3$ | |
| SO | S | H | H | H | $CH_3$ | |
| S | $SO_2$ | H | H | H | $CH_3$ | |
| S | SO | H | H | H | $CH_3$ | |
| S | $SO_2$ | $CH_3$ | $CH_3$ | H | $CH_3$ | |
| $SO_2$ | SO | H | H | H | $CH_3$ | |
| SO | $SO_2$ | H | H | H | $CH_3$ | |

Table IVe

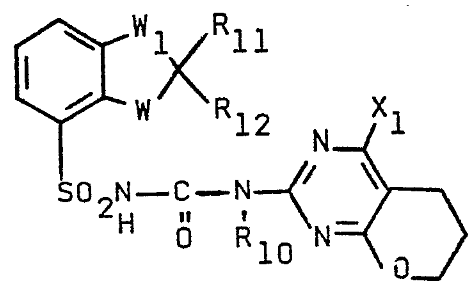

| $W_1$ | W | $R_{11}$ | $R_{12}$ | $R_{10}$ | $X_1$ | m.p.(°C) |
|---|---|---|---|---|---|---|
| O | O | H | H | H | $CH_3$ | |
| O | O | H | H | H | $OCH_3$ | |
| O | O | H | H | $CH_3$ | $CH_3$ | |
| O | O | H | H | $CH_3$ | $OCH_3$ | |
| O | O | H | $CH_3$ | H | $CH_3$ | |
| O | O | H | $CH_3CH_2$ | H | $CH_3$ | |
| O | O | H | $CH_3CH_2CH_2$ | H | $CH_3$ | |
| O | O | H | $(CH_3)_2CH$ | H | $CH_3$ | |
| O | O | H | $CH_3CH_2CH_2CH_2$ | H | $CH_3$ | |
| O | O | H | $CH_3CH(CH_3)CH_3$ | H | $CH_3$ | |
| O | O | H | $(CH_3)_3C$ | H | $CH_3$ | |
| O | O | $CH_3$ | $CH_3$ | H | $OCH_3$ | |
| O | O | $CH_3$ | $CH_3$ | $CH_3$ | $CH_3$ | |
| O | O | $CH_3$ | $CH_3$ | $CH_3$ | $OCH_3$ | |
| O | O | $CH_3$ | $CH_3CH_2$ | H | $CH_3$ | |
| O | O | $CH_3$ | $CH_3CH_2CH_2$ | H | $CH_3$ | |
| O | O | $CH_3$ | $(CH_3)_2CH$ | H | $CH_3$ | |
| O | O | $CH_3$ | $CH_3CH_2CH_2CH_2$ | H | $CH_3$ | |
| O | O | $CH_3$ | $CH_3CH(CH_3)CH_3$ | H | $CH_3$ | |
| O | O | $CH_3$ | $(CH_3)_3C$ | H | $CH_3$ | |
| S | S | H | H | H | $CH_3$ | |
| S | S | H | H | H | $OCH_3$ | |
| S | S | H | H | $CH_3$ | $CH_3$ | |
| S | S | H | H | $CH_3$ | $OCH_3$ | |
| S | S | H | $CH_3$ | H | $CH_3$ | |

Table IVe (continued)

| $W_1$ | $W$ | $R_{11}$ | $R_{12}$ | $R_{10}$ | $X_1$ | m.p.(°C) |
|---|---|---|---|---|---|---|
| S | S | H | $CH_3CH_2$ | H | $CH_3$ | |
| S | S | H | $CH_3CH_2CH_2$ | H | $CH_3$ | |
| S | S | H | $(CH_3)_2CH$ | H | $CH_3$ | |
| S | S | H | $CH_3CH_2CH_2CH_2$ | H | $CH_3$ | |
| S | S | H | $CH_3CH(CH_3)CH_3$ | H | $CH_3$ | |
| S | S | H | $(CH_3)_3C$ | H | $CH_3$ | |
| S | S | $CH_3$ | $CH_3$ | H | $OCH_3$ | |
| S | S | $CH_3$ | $CH_3$ | $CH_3$ | $CH_3$ | |
| S | S | $CH_3$ | $CH_3$ | $CH_3$ | $OCH_3$ | |
| S | S | $CH_3$ | $CH_3CH_2$ | H | $CH_3$ | |
| S | S | $CH_3$ | $CH_3CH_2CH_2$ | H | $CH_3$ | |
| S | S | $CH_3$ | $(CH_3)_2CH$ | H | $CH_3$ | |
| S | S | $CH_3$ | $CH_3CH_2CH_2CH_2$ | H | $CH_3$ | |
| S | S | $CH_3$ | $CH_3CH(CH_3)CH_3$ | H | $CH_3$ | |
| S | S | $CH_3$ | $(CH_3)_3C$ | H | $CH_3$ | |
| $SO_2$ | $SO_2$ | H | H | H | $CH_3$ | |
| $SO_2$ | $SO_2$ | H | H | H | $OCH_3$ | |
| $SO_2$ | $SO_2$ | H | H | $CH_3$ | $CH_3$ | |
| $SO_2$ | $SO_2$ | H | H | $CH_3$ | $OCH_3$ | |
| $SO_2$ | $SO_2$ | H | $CH_3$ | H | $CH_3$ | |
| $SO_2$ | $SO_2$ | H | $CH_3CH_2$ | H | $CH_3$ | |
| $SO_2$ | $SO_2$ | H | $CH_3CH_2CH_2$ | H | $CH_3$ | |
| $SO_2$ | $SO_2$ | H | $(CH_3)_2CH$ | H | $CH_3$ | |
| $SO_2$ | $SO_2$ | H | $CH_3CH_2CH_2CH_2$ | H | $CH_3$ | |
| $SO_2$ | $SO_2$ | H | $CH_3CH(CH_3)CH_3$ | H | $CH_3$ | |
| $SO_2$ | $SO_2$ | H | $(CH_3)_3C$ | H | $CH_3$ | |
| $SO_2$ | $SO_2$ | $CH_3$ | $CH_3$ | H | $OCH_3$ | |
| $SO_2$ | $SO_2$ | $CH_3$ | $CH_3$ | H | $CH_3$ | |
| $SO_2$ | $SO_2$ | $CH_3$ | $CH_3$ | $CH_3$ | $CH_3$ | |
| $SO_2$ | $SO_2$ | $CH_3$ | $CH_3$ | $CH_3$ | $OCH_3$ | |
| $SO_2$ | $SO_2$ | $CH_3$ | $CH_3CH_2$ | H | $CH_3$ | |
| $SO_2$ | $SO_2$ | $CH_3$ | $CH_3CH_2CH_2$ | H | $CH_3$ | |

Table IVe (continued)

| $W_1$ | W | $R_{11}$ | $R_{12}$ | $R_{10}$ | $X_1$ | m.p.(°C) |
|---|---|---|---|---|---|---|
| $SO_2$ | $SO_2$ | $CH_3$ | $(CH_3)_2CH$ | H | $CH_3$ | |
| $SO_2$ | $SO_2$ | $CH_3$ | $CH_3CH_2CH_2CH_2$ | H | $CH_3$ | |
| $SO_2$ | $SO_2$ | $CH_3$ | $CH_3CH(CH_3)CH_3$ | H | $CH_3$ | |
| $SO_2$ | $SO_2$ | $CH_3$ | $(CH_3)_3C$ | H | $CH_3$ | |
| SO | SO | $CH_3$ | $CH_3$ | H | $CH_3$ | |
| SO | SO | $CH_3$ | $CH_3$ | H | $OCH_3$ | |
| O | S | H | H | H | $CH_3$ | |
| O | SO | $CH_3$ | $CH_3$ | H | $CH_3$ | |
| O | $SO_2$ | $CH_3$ | $CH_3$ | H | $CH_3$ | |
| S | O | H | H | H | $CH_3$ | |
| SO | O | H | H | H | $CH_3$ | |
| $SO_2$ | O | H | H | H | $CH_3$ | |
| SO | S | H | H | H | $CH_3$ | |
| S | $SO_2$ | H | H | H | $CH_3$ | |
| S | SO | H | H | H | $CH_3$ | |
| S | $SO_2$ | $CH_3$ | $CH_3$ | H | $CH_3$ | |
| $SO_2$ | SO | H | H | H | $CH_3$ | |
| SO | $SO_2$ | H | H | H | $CH_3$ | |

Table IVf

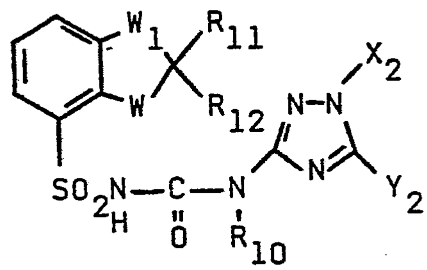

| $W_1$ | W | $R_{11}$ | $R_{12}$ | $R_{10}$ | $X_2$ | $Y_2$ | m.p.(°C) |
|---|---|---|---|---|---|---|---|
| O | O | H | H | H | $CH_3$ | $OCH_3$ | |
| O | O | H | H | H | $CH_3CH_2$ | $SCH_3$ | |
| O | O | H | H | H | $CH_2CF_3$ | $OCH_3$ | |
| O | O | H | H | $CH_3$ | $CH_3$ | $OCH_3$ | |
| O | O | H | $CH_3$ | H | $CH_3CH_2CH_2$ | $OC_2H_5$ | |
| O | O | H | $CH_3CH_2$ | H | $(CH_3)_2CH$ | $SC_2H_5$ | |
| O | O | H | $CH_3CH_2CH_2$ | H | $CH_3$ | $OCH_3$ | |
| O | O | H | $(CH_3)_2CH$ | H | $CH_3$ | $OCH_3$ | |
| O | O | H | $CH_3CH_2CH_2CH_2$ | H | $CH_3$ | $OCH_3$ | |
| O | O | H | $CH_3CH(CH_3)_2CH_2$ | H | $CH_3$ | $OCH_3$ | |
| O | O | H | $(CH_3)_3C$ | H | $CH_3$ | $OCH_3$ | |
| O | O | $CH_3$ | $CH_3$ | H | $CH_3$ | $OCH_3$ | |
| O | O | $CH_3$ | $CH_3$ | H | $CH_3$ | $SCH_3$ | |
| O | O | $CH_3$ | $CH_3$ | H | $CH_3CH_2$ | $OCH_3$ | |
| O | O | $CH_3$ | $CH_3$ | H | $CH_2CF_3$ | $OCH_3$ | |
| O | O | $CH_3$ | $CH_3$ | H | $CH_2CF_3$ | $SCH_3$ | |
| O | O | $CH_3$ | $CH_3$ | $CH_3$ | $CH_3$ | $OCH_3$ | |
| O | O | $CH_3$ | $CH_3CH_2$ | H | $CH_3$ | $OCH_3$ | |
| O | O | $CH_3$ | $CH_3CH_2CH_2$ | H | $CH_3$ | $OCH_3$ | |
| O | O | $CH_3$ | $(CH_3)_2CH$ | H | $CH_3$ | $OCH_3$ | |
| O | O | $CH_3$ | $CH_3CH_2CH_2CH_2$ | H | $CH_3$ | $OCH_3$ | |
| O | O | $CH_3$ | $CH_3CH(CH_3)CH_2$ | H | $CH_3$ | $OCH_3$ | |
| O | O | $CH_3$ | $(CH_3)_3C$ | H | $CH_3$ | $OCH_3$ | |
| S | S | H | H | H | $CH_3$ | $OCH_3$ | |
| S | S | H | H | H | $CH_3CH_2$ | $SCH_3$ | |

Table IVf (continued)

| $W_1$ | W | $R_{11}$ | $R_{12}$ | $R_{10}$ | $X_2$ | $Y_2$ | m.p.(°C) |
|---|---|---|---|---|---|---|---|
| S | S | H | H | H | $CH_2CF_3$ | $OCH_3$ | |
| S | S | H | H | $CH_3$ | $CH_3$ | $OCH_3$ | |
| S | S | H | $CH_3$ | H | $CH_3CH_2CH_2$ | $OC_2H_5$ | |
| S | S | H | $CH_3CH_2$ | H | $(CH_3)_2CH$ | $SC_2H_5$ | |
| S | S | H | $CH_3CH_2CH_2$ | H | $CH_3$ | $OCH_3$ | |
| S | S | H | $(CH_3)_2CH$ | H | $CH_3$ | $OCH_3$ | |
| S | S | H | $CH_3CH_2CH_2CH_2$ | H | $CH_3$ | $OCH_3$ | |
| S | S | H | $CH_3CH(CH_3)_2CH_2$ | H | $CH_3$ | $OCH_3$ | |
| S | S | H | $(CH_3)_3C$ | H | $CH_3$ | $OCH_3$ | |
| S | S | $CH_3$ | $CH_3$ | H | $CH_3$ | $OCH_3$ | |
| S | S | $CH_3$ | $CH_3$ | H | $CH_3$ | $SCH_3$ | |
| S | S | $CH_3$ | $CH_3$ | H | $CH_3CH_2$ | $OCH_3$ | |
| S | S | $CH_3$ | $CH_3$ | H | $CH_2CF_3$ | $OCH_3$ | |
| S | S | $CH_3$ | $CH_3$ | H | $CH_2CF_3$ | $SCH_3$ | |
| S | S | $CH_3$ | $CH_3$ | $CH_3$ | $CH_3$ | $OCH_3$ | |
| S | S | $CH_3$ | $CH_3CH_2$ | H | $CH_3$ | $OCH_3$ | |
| S | S | $CH_3$ | $CH_3CH_2CH_2$ | H | $CH_3$ | $OCH_3$ | |
| S | S | $CH_3$ | $(CH_3)_2CH$ | H | $CH_3$ | $OCH_3$ | |
| S | S | $CH_3$ | $CH_3CH_2CH_2CH_2$ | H | $CH_3$ | $OCH_3$ | |
| S | S | $CH_3$ | $CH_3CH(CH_3)CH_2$ | H | $CH_3$ | $OCH_3$ | |
| S | S | $CH_3$ | $(CH_3)_3C$ | H | $CH_3$ | $OCH_3$ | |
| $SO_2$ | $SO_2$ | H | H | H | $CH_3$ | $OCH_3$ | |
| $SO_2$ | $SO_2$ | H | H | H | $CH_3CF_2$ | $SCH_3$ | |
| $SO_2$ | $SO_2$ | H | H | H | $CH_2CH_3$ | $OCH_3$ | |
| $SO_2$ | $SO_2$ | H | H | $CH_3$ | $CH_3$ | $OCH_3$ | |
| $SO_2$ | $SO_2$ | H | $CH_3$ | H | $CH_3CH_2CH_2$ | $OC_2H_5$ | |
| $SO_2$ | $SO_2$ | H | $CH_3CH_2$ | H | $(CH_3)_2CH$ | $SC_2H_5$ | |
| $SO_2$ | $SO_2$ | H | $CH_3CH_2CH_2$ | H | $CH_3$ | $OCH_3$ | |
| $SO_2$ | $SO_2$ | H | $(CH_3)_2CH$ | H | $CH_3$ | $OCH_3$ | |
| $SO_2$ | $SO_2$ | H | $CH_3CH_2CH_2CH_2$ | H | $CH_3$ | $OCH_3$ | |
| $SO_2$ | $SO_2$ | H | $CH_3CH(CH_3)_2CH_2$ | H | $CH_3$ | $OCH_3$ | |
| $SO_2$ | $SO_2$ | H | $(CH_3)_3C$ | H | $CH_3$ | $OCH_3$ | |

Table IVf (continued)

| $W_1$ | W | $R_{11}$ | $R_{12}$ | $R_{10}$ | $X_2$ | $Y_2$ | m.p.(°C) |
|---|---|---|---|---|---|---|---|
| $SO_2$ | $SO_2$ | $CH_3$ | $CH_3$ | H | $CH_3$ | $OCH_3$ | |
| $SO_2$ | $SO_2$ | $CH_3$ | $CH_3$ | H | $CH_3$ | $SCH_3$ | |
| $SO_2$ | $SO_2$ | $CH_3$ | $CH_3$ | H | $CH_3CH_2$ | $OCH_3$ | |
| $SO_2$ | $SO_2$ | $CH_3$ | $CH_3$ | H | $CH_2CF_3$ | $OCH_3$ | |
| $SO_2$ | $SO_2$ | $CH_3$ | $CH_3$ | H | $CH_2CF_3$ | $SCH_3$ | |
| $SO_2$ | $SO_2$ | $CH_3$ | $CH_3$ | $CH_3$ | $CH_3$ | $OCH_3$ | |
| $SO_2$ | $SO_2$ | $CH_3$ | $CH_3CH_2$ | H | $CH_3$ | $OCH_3$ | |
| $SO_2$ | $SO_2$ | $CH_3$ | $CH_3CH_2CH_2$ | H | $CH_3$ | $OCH_3$ | |
| $SO_2$ | $SO_2$ | $CH_3$ | $(CH_3)_2CH$ | H | $CH_3$ | $OCH_3$ | |
| $SO_2$ | $SO_2$ | $CH_3$ | $CH_3CH_2CH_2CH_2$ | H | $CH_3$ | $OCH_3$ | |
| $SO_2$ | $SO_2$ | $CH_3$ | $CH_3CH(CH_3)CH_2$ | H | $CH_3$ | $OCH_3$ | |
| $SO_2$ | $SO_2$ | $CH_3$ | $(CH_3)_3C$ | H | $CH_3$ | $OCH_3$ | |
| SO | SO | H | H | H | $CH_3$ | $OCH_3$ | |
| SO | SO | $CH_3$ | $CH_3$ | H | $CH_3$ | $SCH_3$ | |
| O | S | H | H | H | $CH_3$ | $OCH_3$ | |
| O | SO | $CH_3$ | $CH_3$ | H | $CH_3$ | $OCH_3$ | |
| O | $SO_2$ | $CH_3$ | $CH_3$ | H | $CH_3$ | $OCH_3$ | |
| S | O | H | H | H | $CH_3$ | $OCH_3$ | |
| SO | O | H | H | H | $CH_3$ | $OCH_3$ | |
| $SO_2$ | O | H | H | H | $CH_3$ | $OCH_3$ | |
| S | SO | H | H | H | $CH_3$ | $OCH_3$ | |
| S | $SO_2$ | H | H | H | $CH_3$ | $OCH_3$ | |
| S | SO | H | H | H | $CH_3$ | $OCH_3$ | |
| S | $SO_2$ | $CH_3$ | $CH_3$ | H | $CH_3$ | $OCH_3$ | |
| $SO_2$ | SO | H | H | H | $CH_3$ | $OCH_3$ | |
| SO | $SO_2$ | H | H | H | $CH_3$ | $OCH_3$ | |

Table Va

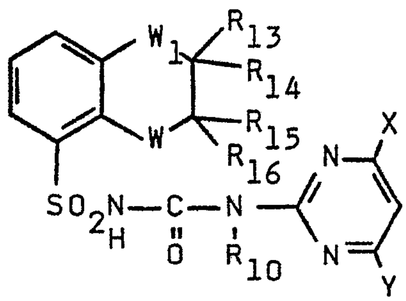

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| O | O | H | H | H | H | H | $CH_3$ | $CH_3$ | |
| O | O | H | H | H | H | H | $CH_3$ | $OCH_3$ | 203-205° |
| O | O | H | H | H | H | H | $CH_3$ | $SCH_3$ | |
| O | O | H | H | H | H | H | $CH_3$ | $NH_2$ | |
| O | O | H | H | H | H | H | $OCH_3$ | $OCH_3$ | |
| O | O | H | H | H | H | H | $OCH_3$ | $CH_2OCH_3$ | |
| O | O | H | H | H | H | H | $OCH_3$ | $CH(OCH_3)_2$ | |
| O | O | H | H | H | H | H | Cl | $OCH_3$ | |
| O | O | H | H | H | H | H | Cl | $NH_2$ | |
| O | O | H | H | H | H | H | Cl | $NH(CH_3)$ | |
| O | O | H | H | H | H | H | Cl | $N(CH_3)_2$ | |
| O | O | H | H | H | H | $CH_3$ | $CH_3$ | $CH_3$ | |
| O | O | H | H | H | H | $CH_3$ | $CH_3$ | $OCH_3$ | |
| O | O | $CH_3$ | H | H | H | H | $CH_3$ | $CH_3$ | |
| O | O | $CH_3$ | H | H | H | H | $CH_3$ | $OCH_3$ | |
| O | O | $CH_3$ | H | H | H | H | $OCH_3$ | $OCH_3$ | |
| O | O | $CH_3$ | H | H | H | H | $OCH_3$ | $CH_2(OCH_3)$ | |
| O | O | $CH_3$ | H | H | H | H | Cl | $N(CH_3)_2$ | |
| O | O | $CH_3$ | H | $CH_3$ | H | H | $CH_3$ | $CH_3$ | |
| O | O | $CH_3$ | H | $CH_3$ | H | H | $CH_3$ | $OC_2H_5$ | |
| O | O | $CH_3$ | H | $CH_3$ | H | H | $CH_3$ | $CH_3(OCH_3)$ | |
| O | O | $CH_3$ | H | $CH_3$ | H | H | $OCH_3$ | $OCH_3$ | |
| O | O | $CH_3$ | $CH_3$ | H | H | H | $CH_3$ | $CH_3$ | |
| O | O | $CH_3$ | $CH_3$ | H | H | H | $CH_3$ | $OCH_3$ | |

Table Va (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| O | O | $CH_3$ | $CH_3$ | H | H | H | $OCH_3$ | $OC_2H_5$ | |
| O | O | $CH_3$ | $CH_3$ | H | H | H | Cl | $N(CH_3)_2$ | |
| O | O | $CH_3$ | $CH_3$ | CH | H | H | $CH_3$ | $CH_3$ | |
| O | O | $CH_3$ | $CH_3$ | $CH_3$ | H | H | $OCH_3$ | $SCH_3$ | |
| O | O | $CH_3$ | $CH_3$ | $CH_3$ | $CH_3$ | H | $OCH_3$ | $OCH_3$ | |
| O | O | $CH_3$ | $CH_3$ | $CH_3$ | $CH_3$ | H | $CH_3$ | $CH(OCH_3)_2$ | |
| O | O | $CH_3$ | $CH_3$ | $CH_3$ | $CH_3$ | H | H | 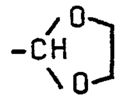 | |
| O | O | $CH_3$ | $CH_3$ | $CH_3$ | $CH_3$ | H | $OCH_3$ | $SCH_3$ | |
| O | O | $CH_3$ | $CH_3$ | $CH_3$ | $CH_3$ | $CH_3$ | $CH_3$ | $CH_3$ | |
| O | O | $CH_3$ | $CH_3$ | $CH_3$ | $CH_3$ | $CH_3$ | $CH_3$ | $OCH_3$ | |
| O | O | $CH_3$ | $CH_3$ | $CH_3$ | $CH_3$ | $CH_3$ | $OCH_3$ | $OCH_3$ | |
| S | S | H | H | H | H | H | $CH_3$ | $CH_3$ | |
| S | S | H | H | H | H | H | $CH_3$ | $OCH_3$ | |
| S | S | H | H | H | H | H | $CH_3$ | $N(CH_3)_2$ | |
| S | S | H | H | H | H | H | $OCH_3$ | H | |
| S | S | H | H | H | H | H | $OCH_3$ | $OCH_3$ | |
| S | S | H | H | H | H | H | $OCH_3$ | $CH_3(OCH_3)_2$ | |
| S | S | H | H | H | H | H | Cl | $OCH_3$ | |
| S | S | H | H | H | H | H | Cl | $NH_2$ | |
| S | S | H | H | H | H | H | Cl | $N(CH_3)_2$ | |
| S | S | H | H | H | H | $CH_3$ | $CH_3$ | $OCH_3$ | |
| S | S | H | H | H | H | $CH_3$ | $OCH_3$ | $OCH_3$ | |
| S | S | $CH_3$ | H | H | H | H | $CH_3$ | 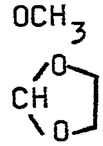 | |
| S | S | $CH_3$ | H | H | H | H | $CH_3$ | $OCH_3$ | |
| S | S | $CH_3$ | H | H | H | H | $OCH_3$ | $OCH_3$ | |
| S | S | $CH_3$ | H | H | H | H | Cl | $OCH_3$ | |
| S | S | $CH_3$ | H | H | H | H | Cl | $N(CH_3)_2$ | |
| S | S | $CH_3$ | H | $CH_3$ | H | H | $CH_3$ | $CH_3$ | |
| S | S | $CH_3$ | H | $CH_3$ | H | H | $CH_3$ | $OCH_3$ | |
| S | S | $CH_3$ | H | $CH_3$ | H | H | $CH_3$ | $OCH_3$ | |

Table Va (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| S | S | $CH_3$ | H | $CH_3$ | H | H | $OCH_3$ | 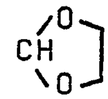 | |
| S | S | $CH_3$ | $CH_3$ | H | H | H | $CH_3$ | $CH_3$ | |
| S | S | $CH_3$ | $CH_3$ | H | H | H | $CH_3$ | $OCH_3$ | |
| S | S | $CH_3$ | $CH_3$ | H | H | H | $OCH_3$ | $OCH_3$ | |
| S | S | $CH_3$ | $CH_3$ | H | H | H | $OCH_3$ | $N(CH_3)_2$ | |
| S | S | $CH_3$ | $CH_3$ | $CH_3$ | H | H | $CH_3$ | $CH_3$ | |
| S | S | $CH_3$ | $CH_3$ | $CH_3$ | H | H | $OCH_3$ | $NH_2$ | |
| S | S | $CH_3$ | $CH_3$ | $CH_3$ | $CH_3$ | H | $OCH_3$ | $OCH_3$ | |
| S | S | $CH_3$ | $CH_3$ | $CH_3$ | $CH_3$ | H | $CH_3$ | $CH(OCH_3)_2$ | |
| S | S | $CH_3$ | $CH_3$ | $CH_3$ | $CH_3$ | H | $CH_3$ | 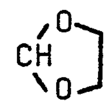 | |
| S | S | $CH_3$ | $CH_3$ | $CH_3$ | $CH_3$ | H | $OCH_3$ | $SCH_3$ | |
| S | S | $CH_3$ | $CH_3$ | $CH_3$ | $CH_3$ | $CH_3$ | $CH_3$ | $CH_3$ | |
| S | S | $CH_3$ | $CH_3$ | $CH_3$ | $CH_3$ | $CH_3$ | $CH_3$ | $OCH_3$ | |
| $SO_2$ | $SO_2$ | H | H | H | H | H | $CH_3$ | $CH_3$ | |
| $SO_2$ | $SO_2$ | H | H | H | H | H | $CH_3$ | $NHCH_3$ | |
| $SO_2$ | $SO_2$ | H | H | H | H | H | $CH_3$ | $OCH_3$ | |
| $SO_2$ | $SO_2$ | H | H | H | H | H | $CH_3$ | $SCH_3$ | |
| $SO_2$ | $SO_2$ | H | H | H | H | H | $OCH_3$ | H | |
| $SO_2$ | $SO_2$ | H | H | H | H | H | $OCH_3$ | $OCH_3$ | |
| $SO_2$ | $SO_2$ | H | H | H | H | H | $OCH_3$ | $CH_2(OCH_3)_2$ | |
| $SO_2$ | $SO_2$ | H | H | H | H | H | $OCH_3$ | $NH(CH_3)_2$ | |
| $SO_2$ | $SO_2$ | H | H | H | H | H | Cl | $OCH_3$ | |
| $SO_2$ | $SO_2$ | H | H | H | H | H | Cl | $NH_2$ | |
| $SO_2$ | $SO_2$ | H | H | H | H | H | Cl | $N(CH_3)_2$ | |
| $SO_2$ | $SO_2$ | H | H | H | H | $CH_3$ | $CH_3$ | $OCH_3$ | |
| $SO_2$ | $SO_2$ | H | H | H | H | $CH_3$ | $OCH_3$ | $OCH_3$ | |
| $SO_2$ | $SO_2$ | $CH_3$ | H | H | H | H | $CH_3$ | $CH_3$ | |
| $SO_2$ | $SO_2$ | $CH_3$ | H | H | H | H | $CH_3$ | $OCH_3$ | |
| $SO_2$ | $SO_2$ | $CH_3$ | H | H | H | H | $OCH_3$ | $OCH_3$ | |
| $SO_2$ | $SO_2$ | $CH_3$ | H | H | H | H | Cl | $OCH_3$ | |

Table Va (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| $SO_2$ | $SO_2$ | $CH_3$ | H | H | H | H | Cl | $N(CH_3)_2$ | |
| $SO_2$ | $SO_2$ | $CH_3$ | H | $CH_3$ | H | H | $CH_3$ | $CH_3$ | |
| $SO_2$ | $SO_2$ | $CH_3$ | H | $CH_3$ | H | H | $CH_3$ | $OCH_3$ | |
| $SO_2$ | $SO_2$ | $CH_3$ | H | $CH_3$ | H | H | $OCH_3$ | $OCH_3$ | |
| $SO_2$ | $SO_2$ | $CH_3$ | $CH_3$ | H | H | H | $CH_3$ | $CH_3$ | |
| $SO_2$ | $SO_2$ | $CH_3$ | $CH_3$ | H | H | H | $CH_3$ | $NH(CH_3)$ | |
| $SO_2$ | $SO_2$ | $CH_3$ | $CH_3$ | H | H | H | $OCH_3$ | $OCH_3$ | |
| $SO_2$ | $SO_2$ | $CH_3$ | $CH_3$ | H | H | H | $OCH_3$ | $N(CH_3)_2$ | |
| $SO_2$ | $SO_2$ | $CH_3$ | $CH_3$ | $CH_3$ | H | H | $CH_3$ | $CH_3$ | |
| $SO_2$ | $SO_2$ | $CH_3$ | $CH_3$ | $CH_3$ | H | H | $OCH_3$ | $OCH_3$ | |
| $SO_2$ | $SO_2$ | $CH_3$ | $CH_3$ | $CH_3$ | $CH_3$ | H | $OCH_3$ | $OCH_3$ | |
| $SO_2$ | $SO_2$ | $CH_3$ | $CH_3$ | $CH_3$ | $CH_3$ | H | $CH_3$ | $CH(OCH_3)_2$ | |
| $SO_2$ | $SO_2$ | $CH_3$ | CH | $CH_3$ | $CH_3$ | H | $CH_3$ | 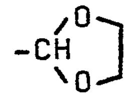 | |
| $SO_2$ | $SO_2$ | $CH_3$ | $CH_3$ | $CH_3$ | $CH_3$ | H | $OCH_3$ | $SCH_3$ | |
| $SO_2$ | $SO_2$ | $CH_3$ | $CH_3$ | $CH_3$ | $CH_3$ | $CH_3$ | $CH_3$ | $CH_3$ | |
| $SO_2$ | $SO_2$ | $CH_3$ | $CH_3$ | $CH_3$ | $CH_3$ | $CH_3$ | $CH_3$ | $OCH_3$ | |
| SO | SO | H | H | H | H | H | $CH_3$ | $CH_3$ | |
| SO | SO | H | H | H | H | H | $OCH_3$ | $OCH_3$ | |
| SO | SO | H | H | H | H | H | $OCH_3$ | $SCH_3$ | |
| SO | SO | H | H | H | H | H | $CH_3$ | $N(CH_3)_2$ | |
| SO | SO | $CH_3$ | H | H | H | H | $CH_3$ | $OCH_3$ | |
| SO | SO | $CH_3$ | $CH_3$ | $CH_3$ | $CH_3$ | H | $CH_3$ | $OCH_3$ | |
| O | S | H | H | H | H | H | $CH_3$ | $OCH_3$ | |
| O | S | $CH_3$ | $CH_3$ | $CH_3$ | $CH_3$ | H | $CH_3$ | $CH_3$ | |
| O | SO | H | H | H | H | H | $CH_3$ | $OCH_3$ | |
| O | $SO_2$ | H | H | H | H | H | $CH_3$ | $OCH_3$ | |
| O | $SO_2$ | $CH_3$ | H | $CH_3$ | H | H | $CH_3$ | $OCH_3$ | |
| S | O | H | H | H | H | H | $CH_3$ | $OCH_3$ | |
| SO | O | H | H | H | H | H | $CH_3$ | $OCH_3$ | |
| $SO_2$ | O | H | H | H | H | H | $CH_3$ | $OCH_3$ | |
| $SO_2$ | S | H | H | H. | H | H | $CH_3$ | $OCH_3$ | |

Table Va (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| $SO_2$ | S | H | H | H | H | H | $CH_3$ | $OCH_3$ | |
| S | SO | H | H | H | H | H | $CH_3$ | $OCH_3$ | |
| S | $SO_2$ | H | H | H | H | H | $CH_3$ | $OCH_3$ | |
| S | $SO_2$ | $CH_3$ | H | $CH_3$ | H | H | $CH_3$ | $OCH_3$ | |
| $SO_2$ | SO | H | H | H | H | H | $CH_3$ | $OCH_3$ | |
| SO | $SO_2$ | H | H | H | H | H | $CH_3$ | $OCH_3$ | |

Table Vb

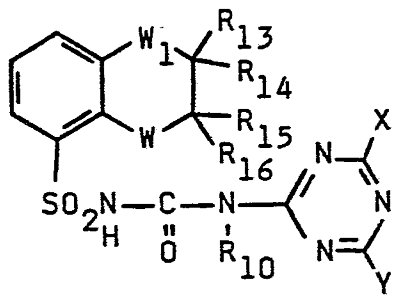

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| O | O | H | H | H | H | H | $CH_3$ | $CH_3$ | |
| O | O | H | H | H | H | H | $CH_3$ | $OCH_3$ | |
| O | O | H | H | H | H | H | $CH_3$ | $OC_2H_5$ | |
| O | O | H | H | H | H | H | $CH_3$ | $N(CH_3)_2$ | |
| O | O | H | H | H | H | H | $CH_3$ | $CH_2OCH_3$ | |
| O | O | H | H | H | H | H | $CH_3$ | $SCH_3$ | |
| O | O | H | H | H | H | H | $OCH_3$ | $SCH_3$ | |
| O | O | H | H | H | H | H | $OCH_3$ | $OCH_3$ | |
| O | O | H | H | H | H | H | $OCH_3$ | $OC_2H_5$ | |
| O | O | H | H | H | H | H | $OCH_3$ | $CH_2OCH_3$ | |
| O | O | H | H | H | H | H | $OCH_3$ | $NH_2$ | |
| O | O | H | H | H | H | H | $OCH_3$ | $NHCH_3$ | |
| O | O | H | H | H | H | H | $OCH_3$ | $N(CH_3)_2$ | |
| O | O | H | H | H | H | $CH_3$ | $CH_3$ | $OCH_3$ | |
| O | O | H | H | H | H | $CH_3$ | $OCH_3$ | $OCH_3$ | |
| O | O | $CH_3$ | H | H | H | H | $CH_3$ | $CH_3$ | |
| O | O | $CH_3$ | H | H | H | H | $CH_3$ | $OCH_3$ | |
| O | O | $CH_3$ | H | H | H | H | $CH_3$ | $CH(OCH_3)_2$ | |
| O | O | $CH_3$ | H | H | H | H | $CH_3$ | $CH_2OCH_3$ | |
| O | O | $CH_3$ | H | H | H | H | $OCH_3$ | $OCH_3$ | |
| O | O | $CH_3$ | H | H | H | H | $OCH_3$ | 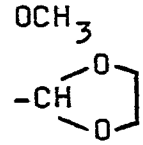 | |
| O | O | $CH_3$ | H | H | H | H | $OCH_3$ | $CH_2OCH_3$ | |
| O | O | $CH_3$ | H | H | H | H | $OCH_3$ | $SCH_3$ | |

Table Vb (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| O | O | $CH_3$ | H | $CH_3$ | H | H | $CH_3$ | $OCH_3$ | |
| O | O | $CH_3$ | H | $CH_3$ | H | H | $CH_3$ | $OC_2H_5$ | |
| O | O | $CH_3$ | H | $CH_3$ | H | H | $OCH_3$ | H | |
| O | O | $CH_3$ | H | $CH_3$ | H | H | $OCH_3$ | $OCH_3$ | |
| O | O | $CH_3$ | $CH_3$ | H | H | H | $CH_3$ | $CH_3$ | |
| O | O | $CH_3$ | $CH_3$ | H | H | H | $CH_3$ | $OCH_3$ | |
| O | O | $CH_3$ | $CH_3$ | H | H | H | $OCH_3$ | $OCH_3$ | |
| O | O | $CH_3$ | $CH_3$ | $CH_3$ | H | H | $CH_3$ | $CH_3$ | |
| O | O | $CH_3$ | $CH_3$ | $CH_3$ | H | H | $CH_3$ | $OCH_3$ | |
| O | O | $CH_3$ | $CH_3$ | $CH_3$ | H | H | $OCH_3$ | $OCH_3$ | |
| O | O | $CH_3$ | $CH_3$ | $CH_3$ | $CH_3$ | H | $CH_3$ | $OCH_3$ | |
| O | O | $CH_3$ | $CH_3$ | $CH_3$ | $CH_3$ | H | $CH_3$ | $N(CH_3)_2$ | |
| O | O | $CH_3$ | $CH_3$ | $CH_3$ | $CH_3$ | H | $CH_3$ | 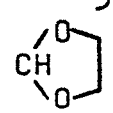 | |
| O | O | $CH_3$ | $CH_3$ | $CH_3$ | $CH_3$ | H | $OCH_3$ | $OCH_3$ | |
| O | O | $CH_3$ | $CH_3$ | $CH_3$ | $CH_3$ | H | $OCH_3$ | $SCH_3$ | |
| O | O | $CH_3$ | $CH_3$ | $CH_3$ | $CH_3$ | H | $OCH_3$ | $N(CH_3)_2$ | |
| O | O | $CH_3$ | $CH_3$ | $CH_3$ | $CH_3$ | $CH_3$ | $CH_3$ | $CH_3$ | |
| O | O | $CH_3$ | $CH_3$ | $CH_3$ | $CH_3$ | $CH_3$ | $CH_3$ | $OCH_3$ | |
| S | S | H | H | H | H | H | $CH_3$ | $CH_3$ | |
| S | S | H | H | H | H | H | $CH_3$ | $OCH_3$ | |
| S | S | H | H | H | H | H | $CH_3$ | $OC_2H_5$ | |
| S | S | H | H | H | H | H | $CH_3$ | $CH_2OCH_3$ | |
| S | S | H | H | H | H | H | $OCH_3$ | H | |
| S | S | H | H | H | H | H | $OCH_3$ | $OCH_3$ | |
| S | S | H | H | H | H | H | $OCH_3$ | $OC_2H_5$ | |
| S | S | H | H | H | H | H | $OCH_3$ | $CH_2OCH_3$ | |
| S | S | H | H | H | H | H | $OCH_3$ | $SCH_3$ | |
| S | S | H | H | H | H | $CH_3$ | $CH_3$ | $CH_3$ | |
| S | S | H | H | H | H | $CH_3$ | $CH_3$ | $OCH_3$ | |
| S | S | $CH_3$ | H | H | H | H | $CH_3$ | $CH_3$ | |
| S | S | $CH_3$ | H | H | H | H | $CH_3$ | $OCH_3$ | |

Table Vb (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| S | S | $CH_3$ | H | H | H | H | $CH_3$ | $OC_2H_5$ | |
| S | S | $CH_3$ | H | H | H | H | $CH_3$ | $CH_2OCH_3$ | |
| S | S | $CH_3$ | H | H | H | H | $OCH_3$ | $OCH_3$ | |
| S | S | $CH_3$ | H | H | H | H | $OCH_3$ | $OC_2H_5$ | |
| S | S | $CH_3$ | H | H | H | H | $OCH_3$ | $CH_2OCH_3$ | |
| S | S | $CH_3$ | H | $CH_3$ | H | H | $CH_3$ | $OC_2H_5$ | |
| S | S | $CH_3$ | H | $CH_3$ | H | H | $OCH_3$ | H | |
| S | S | $CH_3$ | H | $CH_3$ | H | H | $OCH_3$ | $OCH_3$ | |
| S | S | $CH_3$ | $CH_3$ | H | H | H | $CH_3$ | $CH_3$ | |
| S | S | $CH_3$ | $CH_3$ | H | H | H | $CH_3$ | $OCH_3$ | |
| S | S | $CH_3$ | $CH_3$ | H | H | H | $OCH_3$ | $OCH_3$ | |
| S | S | $CH_3$ | $CH_3$ | $CH_3$ | H | H | $CH_3$ | $CH_3$ | |
| S | S | $CH_3$ | $CH_3$ | $CH_3$ | H | H | $CH_3$ | $OCH_3$ | |
| S | S | $CH_3$ | $CH_3$ | $CH_3$ | H | H | $OCH_3$ | $OCH_3$ | |
| S | S | $CH_3$ | $CH_3$ | $CH_3$ | $CH_3$ | H | $CH_3$ | $OCH_3$ | |
| S | S | $CH_3$ | $CH_3$ | $CH_3$ | $CH_3$ | H | $CH_3$ | $NH_2$ | |
| S | S | $CH_3$ | $CH_3$ | $CH_3$ | $CH_3$ | H | $CH_3$ | $NHCH_3$ | |
| S | S | $CH_3$ | $CH_3$ | $CH_3$ | $CH_3$ | H | $CH_3$ | $N(CH_3)_2$ | |
| S | S | $CH_3$ | $CH_3$ | $CH_3$ | $CH_3$ | H | $OCH_3$ | $OCH_3$ | |
| S | S | $CH_3$ | $CH_3$ | $CH_3$ | $CH_3$ | H | $OCH_3$ | $SCH_3$ | |
| S | S | $CH_3$ | $CH_3$ | $CH_3$ | $CH_3$ | $CH_3$ | $CH_3$ | $CH_3$ | |
| S | S | $CH_3$ | $CH_3$ | $CH_3$ | $CH_3$ | $CH_3$ | $OCH_3$ | $OCH_3$ | |
| $SO_2$ | $SO_2$ | H | H | H | H | H | $CH_3$ | $CH_3$ | |
| $SO_2$ | $SO_2$ | H | H | H | H | H | $CH_3$ | $OCH_3$ | |
| $SO_2$ | $SO_2$ | H | H | H | H | H | $CH_3$ | $OC_2H_5$ | |
| $SO_2$ | $SO_2$ | H | H | H | H | H | $CH_3$ | $CH_2OCH_3$ | |
| $SO_2$ | $SO_2$ | H | H | H | H | H | $CH_3$ | $N(CH_3)_2$ | |
| $SO_2$ | $SO_2$ | H | H | H | H | H | $OCH_3$ | $OCH_3$ | |
| $SO_2$ | $SO_2$ | H | H | H | H | H | $OCH_3$ | $OC_2H_5$ | |
| $SO_2$ | $SO_2$ | H | H | H | H | H | $OCH_3$ | $CH_2OCH_3$ | |
| $SO_2$ | $SO_2$ | H | H | H | H | $CH_3$ | $CH_3$ | $OCH_3$ | |
| $SO_2$ | $SO_2$ | H | H | H | H | $CH_3$ | $OCH_3$ | $OCH_3$ | |

Table Vb (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| $SO_2$ | $SO_2$ | $CH_3$ | H | H | H | H | $CH_3$ | $CH_3$ | |
| $SO_2$ | $SO_2$ | $CH_3$ | H | H | H | H | $CH_3$ | $OCH_3$ | |
| $SO_2$ | $SO_2$ | $CH_3$ | H | H | H | H | $CH_3$ | $OC_2H_5$ | |
| $SO_2$ | $SO_2$ | $CH_3$ | H | H | H | H | $CH_3$ | $CH_2OCH_3$ | |
| $SO_2$ | $SO_2$ | $CH_3$ | H | H | H | H | $OCH_3$ | $OCH_3$ | |
| $SO_2$ | $SO_2$ | $CH_3$ | H | H | H | H | $OCH_3$ | $OC_2H_5$ | |
| $SO_2$ | $SO_2$ | $CH_3$ | H | H | H | H | $OCH_3$ | $CH_2OCH_3$ | |
| $SO_2$ | $SO_2$ | $CH_3$ | H | $CH_3$ | H | H | $CH_3$ | $OCH_3$ | |
| $SO_2$ | $SO_2$ | $CH_3$ | H | $CH_3$ | H | H | $CH_3$ | $OC_2H_5$ | |
| $SO_2$ | $SO_2$ | $CH_3$ | H | $CH_3$ | H | H | $OCH_3$ | H | |
| $SO_2$ | $SO_2$ | $CH_3$ | H | $CH_3$ | H | H | $OCH_3$ | $OCH_3$ | |
| $SO_2$ | $SO_2$ | $CH_3$ | $CH_3$ | H | H | H | $CH_3$ | $CH_3$ | |
| $SO_2$ | $SO_2$ | $CH_3$ | $CH_3$ | H | H | H | $CH_3$ | $OCH_3$ | |
| $SO_2$ | $SO_2$ | $CH_3$ | $CH_3$ | H | H | H | $OCH_3$ | $OCH_3$ | |
| $SO_2$ | $SO_2$ | $CH_3$ | $CH_3$ | $CH_3$ | H | H | $CH_3$ | $CH_3$ | |
| $SO_2$ | $SO_2$ | $CH_3$ | $CH_3$ | $CH_3$ | H | H | $CH_3$ | $OCH_3$ | |
| $SO_2$ | $SO_2$ | $CH_3$ | $CH_3$ | $CH_3$ | H | H | $OCH_3$ | $OCH_3$ | |
| $SO_2$ | $SO_2$ | $CH_3$ | $CH_3$ | $CH_3$ | $CH_3$ | H | $CH_3$ | $OCH_3$ | |
| $SO_2$ | $SO_2$ | $CH_3$ | $CH_3$ | $CH_3$ | $CH_3$ | H | $OCH_3$ | $OCH_3$ | |
| $SO_2$ | $SO_2$ | $CH_3$ | $CH_3$ | $CH_3$ | $CH_3$ | H | $OCH_3$ | $NHCH_3$ | |
| $SO_2$ | $SO_2$ | $CH_3$ | $CH_3$ | $CH_3$ | $CH_3$ | $CH_3$ | $CH_3$ | $OCH_3$ | |
| $SO_2$ | $SO_2$ | $CH_3$ | $CH_3$ | $CH_3$ | $CH_3$ | $CH_3$ | $OCH_3$ | $OCH_3$ | |
| SO | SO | H | H | H | H | H | $CH_3$ | $CH_3$ | |
| SO | SO | H | H | H | H | H | $CH_3$ | $SCH_3$ | |
| SO | SO | H | H | H | H | H | $CH_3$ | $OCH_3$ | |
| SO | SO | H | H | H | H | H | $OCH_3$ | $OCH_3$ | |
| SO | SO | $CH_3$ | H | H | H | H | $CH_3$ | $OCH_3$ | |
| SO | SO | $CH_3$ | $CH_3$ | $CH_3$ | $CH_3$ | H | $CH_3$ | $OCH_3$ | |
| O | S | H | H | H | H | H | $CH_3$ | $OCH_3$ | |
| O | S | $CH_3$ | $CH_3$ | $CH_3$ | $CH_3$ | H | $OCH_3$ | $SCH_3$ | |
| O | SO | H | H | H | H | H | $CH_3$ | $OCH_3$ | |
| O | $SO_2$ | H | H | H | H | H | $CH_3$ | $OCH_3$ | |

Table Vb (continued)

| $W_1$ | W | $R_{13}$ | $R_{14}$ | $R_{15}$ | $R_{16}$ | $R_{10}$ | X | Y | m.p.(°C) |
|---|---|---|---|---|---|---|---|---|---|
| O | $SO_2$ | $CH_3$ | H | $CH_3$ | H | H | $CH_3$ | $OCH_3$ | |
| S | O | H | H | H | H | H | $CH_3$ | $OCH_3$ | |
| SO | O | H | H | H | H | H | $CH_3$ | $OCH_3$ | |
| $SO_2$ | O | H | H | H | H | H | $CH_3$ | $OCH_3$ | |
| SO | S | H | H | H | H | H | $CH_3$ | $OCH_3$ | |
| $SO_2$ | S | H | H | H | H | H | $CH_3$ | $OCH_3$ | |
| S | SO | H | H | H | H | H | $CH_3$ | $OCH_3$ | |
| S | $SO_2$ | H | H | H | H | H | $CH_3$ | $OCH_3$ | |
| S | $SO_2$ | $CH_3$ | H | $CH_3$ | H | H | $CH_3$ | $OCH_3$ | |
| $SO_2$ | SO | H | H | H | H | H | $CH_3$ | $OCH_3$ | |
| SO | $SO_2$ | H | H | H | H | H | $CH_3$ | $OCH_3$ | |

Table Vc

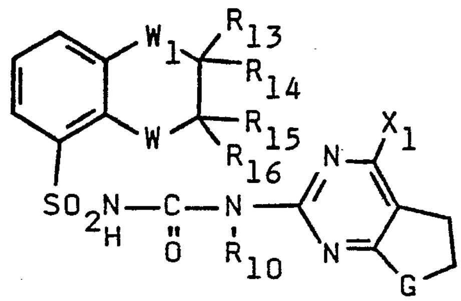

| $W_1$ | W | $R_{13}$ | $R_{14}$ | $R_{15}$ | $R_{16}$ | $R_{10}$ | G | $X_1$ |
|---|---|---|---|---|---|---|---|---|
| O | O | H | H | H | H | H | $CH_2$ | $CH_3$ |
| O | O | H | H | H | H | H | $CH_2$ | $OCH_3$ |
| O | O | H | H | H | H | H | O | $CH_3$ |
| O | O | H | H | H | H | H | O | $OCH_3$ |
| O | O | H | H | H | H | $CH_3$ | $CH_3$ | $CH_3$ |
| O | O | H | H | H | H | $CH_3$ | O | $OCH_3$ |
| O | O | $CH_3$ | H | H | H | H | O | $CH_3$ |
| O | O | $CH_3$ | H | $CH_3$ | H | H | $CH_2$ | $OCH_3$ |
| O | O | $CH_3$ | H | $CH_3$ | H | H | O | $CH_3$ |
| O | O | $CH_3$ | H | $CH_3$ | H | O | O | $OCH_3$ |
| O | O | $CH_3$ | $CH_3$ | $CH_3$ | H | H | O | $OCH_3$ |
| O | O | $CH_3$ | $CH_3$ | $CH_3$ | $CH_3$ | H | $CH_2$ | $CH_3$ |
| O | O | $CH_3$ | $CH_3$ | $CH_3$ | $CH_3$ | H | O | $CH_3$ |
| O | O | $CH_3$ | $CH_3$ | $CH_3$ | $CH_3$ | H | O | $OCH_3$ |
| O | O | $CH_3$ | $CH_3$ | $CH_3$ | $CH_3$ | $CH_3$ | $CH_2$ | $CH_3$ |
| O | O | $CH_3$ | $CH_3$ | $CH_3$ | $CH_3$ | $CH_3$ | O | $OCH_3$ |
| S | S | H | H | H | H | H | $CH_2$ | $CH_3$ |
| S | S | H | H | H | H | H | $CH_2$ | $OCH_3$ |
| S | S | H | H | H | H | H | O | $CH_3$ |
| S | S | H | H | H | H | H | O | $OCH_3$ |
| S | S | H | H | H | H | $CH_3$ | $CH_2$ | $CH_3$ |
| S | S | H | H | H | H | $CH_3$ | O | $OCH_3$ |
| S | S | $CH_3$ | H | H | H | H | O | $CH_3$ |
| S | S | $CH_3$ | H | $CH_3$ | H | H | $CH_2$ | $OCH_3$ |

Table Vc (continued)

| $W_1$ | W | $R_{13}$ | $R_{14}$ | $R_{15}$ | $R_{16}$ | $R_{10}$ | G | $X_1$ |
|---|---|---|---|---|---|---|---|---|
| S | S | $CH_3$ | H | $CH_3$ | H | H | O | $CH_3$ |
| S | S | $CH_3$ | H | $CH_3$ | H | O | O | $OCH_3$ |
| S | S | $CH_3$ | $CH_3$ | $CH_3$ | H | H | O | $OCH_3$ |
| S | S | $CH_3$ | $CH_3$ | $CH_3$ | $CH_3$ | H | $CH_2$ | $CH_3$ |
| S | S | $CH_3$ | $CH_3$ | $CH_3$ | $CH_3$ | H | O | $CH_3$ |
| S | S | $CH_3$ | $CH_3$ | $CH_3$ | $CH_3$ | H | O | $OCH_3$ |
| S | S | $CH_3$ | $CH_3$ | $CH_3$ | $CH_3$ | $CH_3$ | $CH_2$ | $CH_3$ |
| S | S | $CH_3$ | $CH_3$ | $CH_3$ | $CH_3$ | $CH_3$ | O | $CH_3$ |
| $SO_2$ | $SO_2$ | H | H | H | H | H | $CH_2$ | $CH_3$ |
| $SO_2$ | $SO_2$ | H | H | H | H | H | $CH_2$ | $OCH_3$ |
| $SO_2$ | $SO_2$ | H | H | H | H | H | O | $CH_3$ |
| $SO_2$ | $SO_2$ | H | H | H | H | H | O | $OCH_3$ |
| $SO_2$ | $SO_2$ | H | H | H | H | $CH_3$ | $CH_2$ | $CH_3$ |
| $SO_2$ | $SO_2$ | H | H | H | H | $CH_3$ | O | $CH_3$ |
| $SO_2$ | $SO_2$ | H | H | H | H | $CH_3$ | O | $OCH_3$ |
| $SO_2$ | $SO_2$ | $CH_3$ | H | H | H | H | O | $CH_3$ |
| $SO_2$ | $SO_2$ | $CH_3$ | H | $CH_3$ | H | H | $CH_2$ | $OCH_3$ |
| $SO_2$ | $SO_2$ | $CH_3$ | H | $CH_3$ | H | H | O | $CH_3$ |
| $SO_2$ | $SO_2$ | $CH_3$ | H | $CH_3$ | H | O | O | $OCH_3$ |
| $SO_2$ | $SO_2$ | $CH_3$ | $CH_3$ | $CH_3$ | H | H | O | $OCH_3$ |
| $SO_2$ | $SO_2$ | $CH_3$ | $CH_3$ | $CH_3$ | $CH_3$ | H | $CH_2$ | $CH_3$ |
| $SO_2$ | $SO_2$ | $CH_3$ | $CH_3$ | $CH_3$ | $CH_3$ | H | O | $CH_3$ |
| $SO_2$ | $SO_2$ | $CH_3$ | $CH_3$ | $CH_3$ | $CH_3$ | H | O | $OCH_3$ |
| $SO_2$ | $SO_2$ | $CH_3$ | $CH_3$ | $CH_3$ | $CH_3$ | $CH_3$ | $CH_2$ | $CH_3$ |
| $SO_2$ | $SO_2$ | $CH_3$ | $CH_3$ | $CH_3$ | $CH_3$ | $CH_3$ | O | $OCH_3$ |
| SO | SO | H | H | H | H | H | $CH_2$ | $CH_3$ |
| SO | SO | H | H | H | H | H | O | $OCH_3$ |
| SO | SO | H | H | H | H | H | O | $CH_3$ |
| SO | SO | $CH_3$ | H | $CH_3$ | H | H | O | $CH_3$ |
| SO | SO | $CH_3$ | $CH_3$ | $CH_3$ | $CH_3$ | H | O | $CH_3$ |
| O | S | H | H | H | H | H | O | $CH_3$ |
| O | SO | H | H | H | H | H | O | $CH_3$ |

Table Vc (continued)

| $W_1$ | $W$ | $R_{13}$ | $R_{14}$ | $R_{15}$ | $R_{16}$ | $R_{10}$ | G | $X_1$ |
|---|---|---|---|---|---|---|---|---|
| O | $SO_2$ | H | H | H | H | H | O | $CH_3$ |
| O | $SO_2$ | $CH_3$ | H | $CH_3$ | H | H | O | $CH_3$ |
| O | $SO_2$ | $CH_3$ | $CH_3$ | $CH_3$ | $CH_3$ | H | O | $CH_3$ |
| S | O | H | H | H | H | H | O | $CH_3$ |
| SO | O | H | H | H | H | H | O | $CH_3$ |
| $SO_2$ | O | H | H | H | H | H | O | $CH_3$ |
| SO | S | H | H | H | H | H | O | $CH_3$ |
| $SO_2$ | S | H | H | H | H | H | O | $CH_3$ |
| S | SO | H | H | H | H | H | O | $CH_3$ |
| S | $SO_2$ | H | H | H | H | H | O | $CH_3$ |
| S | $SO_2$ | $CH_3$ | H | $CH_3$ | H | H | O | $CH_3$ |
| $SO_2$ | SO | H | H | H | H | H | O | $CH_3$ |
| SO | $SO_2$ | H | H | H | H | H | O | $CH_3$ |

Table Vd

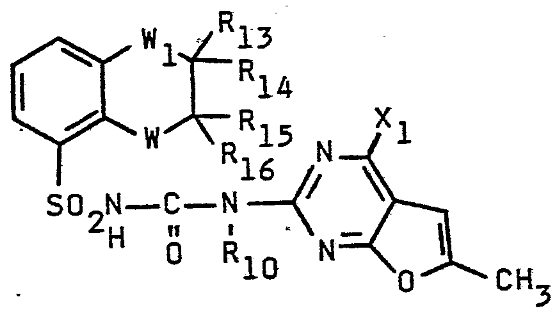

| $W_1$ | W | $R_{13}$ | $R_{14}$ | $R_{15}$ | $R_{16}$ | $R_{10}$ | $X_1$ |
|---|---|---|---|---|---|---|---|
| O | O | H | H | H | H | H | $CH_3$ |
| O | O | H | H | H | H | H | $OCH_3$ |
| O | O | H | H | H | H | $CH_3$ | $CH_3$ |
| O | O | H | H | H | H | $CH_3$ | $OCH_3$ |
| O | O | $CH_3$ | H | H | H | H | $CH_3$ |
| O | O | $CH_3$ | H | $CH_3$ | H | H | $OCH_3$ |
| O | O | $CH_3$ | H | $CH_3$ | H | H | $OCH_3$ |
| O | O | $CH_3$ | H | $CH_3$ | H | H | $CH_3$ |
| O | O | $CH_3$ | $CH_3$ | H | H | H | $CH_3$ |
| O | O | $CH_3$ | $CH_3$ | $CH_3$ | H | H | $CH_3$ |
| O | O | $CH_3$ | $CH_3$ | $CH_3$ | $CH_3$ | H | $CH_3$ |
| O | O | $CH_3$ | $CH_3$ | $CH_3$ | $CH_3$ | H | $OCH_3$ |
| O | O | $CH_3$ | $CH_3$ | $CH_3$ | $CH_3$ | $CH_3$ | $CH_3$ |
| O | O | $CH_3$ | $CH_3$ | $CH_3$ | $CH_3$ | $CH_3$ | $OCH_3$ |
| S | S | H | H | H | H | H | $CH_3$ |
| S | S | $CH_3$ | H | $CH_3$ | H | H | $CH_3$ |
| S | S | $CH_3$ | H | $CH_3$ | H | H | $OCH_3$ |
| S | S | $CH_3$ | $CH_3$ | $CH_3$ | $CH_3$ | H | $CH_3$ |
| S | S | $CH_3$ | $CH_3$ | $CH_3$ | $CH_3$ | H | $OCH_3$ |
| S | S | $CH_3$ | $CH_3$ | $CH_3$ | $CH_3$ | $CH_3$ | $CH_3$ |
| S | S | $CH_3$ | $CH_3$ | $CH_3$ | $CH_3$ | $CH_3$ | $OCH_3$ |
| $SO_2$ | $SO_2$ | H | H | H | H | H | $CH_3$ |
| $SO_2$ | $SO_2$ | H | H | H | H | H | $OCH_3$ |
| $SO_2$ | $SO_2$ | $CH_3$ | H | $CH_3$ | H | H | $CH_3$ |

Table Vd (continued)

| $W_1$ | W | $R_{13}$ | $R_{14}$ | $R_{15}$ | $R_{16}$ | $R_{10}$ | $X_1$ |
|---|---|---|---|---|---|---|---|
| $SO_2$ | $SO_2$ | $CH_3$ | $CH_3$ | $CH_3$ | $CH_3$ | H | $CH_3$ |
| $SO_2$ | $SO_2$ | $CH_3$ | $CH_3$ | $CH_3$ | $CH_3$ | H | $OCH_3$ |
| SO | SO | H | H | H | H | H | $CH_3$ |
| SO | SO | H | H | H | H | H | $OCH_3$ |
| SO | SO | $CH_3$ | H | $CH_3$ | H | H | $CH_3$ |
| SO | SO | $CH_3$ | H | $CH_3$ | H | H | $OCH_3$ |
| O | S | H | H | H | H | H | $OCH_3$ |
| O | SO | H | H | H | H | H | $OCH_3$ |
| O | $SO_2$ | H | H | H | H | H | $OCH_3$ |
| O | $SO_2$ | H | $CH_3$ | H | $CH_3$ | H | $CH_3$ |
| O | $SO_2$ | H | $CH_3$ | H | $CH_3$ | H | $OCH_3$ |
| O | $SO_2$ | $CH_3$ | $CH_3$ | $CH_3$ | $CH_3$ | H | $OCH_3$ |
| S | O | H | H | H | H | H | $CH_3$ |
| SO | O | H | H | H | H | H | $CH_3$ |
| $SO_2$ | O | H | H | H | H | H | $CH_3$ |
| SO | S | H | H | H | H | H | $CH_3$ |
| $SO_2$ | S | H | H | H | H | H | $CH_3$ |
| S | SO | H | H | H | H | H | $CH_3$ |
| S | $SO_2$ | H | H | H | H | H | $CH_3$ |
| S | $SO_2$ | $CH_3$ | H | $CH_3$ | H | H | $CH_3$ |
| S | $SO_2$ | $CH_3$ | $CH_3$ | H | $CH_3$ | H | $CH_3$ |
| $SO_2$ | SO | H | H | H | H | H | $CH_3$ |
| SO | $SO_2$ | H | H | H | H | H | $CH_3$ |

Table Ve

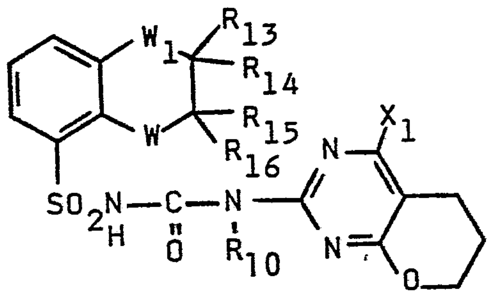

| $W_1$ | $W$ | $R_{13}$ | $R_{14}$ | $R_{15}$ | $R_{16}$ | $R_{10}$ | $X_1$ |
|---|---|---|---|---|---|---|---|
| O | O | H | H | H | H | H | $CH_3$ |
| O | O | H | H | H | H | H | $OCH_3$ |
| O | O | H | H | H | H | $CH_3$ | $CH_3$ |
| O | O | H | H | H | H | $CH_3$ | $OCH_3$ |
| O | O | $CH_3$ | H | H | H | H | $CH_3$ |
| O | O | $CH_3$ | H | $CH_3$ | H | H | $OCH_3$ |
| O | O | $CH_3$ | H | $CH_3$ | H | H | $CH_3$ |
| O | O | $CH_3$ | $CH_3$ | H | H | H | $CH_3$ |
| O | O | $CH_3$ | $CH_3$ | $CH_3$ | H | H | $CH_3$ |
| O | O | $CH_3$ | $CH_3$ | $CH_3$ | $CH_3$ | H | $CH_3$ |
| O | O | $CH_3$ | $CH_3$ | $CH_3$ | $CH_3$ | H | $OCH_3$ |
| O | O | $CH_3$ | $CH_3$ | $CH_3$ | $CH_3$ | $CH_3$ | $CH_3$ |
| O | O | $CH_3$ | $CH_3$ | $CH_3$ | $CH_3$ | $CH_3$ | $OCH_3$ |
| S | S | H | H | H | H | H | $CH_3$ |
| S | S | H | H | H | H | H | $OCH_3$ |
| S | S | $CH_3$ | H | $CH_3$ | H | H | $CH_3$ |
| S | S | $CH_3$ | H | $CH_3$ | H | H | $OCH_3$ |
| S | S | $CH_3$ | $CH_3$ | $CH_3$ | $CH_3$ | H | $CH_3$ |
| S | S | $CH_3$ | $CH_3$ | $CH_3$ | $CH_3$ | H | $OCH_3$ |
| S | S | $CH_3$ | $CH_3$ | $CH_3$ | $CH_3$ | $CH_3$ | $CH_3$ |
| $SO_2$ | $SO_2$ | H | H | H | H | H | $CH_3$ |
| $SO_2$ | $SO_2$ | H | H | H | H | H | $OCH_3$ |
| $SO_2$ | $SO_2$ | H | H | H | H | $CH_3$ | $OCH_3$ |
| $SO_2$ | $SO_2$ | $CH_3$ | H | $CH_3$ | H | H | $OCH_3$ |

Table Ve (continued)

| $W_1$ | W | $R_{13}$ | $R_{14}$ | $R_{15}$ | $R_{16}$ | $R_{10}$ | $X_1$ |
|---|---|---|---|---|---|---|---|
| $SO_2$ | $SO_2$ | $CH_3$ | $CH_3$ | $CH_3$ | $CH_3$ | H | $CH_3$ |
| $SO_2$ | $SO_2$ | $CH_3$ | $CH_3$ | $CH_3$ | $CH_3$ | H | $OCH_3$ |
| $SO_2$ | $SO_2$ | $CH_3$ | $CH_3$ | $CH_3$ | $CH_3$ | $CH_3$ | $CH_3$ |
| $SO_2$ | $SO_2$ | $CH_3$ | $CH_3$ | $CH_3$ | $CH_3$ | $CH_3$ | $OCH_3$ |
| SO | SO | H | H | H | H | H | $CH_3$ |
| SO | SO | H | H | H | H | H | $OCH_3$ |
| SO | SO | $CH_3$ | H | $CH_3$ | H | H | $CH_3$ |
| SO | SO | $CH_3$ | H | $CH_3$ | H | H | $OCH_3$ |
| O | S | H | H | H | H | H | $OCH_3$ |
| O | SO | H | H | H | H | H | $OCH_3$ |
| O | $SO_2$ | H | H | H | H | H | $OCH_3$ |
| O | $SO_2$ | H | $CH_3$ | H | $CH_3$ | H | $CH_3$ |
| O | $SO_2$ | H | $CH_3$ | H | $CH_3$ | H | $OCH_3$ |
| O | $SO_2$ | $CH_3$ | $CH_3$ | $CH_3$ | $CH_3$ | H | $OCH_3$ |
| S | O | H | H | H | H | H | $CH_3$ |
| SO | O | H | H | H | H | H | $CH_3$ |
| $SO_2$ | O | H | H | H | H | H | $CH_3$ |
| SO | S | H | H | H | H | H | $CH_3$ |
| $SO_2$ | S | H | H | H | H | H | $CH_3$ |
| S | SO | H | H | H | H | H | $CH_3$ |
| S | $SO_2$ | H | H | H | H | H | $CH_3$ |
| S | $SO_2$ | $CH_3$ | H | $CH_3$ | H | H | $CH_3$ |
| S | $SO_2$ | $CH_3$ | $CH_3$ | H | $CH_3$ | H | $CH_3$ |
| $SO_2$ | SO | H | H | H | H | H | $CH_3$ |
| SO | $SO_2$ | H | H | H | H | H | $CH_3$ |

Table Vf

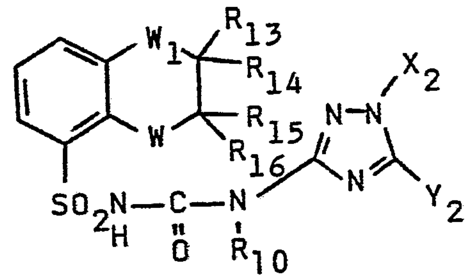

| $W_1$ | W | $R_{13}$ | $R_{14}$ | $R_{15}$ | $R_{16}$ | $R_{10}$ | $X_2$ | $Y_2$ |
|---|---|---|---|---|---|---|---|---|
| O | O | H | H | H | H | H | $CH_3$ | $OCH_3$ |
| O | O | H | H | H | H | H | $CH_2CF_3$ | $SCH_3$ |
| O | O | H | H | H | H | H | $CH_2CF_3$ | $OCH_2CH_3$ |
| O | O | H | H | H | H | $CH_3$ | $CH_3$ | $OCH_3$ |
| O | O | H | H | H | H | $CH_3$ | $CH_3$ | $SCH_3$ |
| O | O | $CH_3$ | H | H | H | H | $C_2H_5$ | $OCH_3$ |
| O | O | $CH_3$ | H | $CH_3$ | H | H | $CH_3$ | $OC_2H_5$ |
| O | O | $CH_3$ | H | $CH_3$ | H | H | $CH_2CF_3$ | $SCH_3$ |
| O | O | $CH_3$ | $CH_3$ | $CH_3$ | H | H | $CH_3CH_2CH_2$ | $OCH_3$ |
| O | O | $CH_3$ | $CH_3$ | $CH_3$ | $CH_3$ | H | $CH_3$ | $OCH_3$ |
| O | O | $CH_3$ | $CH_3$ | $CH_3$ | $CH_3$ | H | $CH_3$ | $SCH_3$ |
| O | O | $CH_3$ | $CH_3$ | $CH_3$ | $CH_3$ | H | $CH_3CH_2CH_2$ | $SCH_3$ |
| O | O | $CH_3$ | $CH_3$ | $CH_3$ | $CH_3$ | H | $(CH_3)_2CH$ | $SC_2H_5$ |
| O | O | $CH_3$ | $CH_3$ | $CH_3$ | $CH_3$ | $CH_3$ | $CH_3$ | $OCH_3$ |
| S | S | H | H | H | H | H | $CH_3$ | $OCH_3$ |
| S | S | H | H | H | H | H | $CH_3$ | $SCH_3$ |
| S | S | H | H | H | H | $CH_3$ | $CH_3$ | $OCH_3$ |
| S | S | $CH_3$ | H | H | H | H | $CH_3$ | $OCH_3$ |
| S | S | $CH_3$ | H | $CH_3$ | H | H | $CH_3$ | $OCH_3$ |
| S | S | $CH_3$ | $CH_3$ | $CH_3$ | $CH_3$ | H | $CH_3$ | $OCH_3$ |
| $SO_2$ | $SO_2$ | H | H | H | H | H | $CH_3$ | $OCH_3$ |
| $SO_2$ | $SO_2$ | H | H | H | H | H | $CH_3$ | $SCH_3$ |
| $SO_2$ | $SO_2$ | H | H | H | H | H | $CH_2CF_3$ | $OCH_3$ |
| $SO_2$ | $SO_2$ | H | H | H | H | $CH_3$ | $CH_3$ | $OCH_3$ |

Table Vf (continued)

| $W_1$ | W | $R_{13}$ | $R_{14}$ | $R_{15}$ | $R_{16}$ | $R_{10}$ | $X_2$ | $Y_2$ |
|---|---|---|---|---|---|---|---|---|
| $SO_2$ | $SO_2$ | $CH_3$ | $CH_3$ | $CH_3$ | $CH_3$ | H | $CH_3$ | $OCH_3$ |
| $SO_2$ | $SO_2$ | $CH_3$ | $CH_3$ | $CH_3$ | $CH_3$ | $CH_3$ | $CH_3$ | $OCH_3$ |
| SO | SO | H | H | H | H | H | $CH_3$ | $OCH_3$ |
| SO | SO | $CH_3$ | H | $CH_3$ | H | H | $CH_3$ | $OCH_3$ |
| SO | SO | $CH_3$ | $CH_3$ | $CH_3$ | $CH_3$ | H | $CH_3$ | $OCH_3$ |
| O | S | H | H | H | H | H | $CH_3$ | $OCH_3$ |
| O | SO | H | H | H | H | H | $CH_3$ | $OCH_3$ |
| O | $SO_2$ | H | H | H | H | H | $CH_3$ | $OCH_3$ |
| O | $SO_2$ | $CH_3$ | H | $CH_3$ | H | H | $CH_3$ | $OCH_3$ |
| O | $SO_2$ | $CH_3$ | $CH_3$ | $CH_3$ | $CH_3$ | H | $CH_3$ | $OCH_3$ |
| S | O | H | H | H | H | H | $CH_3$ | $OCH_3$ |
| SO | O | H | H | H | H | H | $CH_3$ | $OCH_3$ |
| $SO_2$ | O | H | H | H | H | H | $CH_3$ | $OCH_3$ |
| SO | S | H | H | H | H | H | $CH_3$ | $OCH_3$ |
| $SO_2$ | S | H | H | H | H | H | $CH_3$ | $OCH_3$ |
| S | SO | H | H | H | H | H | $CH_3$ | $OCH_3$ |
| S | $SO_2$ | H | H | H | H | H | $CH_3$ | $OCH_3$ |
| $SO_2$ | SO | H | H | H | H | H | $CH_3$ | $OCH_3$ |
| SO | $SO_2$ | H | H | H | H | H | $CH_3$ | $OCH_3$ |

Formulations

Useful formulations of the compounds of Formula I, Ia, II, IIa or III can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

Table VI

| | Weight Percent* | | |
| --- | --- | --- | --- |
| | Active Ingredient | Diluent(s) | Surfactant(s) |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", <u>Chemical Engineering</u>,

December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley and Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

## Example 34

Wettable Powder

| | |
|---|---|
| 2,3-dihydro-N-[(4,6-dimethoxypyrimidin-2-yl)aminocarbonyl]-1H-indene-4-sulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 35

Wettable Powder

| | |
|---|---|
| 2,3-dihydro-N-[(4-methoxy-6-methylpyrimidin-2-yl)-aminocarbonyl]-1H-indene-4-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 36

Granule

| | |
|---|---|
| Wettable Powder of Example 35 | 5% |
| attapulgite granules (U.S.S. 20-40 mesh; 0.84-0.42 mm) | 95% |

A slurry of wettable powder containing ≈25% solids is sprayed on the surface of attapulgite granules in a double-cone blender. The granules are dried and packaged.

Example 37

Extruded Pellet

| | |
|---|---|
| 2,3-dihydro-N-[(4-methoxy-6-methyl-1,3,5-triazin-2-yl)aminocarbonyl]-1H-indene-4-sulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve

(0.42 mm openings) may be packaged for use and the fines recycled.

Example 38

Oil Suspension

2,3-dihydro-N-[(4,6-dimethoxy-1,3,5-triazin-2-yl)-aminocarbonyl]-1H-indene-4-sulfonamide 25%
polyoxyethylene sorbitol hexaoleate 5%
highly aliphatic hydrocarbon oil 70%

The ingredients are ground together in a sand mill until the solid particles have been reduced to under about 5 microns. The resulting thick suspension may be applied directly, but preferably after being extended with oils or emulsified in water.

Example 39

Wettable Powder

2,3-dihydro-N-[(4,6-dimethyl-1,3,5-triazin-2-yl)-aminocarbonyl]-1H-indene-4-sulfonamide 20%
sodium alkylnaphthalenesulfonate 4%
sodium ligninsulfonate 4%
low viscosity methyl cellulose 3%
attapulgite 69%

The ingredients are thoroughly blended. After grinding in a hammer-mill to produce particles essentially all below 100 microns, the material is reblended and sifted through a U.S.S. No. 50 sieve (0.3 mm opening) and packaged.

Example 40

Low Strength Granule

2,3-dihydro-N-[(4,6-dimethylpyrimidin-2-yl)aminocarbonyl]-1H-indene-4-sulfonamide 1%
N,N-dimethylformamide 9%
attapulgite granules (U.S.S. 20-40 sieve) 90%

The active ingredient is dissolved in the solvent and the solution is sprayed upon dedusted granules in a double cone blender. After spraying of the solution has been completed, the blender is allowed to run for a short period and then the granules are packaged.

Example 41

Aqueous Suspension

| | |
|---|---|
| 2,3-dihydro-N-[(4,6-dimethoxypyrimidin-2-yl)aminocarbonyl]-5-nitro-1H-indene-4-sulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 42

Solution

| | |
|---|---|
| N-[(4,6-dimethoxypyrimidin-2-yl)aminocarbonyl]-5,6,7,8-tetrahydro-1-naphthalenesulfonamide, sodium salt | 5% |
| water | 95% |

The salt is added directly to the water with stirring to produce the solution, which may then be packaged for use.

Example 43

Low Strength Granule

| | |
|---|---|
| 2,3-dihydro-N-[(4-methoxy-6-methylpyrimidin-2-yl)aminocarbonyl]-5-nitro-1H-indene-4-sulfonamide | 0.1% |
| attapulgite granules (U.S.S. 20-40 mesh) | 99.9% |

The active ingredient is dissolved in a solvent and the solution is sprayed upon dedusted granules in

a double-cone blender. After spraying of the solution has been completed, the material is warmed to evaporate the solvent. The material is allowed to cool and then packaged.

Example 44

Granule

| | |
|---|---|
| 2,3-dihydro-N-[(4,6-dimethoxypyrimidin-2-yl)aminocarbonyl]-5-nitro-1H-indene-4-sulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 45

High Strength Concentrate

| | |
|---|---|
| 2,3-dihydro-N-[(4,6-dimethoxy-1,3,5-triazin-2-yl)aminocarbonyl]-5-nitro-1H-indene-4-sulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 46

Wettable Powder

| | |
|---|---|
| 2,3-dihydro-N-[(4-methoxy-6-methyl-1,3,5-triazin-2-yl)aminocarbonyl]-5-nitro-1H-indene-4-sulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 47

Wettable Powder

| | |
|---|---|
| N-[(4,6-dimethoxy-1,3,5-triazin-2-yl)aminocarbonyl]-5,6,7,8-tetrahydro-1-naphthalenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 48

Oil Suspension

| | |
|---|---|
| 1-chloro-N-[(4-methoxy-6-methylpyrimidin-2-yl)aminocarbonyl]-3H-indene-4-sulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 49

Dust

| | |
|---|---|
| 1-chloro-N-[(4-methoxy-6-methyl-1,3,5-triazin-2-yl)aminocarbonyl]-3H-indene-4-sulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

The compounds of the present invention are active herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for the selective pre- and/or post-emergence weed control in crops, such as cotton, wheat, and barley.

The rates of application for the compounds of the invention are determined by a number of factors, including their use as selective or general herbicides, the crop species involved, the types of weeds to be controlled, weather and climate, formulations selected, mode of application, amount of foliage present, etc. In general terms, the subject compounds should be applied at levels of around 0.125 to 10 kg/ha, the lower rates being suggested for use on lighter soils and/or those having a low organic matter content, for selective weed control or for situations where only short-term persistence is required.

The compounds of the invention may be used in combination with any other commercial herbicide examples of which are those of the triazine, triazole, uracil, urea, amide, diphenylether, carbamate, acetanilide, dinitroaniline, phenolic and bipyridylium types.

The herbicidal properties of the subject compounds were discovered in a number of greenhouse tests. The test procedures and results follow.

Test A

Seeds of crabgrass (*Digitaria* sp.), barnyardgrass (*Echinochloa crusgalli*), wild oats (*Avena fatua*), sicklepod (*Cassia obtusifolia*), morningglory (*Ipomoea* sp.), cocklebur (*Xanthium* sp.), sorghum, corn, soybean, rice, wheat and nutsedge tubers (*Cyperus rotundus*) were planted in a growth medium and treated pre-emergence with the chemicals dissolved in a non-phytotoxic solvent solution of the compounds of Table A. At the same time, cotton having five leaves (including cotyledonary ones), bush beans with the third trifoliolate leaf expanding, crabgrass, barnyardgrass and wild oats with two leaves, sicklepod with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three-five leaves were sprayed with a non-phytotoxic solvent solution of the compounds of Table A. Other containers of the above mentioned weeds and crops were treated pre- or post-emergence with the same non-phytotoxic solvent so as to provide a solvent control. A set of untreated control plants was also included for comparison. Pre-emergence and post-emergence treated plants

and controls were maintained in a greenhouse for sixteen days, then all treated plants were compared with their respective controls and rated visually for response to treatment.

The following rating system was used:

0 = no effect;
10 = maximum effect;
A = accelerated growth;
C = chlorosis and/or necrosis;
D = defoliation;
E = emergence inhibition;
G = growth retardation;
H = formative effects;
S = albinism;
U = unusual pigmentation;
X = axillary stimulation;
6Y = abscised buds or flowers; and
6F = delayed flowering.

The data show that the compounds tested are highly active pre- and post-emergence herbicides.

Table A Structures

Compound 1

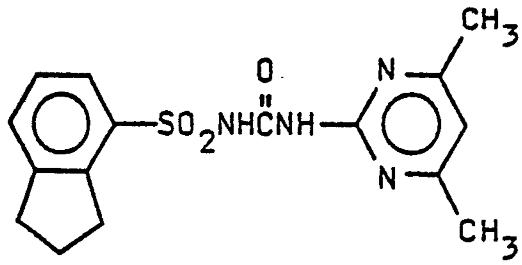

Compound 2

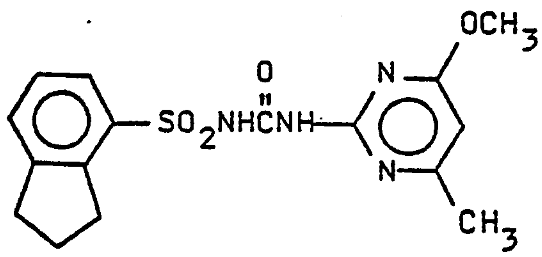

Compound 3

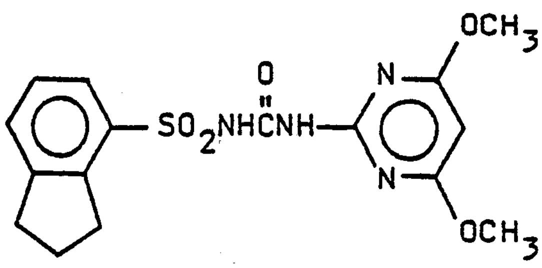

Compound 4

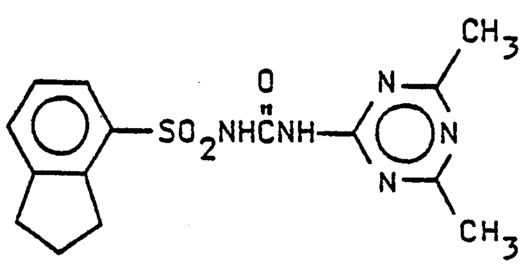

Table A Structures (continued)

Compound 5

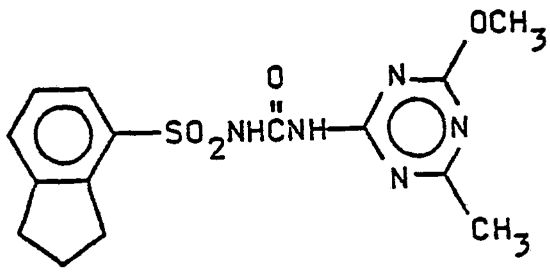

Compound 6

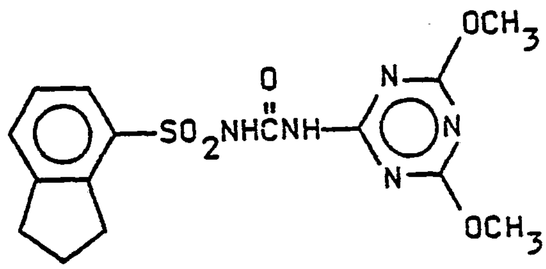

Compound 7

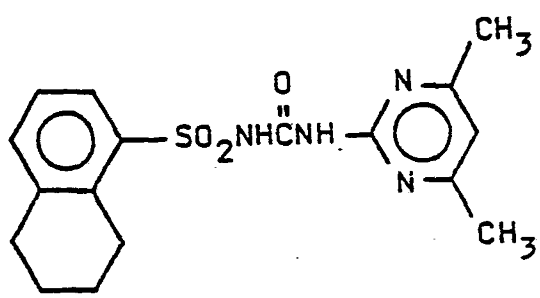

Compound 8

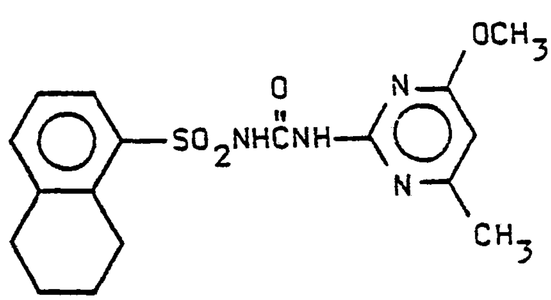

Table A Structures (continued)

Compound 9

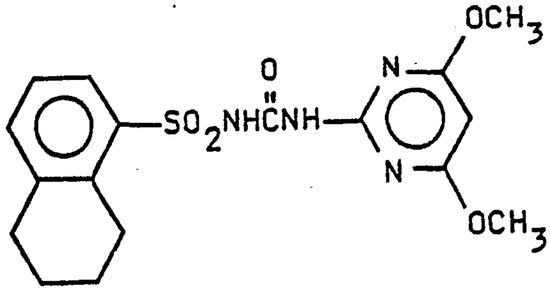

Compound 10

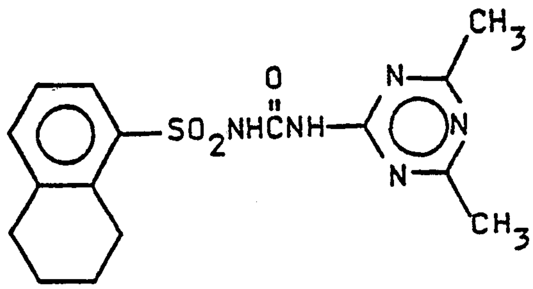

Compound 11

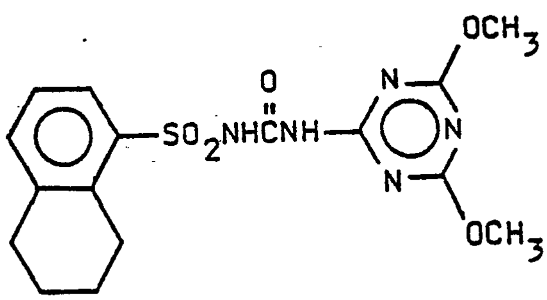

Compound 12

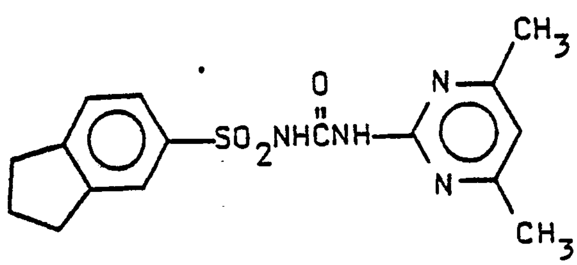

Table A Structures (continued)

Compound 13

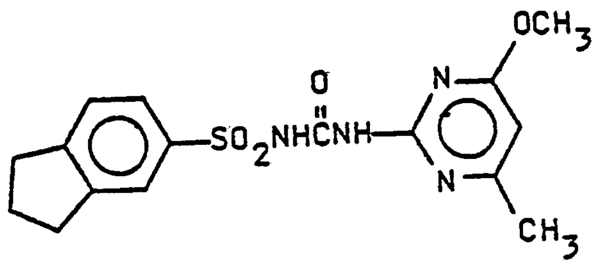

Compound 14

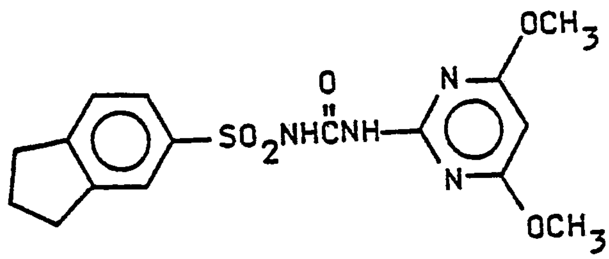

Compound 15

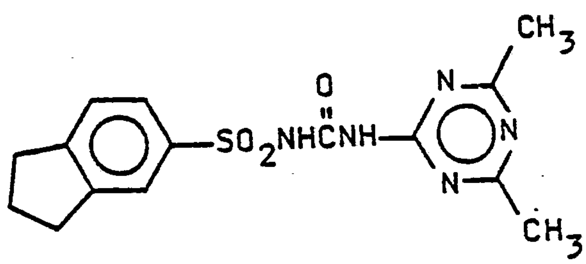

Compound 16

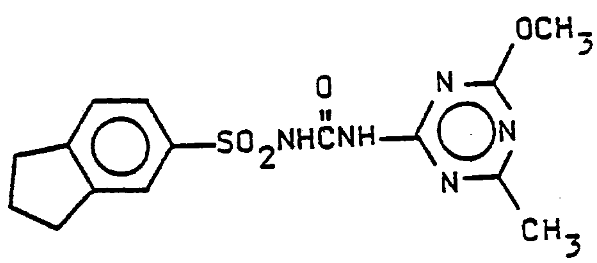

Table A Structures (continued)

Compound 17

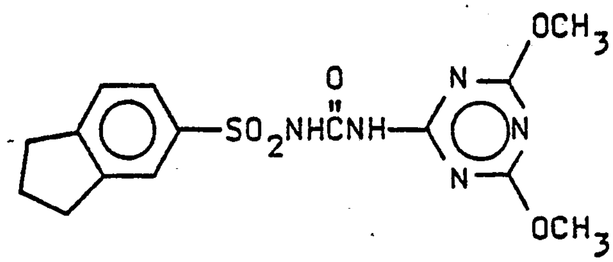

Compound 18

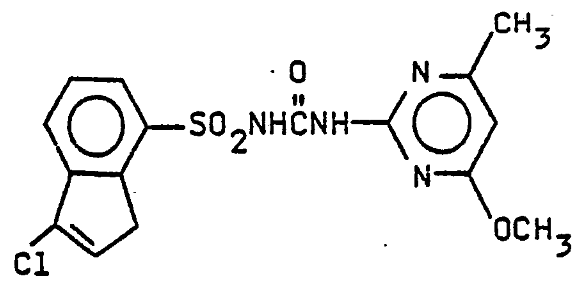

Compound 19

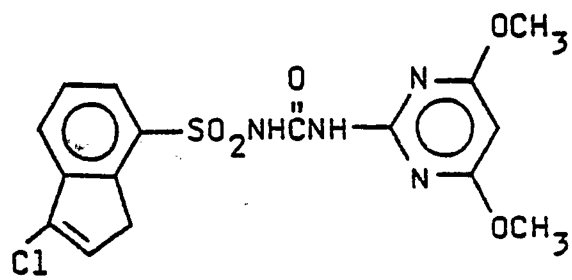

Compound 20

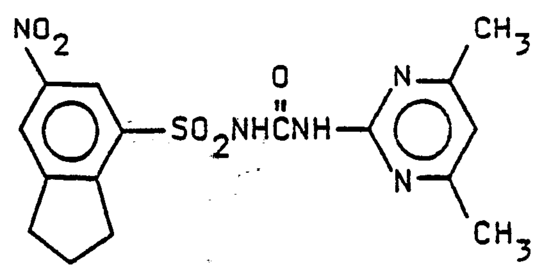

Table A Structures (continued)

Compound 21

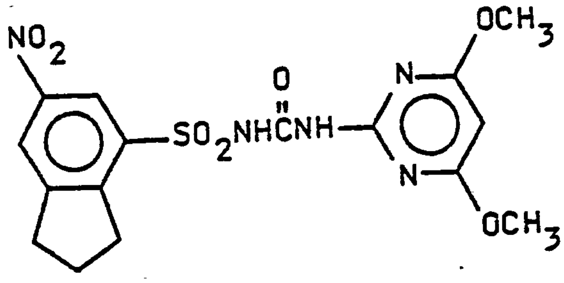

Compound 22

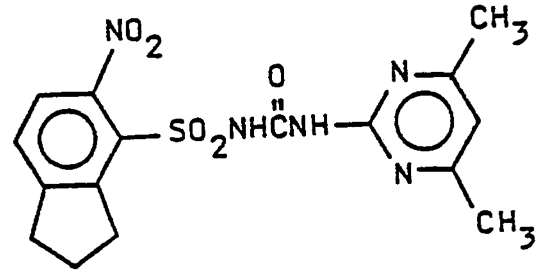

Compound 23

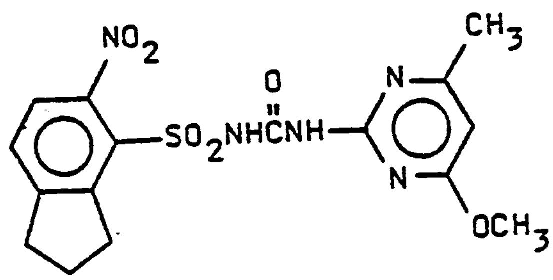

Compound 24

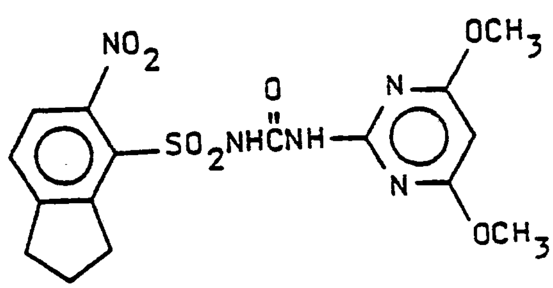

Table A Structures (continued)

Compound 25

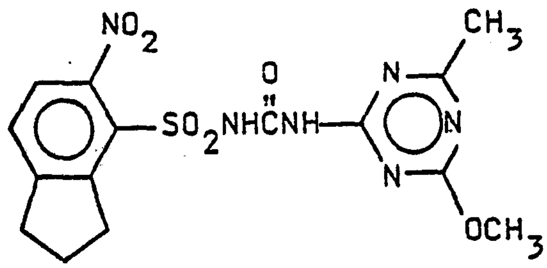

Compound 26

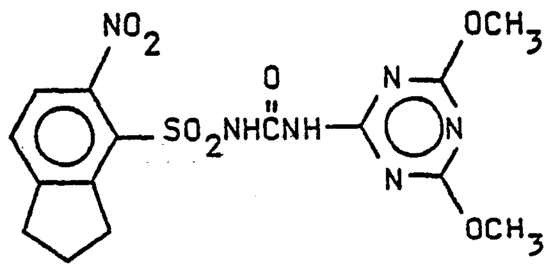

Compound 27

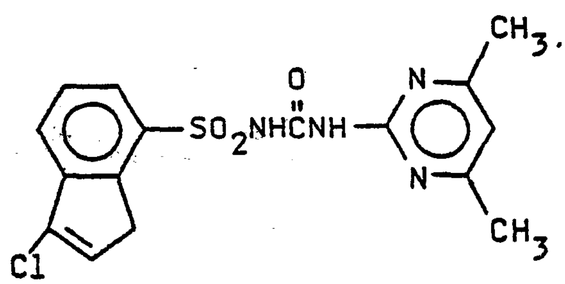

Compound 28

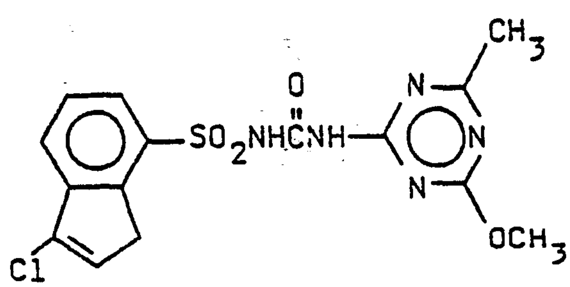

Table A Structures (continued)

Compound 29

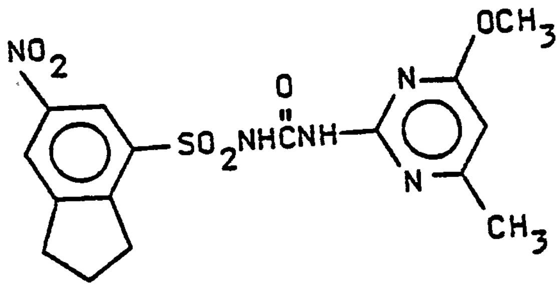

Compound 30

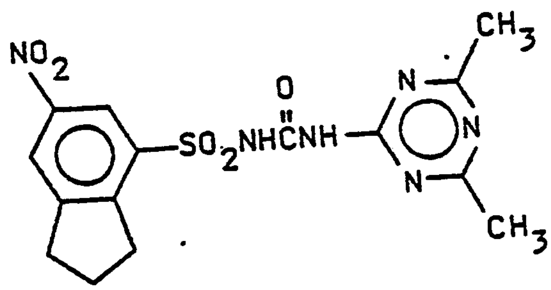

Compound 31

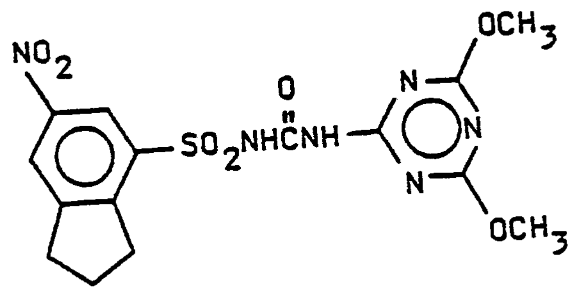

Compound 32

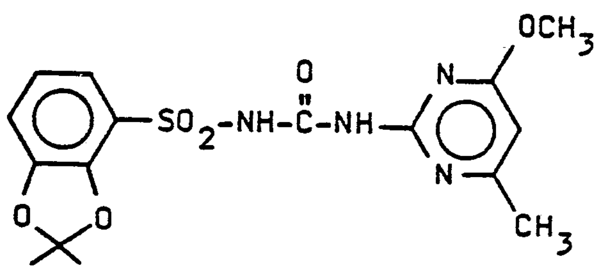

Table A Structures (continued)

Compound 33

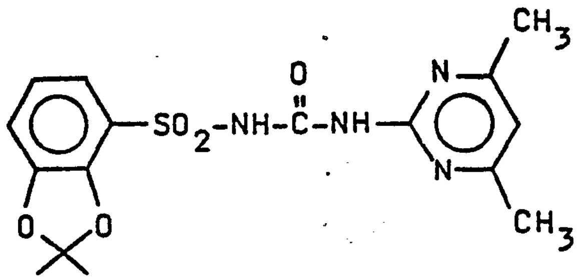

Compound 34

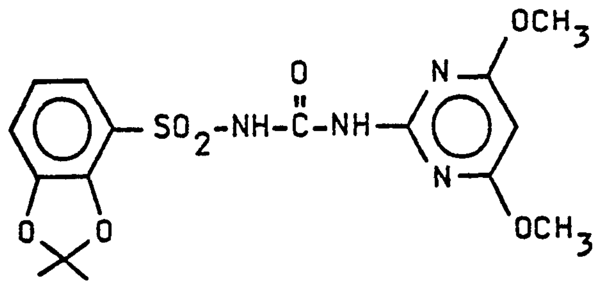

Compound 35

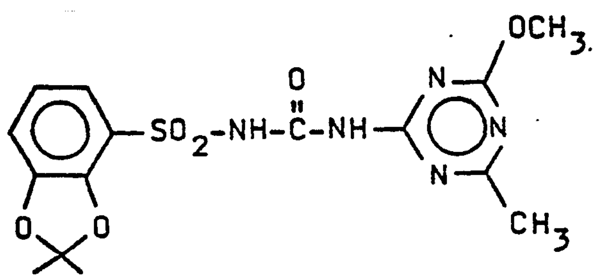

Compound 36

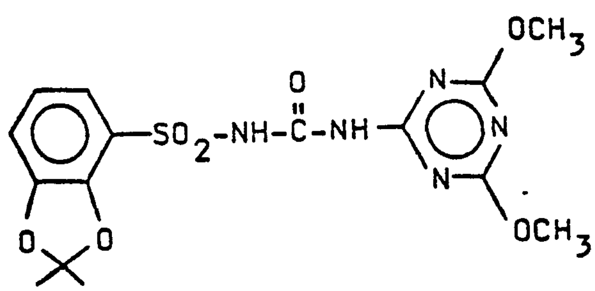

Table A Structures (continued)

Compound 37

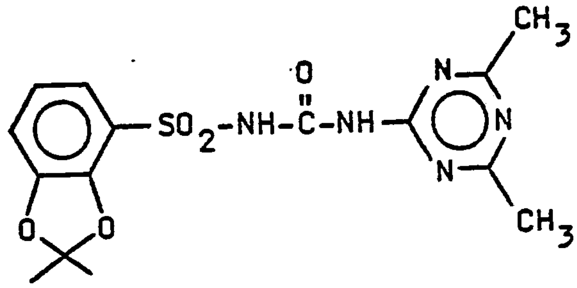

Table A

| | Cmpd. 1 | Cmpd. 2 | Cmpd. 3 | Cmpd. 4 |
|---|---|---|---|---|
| Rate kg/ha | .05 | .05 | .05 | .05 |
| POST-EMERGENCE | | | | |
| Bush bean | 1C | 6C,8G,6Y | 4S,7G,6Y | 3C,6G,6Y |
| Cotton | 1C | 4C,5H,8G | 4C,4H,6G | 1C |
| Morningglory | 0 | 3C,5G | 4C,5G | 1C |
| Cocklebur | 1H | 3C,8G | 4C,9G | 0 |
| Sicklepod | 1C | 3C,8G | 3C,6G | 0 |
| Nutsedge | 5G | 2C,8G | 2C,9G | 0 |
| Crabgrass | 1C,5G | 3C,8G | 1C,5G | 0 |
| Barnyardgrass | 2G | 3C,9H | 2C,7H | 0 |
| Wild Oats | 0 | 2C,5G | 2C | 0 |
| Wheat | 0 | 2C,4G | 0 | 0 |
| Corn | 2C,4G | 2C,9G | 1C,5G | 1C,3G |
| Soybean | 1C | 5C,9G | 2C,4H,9G | 0 |
| Rice | 1C,6G | 5C,9G | 9G | 5G |
| Sorghum | 6G | 3C,9H | 1C,7G | 3G |
| PRE-EMERGENCE | | | | |
| Morningglory | 5G | 9H | 9G | 5G |
| Cocklebur | 5G | 8H | 9H | 2H |
| Sicklepod | 3G | 9G | 8G | 1C |
| Nutsedge | 0 | 5G | 10E | 5G |
| Crabgrass | 1C | 2C,5G | 1C,4G | 0 |
| Barnyardgrass | 2C,6G | 2C,9H | 2C,9H | 2C |
| Wild Oats | 3C,8G | 2C,9G | 8G | 0 |
| Wheat | 3C,8G | 2C,9G | 9G | 3G |
| Corn | 2C,7G | 1C,9G | 2C,8G | 2C |
| Soybean | 0 | 2C,5H | 3G | 0 |
| Rice | 2C,6G | 9H | 10E | 5G |
| Sorghum | 2C,5G | 5C,9H | 3C,9H | 2C,6G |

Table A (continued)

| | Cmpd. 5 | Cmpd. 6 | Cmpd. 7 | Cmpd. 8 |
|---|---|---|---|---|
| Rate kg/ha | .05 | .05 | .05 | .05 |
| POST-EMERGENCE | | | | |
| Bush bean | 5C,7G,6Y | 5C,9G,6Y | 0 | 2C,4G |
| Cotton | 5C,6G | 2C,2H | 0 | 2C |
| Morningglory | 2C,5G | 3C,9G | 0 | 2C |
| Cocklebur | 2C,7G | 2C,6G | 0 | 3G,6F |
| Sicklepod | 2C,3H | 3C,6G | 0 | 1C,1H |
| Nutsedge | 2C | 3G | 0 | 0 |
| Crabgrass | 2G | 3G | 0 | 2G |
| Barnyardgrass | 9H | 3C,7H | 0 | 3C,6H |
| Wild Oats | 0 | 0 | 0 | 1C |
| Wheat | 0 | 0 | 0 | 0 |
| Corn | 5U,9G | 2C,9H | 0 | 3G |
| Soybean | 5C,9G | 5C,9G | 0 | 1C,5H |
| Rice | 6C,9G | 5C,9G | 0 | 4G |
| Sorghum | 3U,9G | 2U,9H | 0 | 2H |
| PRE-EMERGENCE | | | | |
| Morningglory | 9H | 9G | 0 | 8G |
| Cocklebur | 9H | 9H | 4H | 9H |
| Sicklepod | 2C,8G | 2C,8G | 0 | 1C,5G |
| Nutsedge | 4C,6G | 5G | 0 | 3G |
| Crabgrass | 1C | 4C | 0 | 2C |
| Barnyardgrass | 9H | 9H | 0 | 2C,9H |
| Wild Oats | 2C,6G | 2C,8H | 0 | 2C,8H |
| Wheat | 8G | 2C,9G | 0 | 2C,9G |
| Corn | 2C,8G | 2C,8G | 0 | 1C,8G |
| Soybean | 2C,5H | 2C,7H | 0 | 1C |
| Rice | 9H | 10E | 0 | 3C,7H |
| Sorghum | 2U,9G | 2C,9H | 0 | 2C,9G |

Table A (continued)

| | Cmpd. 9 | Cmpd. 10 | Cmpd. 11 | Cmpd. 12 |
|---|---|---|---|---|
| Rate kg/ha | .05 | .05 | .05 | .05 |
| POST-EMERGENCE | | | | |
| Bush bean | 3C,7G,6F | 2C | 5C,9G,6Y | 1C |
| Cotton | 2C,3G | 0 | 1C | 0 |
| Morningglory | 2C | 0 | 2C,7G | 1H |
| Cocklebur | 3G,6F | 0 | 3H,9G | 0 |
| Sicklepod | 1C,1H | 0 | 1C,3G | 2A |
| Nutsedge | 2G | 0 | 2G | 0 |
| Crabgrass | 2G | 2G | 0 | 0 |
| Barnyardgrass | 2C,6H | 0 | 1H | 0 |
| Wild Oats | 0 | 0 | 0 | 0 |
| Wheat | 0 | 0 | 0 | 0 |
| Corn | 1C,5G | 0 | 2C,5G | 1H |
| Soybean | 2C,8H | 0 | 3C,9G,5X | 2G |
| Rice | 2C,5G | 0 | 9G,5I | 2G |
| Sorghum | 1C,5H | 0 | 1C,9H | 0 |
| PRE-EMERGENCE | | | | |
| Morningglory | 7G | 2C | 9G | 1C |
| Cocklebur | 8H | 0 | 9H | 1H |
| Sicklepod | 2C | 0 | 2C,7G | 0 |
| Nutsedge | 3G | 0 | 5G | 0 |
| Crabgrass | 2C,7G | 0 | 2C,4G | 0 |
| Barnyardgrass | 3C,9H | 1C | 3C,9H | 0 |
| Wild Oats | 3C,7G | 0 | 2C,9G | 0 |
| Wheat | 3C,8G | 0 | 2C,9G | 0 |
| Corn | 2C,8G | 0 | 1C,8G | 2G |
| Soybean | 1C,1H | 0 | 2C,5H | 0 |
| Rice | 2C,8H | 1C | 10E | 0 |
| Sorghum | 9G | 1C | 9G | 0 |

Table A (continued)

| | Cmpd. 13 | Cmpd. 14 | Cmpd. 15 | Cmpd. 16 |
|---|---|---|---|---|
| Rate kg/ha | .05 | .05 | .05 | .05 |
| POST-EMERGENCE | | | | |
| Bush bean | 1C,2G | 2C,2H | 0 | 1C,6G |
| Cotton | 2C | 2C,2H | 0 | 1C |
| Morningglory | 2C,3G | 1H | 0 | 1H |
| Cocklebur | 3H | 3G,6F | 0 | 1H |
| Sicklepod | 2C | 2C,3H | 0 | 2C |
| Nutsedge | 2G | 0 | 0 | 0 |
| Crabgrass | 2G | 0 | 0 | 0 |
| Barnyardgrass | 2G | 1H | 0 | 1C,5H |
| Wild Oats | 0 | 0 | 0 | 0 |
| Wheat | 0 | 0 | 0 | 0 |
| Corn | 2H | 3G | 0 | 8H |
| Soybean | 0 | 1C,4G | 0 | 2C,7G |
| Rice | 4G | 2G | 0 | 6G |
| Sorghum | 1C,5H | 2G | 0 | 2C,9H |
| PRE-EMERGENCE | | | | |
| Morningglory | 2G | 8G | 0 | 6G |
| Cocklebur | 8G | - | 4G | 8H |
| Sicklepod | 2C | 3G | 0 | 6G |
| Nutsedge | 2G | 5G | 0 | 0 |
| Crabgrass | 1C,3G | 1C | 0 | 1C |
| Barnyardgrass | 9H,5C | 8H,3C | 0 | 6G,2C |
| Wild Oats | 2C,6G | 2C,9H | 0 | 1C |
| Wheat | 3G | 2C,9H | 0 | 2C,6G |
| Corn | 3C | 3C,6G | 0 | 4C,8G |
| Soybean | 0 | 2C,2H | 0 | 2C,3H |
| Rice | 2C,6H | 2C,8H | 1C | 2C,7H |
| Sorghum | 2C,6G | 2C,8H | 3G | 2C,8H |

Table A (continued)

| | Cmpd. 17 | Cmpd. 18 | Cmpd. 19 | Cmpd. 20 |
|---|---|---|---|---|
| Rate kg/ha | .05 | .05 | .05 | .05 |
| POST-EMERGENCE | | | | |
| Bush bean | 2C,5G,6Y | 2C | 3C,6Y | 5G,6Y |
| Cotton | 0 | 0 | 0 | 2G |
| Morningglory | 1C | 1C | 2C,5G | 3G |
| Cocklebur | 1H | 0 | 5G | 5G |
| Sicklepod | 0 | 3C | 3C,6H | 2C |
| Nutsedge | 0 | 3G | 3G | 0 |
| Crabgrass | 0 | 3G | 2G | 0 |
| Barnyardgrass | 2H | 7H | 3C,8H | 3H |
| Wild Oats | 0 | 0 | 1C | 0 |
| Wheat | 0 | 0 | 0 | 0 |
| Corn | 4H | 2C | 9H | 2C,5G |
| Soybean | 2C,6G | 2C | 2C,2H | 3G |
| Rice | 4G | 1C | 2C,8G | 4G |
| Sorghum | 7H | 3C | 3C,7H | 4G |
| PRE-EMERGENCE | | | | |
| Morningglory | 2C,7H | 2C,3H | 8G | 2C |
| Cocklebur | 4H | - | 9H | 8H |
| Sicklepod | 4G | 5C,8H | 8H,2C | 4G |
| Nutsedge | 0 | 2C,8G | 2C,7G | 0 |
| Crabgrass | 0 | 2C,6G | 2C,5G | 0 |
| Barnyardgrass | 2C | 5C,9H | 5C,9H | 0 |
| Wild Oats | 0 | 3C,9G | 9G | 4G |
| Wheat | 0 | 2C,8G | 9G | 4G |
| Corn | 2C,6G | 2C,8G | 2C,8G | 2C,6G |
| Soybean | 2C | 2C | 1C | 0 |
| Rice | 2C,9G | 5C,8H | 5C,9H | 2C |
| Sorghum | 2C,7H | 2C,8H | 3C,8H | 1C,3G |

Table A (continued)

| | Cmpd. 21 | Cmpd. 22 | Cmpd. 23 | Cmpd. 24 |
|---|---|---|---|---|
| Rate kg/ha | .05 | .05 | .05 | .05 |
| POST-EMERGENCE | | | | |
| Bush bean | 2C,4H,6Y | 7C,9G,6Y | 4C,6G,6Y | 4C,7G,6Y |
| Cotton | 1C,3G | 4C,8G | 3C,9G | 3C,9G |
| Morningglory | 2C,7G | 3G | 1C,3G | 2C,7G |
| Cocklebur | 2C,8G | 9H | 9C | 10C |
| Sicklepod | 3C,5G | 2C,4G | 2C,7G | 5C,9G |
| Nutsedge | 5G | 5C,9G | 3C,9G | 9C |
| Crabgrass | 4G | 2C,7G | 2C,6H | 2C,7G |
| Barnyardgrass | 3C,9H | 3C,6H | 5C,9H | 9C |
| Wild Oats | 3G | 3C,8G | 3C,8G | 2C |
| Wheat | 3G | 3C,8G | 3C,9G | 2C,5G |
| Corn | 3U,9G | 5U,9G | 9C | 6U,9C |
| Soybean | 2H,5G | 3C,9G | 9C | 9C |
| Rice | 2C,8G | 2C,9G | 2U,9G | 2C,9G |
| Sorghum | 2C,8H | 3U,9G | 9G | 6U,9G |
| PRE-EMERGENCE | | | | |
| Morningglory | 2C | 6G | 2C,7G | 8G |
| Cocklebur | 9H | 9H | - | 9H |
| Sicklepod | 3G | 7H | 2C,8G | 9G |
| Nutsedge | 3G | 10E | 10E | 10E |
| Crabgrass | 4G | 2C | 2C,6G | 2C,6G |
| Barnyardgrass | 2C,8H | 2C,9H | 5C,9H | 5C,9H |
| Wild Oats | 2C,5H | 2C,9H | 3C,9H | 2C,8G |
| Wheat | 3G | 2C,9G | 6C,9H | 3C,9G |
| Corn | 3C,7G | 3C,9G | 10H | 3C,9H |
| Soybean | 1H | 9H | 8H | 8H |
| Rice | 2C,6G | 10E | 10E | 10E |
| Sorghum | 2C,6G | 10H | 9H | 5C,9H |

Table A (continued)

| | Cmpd. 25 | Cmpd. 26 | Cmpd. 27 | Cmpd. 28 |
|---|---|---|---|---|
| Rate kg/ha | .05 | .05 | .05 | .05 |
| POST-EMERGENCE | | | | |
| Bush bean | 7C,9G,6Y | 8C,9G,6Y | 2G | 2C,4G |
| Cotton | 4C,9G | 3C,9G | 0 | 1C |
| Morningglory | 2C,8G | 3C,8G | 1C | 2C,7H |
| Cocklebur | 9C | 9C | 0 | 3G |
| Sicklepod | 5C,9G | 2C,9G | 1C | 3C |
| Nutsedge | 6C,9G | 1C,6G | 0 | 0 |
| Crabgrass | 2C,6G | 0 | 0 | 0 |
| Barnyardgrass | 3C,7H | 1C | 1H | 1H |
| Wild Oats | 3C,8G | 2G | 0 | 0 |
| Wheat | 3C,9G | 3G | 0 | 0 |
| Corn | 5U,9C | 2C,8H | 0 | 2C,9H |
| Soybean | 4C,9G | 9C | 2G | 2C,5H |
| Rice | 5C,9G | 4C,9G | 0 | 9G |
| Sorghum | 2U,9G | 2C,7H | 0 | 3C,9H |
| PRE-EMERGENCE | | | | |
| Morningglory | 9G | 2C,9G | 2H | 2C,8H |
| Cocklebur | 9H | 9H | 5G | 2C,5G |
| Sicklepod | 3C,9G | 2C,9G | 1C | 6H,4C |
| Nutsedge | 9G | 10E | 0 | 0 |
| Crabgrass | 1C | 0 | 0 | 1C |
| Barnyardgrass | 5C,7G | 2C | 0 | 3C,7H |
| Wild Oats | 2C,9G | 2C,9H | 0 | 2G |
| Wheat | 1C,9G | 3G | 0 | 2C,8G |
| Corn | 3C,9G | 1C,8G | 0 | 9G |
| Soybean | 3C,8H | 2C,8H | 1H | 1C,1H |
| Rice | 10E | 3C,9H | 1C | 4C,9H |
| Sorghum | 2C,9H | 2C,6G | 0 | 9H |

Table A (continued)

| | Cmpd. 29 | Cmpd. 30 | Cmpd. 31 |
|---|---|---|---|
| Rate kg/ha | .05 | .05 | .05 |
| POST-EMERGENCE | | | |
| Bush bean | 4C,9G,6Y | 1C,8G,6Y | 1C,8G,6Y |
| Cotton | 3C,7G | 1C,5G | 4B,5D,5G |
| Morningglory | 3C,7G | 1C,3G | 3C,6G |
| Cocklebur | 3C,9G | 1C,8H | 10C |
| Sicklepod | 4C,8G | 1C,3G | 2C,6G |
| Nutsedge | 1C,9G | 3G | 1C,5G |
| Crabgrass | 2C,7G | 1H | 2G |
| Barnyardgrass | 2C,9H | 0 | 3C,9H |
| Wild Oats | 2C,9G | 0 | 2C,8G |
| Wheat | 2C,9G | 0 | 2C,9G |
| Corn | 3U,9G | 4H | 5U,9H |
| Soybean | 9C | 1C,8G | 9C |
| Rice | 2C,9G | 8G | 6C,9G |
| Sorghum | 1C,9H | 4G | 2C,9H |
| PRE-EMERGENCE | | | |
| Morningglory | 7G | 1C,5H | 2C,7G |
| Cocklebur | 9H | 9H | 8H |
| Sicklepod | 7G | 2G | 2C,7G |
| Nutsedge | 9G | 0 | 5G |
| Crabgrass | 2C | 0 | 1C |
| Barnyardgrass | 5C,9H | 0 | 2C,8H |
| Wild Oats | 2C,9G | 1C,5G | 2C,9G |
| Wheat | 2C,8G | 1C | 7G |
| Corn | 9H | 1C,5G | 3C,8G |
| Soybean | 8H | 3C,4H | 2C,9H |
| Rice | 10E | 2C,9H | 6C,9H |
| Sorghum | 4C,9H | 2C,7H | 1C,8H |

In the above tests, certain compounds listed, e.g. compounds 10, 12, show weak activity at 0.05 kg/ha; it is thought they would demonstrate better activity at higher rates of application.

Table A (continued)

| | Compound 32 | | Compound 33 | |
|---|---|---|---|---|
| Rate kg/ha | 0.4 | 0.1 | 0.05 | 0.4 |
| POST-EMERGENCE | | | | |
| Bush bean | 9C | 8D,9G,6Y | 3C,9G,6Y | 5C,9G,6Y |
| Cotton | 9C | 6C,9G | 3C,3H,8G | 4C,9G |
| Morningglory | 10C | 9C | 2C,4G | 6C,9G |
| Cocklebur | 9C | 9C | 2C,6G | 5C,9G |
| Sicklepod | 9C | 6C,9G | 4C | 4C,6H |
| Nutsedge | 4C,9G | 9G,5X | 4G | 2C,9G |
| Crabgrass | 1C,9G | 2H,8G | 0 | 4G |
| Barnyardgrass | 9C | 5C,9H | 2C,8H | 9H |
| Wild Oats | 3C,9G | 2C,9G | 2G | 2C,5G |
| Wheat | 3C,9G | 0 | 0 | 0 |
| Corn | 5U,9C | 3U,9G | 2C,8H | 2C,9H |
| Soybean | 9C | 9C | 2C,5G | 3C,8G |
| Rice | 9C | 3C,9G | 0 | 5G |
| Sorghum | 4U,9G | 9G | 2C,8H | 2C,9H |
| PRE-EMERGENCE | | | | |
| Morningglory | 9H | 2C,9H | 5G | 9G |
| Cocklebur | 9H | 9H | 5H | - |
| Sicklepod | 9C | 9G | 4H | 7G |
| Nutsedge | 9G | 8G | 0 | 9G |
| Crabgrass | 3C,8G | 1C,6G | 0 | 0 |
| Barnyardgrass | 5C,9H | 9H | 2C,4G | 2C,8H |
| Wild Oats | 3C,9H | 2C,9G | 1C,3G | 2C,7G |
| Wheat | 2C,9G | 2C,9G | 0 | 7G |
| Corn | 10E | 5C,9H | 2C,6G | 2C,9H |
| Soybean | 9H | 8H | 1C,1H | 2C |
| Rice | 4C,9H | 3C,8H | 2C,3G | 2G |
| Sorghum | 10E | 9H | 1C | 2C,7H |

Table A (continued)

| | Compound 34 | | Cmpd. 35 | Cmpd. 36 |
|---|---|---|---|---|
| Rate kg/ha | 0.05 | 0.4 | 0.05 | 0.05 |
| POST-EMERGENCE | | | | |
| Bush bean | 7C,9G,6Y | 9C | 5C,9G,6Y | 9C |
| Cotton | 4C,3H,9G | 6C,9G | 4C,8G | 5C,9G |
| Morningglory | 5G | 5G | 10C | 10C |
| Cocklebur | 5C,9G | 9C | 10C | 9C |
| Sicklepod | 5C,9G | 9C | 4C,9G | 9C |
| Nutsedge | 2C,9G | 3C,9G | 6G | 0 |
| Crabgrass | 4G | 5G | 2C,8G | 6G |
| Barnyardgrass | 3C,9H | 9C | 4C,9H | 9H |
| Wild Oats | 1C,6G | 2C,9G | 9G | 2C,9G |
| Wheat | 3G | 1C,8G | 9G | 2G |
| Corn | 3C,9H | 1C,9H | 5U,9C | 3C,9G |
| Soybean | 5C,8H | 9C | 9C | 9C |
| Rice | 1G | 1C,6G | 6C,9G | 2C,8G |
| Sorghum | 2C,9G | 2C,9G | 5C,9G | 5C,9G |
| PRE-EMERGENCE | | | | |
| Morningglory | 2G | 7G | 10C | 8H |
| Cocklebur | 8H | 9H | 9H | 9H |
| Sicklepod | 8G | 9G | 2C,9G | 2C,9G |
| Nutsedge | 10E | 10E | 0 | 5G |
| Crabgrass | 0 | 2C,4G | 2C | 3G |
| Barnyardgrass | 3C,9H | 9H | 3C,9H | 2C,8H |
| Wild Oats | 2C,6G | 3C,8H | 3C,9G | 2C,6G |
| Wheat | 2C,5G | 2C,8G | 1C,9G | 1C,6G |
| Corn | 2C,9G | 5C,9H | 5C,9H | 8G |
| Soybean | 7H | 9H | 9H | 3C,5H |
| Rice | 2G | 6G | 5C,7H | 0 |
| Sorghum | 10H | 6C,9H | 10H | 4C,9H |

Table A (continued)

| | Cmpd. 37 |
|---|---|
| Rate kg/ha | .05 |
| POST-EMERGENCE | |
| Bush bean | 0 |
| Cotton | 0 |
| Morningglory | 2C,2H |
| Cocklebur | 1C |
| Sicklepod | 0 |
| Nutsedge | 0 |
| Crabgrass | 0 |
| Barnyardgrass | 0 |
| Wild Oats | 0 |
| Wheat | 0 |
| Corn | 0 |
| Soybean | 0 |
| Rice | 0 |
| Sorghum | 0 |
| Sugar beet | 2C,7G |
| PRE-EMERGENCE | |
| Morningglory | 0 |
| Cocklebur | 0 |
| Sicklepod | 0 |
| Nutsedge | 0 |
| Crabgrass | 0 |
| Barnyardgrass | 0 |
| Wild Oats | 0 |
| Wheat | 0 |
| Corn | 0 |
| Soybean | 0 |
| Rice | 0 |
| Sorghum | 0 |
| Sugar beet | 0 |

It is noted that Compound 37 has demonstrated low activity at a rate of .05 kg/ha. It is thought that at higher rates, activity would be improved.

Claims:

1. A compound of the formula:

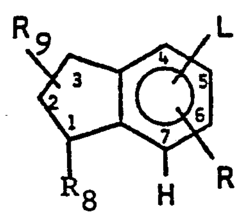
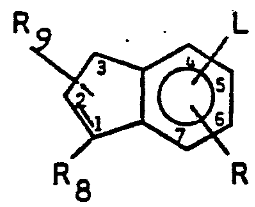
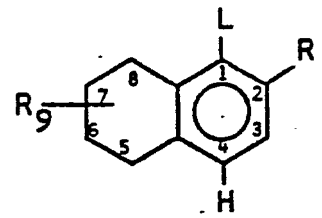

I II III

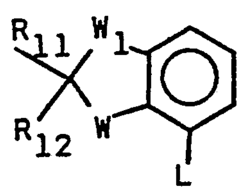
or
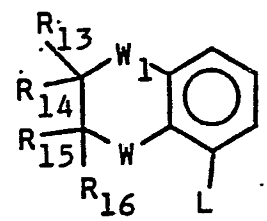

Ia IIa

wherein

L is $SO_2NH\overset{O}{\overset{\|}{C}}\underset{R_{10}}{N}R_1$ ;

R is H, Cl, Br, $NO_2$, $QR_2$, $CO_2R_3$, $SO_2NR_5R_6$, $SO_2N(OCH_3)CH_3$, $OSO_2R_7$ or $C_1$-$C_3$ alkyl;

$R_2$ is $C_1$-$C_3$ alkyl, $CF_2H$, $CF_3$ or $CF_2CF_2H$;

$R_3$ is $C_1$-$C_3$ alkyl, $CH_2CH=CH_2$, $CH_2CH_2OCH_3$ or $CH_2CH_2Cl$;

$R_5$ and $R_6$ are independently $C_1$-$C_2$ alkyl;

$R_7$ is $C_1$-$C_3$ alkyl or $CF_3$;

$R_8$ is H, $CH_3$ or Cl;

$R_9$ is H or $CH_3$;

$R_{10}$ is H or $CH_3$;

Q is O, S or $SO_2$;

W is O, S, SO or $SO_2$;

$W_1$ is O, S, SO or $SO_2$;

$R_{11}$ is H or $CH_3$;

$R_{12}$ is H or $C_1$-$C_4$ alkyl;

$R_{13}$, $R_{14}$, $R_{15}$, $R_{16}$ and $R_{17}$ are independently H or $CH_3$;

$R_1$ is 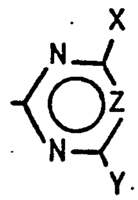 , 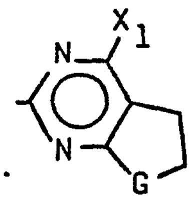 , 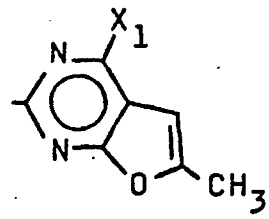 ,

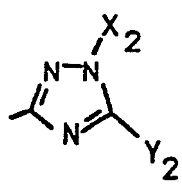 or 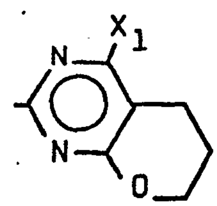 ;

X is $CH_3$, $OCH_3$ or Cl;

Y is $CH_3$, $C_2H_5$, $OCH_3$, $OC_2H_5$, $CH_2OCH_3$, $NH_2$, $NHCH_3$, $SCH_3$, $CH(OCH_3)_2$; 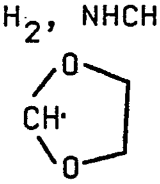 or $N(CH_3)_2$;

Z is CH or N;

$X_1$ is $CH_3$ or $OCH_3$;

G is O or $CH_2$;

$X_2$ is $C_1$-$C_3$ alkyl or $CH_2CF_3$; and

$Y_2$ is $SCH_3$, $SC_2H_5$, $OCH_3$ or $OC_2H_5$.

and their agriculturally suitable salts;

provided that

(1) in Formula I, L is not in the 6-position;

(2) in Formula I, when L is in the 4-position and R is in the 6-position, then R is H, Cl, Br, $NO_2$, $CH_3$ or $OCH_3$;

(3) in Formula II, when $R_8$ is Cl, then R is other than $C_1$-$C_3$ alkyl; and

(4) when X is Cl, then Z is CH and Y is $NH_2$, $NHCH_3$, $N(CH_3)_2$ or $OCH_3$.

2. Compounds of Claim 1, Formula I.

3. Compounds of Claim 1, Formula II.

4. Compounds of Claim 1, Formula III.

5. Compounds of Claim 1, Formula Ia, where W and $W_1$ have identical values.

6. Compounds of Claim 1, Formula IIa, where W and $W_1$ have identical values.

7. Compounds of Claim 2 where L is fixed at the 4-position and $R_9$ is at the 2 or 3 position.

8. Compounds of Claim 7 where R is fixed at the 5-position and $R_{10}$ is H.

9. Compounds of Claim 8 where

$R_1$ is 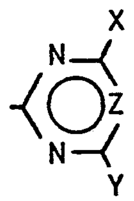 .

10. Compounds of Claim 9 where R is $CO_2CH_3$, $SO_2CH_3$, $SO_2N(CH_3)_2$, Cl, $NO_2$, $OSO_2CH_3$, $OCF_2H$, $SCF_2H$ or H.

11. Compounds of Claim 5 where

$R_1$ is 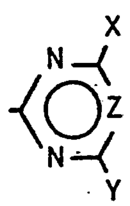 ; and

$R_{10}$ is H.

12. Compounds of Claim 6 where

$R_1$ is 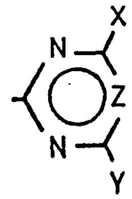 ; and

$R_{10}$ is H.

13. Compounds of Claim 11 where $R_{12}$ is H or $CH_3$.

14. Compounds of Claim 13 where Y is $CH_3$ or $OCH_3$.

15. Compounds of Claim 14 where W and $W_1$ are O or S.

16. Compounds of Claim 14 where W and $W_1$ are SO or $SO_2$.

17. The compound of Claim 1 which is 2,3-dihydro-N-[(4,6-dimethoxypyrimidin-2-yl)aminocarbonyl]-1H-indene-4-sulfonamide.

18. The compound of Claim 1 which is 2,3-dihydro-N-[(4,6-dimethylpyrimidin-2-yl)aminocarbonyl]-1H-indene-4-sulfonamide.

19. The compound of Claim 1 which is 2,3-dihydro-N-[(4-methoxy-6-methylpyrimidin-2-yl)aminocarbonyl]-1H-indene-4-sulfonamide.

20. The compound of Claim 1 which is 2,3-dihydro-N-[(4,6-dimethoxy-1,3,5-triazin-2-yl)aminocarbonyl]-1H-indene-4-sulfonamide.

21. The compound of Claim 1 which is 2,3-dihydro-N-[(4,6-dimethyl-1,3,5-triazin-2-yl)aminocarbonyl]-1H-indene-4-sulfonamide.

22. The compound of Claim 1 which is 2,3-dihydro-N-[(4-methoxy-6-methyl-1,3,5-triazin-2-yl)aminocarbonyl]-1H-indene-4-sulfonamide.

23. The compound of Claim 1 which is N-[(4-methoxy-6-methylpyrimidin-2-yl)aminocarbonyl]-2,2-dimethyl-1,3-benzodioxole-4-sulfonamide.

24. The compound of Claim 1 which is N-[(4,6-dimethylpyrimidin-2-yl)aminocarbonyl]-2,2-dimethyl-1,3-benzodioxole-4-sulfonamide.

25. The compound of Claim 1 which is N-[(4,6-dimethoxypyrimidin-2-yl)aminocarbonyl-2,2-dimethyl-1,3-benzodioxole-4-sulfonamide.

26. The compound of Claim 1 which is N-[(4,6-dimethoxy-1,3,5-triazin-2-yl)aminocarbonyl]-2,2-dimethyl-1,3-benzodioxole-4-sulfonamide.

27. The compound of Claim 1 which is N-[(4-methoxy-6-methyl-1,3,5-triazin-2-yl)aminocarbonyl]-2,2-dimethyl-1,3-benzodioxole-4-sulfonamide.

28. The compound of Claim 1 which is N-[(4,6-dimethyl-1,3,5-triazin-2-yl)aminocarbonyl]-2,2-dimethyl-1,3-benzodioxole-4-sulfonamide.

29. A compound of claim 1 of the formula:

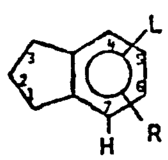

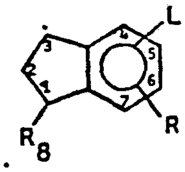

or

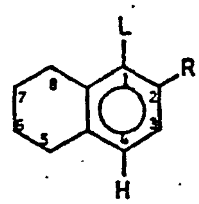

I II III

wherein

L is $SO_2NHCONHR_1$;

R is H, Cl, Br, $NO_2$, $CH_3$, $OR_2$, $CO_2R_3$, $SO_2R_4$, $SO_2NR_5R_6$, $SO_2N(OCH_3)CH_3$ or $OSO_2R_7$;

$R_2$ is $C_1$-$C_3$ alkyl;

$R_3$ is $C_1$-$C_3$ alkyl, $CH_2CH=CH_2$, $CH_2CH_2OCH_3$ or $CH_2CH_2Cl$;

$R_4$ is $C_1$-$C_3$ alkyl;

$R_5$ and $R_6$ are independently $C_1$-$C_2$ alkyl;

$R_7$ is $C_1$-$C_3$ alkyl or $CF_3$;

$R_8$ is H, $CH_3$ or Cl;

$R_1$ is

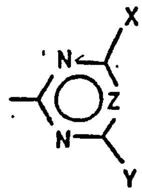 ;

X is $CH_3$ or $OCH_3$;

Y is $CH_3$, $C_2H_5$, $OCH_3$, $OC_2H_5$, $CH_2OCH_3$, $NH_2$, $NHCH_3$ or $N(CH_3)_2$; and

Z is CH or N;

and agriculturally suitable salts thereof.

30. A compound of claim 1 of the formula

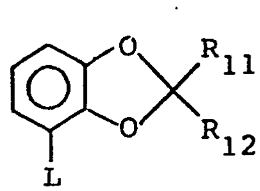

or

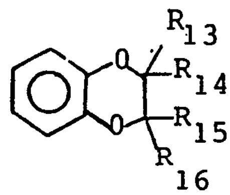

wherein

L is $SO_2NH\overset{O}{\overset{\|}{C}}NHR_1$;

$R_{11}$, $R_{12}$, $R_{13}$, $R_{14}$, $R_{15}$ and $R_{16}$ are as defined in claim 1;

$R_1$ is

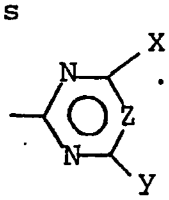

X and Z are as defined in claim 1; and

Y is $CH_3$, $OCH_3$, $OC_2H_5$, $CH_2OCH_3$, $NH_2$, $NHCH_3$ or $N(CH_3)_2$;

and agriculturally suitable salts thereof.

31. A composition suitable for controlling the growth of undesired vegetation comprising an effective amount of a herbicidal compound and at least one of the following: surfactant, solid or liquid diluent, characterised in

that said herbicidal compound comprises a compound of any of claims 1 to 30.

32. A method for controlling the growth of undesired vegetation by applying to the locus to be protected an effective amount of a herbicidal compound, characterised in

that said herbicidal compound comprises a compound of any of claims 1 to 30.

33. A method for the preparation of a compound of claim 1 which comprises

(a) reacting an appropriately substituted isocyanate of formula

$$L_1SO_2NCO$$

wherein $L_1$ is selected from

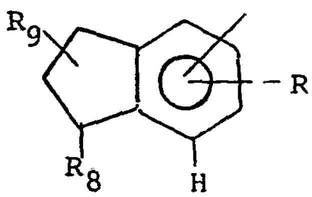

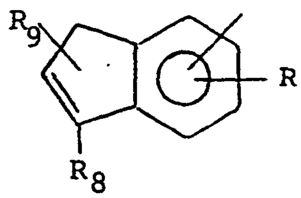

or

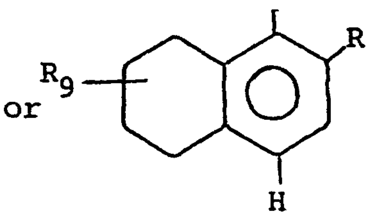

with the appropriate heterocyclic amine $R_1R_{10}NH$; R, $R_1$, $R_8$, $R_9$ and $R_{10}$ being as defined in claim 1; or

(b) reacting a sulfonamide of formula

$$L_1SO_2NH_2$$

wherein $L_1$ is as defined above, with a heterocyclic isocyanate of formula

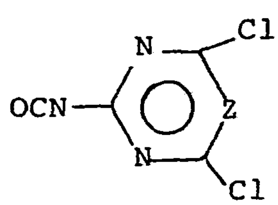

wherein Z is CH or N, to obtain an intermediate of formula

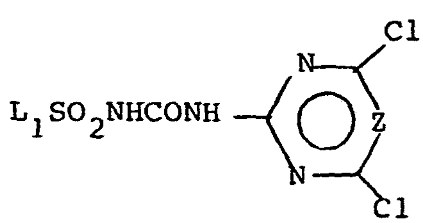

whereafter one or both the chlorine atoms in the heterocylic ring are displaced to obtain a compound of claim 1 wherein X is Cl or $OCH_3$ and Y is $OCH_3$, $OC_2H_5$, $NH_2$, $NHCH_3$ or $N(CH_3)_2$; or

(c) reacting a sulfonamide of formula

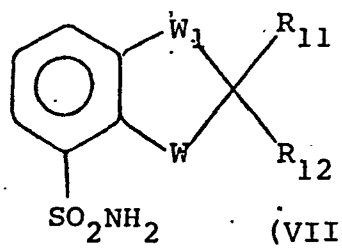 (VII) or 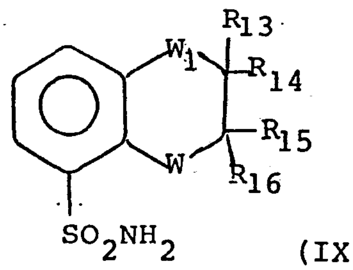 (IX)

wherein W, $W_1$, $R_{11}$, $R_{12}$, $R_{13}$, $R_{14}$, $R_{15}$ and $R_{16}$ are as defined in claim 1, with a methyl heterocyclic carbamate of formula

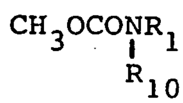

wherein $R_1$ and $R_{10}$ are as defined in claim 1.

34. Isocyanates of formula

$$L_1SO_2NCO$$

as defined in section (a) of claim 33 and sulfonamides of formulae (VII) and (IX) as defined in section (c) of claim 33.

BA-8451-A/8465-A
Cognate-Austria

Claims:

1. A process for the preparation of a compound of the formula:

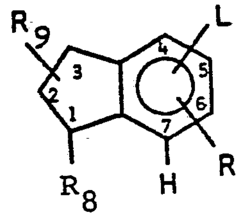

I

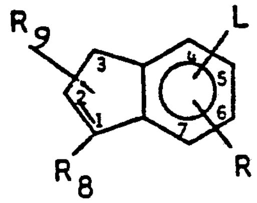

II

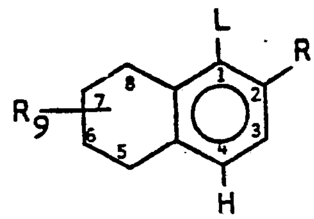

III

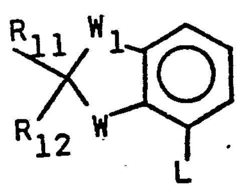

Ia

or

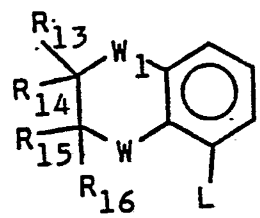

IIa

wherein

L is $SO_2NH\overset{O}{\overset{\|}{C}}\underset{R_{10}}{N}R_1$ ;

R is H, Cl, Br, $NO_2$, $QR_2$, $CO_2R_3$, $SO_2NR_5R_6$, $SO_2N(OCH_3)CH_3$, $OSO_2R_7$ or $C_1$-$C_3$ alkyl;

$R_2$ is $C_1$-$C_3$ alkyl, $CF_2H$, $CF_3$ or $CF_2CF_2H$;

$R_3$ is $C_1$-$C_3$ alkyl, $CH_2CH=CH_2$, $CH_2CH_2OCH_3$ or $CH_2CH_2Cl$;

$R_5$ and $R_6$ are independently $C_1$-$C_2$ alkyl;

$R_7$ is $C_1$-$C_3$ alkyl or $CF_3$;

$R_8$ is H, $CH_3$ or Cl;

$R_9$ is H or $CH_3$;

$R_{10}$ is H or $CH_3$;

Q is O, S or $SO_2$;
W is O, S, SO or $SO_2$;
$W_1$ is O, S, SO or $SO_2$;
$R_{11}$ is H or $CH_3$;
$R_{12}$ is H or $C_1$-$C_4$ alkyl;
$R_{13}$, $R_{14}$, $R_{15}$, $R_{16}$ and $R_{17}$ are independently H or $CH_3$;

$R_1$ is 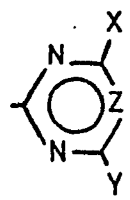 , 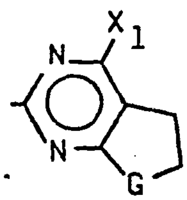 , 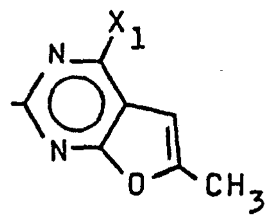 ,

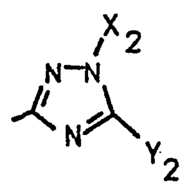 or 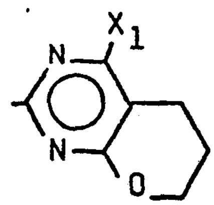 ;

X is $CH_3$, $OCH_3$ or Cl;
Y is $CH_3$, $C_2H_5$, $OCH_3$, $OC_2H_5$, $CH_2OCH_3$, $NH_2$, $NHCH_3$, $SCH_3$,

$CH(OCH_3)_2$; 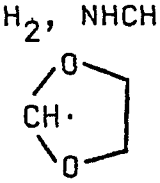 or $N(CH_3)_2$;

Z is CH or N;
$X_1$ is $CH_3$ or $OCH_3$;
G is O or $CH_2$;
$X_2$ is $C_1$-$C_3$ alkyl or $CH_2CF_3$; and
$Y_2$ is $SCH_3$, $SC_2H_5$, $OCH_3$ or $OC_2H_5$.

and their agriculturally suitable salts;
provided that

(1) in Formula I, L is not in the 6-position;
(2) in Formula I, when L is in the 4-position and R is in the 6-position, then R is H, Cl, Br, $NO_2$, $CH_3$ or $OCH_3$;

(3) in Formula II, when $R_8$ is Cl, then R is other than $C_1$-$C_3$ alkyl; and

(4) when X is Cl, then Z is CH and Y is $NH_2$, $NHCH_3$, $N(CH_3)_2$ or $OCH_3$;

which comprises

(a) reacting an appropriately substituted isocyanate of formula

$$L_1SO_2NCO$$

wherein $L_1$ is selected from

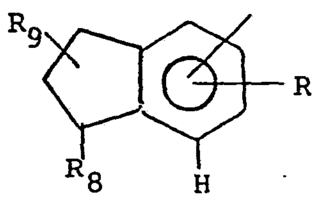

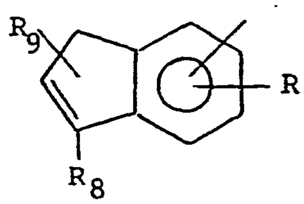

or

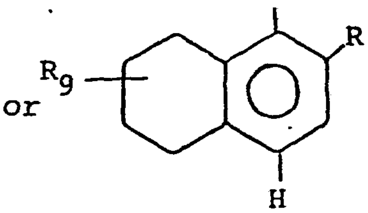

with the appropriate heterocyclic amine $R_1R_{10}NH$; R, $R_1$, $R_8$, $R_9$ and $R_{10}$ being as defined above; or

(b) reacting a sulfonamide of formula

$$L_1SO_2NH_2$$

wherein $L_1$ is as defined above, with a heterocyclic isocyanate of formula

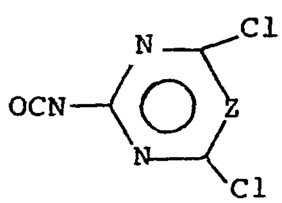

wherein Z is CH or N, to obtain an intermediate of formula

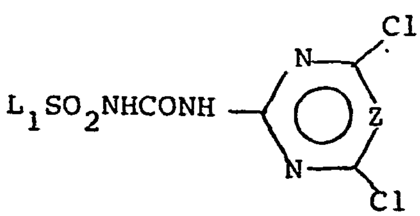

(VIa)

whereafter one or both the chlorine atoms in the heterocylic ring are displaced to obtain a compound of formula (I), (II) or (III) wherein X is Cl or $OCH_3$ and Y is $OCH_3$, $OC_2H_5$, $NH_2$, $NHCH_3$ or $N(CH_3)_2$; or

(c) displacing one or both chlorine atoms in the heterocyclic ring of said intermediate of formula (VIa) to obtain a compound of formula (I), (II) or (III) wherein X is Cl or $OCH_3$ and Y is $OCH_3$, $OC_2H_5$, $NH_2$, $NHCH_3$ or $N(CH_3)_2$; or

(d) reacting a sulfonamide of formula

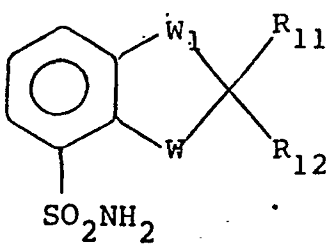

or

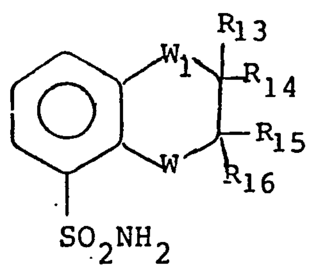

wherein W, $W_1$, $R_{11}$, $R_{12}$, $R_{13}$, $R_{14}$, $R_{15}$ and $R_{16}$ are as defined above, with a methyl heterocyclic carbamate of formula

$$CH_3OCONR_1$$
$$R_{10}$$

wherein $R_1$ and $R_{10}$ are as defined above.

2. A process of claim 1 wherein the product is a compound of the formula:

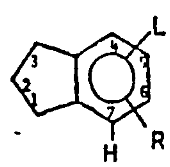

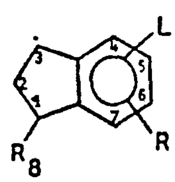

or

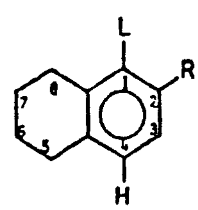

I II III

wherein

L is $SO_2NHCONHR_1$;

R is H, Cl, Br, $NO_2$, $CH_3$, $OR_2$, $CO_2R_3$, $SO_2R_4$, $SO_2NR_5R_6$, $SO_2N(OCH_3)CH_3$ or $OSO_2R_7$;

$R_2$ is $C_1$-$C_3$ alkyl;

$R_3$ is $C_1$-$C_3$ alkyl, $CH_2CH{=}CH_2$, $CH_2CH_2OCH_3$ or $CH_2CH_2Cl$;

$R_4$ is $C_1$-$C_3$ alkyl;

$R_5$ and $R_6$ are independently $C_1$-$C_2$ alkyl;

$R_7$ is $C_1$-$C_3$ alkyl or $CF_3$;

$R_8$ is H, $CH_3$ or Cl;

$R_1$ is

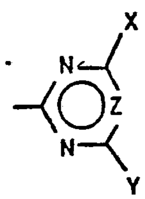 ;

X is $CH_3$ or $OCH_3$;

Y is $CH_3$, $C_2H_5$, $OCH_3$, $OC_2H_5$, $CH_2OCH_3$, $NH_2$, $NHCH_3$ or $N(CH_3)_2$; and

Z is CH or N;

or an agriculturally suitable salt thereof.

3. A process of claim 1 wherein the product is a compound of the formula

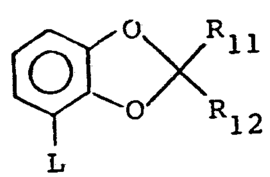 or 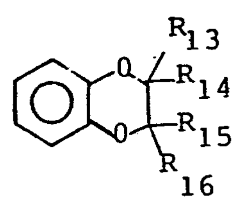

wherein

L is $SO_2NHC(O)NHR_1$;

$R_{11}$, $R_{12}$, $R_{13}$, $R_{14}$, $R_{15}$ and $R_{16}$ are as defined in claim 1;

$R_1$ is

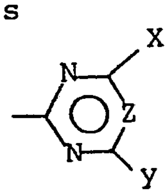

X and Z are as defined in claim 1; and

Y is $CH_3$, $OCH_3$, $OC_2H_5$, $CH_2OCH_3$, $NH_2$, $NHCH_3$ or $N(CH_3)_2$;

or an agriculturally suitable salt thereof.

4. A process of Claim 1, wherein the product is of Formula I.

5. A process of Claim 1, wherein the product is of Formula II.

6. A process of Claim 1, wherein the product is of Formula III.

7. A process of Claim 1, wherein the product is of Formula Ia, where W and $W_1$ have identical values.

8. A process of Claim 1, wherein the product is of Formula IIa, where W and $W_1$ have identical values.

9. A process of Claim 4 where L is fixed at the 4-position and $R_9$ is at the 2 or 3 position.

10. A process of Claim 9 where R is fixed at the 5-position and $R_{10}$ is H.

11. A process of Claim 7, 8, 9 or 10 where

$R_1$ is 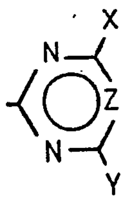 and $R_{10}$ is H.

12. A process of Claim (11) where R is $CO_2CH_3$, $SO_2CH_3$, $SO_2N(CH_3)_2$, Cl, $NO_2$, $OSO_2CH_3$, $OCF_2H$, $SCF_2H$ or H.

13. A process of Claim 7, 11 or 12 where $R_{12}$ is H or $CH_3$, Y is $CH_3$ or $OCH_3$ and W and $W_1$ are O or S.

14. A process of Claim 1 wherein the product is a compound selected from the following: 2,3-dihydro-N-[(4,6-dimethoxypyrimidin-2-yl)aminocarbonyl]-1H-indene-4-sulfonamide;
2,3-dihydro-N-[(4,6-dimethylpyrimidin-2-yl)aminocarbonyl]-1H-indene-4-sulfonamide;
2,3-dihydro-N-[(4-methoxy-6-methylpyrimidin-2-yl)aminocarbonyl]-1H-indene-4-sulfonamide;
2,3-dihydro-N-[(4,6-dimethoxy-1,3,5-triazin-2-yl)aminocarbonyl]-1H-indene-4-sulfonamide;
2,3-dihydro-N-[(4,6-dimethyl-1,3,5-triazin-2-yl)aminocarbonyl]-1H-indene-4-sulfonamide;

2,3-dihydro-N-[(4-methoxy-6-methyl-1,3,5-triazin-2-yl)aminocarbonyl]-1H-indene-4-sulfonamide;
N-[(4-methoxy-6-methylpyrimidin-2-yl)aminocarbonyl]-2,2-dimethyl-1,3-benzodioxole-4-sulfonamide;
N-[(4,6-dimethylpyrimidin-2-yl)aminocarbonyl]-2,2-dimethyl-1,3-benzodioxole-4-sulfonamide;
N-[(4,6-dimethoxypyrimidin-2-yl)aminocarbonyl-2,2-dimethyl-1,3-benzodioxole-4-sulfonamide;
N-[(4,6-dimethoxy-1,3,5-triazin-2-yl)aminocarbonyl]-2,2-dimethyl-1,3-benzodioxole-4-sulfonamide.
N-[(4-methoxy-6-methy-1,3,5-triazin-2-yl)aminocarbonyl]-2,2-dimethyl-1,3-benzodioxole-4-sulfonamide; or
N-[(4,6-dimethyl-1,3,5-triazin-2-yl)aminocarbonyl]-2,2-dimethyl-1,3-benzodioxole-4-sulfonamide.

15. A method for controlling the growth of undesired vegetation by applying to the locus to be protected an effective amount of a herbicidal compound, characterised in

that said herbicidal compound comprises a compound of formula (I), (II), (III), (Ia) or (IIa) as defined in any of claims 1 to 14, or an agriculturally suitable salt thereof.